# EUROPEAN PATENT APPLICATION

(11) **EP 4 446 323 A1**
(43) Date of publication of application: **16.10.2024**
(21) Application number: 22903583.7
(22) Date of filing: 08.12.2022
(51) Int. Cl.: C07D 487/02, A61K 31/53, A61P 31/00, A61P 35/00

(54) **PYRIDINE[4,3-D]PYRIMIDINE COMPOUND AS TLR7/8 AGONIST**

(30) Priority: 08.12.2021 CN 202111493784; 16.09.2022 CN 202211131813
(71) Applicant: Shanghai Visonpharma Co., Ltd., Shanghai 201321 (CN)
(72) Inventor: TANG, Guozhi, Shanghai 201321 (CN); MA, Dawei, Shanghai 201321 (CN); CHEN, Junli, Shanghai 201321 (CN); LIU, Yongfu, Shanghai 201321 (CN); ZHANG, Jinliang, Shanghai 201321 (CN); QUE, Riming, Shanghai 201321 (CN)
(74) Representative: Cabinet Laurent & Charras
(86) International application number: PCT/CN2022/137656
(87) International publication number: WO 2023/104165

(57) **Abstract**

A toll-like receptor agonist, and preparation therefor and an application thereof. Specifically, a compound as shown in formula I, a preparation method therefor, and a use thereof as a TLR7 and/or TLR8 agonist. The compound can be used for preparing a pharmaceutical composition for treating or preventing tumors or infection caused by virus.

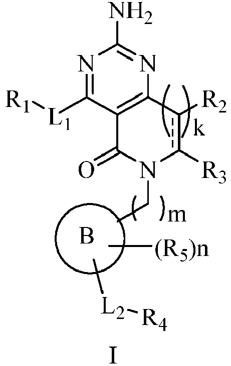

## Description

### Technical field

The present invention belongs to the field of chemical and medicine, in particular to Pyridine[4,3-D]pyrimidine compound as TLR7/8 agonist, and preparation method therefor and application thereof.

### Background

Toll-like receptors (toll-like receptors, TLRs) are a class of structurally conserved proteins that form the first barrier in innate immune responses. By recognizing a variety of conserved pathogen-associated molecular patterns (PAMPs), TLRs can recognize invasive microbes, tissue injury and endogenous molecules released by non-physiological cell death, and activate signaling cascades, leading to the production of pro-inflammatory cytokines. Inflammatory process is crucial to the occurrence and development of many diseases, such as type I diabetes, sepsis, cancer, viral infectious diseases, etc. Therefore, the strategy of manipulating inflammatory response to treat related diseases through small molecule TLRs modulators is very promising.

There are 10 known members of the human TLRs family, which are type I transmembrane proteins characterized by a leucine-rich extracellular structural domain and a cytoplasmic tail containing the conserved Toll/ interleukin (IL)-1 receptor (TIR) structural domain. Among this family, TLR3, TLR7, TLR8 and TLR9 are located in the endosomal compartment.

Both TLR7 and TLR8 can recognize RNA molecules from single-stranded RNA viruses (ssRNA). TLR7 is mainly expressed in plasmacytoid dendritic cells and B cells. TLR8 is predominantly distributed in mDC, macrophages and monocytes and is also expressed in T cells. TLR7 stimulation primarily induces the production of type I interferons, including interferon-alpha (IFN-α), and causes transcription of interferon-stimulated genes (ISGs). Interferon α is one of the main medications for treating chronic hepatitis B or C. TLR8 is predominantly distributed in mDC, macrophages and monocytes and is also expressed in T cells. TLR8 activation mainly produces pro-inflammatory responses, stimulates immune cells to secrete pro-inflammatory cytokines including tumor necrosis factor-α (TNF-α) and IL-6. There is an overlap in the distribution and downstream signaling pathways of TLR7/8, resulting in functional similarities between the two. TLR7/8 activation can exert direct antiviral activity through type I interferon responses, as well as modulate intrinsic immunity through pro-inflammatory responses to promote activation of NK and NKT cells, as well as induce adaptive immunity, improve antigen presentation and activation of dendritic cells, thereby enhancing T-cell responses, and promoting the differentiation of B cells which produce antibody. The development of TLR7/8 agonists has significant clinical value in both antiviral and anti-tumor treatment, and can also be used in antibody conjugated drugs and vaccine adjuvants.

There are several relevant TLR7/8 agonist applications currently pending, but there is still a need for continued development of highly active, safer and therapeutically highly effective TLR7/8 agonists.

### SUMMARY OF THE INVENTION

The purpose of the present invention is to provide a highly active, safer, and therapeutically highly effective TLR7/8 agonist.

In the first aspect of the present invention, provided is a compound of formula I or formula II, or a solvate, a prodrug, a metabolite, or a pharmaceutically acceptable salt thereof, 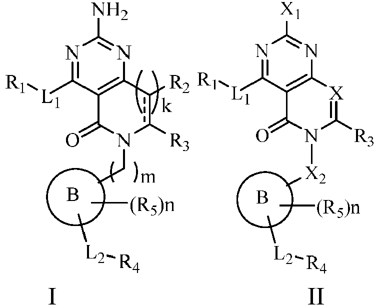 wherein:
L₁ is selected from the group consisting of: -O-, -NH-, -S-, -S(=O)- and -S(=O)₂-;
R₁ is selected from the group consisting of: H, C₁₋₁₂ alkyl, hydroxy substituted C₁₋₁₂ alkyl, C₁₋₁₂ alkoxy, C₂₋₁₂ alkenyl, C₂₋₁₂ alkynyl, C₃₋₁₂ cycloalkyl and 4-12 membered heterocycloalkyl; wherein, R₁ can be further substituted by one or more R^{a} substituents, and R^{a} is selected from the group consisting of: hydrogen, halogen, hydroxyl, cyano, C₁₋₆ alkyl, C₁₋₆ alkoxy, C₃₋₆ cycloalkyl, -NR^{a1}R^{a2}, -NHC(=O)-R^{a3}, 5-6-membered heteroaryl substituted with one or more R^{a4}, -OC(=O)R^{a5}, -C(=O)R^{a5}, -OC(=O)OR^{a5}, and -C(=O)OR^{a5};
R^{a1}, R^{a2}, R^{a3}, or R^{a4} is selected from the group consisting of: C₁₋₆ alkyl, C₁₋₆ haloalkyl, and C₃₋₆ cycloalkyl;
R^{a5} is selected from the group consisting of: C₁₋₂₄ alkyl, C₁₋₂₄ haloalkyl, and C₁₋₂₄ heteroalkyl having 1-10 heteroatoms, wherein the heteroatom is selected from one or more of NH, N, O and S;
X is N or CR₂;
X₁ is H or NH₂;
X₂ is selected from the group consisting of substituted or unsubstituted C₁- C₈ alkylene;
R₂ and R₃ are independently selected from the group consisting of: hydrogen, halogen, cyano, C₁₋₆ alkyl, C₁₋₆ alkoxy, C₃₋₆ cycloalkyl, 4-6 membered heterocycloalkyl, 5-8-membered heteroaryl and 5-8-membered aryl; wherein, R₂ and R₃ can be further substituted by one or more substituents selected from the group consisting of: halogen, hydroxyl, cyano, and amino;
K is 0 or 1;
m is 0, 1, 2, 3, 4, 5, 6,7 or 8;
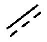 represents a single or double bond;
B is absent, or B is selected from the group consisting of: C₃₋₁₂ cycloalkyl, 4-12 membered heterocycloalkyl, C₆₋₁₂ aryl, 5-12 membered heteroaryl and 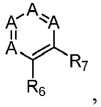 wherein, each A is selected from C, CH and N, and R₆ and R₇ together with the attached carbon atom jointly form a C₄₋₇ cycloalkylene or C₄₋₇ heterocycloalkylene, one or more methylene groups in the C₄₋₇ cycloalkylene or 4-7-membered heterocycloalkylene can be independently replaced by carbonyl or S(=O)₂; and the heteroatom in the 4-7-membered heterocycloalkylene is selected from N, O, and S; the number of heteroatom is 1 to 3;
L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O-), -S(=O)- and -S(=O)₂-; wherein, R^{b}, R^{c} are selected from the group consisting of: hydrogen, halogen, C₁₋₆ alkyl, C₃₋₆ cycloalkyl, 4-6 membered heterocycloalkyl and C₁₋₆ halogenated alkyl, R^{d} is selected from the group consisting of: hydrogen, C₁₋₆ alkyl, C₃₋₆ cycloalkyl, 4-6 membered heterocycloalkyl, C₁₋₆ haloalkyl and hydroxyl substituted C₁₋₆ alkyl; p is 0, 1, 2, 3, 4, 5, or 6; q is 0 or 1;
R₄ is selected from the group consisting of: hydrogen, halogen, cyano, amino, hydroxyl, C₁₋₁₂ alkyl, C₁₋₁₂ alkoxy, C₂₋₁₂ alkenyl, C₂₋₁₂ alkynyl, C₃₋₁₂ cycloalkyl, 4-12 membered heterocyclyl, C₆₋₁₂ aryl and 5-12 membered heteroaryl; and R₄ is optionally substituted by one or more R^{e}, wherein R^{e} is selected from the group consisting of: hydrogen, halogen, hydroxyl, carboxylic acid(-COOH), amino, C₁₋₆alkyl, C₁₋₆alkyl substituted with one or more R^{e1}, C₁₋₆ alkoxy, C₁₋₆ halogenated alkoxy, C₃₋₁₂ cycloalkyl, 4-12 membered heterocycloalkyl, C₆₋₁₂ aryl, 5-12 membered heteroaryl, -N(R^{e2}R^{e3}R^{e4}), -C(=O)O-R^{e2}, -C(=O)NH-R^{e2}, - S(=O)₂-Re²;
R^{e1} is selected from the group consisting of: halogen, hydroxyl, -P(O)(OR^{e2})₂;
R^{e2} and R^{e3} are selected from the group consisting of: hydrogen, C₁₋₆ alkyl, C₃₋₆ cycloalkyl, halogenated C₁₋₆ alkyl and hydroxyl substituted C₁₋₆ alkyl;
R^{e4} is absent or selected from the group consisting of: C₁₋₆ alkyl, C₃₋₆ cycloalkyl and C₁₋₆ haloalkyl;
R₅ is selected from the group consisting of: halogen, hydroxyl, cyano, amino, C₁₋₆ alkyl, C₃₋₆ cycloalkyl, 4-6 membered heterocycloalkyl, C₁₋₆ haloalkyl, C₁₋₆ alkoxy, C₁₋₆ haloalkoxy, -C(=O)O-R^{f}, -C(=O)NH-R^{f}, and -S(=O)₂-R^{f}; wherein, R^{f} is selected from the group consisting of: hydrogen, C₁₋₆ alkyl, C₃₋₆ cycloalkyl and C₁₋₆ haloalkyl;
n is 1, 2, 3, 4, 5 or 6;
wherein, the heterocyclyl can be saturated or partially unsaturated (but without aromatic structure), and in the heterocyclyl, the heteroatom is selected from N, O and S, and the number of heteroatom is 1, 2, 3, or 4 (preferably 1 or 2); among the heteroaryl, the heteroatom is selected from N, O and S, and the number of heteroatom is 1, 2, or 3.

In another preferred embodiment, L₁ is selected from the group consisting of: -O-, - NH-, and -S-.

In another preferred embodiment, R₁ is selected from the group consisting of: C₁₋₁₂ alkyl, hydroxy substituted C₁₋₁₂ alkyl, C₂₋₁₂ alkenyl, C₂₋₁₂ alkynyl, C₃₋₁₂ cycloalkyl and 4-12 membered heterocycloalkyl; wherein, R₁ can be further substituted by one or more R^{a}, and R^{a} is selected from the group consisting of: halogen, hydroxyl, cyano, C₁₋₆ alkyl, C₁₋₆ alkoxy, C₃₋₆ cycloalkyl, -OC(=O)R^{a5}, -C(=O)R^{a5}, -OC(=O)OR^{a5}, -and C(=O)OR^{a5}.

In another preferred embodiment, R₁ is selected from the group consisting of: C₁₋₁₂ alkyl, hydroxy substituted C₁₋₁₂ alkyl, C₂₋₁₂ alkenyl, C₂₋₁₂ alkynyl; wherein, R₁ can be further substituted by one or more R^{a}, and R^{a} is selected from the group consisting of: halogen, hydroxyl, cyano, C₁₋₆ alkyl, C₁₋₆ alkoxy, C₃₋₆ cycloalkyl, -OC(=O)R^{a5}, -C(=O)R^{a5}, -OC(=O)OR^{a5}, and -C(=O)OR^{a5}.

In another preferred embodiment, R₁ is selected from the group consisting of: C₁₋₁₂ alkyl, hydroxy substituted C₁₋₁₂ alkyl; wherein, R₁ can be further substituted by one or more R^{a}, and R^{a} is selected from the group consisting of: C₁₋₆ alkyl, C₃₋₆ cycloalkyl, -OC(=O)R^{a5}, -C(=O)R^{a5}, -OC(=O)OR^{a5}, -C(=O)OR^{a5}.

In another preferred embodiment, the compound of formula I has a structure selected from the group consisting of:

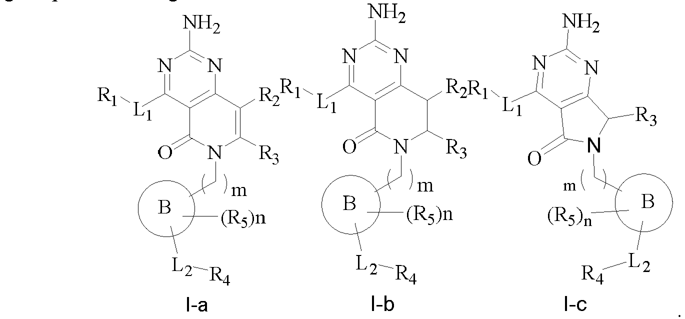

In another preferred embodiment, B is absent, or B is selected from the group consisting of: C₃₋₈ cycloalkyl, 4-7-membered heterocyclyl, C₆₋₁₀ aryl, and

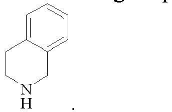

In another preferred embodiment, B is absent, or B is selected from the group consisting of: phenyl, and pyridinyl.

In another preferred embodiment, L₂ is selected from the group consisting of: - (CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O-), -S(=O)- and -S(=O)₂-; wherein, R^{b}, and R^{c} are selected from the group consisting of: hydrogen, halogen and C₁₋₆ alkyl; R^{d} is hydrogen or C₁₋₆ alkyl; p is 0, 1, 2, or 3; q is 0 or 1.

In another preferred embodiment, L₂ is selected from the group consisting of: -(CH₂)ₚ, -(CH₂)ₚ-NR^{d}-, -O-, -S-, -(CH₂)ₚ-C(=O)-, -(CH₂)ₚ-C(=O)NH-, -(CH₂)ₚ-NHC(=O)- and - S(=O)₂-; wherein, R^{d} is hydrogen, or C₁₋₆ alkyl; p is 0, 1, 2, or 3.

In another preferred embodiment, R₄ is selected from the group consisting of: hydrogen, halogen, amino, hydroxyl, C₁₋₁₂ alkyl, C₁₋₁₂ alkoxy, C₂₋₁₂ alkenyl, C₂₋₁₂ alkynyl, C₃₋₁₀ cycloalkyl, 4-10 membered heterocyclyl, C₆₋₁₂ aryl and 5-12 membered heteroaryl; and R₄ is optionally substituted by one or more R^{e}, wherein R^{e} is selected from the group consisting of: hydrogen, halogen, hydroxyl, carboxylic acid, amino, C₁₋₆alkyl, C₁₋₆alkyl substituted with one or more R^{e1}, C₁₋₆ alkoxy, C₁₋₆ haloalkoxy, C₃₋₈ cycloalkyl, 4-7 membered heterocyclyl, phenyl, and 5-7 membered heteroaryl; wherein R^{e1} is selected from the group consisting of: halogen, hydroxyl and -P(O)(OR^{e2})₂.

In another preferred embodiment, R₄ is selected from the group consisting of: hydrogen, C₁₋₁₂ alkyl, C₁₋₁₂ alkoxy, cyclopropyl, cyclobutyl, cyclopentyl, cyclohexyl, 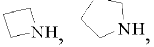 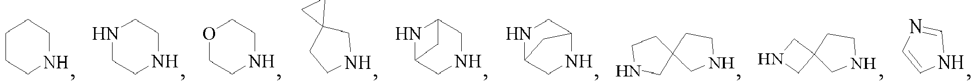 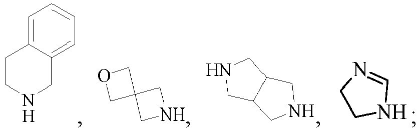 wherein, the loss of H atoms at any position on the above ring forms a connecting site; and R₄ is optionally substituted by one or more R^{e}.

In another preferred embodiment, R₅ is selected from the group consisting of: halogen, hydroxyl, cyano, amino, C₁₋₆ alkyl, C₁₋₆ alkoxy and C₃₋₆ cycloalkyl.

In another preferred embodiment, R₅ is selected from the group consisting of: halogen, C₁₋₄ alkyl, and C₁₋₄ alkoxy.

In the second aspect of the present invention, provided is a pharmaceutical composition comprising one or more of the compound of formula I according to the first aspect of the present invention, a pharmaceutically acceptable salt thereof, a racemate, a R-isomer, a S-isomer, and a mixture thereof, as well as one or more pharmaceutically acceptable carriers, excipients, adjuvants, accessories, and/or diluents.

In the third aspect of the present invention, provided is a use of the compound according to the first aspect of the present invention, a pharmaceutically acceptable salt, a racemate, a R-isomer, a S-isomer thereof, or a mixture thereof in the preparation of a pharmaceutical composition for the treatment or prevention of tumors or infections caused by viruses.

In another preferred embodiment, the present invention provides a conjugate, which is obtained by chemically bonding a compound as described in the present invention with a biological small molecule or monoclonal antibody.

It should be understood that, within the scope of the present invention, each of the above technical features of the present invention and each of the technical features specifically described in the following (such as examples) can be combined with each other to constitute a new or preferred technical solution. Due to space limitations, it will not be repeated herein.

### EMBODIMENTS FOR CARRYING OUT THE INVENTION

After long-term and in-depth research, the inventor has discovered a class of small molecule compounds with TLR7 and/or TLR8 agonist activity. The compound has a novel structure and has comparable or even superior agonist activity to similar compounds in existing technology. Based on the above discoveries, the inventors have completed the present invention.

### TERMS

As used herein, halogen refers to F, Cl, Br or I.

As used herein, unless otherwise specified, the terms used herein have a general meaning known to those skilled in the art.

As used herein, the term "alkyl" refers to a straight or branched alkyl, preferably having 1 to 6 carbon atoms. As used herein, examples of alkyl includes, without limitation, methyl, ethyl, propyl, isopropyl, butyl, isobutyl, sec-butyl, tert-butyl, pentyl and hexyl and the like; preferably ethyl, propyl, isopropyl, butyl, isobutyl, sec-butyl and tert-butyl. The term "alkylene" refers to a divalent group formed when an alkyl group further loses one hydrogen atom.

As used herein, the term "heteroalkyl" refers to a group formed by replacing one or more carbon atoms (optionally including one or more H atoms) on an alkane chain with heteroatoms selected from the group consisting of: NH, N, O and S. Specifically, when NH replaced the carbon atom thereon and the heteroalkyl can have a substituent, the hydrogen atom on NH can also be substituted.

As used herein, the term "alkoxyl" refers to a linear or branched alkoxyl, preferably having 1 to 6 carbon atoms. As used herein, examples of alkoxyl include, without limitation, methoxyl, ethoxyl, propoxyl, isopropoxyl, butoxyl and the like.

As used herein, the term "cycloalkyl" refers to a cyclic alkyl preferably having 3 to 12(for example, 3-7) carbon atoms on the ring, including, without limitation, cyclopropyl, cyclobutyl, cyclopentyl, cyclohexyl, cycloheptyl, cyclooctyl and the like. The terms "C₅-C₆ cycloalkyl", and "C₃-C₆ cycloalkyl" have similar meanings.

As used herein, the terms "aromatic ring" or "aryl" have the same meaning, preferably "aryl" is "C₆-C₁₂ aryl" or "C₆-C₁₀ aryl". The term "C₆-C₁₂ aryl" refers to an aromatic ring group having 6 to 12 carbon atoms without any heteroatoms on the ring, such as phenyl, naphthyl and the like. The term "C₆-C₁₀ aryl" has a similar meaning.

As used herein, the terms "heteroaromatic ring" or "heteroaryl" have the same meaning, referring to heteroaromatic groups containing one to more heteroatoms. The heteroatoms referred herein include oxygen, sulfur, and nitrogen. Such as furyl, thienyl, pyridinyl, pyrazolyl, pyrrolyl, N-alkyl pyrrolyl, pyrimidinyl, pyrazinyl, imidazolyl, tetrazolyl, and the like. Heteroaryl may be condensed onto an ring of aryl, heterocyclyl, or cycloalkyl, wherein the ring connected to the parent structure is a heteroaryl ring. Heteroaryl may be optionally substituted or unsubstituted.

As used herein, the term "Carbocyclic group" refers to a cyclic group that is saturated or unsaturated (non-aromatic ring, including single ring, fused ring, spiro ring, bridged ring, etc.), with a ring skeleton structure consisting only of carbon atoms. Preferably, the carbocyclic group is 3-12 membered, such as 4-9 membered, 5-6 membered, etc. Typical examples include cyclopentyl, cyclohexyl, etc.

As used herein, the term "heterocyclyl" refers to a saturated or unsaturated (non-aromatic rings, including single ring, fused ring, spiro ring, bridged ring, etc.) cyclic group containing 1-3 heteroatoms selected from oxygen, sulfur, and nitrogen on the ring, preferably, the heterocyclyl is 3-12 membered, for example 4-9 membered, 3-7 membered, 5-6 membered. Typical examples include dioxolane.

As used herein, the term "substituted" indicates that one or more hydrogen atoms on a specific group are substituted by specific substituents. The specific substituents are the substituents described in the previous text, or the substituent appeared in each example. Unless otherwise specified, a substituted group may have a substituent selected from a specific group at any substitutable site of that group, and the substituent may be the same or different in each position. A cyclic substituent, such as a heterocycloalkyl, can be connected to another ring, such as a cycloalkyl, thereby forming a spiro-dicyclic ring system, such as wherein the two rings share a common carbon atom. It should be understood by those skilled in the art that the combinations of substituents contemplated by the present invention are those that are stable or chemically achievable. The substituent is exemplifed by such as (but not limited to): C₁₋₈ alkyl, C₂₋₈ alkenyl, C₂₋₈ alkynyl, C₃₋₈ cycloalkyl, 3- to 12-membered heterocyclyl, aryl, heteroaryl, halogen, hydroxyl, carboxyl (-COOH), C₁₋₈ aldehyde group, C₂₋₁₀ acyl, C₂₋₁₀ ester group, amino, alkoxyl, C₁₋₁₀ sulfonyl, etc.

### Compounds of formula I with TLR7 and/or TLR8 regulatory activity

The present invention provides a compound of formula I, or a solvate, a prodrug, a metabolite, or a pharmaceutically acceptable salt thereof, 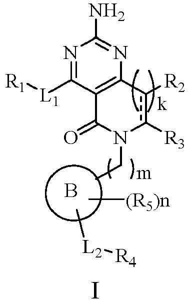 wherein:
L₁ is selected from the group consisting of: -O-, -NH-, -S-, -S(=O)- and -S(=O)₂-;
R₁ is selected from the group consisting of: H, C₁₋₁₂ alkyl, C₁₋₁₂ alkoxy, C₂₋₁₂ alkenyl, C₂₋₁₂ alkynyl, C₃₋₁₂ cycloalkyl and 4-12 membered heterocycloalkyl; wherein, R₁ can be further substituted by one or more R^{a} substituents, and R^{a} is selected from the group consisting of: hydrogen, halogen, hydroxyl, cyano, C₁₋₆ alkyl, C₁₋₆ alkoxy, C₃₋₆ cycloalkyl, -NR^{a1}R^{a2}, -NHC(=O)-R^{a3}, 5-6-membered heteroaryl substituted with one or more R^{a4};
R^{a1}, R^{a2}, R^{a3}, or R^{a4} is selected from the group consisting of: C₁₋₆ alkyl, C₁₋₆ haloalkyl, and C₃₋₆ cycloalkyl;
R₂ and R₃ are independently selected from the group consisting of: hydrogen, halogen, cyano, C₁₋₆ alkyl, C₁₋₆ alkoxy, C₃₋₆ cycloalkyl, 4-6 membered heterocycloalkyl, 5-8-membered heteroaryl and 5-8-membered aryl; wherein, R₂ and R₃ can be further substituted by one or more substituents selected from the group consisting of: halogen, hydroxyl, cyano, and amino;
k is 0 or 1;
m is 0, 1, 2, 3, 4, 5, 6,7 or 8;
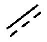 represents a single or double bond;
B is absent, or B is selected from the group consisting of: C₃₋₁₂ cycloalkyl, 4-12 membered heterocycloalkyl, C₆₋₁₂ aryl, 5-12 membered heteroaryl and 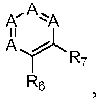 wherein, each A is selected from C, CH and N, and R₆ and R₇ together with the attached carbon atom jointly form a C₄₋₇ cycloalkylene or C₄₋₇ heterocycloalkylene, one or more methylene groups in the C₄₋₇ cycloalkylene or 4-7-membered heterocycloalkylene can be independently replaced by carbonyl or S(=O)₂; and the heteroatom in the 4-7-membered heterocycloalkylene is selected from N, O, and S; the number of heteroatom is 1 to 3;
L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O-), -S(=O)- and -S(=O)₂-; wherein, R^{b}, R^{c} are selected from the group consisting of: hydrogen, halogen, C₁₋₆ alkyl, C₃₋₆ cycloalkyl, 4-6 membered heterocycloalkyl and C₁₋₆ haloalkyl, R^{d} is selected from the group consisting of: hydrogen, C₁₋₆ alkyl, C₃₋₆ cycloalkyl, 4-6 membered heterocycloalkyl, C₁₋₆ haloalkyl and hydroxyl substituted C₁₋₆ alkyl; p is 0, 1, 2, 3, 4, 5, or 6; q is 0 or 1;
R₄ is selected from the group consisting of: hydrogen, halogen, cyano, amino, hydroxyl, C₁₋₁₂ alkyl, C₁₋₁₂ alkoxy, C₂₋₁₂ alkenyl, C₂₋₁₂ alkynyl, C₃₋₁₂ cycloalkyl, 4-12 membered heterocyclyl, C₆₋₁₂ aryl and 5-12 membered heteroaryl; and R₄ is optionally substituted by one or more R^{e}, wherein R^{e} is selected from the group consisting of: hydrogen, halogen, hydroxyl, carboxylic acid, amino, C₁₋₆alkyl, C₁₋₆alkyl substituted with one or more R^{e1}, C₁₋₆ alkoxy, C₁₋₆ haloalkoxy, C₃₋₁₂ cycloalkyl, 4-12 membered heterocycloalkyl, C₆₋₁₂ aryl, 5-12 membered heteroaryl, -N(R^{e2}R^{e3}R^{e4}), -C(=O)O-R^{e2}, -C(=O)NH-R^{e2}, and -S(=O)₂-R^{e2};
R^{e1} is selected from the group consisting of: halogen, and hydroxyl;
R^{e2} and R^{e3} are selected from the group consisting of: hydrogen, C₁₋₆ alkyl, C₃₋₆ cycloalkyl, C₁₋₆ haloalkyl and hydroxyl substituted C₁₋₆ alkyl;
R^{e4} is absent or selected from the group consisting of: C₁₋₆ alkyl, C₃₋₆ cycloalkyl and C₁₋₆ haloalkyl;
R₅ is selected from the group consisting of: halogen, hydroxyl, cyano, amino, C₁₋₆ alkyl, C₃₋₆ cycloalkyl, 4-6 membered heterocycloalkyl, C₁₋₆ haloalkyl, C₁₋₆ alkoxy, C₁₋₆ haloalkoxy, -C(=O)O-R^{f}, -C(=O)NH-R^{f}, and -S(=O)₂-R^{f}; wherein, R^{f} is selected from the group consisting of: hydrogen, C₁₋₆ alkyl, C₃₋₆ cycloalkyl and C₁₋₆ haloalkyl;
n is 1, 2, 3, 4, 5 or 6;
wherein, the heterocyclyl can be saturated or partially unsaturated (but without aromatic structure), and in the heterocyclyl, the heteroatom is selected from N, O and S, and the number of heteroatom is 1, 2, 3, or 4 (preferably 1 or 2); among the heteroaryl, the heteroatom is selected from N, O and S, and the number of heteroatom is 1, 2, or 3.

### Preparation Method for the Compound of formula I

The invention further provides a preparation method for the above-mentioned compound of formula I, comprising the following scheme 1 or 2:

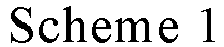

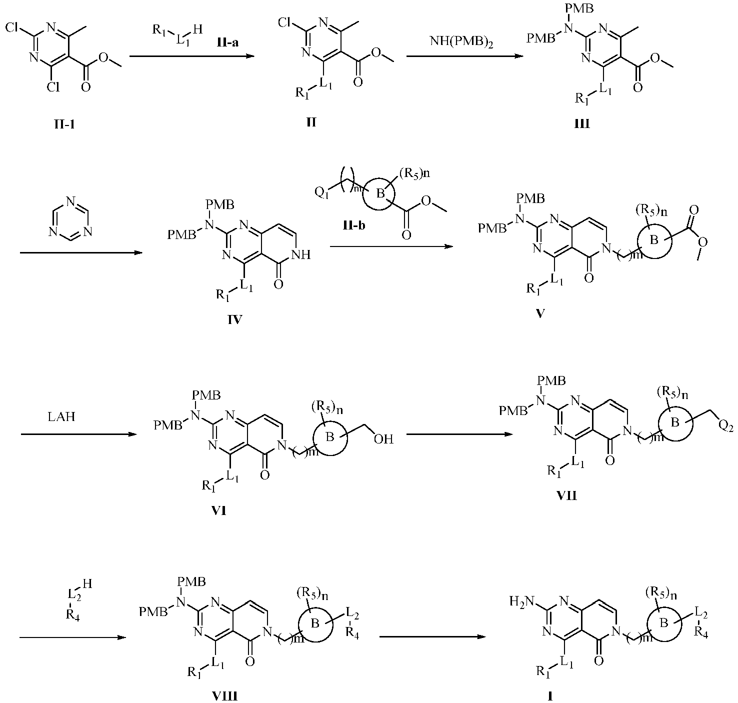

In the scheme 1, the starting compound II-1 undergoes a substitution reaction to obtain compound II; Compound II further undergoes a substitution reaction to obtain compound III; Compound III undergoes a cyclization reaction to obtain compound IV; Compound IV undergoes a substitution reaction or a copper catalyzed reaction to obtain compound V; Compound V undergoes a reduction reaction and cyclization to obtain compound VI; Compound VI undergoes a substitution reaction to obtain compound VII; Compound VII undergoes a substitution reaction with different amines to obtain compound VIII; and Compound VIII undergoes deprotection to obtain compound I.

Q1 and Q2 can be chlorine, bromine, iodine, OMs or OTf

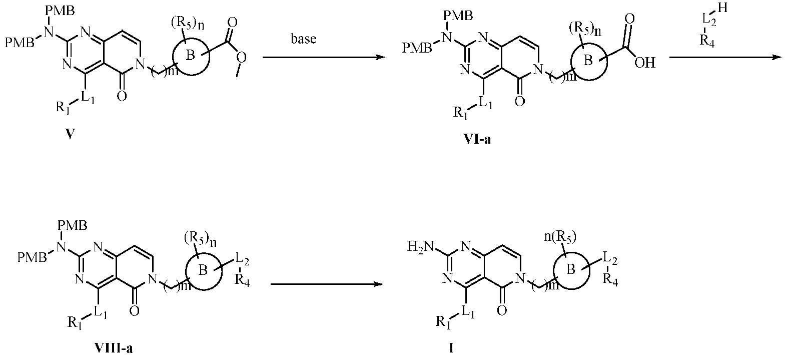

In scheme 2, compound V undergoes a hydrolysis reaction to obtain compound VI-a; Compound VI-a undergoes a condensation reaction to obtain compound VIII-a; Compound VIII-a undergoes deprotection to obtain compound I.

### Pharmaceutical composition containing active ingredient

Since the compounds of the present invention have excellent agonistic activity against Toll-like receptors, thereby the compounds of the present invention and their various crystalline forms, pharmaceutically acceptable inorganic or organic salts, hydrates or solvates, as well as pharmaceutical compositions comprising the compounds of the present invention as the main active ingredient can be used for preventing and/or treating (stabilizing, alleviating, or curing) diseases or disorders related to abnormal expression or activation of Toll-like receptors (especially TLR7, 8), such as tumors or infections caused by viruses; more preferably, the viruses are preferably one or more of HBV, HCV, HIV, and influenza viruses, and the tumors are preferably lung cancer, pancreatic cancer, renal cancer, head and neck cancer, breast cancer, lymphoma, skin cancer, uroepithelial cancer, gastric cancer, hepatocellular carcinoma, colorectal cancer, and other cancer.

The pharmaceutical composition of the present invention comprises a safe and effective amount of the compound of the present invention and a pharmaceutically acceptable excipient or carrier. Wherein "safe and effective amount" refers to the amount of compound which is sufficient to significantly improve the condition, and not to generate severe side effects. Generally, the pharmaceutical composition contains 0.01-99.99% by weight the compound of present invention per dose, preferably, 0.1-99.9% the compound of present invention per dose. Preferably, the "one dose" is one capsule or one tablet.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other, without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

There is no special limitation on the administration mode of the compound or pharmaceutical compositions of the present invention, and the representative administration mode includes (but is not limited to): oral, parenteral (intravenous, intramuscular or subcutaneous) administration.

The solid dosage forms used for oral administration include capsules, tablets, pills, powders, and granules. In these solid dosage forms, the active compounds are mixed with at least one conventional inert excipient (or carrier), such as sodium citrate or CaHPO₄, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the release of active compounds or compounds in the composition can be delayed in a certain part of the digestive tract. Examples of embedding components that may be employed are polymeric substances and waxes. If necessary, the active compound may also be formed into a microcapsules with one or more of the above excipients.

Liquid dosage forms for oral administration include pharmaceutically acceptable emulsion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, maize germ oil, olive oil, castor oil and sesame oil, or combinations thereof, ect.

In addition to these inert diluents, the composition can also include additives such as wetting agents, emulsifiers and suspensions, sweeteners, correctors, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances, ect.

Compositions for parenteral injection may include physiologically acceptable sterile aqueous or anhydrous solutions, dispersion liquid, suspensions or emulsions, and sterile powders for re dissolution into sterile injectable solutions or dispersions. Suitable aqueous and non-aqueous carriers, diluents, solvents or excipients include water, ethanol, polyols, and their suitable mixtures.

The compound of the present invention may be administered alone or in combination with other pharmaceutically acceptable compound(e.g. anti HBV agents).

When co-administered, the pharmaceutical composition further comprises one or more (2, 3, 4, or more) other pharmaceutically acceptable compounds. One or more (2, 3, 4, or more) other pharmaceutically acceptable compounds may be used concurrently, separately, or sequentially with the compounds of the present invention for the prevention and/or treatment of diseases or disorders related to abnormal expression or activation of Toll-like receptors(especially TLR7, 8).

When the pharmaceutical compositions are used, a safe and effective amount of compound of the present invention is applied to a mammal (such as human) in need thereof, wherein the dose of administration is a pharmaceutically effective dose. Of course, the particular dose should also depend on various factors, such as the route of administration, patient healthy status, which are well within the skills of an experienced physician.

The present invention was further described hereafter in combination with specific examples. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are caculated by weight.

In the following examples, the structures of the compounds were determined by nuclear magnetic resonance (NMR) or/and mass spectrometry (MS). The NMR shift (δ) is given in units of 10⁻⁶(ppm). The NMR was determined by Bruker AVANCE-400 NMR. The solvents were deuterated dimethyl sulfoxide (DMSO-d₆), deuterated chloroform (CDCl₃), deuterated methanol (CD₃OD), and the internal standard was tetramethylsilane (TMS).

SHIMADZU LC system (column: XselectO,R: CSH^{™} Prep-C18, 19*150mm, liquid processor LH-40, pump LC-20AP, detector SPD-20A, system controller CBM-20A, solvent system: acetonitrile and 0.05% trifluoroacetic acid aqueous solution).

LC/MS spectra of the compounds were obtained using LC/MS(Agilent Technologies 1200 Series). The LC/MS conditions were as follows (10 min run time):
Acidic conditions: A:0.05% trifluoroacetic acid in water; B:0.05% trifluoroacetic acid in acetonitrile;
Alkaline conditions: A:0.05%NH₃• H₂O aqueous solution; B: acetonitrile
Neutral conditions: A: 10 mM NH₄OAC aqueous solution; B: acetonitrile

Unless otherwise specified, in the following examples, the intermediates and final compounds were purified using silica gel column chromatography, or using a XselectO,R-CSH^{™} Prep-C18(5 µm, OBD^{™} 19*150mm) column or using a XBridgeTM Prep Phenyl(5 µm, OBD^{™} 30* 100mm) on a reversed-phase chromatographic column by preparative HPLC.

Silica gel column chromatography generally uses Yantai Huanghai silica gel 200-300 mesh silica gel as the carrier.

The Combiflash Rf200(TELEDYNE ISCO) was used CombiFlash a rapid preparator.

Thin layer chromatography (TLC) silica gel plate uses Yantai Huanghai HSGF254 or Qingdao GF254 silica gel plate. The specifications of silica gel plate used for thin layer chromatography detection products are 0.15mm -0.2mm, and the specifications of thin layer chromatography separation and purification products are 0.4mm -0.5mm.

The known starting materials of the present invention can be synthesized using or following methods known in the art, or can be purchased from ABCR GmbH & Co.KG, Acros Organics, Aldrich Chemical Company, Shaoyuan Chemical Technology (Accela ChemBio Inc), Darry Chemicals and other companies.

### Abbreviations:

MeOH: methanol; DIEA : N,N-diisopropylethylamine; DMAP : 4-Dimethylaminopyridine; DCM: dichloromethane ; Dioxane: 1,4-Dioxane ; EA: ethyl acetate; Conc.HCl: con. hydrochloric acid; DMF: N,N-dimethylformamide ; AcOH: Acetic Acid ; DMA : N,N-dimethylacetamide ; XantPhos : 9,9-Dimethyl-4,5-bis(diphenylphosphino)xanthene; DCE: 1,2-dichloroethane; BH₃: Borane ; Boc₂O: tert-butyldicarbonate ; t-BuLi: tert-Butyllithium; DMF-DMA: N,N-Dimethylformamide dimethyl acetal; PPA: polyphosphoric acid; NaHMDS: Sodium bis(trimethylsilyl)amide; POCl₃: phosphorus oxychloride; Pd/C: Palladium 10% on Carbon; ACN: Acetonitrile; EtOH: ethanol; t-butyl nitrite: tert-Butyl nitrite; TsOH: P-toluenesulfonic acid; THF: Tetrahydrofuran; LDA: Lithium diisopropylamide; LAH: Lithium Aluminum Hydride; Pd₂(dba)₃: Tris(dibenzylideneacetone)dipalladium; PMB: p-Methoxybenzyl ; Xphos: 2-Dicyclohexylphosphino-2',4',6'-triisopropylbiphenyl ; H₂: hydrogen; Pd(dppf)Cl₂: [1,1'-Bis(diphenylphosphino)ferrocene]dichloropalladium(II) ; t-BuOK: potassium t-butoxide; K₂CO₃; Potassium carbonate; HCOONH₄: Ammonium formate; LiHMDS: Lithium bis(trimethylsilyl)amide; n-BuLi: n-Butyllithium; MsCl: Methanesulfonyl chloride; Dppf: 1,1'-Bis(diphenylphosphino)ferrocene; Et₃N: triethylamine; AcCl: Acetyl chloride; NaH: Sodium hydride ; TFA : trifluoroacetic acid; SOCl₂: thionyl chloride ; NBS : N-Bromosuccinimide ; NCS: N-Chlorosuccinimide; K₃PO₄: Tripotassium Orthophosphate; RuPhos Pd G2: Chloro(2-dicyclohexylphosphino-2',6'-di-i-propoxy-1,1'-biphenyl)(2-aMino-1,1'-biphenyl-2-yl)palladiuM(II); MTBE: Methyl tert-Butyl Ether.

### Intermediate Int. A

### 2-(bis(4-methoxybenzyl)amino)-4-(butylamino)pyrido[4,3-d]pyrimidin-5(6H)-one

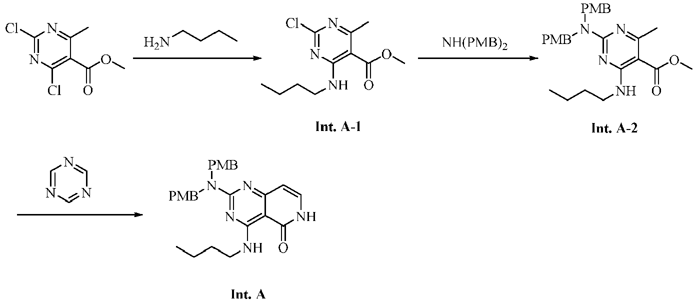

### Step 1: Preparation of Int. A-1

To a stirred mixture solution of methyl 2,4-dichloro-6-methylpyrimidine-5-carboxylate (10 g, 45.2 mmol) and butan-1-amine (3.6 g, 49.8 mmol) in DCM (100 mL) was added DIEA (8.8 g, 67.9 mmol) at 0°C, then the resulting mixture was stirred at 0°C for 3 hours. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Int. A-1** (11 g, 95 %) as a colorless oil. MS: 258.1 (M+H)⁺

### Step 2: Preparation of Int. A-2

To a stirred mixture solution of **Int. A-1** (11 g, 42.8 mmol) and N,N-bis(4-methoxybenzyl)amine (13.2 g, 51.4 mmol) in ACN (100 mL) was added K₂CO₃ (8.9 g, 64.2 mmol) at 0°C, then the resulting mixture was stirred at 70°C for 13 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Int. A-2** (18.4 g, 90 %) as a yellow solid. MS: 479.3 (M+H)⁺

### Step 3: Preparation of Int. A

To a stirred mixture solution of **Int. A-2** (23 g, 48.1 mmol) and 1,3,5-triazine (4.68 g, 57.7 mmol) in DMSO (150 mL) was added t-BuOK (8.09 g, 72.1 mmol) at 0°C, then the resulting mixture was stirred at 80°C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Int. A** (17 g, 74.7 %) as a yellow solid. MS: 474.2 (M+H)⁺

### Intermediate Int. B

### 2-((4-methoxybenzyl)amino)-4-((2-methoxyethyl)amino)pyrido[4,3-d]pyrimidin-5(6H)-one

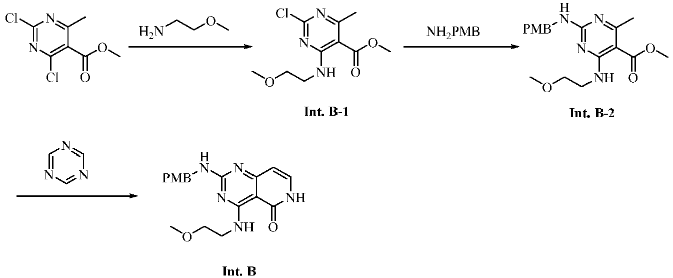

The title compound **Int. B** was prepared in analogy to the preparation of **Int. A** by using 2-methoxyethan-1-amine instead of butan-1-amine and (4-methoxyphenyl)methanamine instead of N,N-bis(4-methoxybenzyl)amine. MS: 356.2 (M+H)⁺.

### Intermediate Int. C

### 2-(bis(4-methoxybenzyl)amino)-4-(pentan-2-ylamino)pyrido[4,3-d]pyrimidin-5(6H)-one

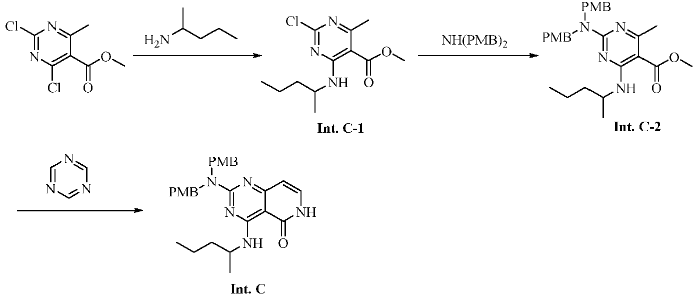

The title compound **Int. C** was prepared in analogy to the preparation of **Int. A** by using pentan-2-amine instead of butan-1-amine. MS: 488.2 (M+H)⁺.

### Intermediate Int. D

### 2-(bis(4-methoxybenzyl)amino)-4-((2-cyclopropylethyl)amino)pyrido[4,3-d]pyrimidin-5(6H)-one

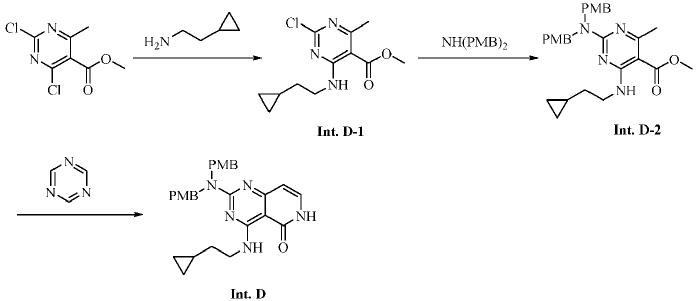

The title compound **Int. D** was prepared in analogy to the preparation of **Int. A** by using 2-cyclopropylethan-1-amine instead of butan-1-amine. MS: 486.2 (M+H)⁺.

### Intermediate Int. E

### 2-(bis(4-methoxybenzyl)amino)-4-((2-ethoxyethyl)amino)pyrido[4,3-d]pyrimidin-5(6H)-one

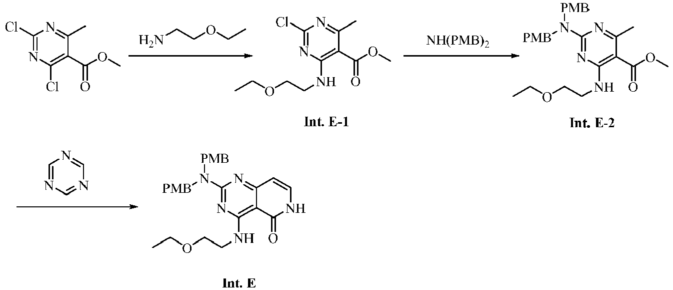

The title compound **Int. E** was prepared in analogy to the preparation of **Int. A** by using 2-ethoxyethan-1-amine instead of butan-1-amine. MS: 490.2 (M+H)⁺.

### Intermediate Int. F

### 2-(bis(4-methoxybenzyl)amino)-4-((1-hydroxyhexan-3-yl)amino)pyrido[4,3-d]pyrimidin-5(6H)-one

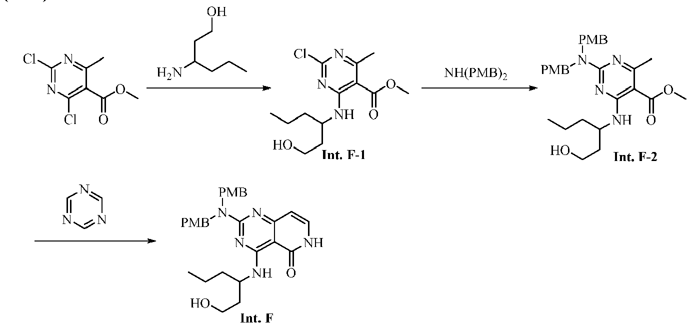

The title compound **Int. F** was prepared in analogy to the preparation of **Int. A** by using 3-aminohexan-1-ol instead of butan-1-amine. MS: 518.2 (M+H)⁺.

### Intermediate Int. G

### 2-(bis(4-methoxybenzyl)amino)-4-((2-(ethylamino)ethyl)amino)pyrido[4,3-d]pyrimidin-5(6H)-one

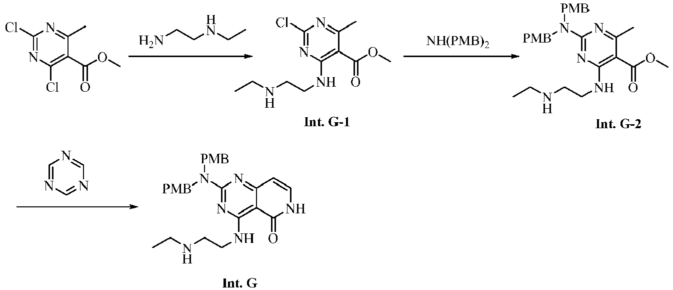

The title compound **Int. G** was prepared in analogy to the preparation of **Int. A** by using N¹-ethylethane-1,2-diamine instead of butan-1-amine. MS: 489.2 (M+H)⁺.

### Intermediate Int. H

### 2-(bis(4-methoxybenzyl)amino)-4-((4-fluorobutyl)amino)pyrido[4,3-d]pyrimidin-5(6H)-one

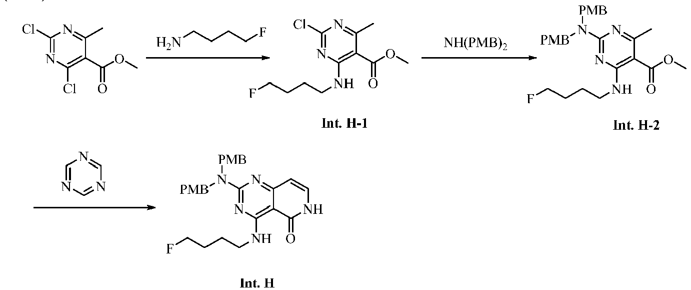

The title compound **Int. H** was prepared in analogy to the preparation of **Int. A** by using 4-fluorobutan-1-amine instead of butan-1-amine. MS: 492.2 (M+H)⁺.

### Intermediate Int. J

### (R)-pentan-2-amine hydrochloride

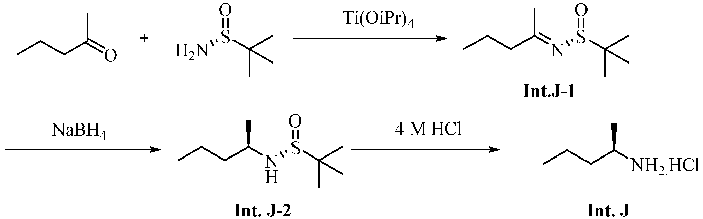

### Step 1: Preparation of Intermediate Int. J-1

To a stirred mixture solution of pentan-2-one (10 g, 116 mmol) in EA (200 mL) was added (R)-2-methylpropane-2-sulfinamide (14 g, 116 mmol) and Ti(OiPr)₄ (66 g, 233 mmol) at 10 °C and the resulting mixture was stirred at 10 °C for 15 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. J-1** (7.0 g, 32%) as a colorless oil. MS: 190.2 (M+H)⁺.

### Step 2: Preparation of Intermediate Int. J-2

To a stirred mixture solution of **Int. J-1** (7 g, 37 mmol) in THF (100 mL) was added NaBH₄ (2.1 g, 55.6 mmol) at -70 °C and the resulting mixture was stirred at 20 °C for 2 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. J-2** (3.5 g, 49%) as a colorless oil. MS: 192.2 (M+H)⁺.

### Step 3: Preparation of Intermediate Int. J

A mixture solution of **Int. J-2** (3.5 g, 18.3 mmol) in 4N HCl-Dioxane solution (20 mL) was stirred at 20 °C for 12 hours. After the reaction was completed, the reaction mixture was concentrated in vacuo to give a crude product, which was with ether to afford **Int. J** (2 g, 90%) as a white solid.

### Intermediate Int. K

### (S)-pentan-2-amine hydrochloride

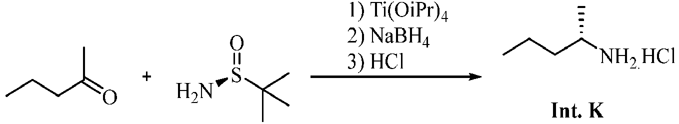

The title compound **Int. K** was prepared in analogy to the preparation of **Int. J** by using (S)-2-methylpropane-2-sulfinamide instead of (R)-2-methylpropane-2-sulfinamide e. MS: 492.2 (M+H)⁺.

### Intermediate Int. L

### (R)-2-(bis(4-methoxybenzyl)amino)-4-(pentan-2-ylamino)pyrido [4,3-d]pyrimidin-5(6H)-one

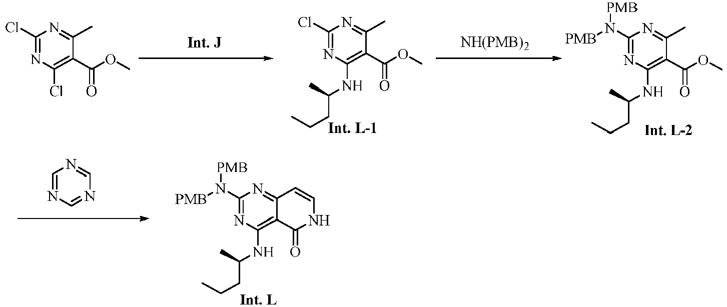

The title compound **Int. L** was prepared in analogy to the preparation of **Int. A** by using **Int. J** instead of butan-1-amine. MS: 488.2 (M+H)⁺.

### Intermediate Int. M

### (S)-2-(bis(4-methoxybenzyl)amino)-4-(pentan-2-ylamino)pyrido[4,3-d]pyrimidin-5(6H)-one

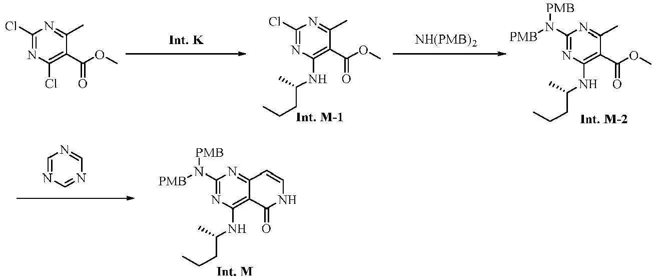

The title compound **Int. M** was prepared in analogy to the preparation of **Int. A** by using **Int. K** instead of butan-1-amine. MS: 488.2 (M+H)⁺.

### Intermediate Int. N

### (R)-2-(bis(4-methoxybenzyl)amino)-4-((1-hydroxy-2-methylhexan-2-yl)amino)pyrido[4,3-d]pyrimidin-5(6H)-one

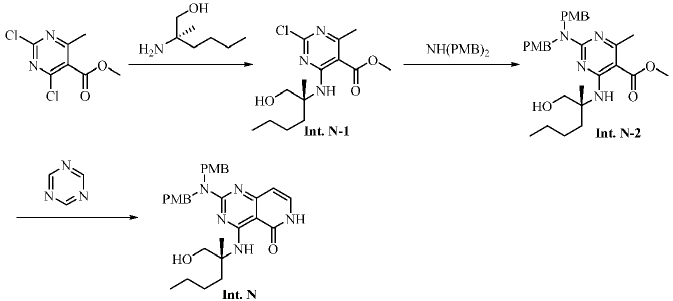

The title compound **Int. N** was prepared in analogy to the preparation of **Int. A** by using (R)-2-amino-2-methylhexan-1-ol instead of butan-1-amine. MS: 532.2 (M+H)⁺.

### Intermediate Int. P

### 6-amino-8-(butylamino)-4-fluoro-2,7-naphthyridin-1(2H)-one

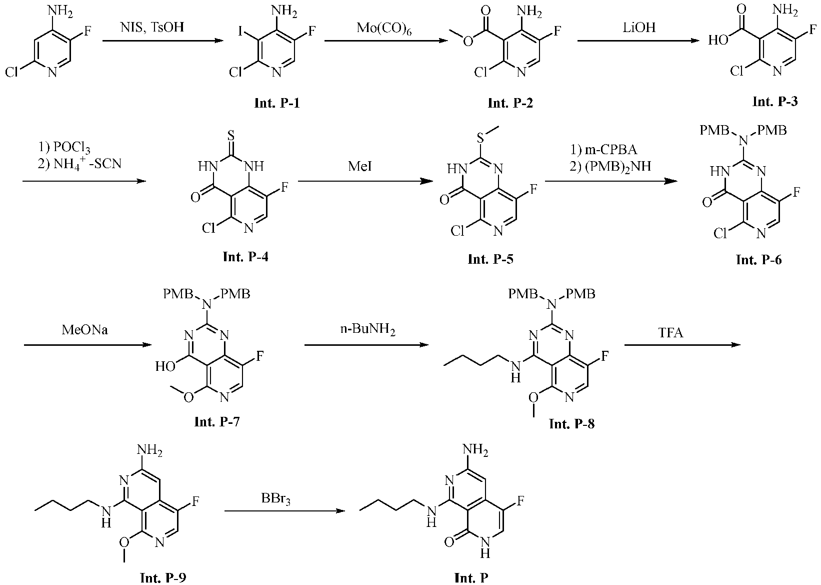

### Step 1: Preparation of Intermediate Int.P-1

To a stirred mixture solution of 2-chloro-5-fluoropyridin-4-amine (6 g, 40.9 mmol) and TsOH (0.389 g, 2.047 mmol) in ACN (60 mL) was added NIS (10.13 g, 45.0 mmol) at 20 °C and the resulting mixture was stirred at 70 °C for 2 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-1** (8.8 g, 79%) as a yellow solid. MS: 272.1&274.2 (M+H)⁺.

### Step 2: Preparation of Intermediate Int.P-2

A mixture solution of **Int.P-1** (9.5 g, 34.9 mmol), Mo(CO)₆ ( 13.81 g, 52.3 mmol), TEA (17.64 g, 174 mmol), Xantphos (1.009g, 1.743 mmol) and Pd(OAc)₂ (0.391g, 1.743 mmol) in MeOH (200 mL) was stirred at 60 °C for 4 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-2** (4.44 g, 62%) as a white solid. MS: 205.1&207.2 (M+H)⁺.

### Step 3: Preparation of Intermediate Int.P-3

The title compound **Int.P-3** was prepared in analogy to the preparation of **Intermediate I-14-1** by using **Int. P-2** instead of **Intermediate I-11-1.** MS: 191.1&193.1 (M+H)⁺.

### Step 4: Preparation of Intermediate Int.P-4

A mixture solution of **Int.P-3** (3.92 g, 20.57 mmol) in phosphoryl trichloride (20 mL) was stirred at 70 °C for 2 hours. After the reaction was completed,the solvent was removed in vacuo and co-evaporated with DCM (20 mL) once. The residue was diluted with THF (30 mL) and then thiocyanic acid, ammonia salt (3.13 g, 41.1 mmol) was added to the above mixture solution. The resulting mixture was stirred at 20 °C for 16 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-4** (2.7 g, 57%) as a yellow solid. MS: 231.1&233.2 (M+H)⁺.

### Step 5: Preparation of Intermediate Int.P-5

A mixture solution of **Int.P-4** (2.72 g, 11.74 mmol), CH₃ONa ( 0.634 g, 11.74 mmol) and CH₃I (1.667g, 11.74 mmol) in DMF (20 mL) was stirred at 20 °C for 4 hours. After the reaction was completed, the reaction mixture was quenched by adding KHCO₃ ice-water solution and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-5** (1.8 g, 62%) as a white solid. MS: 246.1&248.2 (M+H)⁺.

### Step 6: Preparation of Intermediate Int.P-6

A mixture solution of **Int.P-5** (0.362 g, 1.474 mmol) and m-CPBA (0.381g, 2.21 mmol) in toluene (10 mL) was stirred at 40 °C for 2 hours. After the reaction was completed, DIEA (0.571g, 4.42 mmol) and N,N-bis(4-methoxybenzyl)amine (0.758 g, 2.95 mmol) was added to the above mixture solution, then the resulting mixture was stirred at 70 °C for 2 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-6** (188 mg, 27%) as a white solid. MS: 455.1&457.2 (M+H)⁺.

### Step 7: Preparation of Intermediate Int.P-7

A mixture solution of **Int.P-6** (0.3 g, 0.66 mmol) and CH₃ONa ( 0.381 g, 2.21 mmol) in CH₃OH (5 mL) was stirred at 100 °C for 1 hour under M.W. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-7** ( 200 mg, 67%) as a yellow oil. MS: 451.2 (M+H)⁺.

### Step 8: Preparation of Intermediate Int.P-8

A mixture solution of **Int.P-7** (0.211 g, 0.468 mmol), BOP (0.414 g, 0.937 mmol), DBU (0.214 g, 1.405 mmol) and butan-1-amine (0.103 g, 1.405 mmol) in DMF (5 mL) was stirred at 20 °C for 2 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P-8** (100 mg, 42%) as a white solid. MS: 506.2 (M+H)⁺.

### Step 9: Preparation of Intermediate Int.P-9

The title compound **Int.P-9** was prepared in analogy to the preparation of **I-1** by using **Int. P-8** instead of **Intermediate I-1-3.** MS: 266.1 (M+H)⁺.

### Step 10: Preparation of Intermediate Int.P

A mixture solution of **Int.P-9** (40 mg, 0.151 mmol) and BBr₃ (0.754 mmol) in DCM (6 mL) was stirred at -70 °C for 2 hours. After the reaction was completed, the reaction mixture was poured into ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **Int. P.** MS: 252.2 (M+H)⁺.

### Intermediate Int. Q

### 2-(bis(4-methoxybenzyl)amino)-4-(heptan-4-ylamino)pyrido[4,3-d]pyrimidin-5(6H)-one

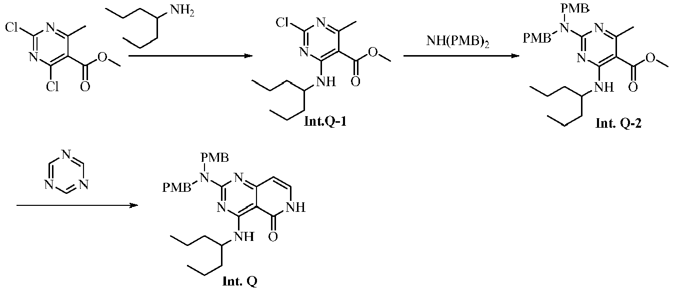

The title compound **Int. Q** was prepared in analogy to the preparation of **Int. A** by using heptan-4-amine instead of butan-1-amine. MS: 516.2 (M+H)⁺.

### Intermediate Int. R

### (S)-2-(bis(4-methoxybenzyl)amino)-4-((1-hydroxyhexan-3-yl)amino)pyrido[4,3-d]pyrimidin-5(6H)-one

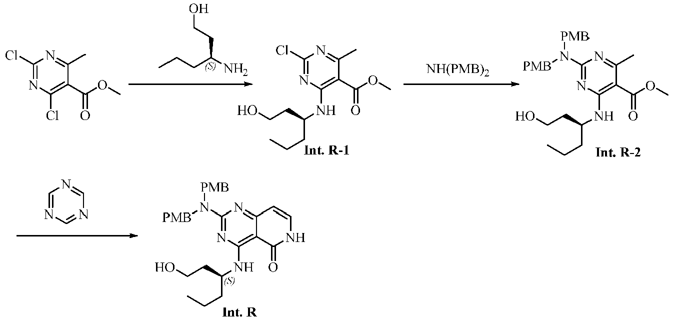

The title compound **Int. R** was prepared in analogy to the preparation of **Int. A** by using (S)-3-aminohexan-1-ol instead of butan-1-amine. MS: 518.2 (M+H)⁺.

### Intermediate Int. S

### 2-(bis(4-methoxybenzyl)amino)-4-butoxypyrido[4,3-d]pyrimidin-5(6H)-one

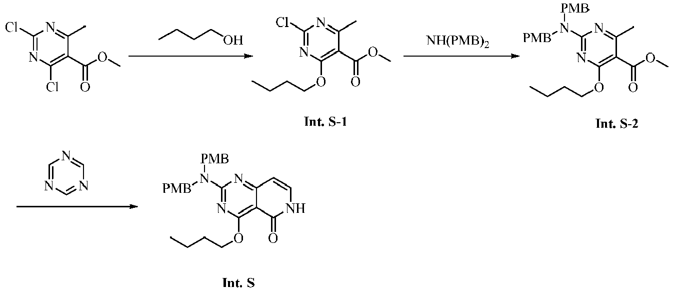

The title compound **Int. S** was prepared in analogy to the preparation of **Int. A** by using butan-1-ol instead of butan-1-amine. MS: 475.2 (M+H)⁺.

### Intermediate Int. T

### 2-(bis(4-methoxybenzyl)amino)-4-(pentan-2-yloxy)pyrido[4,3-d]pyrimidin-5(6H)-one

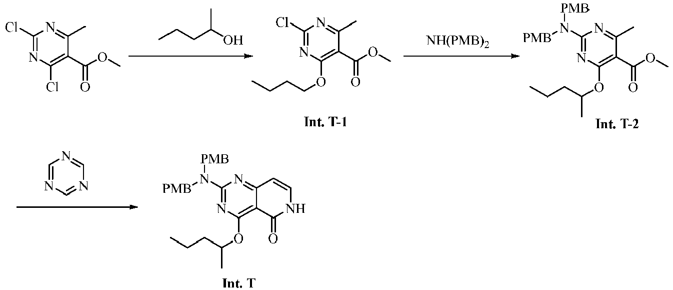

The title compound **Int. T** was prepared in analogy to the preparation of **Int. A** by using pentan-2-ol instead of butan-1-amine. MS: 489.2 (M+H)⁺.

### Intermediate Int. T1

### (S)-2-(bis(4-methoxybenzyl)amino)-4-((1-hydroxypentan-2-yl)amino)pyrido[4,3-d]pyrimidin-5(6H)-one

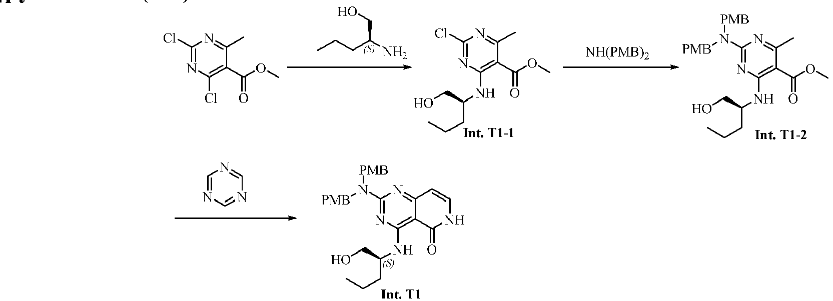

The title compound **Int. T1** was prepared in analogy to the preparation of **Int. A** by using (S)-2-aminopentan-1-ol instead of butan-1-amine. MS: 504.2 (M+H)⁺.

### Intermediate Int. T2

### (R)-2-(bis(4-methoxybenzyl)amino)-4-(hexan-3-ylamino)pyrido[4,3-d]pyrimidin-5(6H)-one

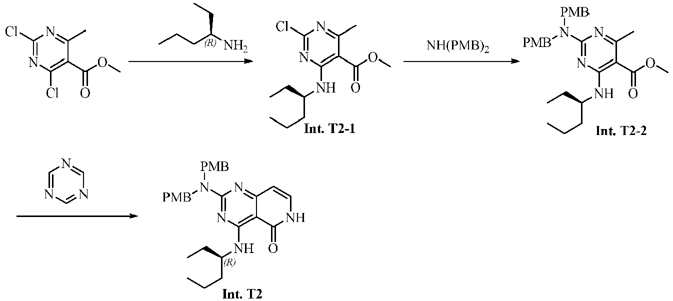

The title compound **Int. T2** was prepared in analogy to the preparation of **Int. A** by using (R)-hexan-3-amine instead of butan-1-amine. MS: 502.2 (M+H)⁺.

### Intermediate Int. T3

### N-(2-((2-(bis(4-methoxybenzyl)amino)-5-oxo-5,6-dihydropyrido [4,3-d]pyrimidin-4-yl)amino)pentyl)acetamide

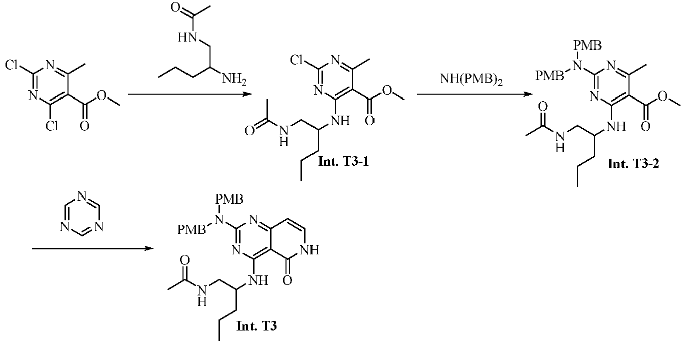

The title compound **Int. T3** was prepared in analogy to the preparation of **Int. A** by using N-(2-aminopentyl)acetamide instead of butan-1-amine. MS: 545.2 (M+H)⁺.

### Intermediate Int. T4

### 2-(bis(4-methoxybenzyl)amino)-4-(butylthio)pyrido[4,3-d]pyrimidin-5(6H)-one

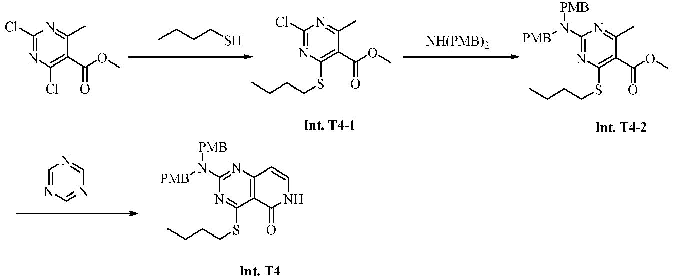

The title compound **Int. T4** was prepared in analogy to the preparation of **Int. A** by using butane-1-thiol instead of butan-1-amine. MS: 491.2 (M+H)⁺.

### Intermediate Int. T5

### (R)-2-(bis(4-methoxybenzyl)amino)-4-((1-hydroxypentan-2-yl)amino)pyrido[4,3-d]pyrimidin-5(6H)-one

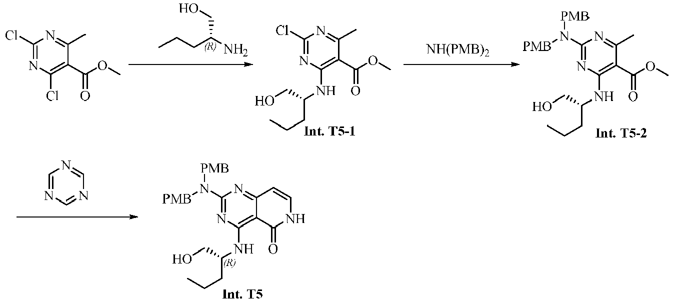

The title compound **Int. T5** was prepared in analogy to the preparation of **Int. A** by using (R)-2-aminopentan-1-ol instead of butan-1-amine. MS: 504.2 (M+H)⁺.

### Intermediate Int. T6

### (S)-2-(bis(4-methoxybenzyl)amino)-4-((1-methoxypentan-2-yl)amino)pyrido[4,3-d]pyrimidin-5(6H)-one

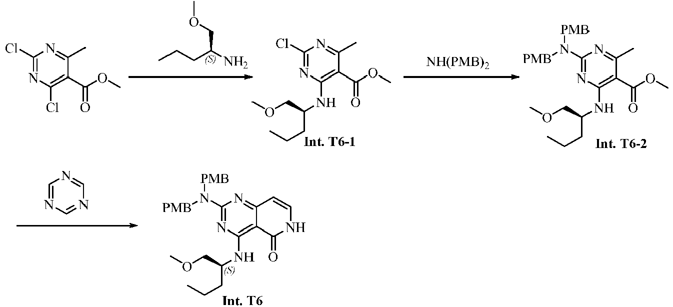

The title compound **Int. T6** was prepared in analogy to the preparation of **Int. A** by using (S)-1-methoxypentan-2-amine instead of butan-1-amine. MS: 518.2 (M+H)⁺.

### Intermediate Int. T7

### (S)-2-(bis(4-methoxybenzyl)amino)-4-((1-hydroxyhexan-2-yl)amino)pyrido [4,3-d]pyrimidin-5(6H)-one

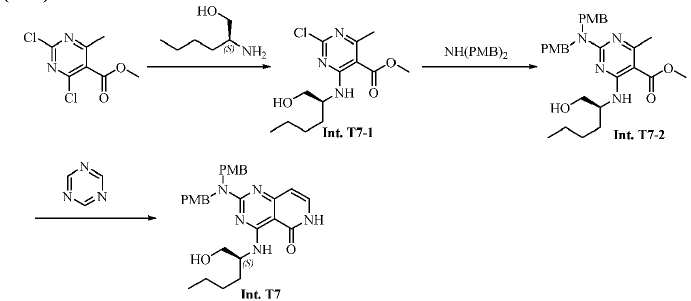

The title compound **Int. T7** was prepared in analogy to the preparation of **Int. A** by using (S)-2-aminohexan-1-ol instead of butan-1-amine. MS: 518.2 (M+H)⁺.

### Intermediate Int. T8

### methyl 4-((5-(butylamino)-7-((4-methoxybenzyl)amino)-4-oxopyrimido[4,5-d]pyrimidin-3(4H)-yl)methyl)benzoate

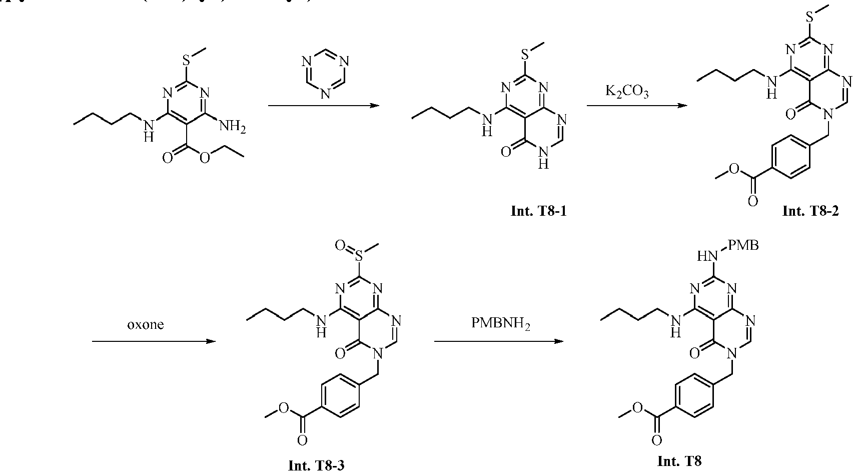

The title compound **Int. T8-1** was prepared in analogy to the preparation of **Int. T7** by using ethyl 4-amino-6-(butylamino)-2-(methylthio)pyrimidine-5-carboxylate instead of **Int. T7-2.** MS: 266.2 (M+H)⁺.

The title compound **Int. T8-2** was prepared in analogy to the preparation of **Intermediate I-11-1** by using **Int. T8-1** instead of **Int. A.** MS: 414.2 (M+H)⁺.

The title compound **Int. T8** was prepared in analogy to the preparation of **Intermediate I-6-8** by using **Int. T8-2** instead of **Intermediate Int. I-6-6.** MS: 503.2 (M+H)⁺.

### Intermediate Int. T9

### 2-(bis(4-methoxybenzyl)amino)-4-((1-((tert-butyldiphenylsilyl)oxy)hexan-3-yl)oxy)pyrido[4,3-d]pyrimidin-5(6H)-one

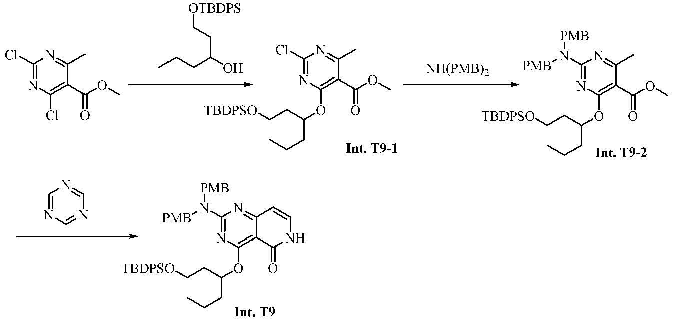

The title compound **Int. T9** was prepared in analogy to the preparation of **Int. A** by using 1-((tert-butyldiphenylsilyl)oxy)hexan-3-ol instead of butan-1-amine. MS: 758.2 (M+H)⁺.

### Intermediate Int. T10

### 4-(butylamino)pyrido[4,3-d]pyrimidin-5(6H)-one

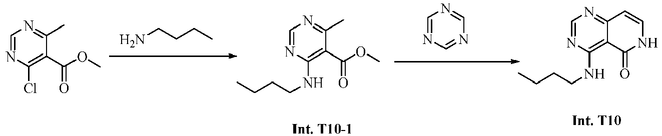

The title compound **Int. T10** was prepared in analogy to the preparation of **Int. A** by using methyl 4-chloro-6-methylpyrimidine-5-carboxylate instead of methyl 2,4-dichloro-6-methylpyrimidine-5-carboxylate. MS: 219.2 (M+H)⁺.

### Intermediate Int. T11

### 4-(butylamino)-2-((4-methoxybenzyl)amino)-7-methylpyrido[4,3-d]pyrimidin-5(6H)-one

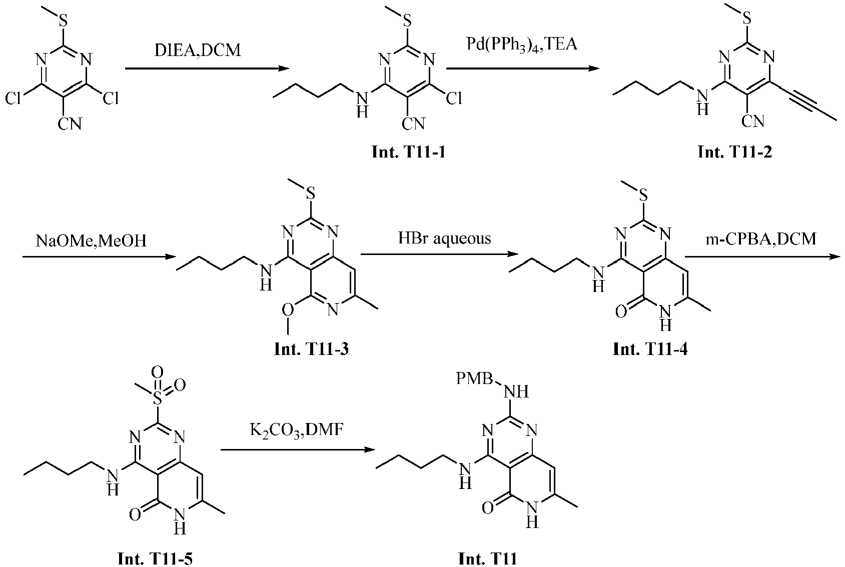

### Step 1: Preparation of Int.T11-1

The title compound **Int. T11-1** was prepared in analogy to the preparation of **Int. A-1** by using 4,6-dichloro-2-(methylthio)pyrimidine-5-carbonitrile instead of methyl 2,4-dichloro-6-methylpyrimidine-5-carboxylate. MS: 257.2 (M+H)⁺.

### Step 2: Preparation of Int.T11-2

A mixture solution of **Int.T11-1** (5.0 g, 19.47 mmol), Pd(Ph₃P)₄ (1.125 g, 0.974 mmol), DIEA (3.78 g, 29.2 mmol) and tributyl(prop-1-yn-1-yl)stannane (9.61 g, 29.2 mmol) in DMF (50 mL) was stirred at 80 °C for 3 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Int.T11-2** (2.2 g, 43%) as a white solid. MS: 261.2 (M+H)⁺.

### Step 3: Preparation of Int.T11-3

A mixture solution of **Int.T11-2** (2.0 g, 7.68 mmol) and CHsONa (0.83 g, 15.36 mmol) in CH3OH (20 mL) was stirred at 40 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Int.T11-3** (1.1 g, 49%) as a white solid. MS: 293.2 (M+H)⁺.

### Step 4: Preparation of Int.T11-4

A mixture solution of **Int.T11-3** (1.0 g, 3.42 mmol) and 48% HBr solution (5 mL) in water (5 mL) was stirred at 50 °C for 3 hours. After the reaction was completed, the reaction mixture was poured into NaHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Int.T11-4** (0.7 g, 74%) as a white solid. MS: 279.2 (M+H)⁺.

### Step 5: Preparation of Int.T11-5

A mixture solution of **Int.T11-4** (0.7 g, 2.51 mmol) and m-CPBA (1.276 g, 6.29 mmol) in DCM (15 mL) was stirred at 20 °C for 3 hours. After the reaction was completed, the reaction mixture was poured into NaHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Int.T11-5** (0.71 g, 91%). MS: 311.2 (M+H)⁺.

### Step 6: Preparation of Int.T11

A mixture solution of **Int.T11-5** (0.71 g, 2.288 mmol), (4-methoxyphenyl)methanamine (0.471 g, 3.43 mmol) and K₂CO₃ (1.276 g, 6.29 mmol) in DMF (10 mL) was stirred at 50 °C for 3 hours. After the reaction was completed, the reaction mixture was poured into NaHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Int.T11** (0.6 g, 71%) as a white solid. MS: 368.2 (M+H)⁺.

### Intermediate Int. T12

### 4-(butylamino)-2-((4-methoxybenzyl)amino)-7-phenylpyrido[4,3-d]pyrimidin-5(6H)-one

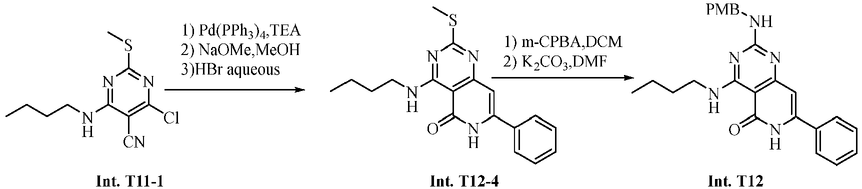

The title compound **Int. T12** was prepared in analogy to the preparation of **Int. T11** by using tributyl(phenylethynyl)stannane instead of tributyl(prop-1-yn-1-yl)stannane. MS: 430.2 (M+H)⁺.

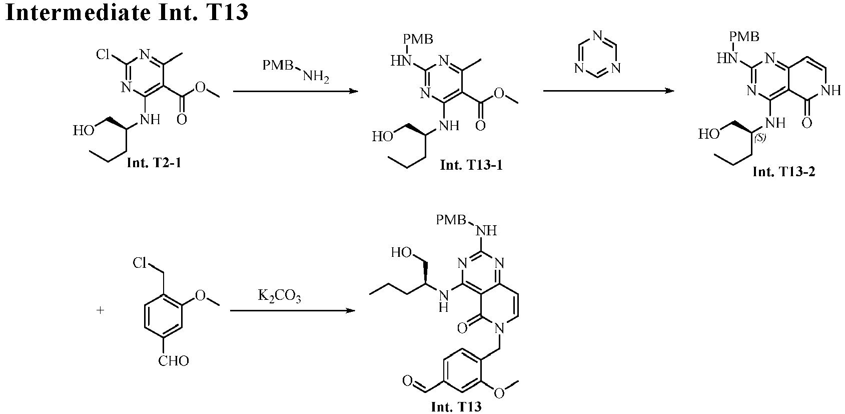

Intermediate **Int. T13-2** was prepared in analogy to the preparation of intermediate **Int. B** by using intermediate **Int. T2-1** instead of intermediate **Int. B-1.** MS: 384.3 (M+H)⁺.
Intermediate **Int. T13** was prepared in analogy to the preparation of intermediate **I-11-1** by using 4-(chloromethyl)-3-methoxybenzaldehyde instead of methyl 4-(bromomethyl)benzoate and intermediate **Int. T13-2** instead of intermediate **Int. A.** MS: 532.3 (M+H)⁺.

### Example I-1

### 2-amino-4-(butylamino)-6-(4-(pyrrolidin-1-ylmethyl)benzyl)pyrido [4,3-d]pyrimidin-5(6H)-one

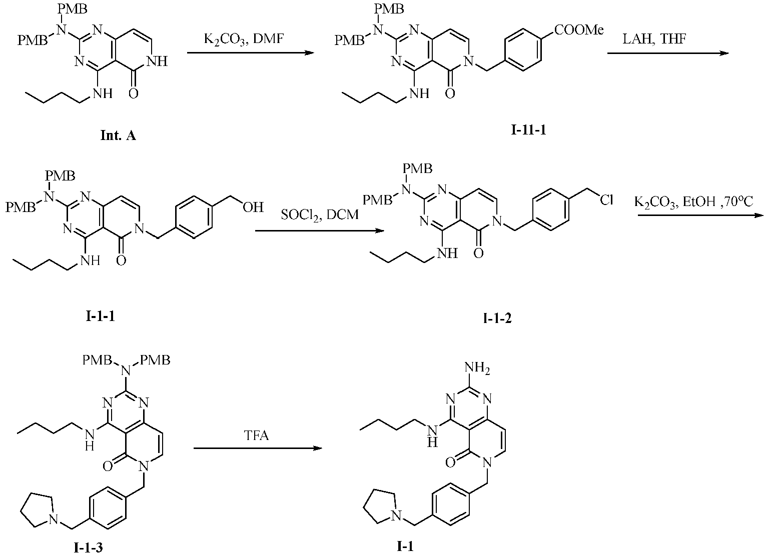

### Step 1: Preparation of Intermediate I-1-3

The title compound **Intermediate I-1-3** was prepared in analogy to the preparation of compound **I-11** by using **Intermediate I-11-1** instead of **Intermediate I-11-4.**

### Step 2: Preparation of compound I-1

The title compound **I-1** was prepared in analogy to the preparation of **Intermediate I-11-2** by using **Intermediate I-1-3** instead of **Intermediate I-11-1.** MS: 407.3 (M+H)⁺.¹H NMR (400 MHz, DMSO-*d₆*) δ 10.13 (s, 1H), 9.98 (s, 1H), 8.11 (d, J = 7.4 Hz, 1H), 7.99 - 7.77 (m, 2H), 7.51 (d, J = 7.8 Hz, 2H), 7.37 (d, J = 7.7 Hz, 2H), 6.38 (d, J = 7.4 Hz, 1H), 5.19 (s, 2H), 4.33 (s, 2H), 3.51 (q, J = 6.6 Hz, 4H), 3.08 (s, 2H), 2.02 (s, 2H), 1.87 (s, 2H), 1.57 (q, J = 7.3 Hz, 2H), 1.34 (dt, J = 14.8, 7.5 Hz, 2H), 0.92 (t, J = 7.3 Hz, 3H).

### Example I-2

### 2-amino-4-(butylamino)-6-(2-methoxy-4-(pyrrolidin-1-ylmethyl)benzyl)pyrido[4,3-d]pyrimidin-5(6H)-one

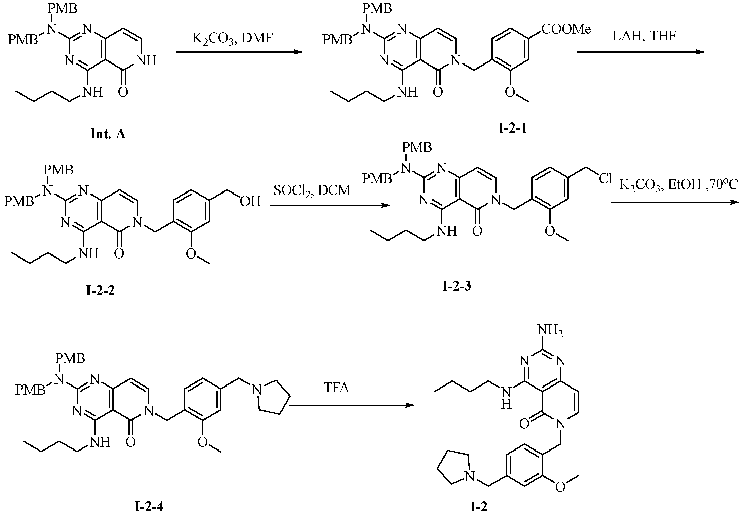

The title compound **I-2** was prepared in analogy to the preparation of compound **I-1** by using methyl 4-(bromomethyl)-3-methoxybenzoate instead of methyl 4-(bromomethyl)benzoate. MS: 437.3 (M+H)⁺. ¹H NMR (400 MHz, DMSO-*d₆*) δ 13.60 (s, 1H), 10.42 (s, 1H), 10.10 (t, J = 5.8 Hz, 1H), 8.42 (s, 2H), 8.08 (d, J = 7.4 Hz, 1H), 7.22 (s, 1H), 7.09 (d, J = 7.7 Hz, 1H), 7.03 (d, J = 7.7 Hz, 1H), 6.40 (d, J = 7.4 Hz, 1H), 5.08 (s, 2H), 4.31 (d, J = 5.1 Hz, 2H), 3.84 (s, 3H), 3.51 (q, J = 6.7 Hz, 2H), 3.35 (s, 2H), 3.06 (s, 2H), 2.00 (d, J = 7.5 Hz, 2H), 1.84 (d, J = 5.4 Hz, 2H), 1.55 (p, J = 7.2 Hz, 2H), 1.32 (h, J = 7.4 Hz, 2H), 0.89 (t, J = 7.3 Hz, 3H).

### Example I-3

### 2-amino-4-((1-hydroxyhexan-3-yl)amino)-6-(4-(pyrrolidin-1-ylmethyl)benzyl)pyrido[4,3-d]pyrimidin-5(6H)-one

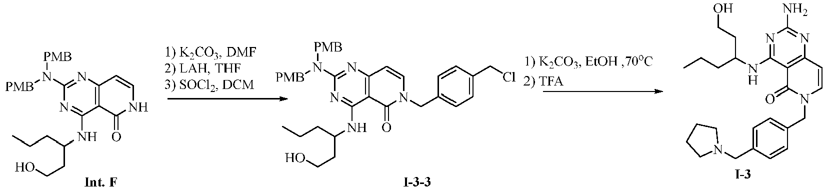

The title compound **I-3** was prepared in analogy to the preparation of compound **I-1** by using **Intermediate Int. F** instead of **Intermediate Int. A.** MS: 451.3 (M+H)⁺.

### Example I-4

### 2-amino-4-(butylamino)-6-(piperidin-4-ylmethyl)pyrido[4,3-d]pyrimidin-5(6H)-one

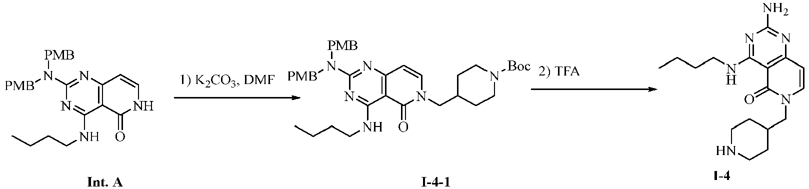

The title compound **I-4** was prepared in analogy to the preparation of compound **I-1** by using tert-butyl 4-(((methylsulfonyl)oxy)methyl)piperidine-1-carboxylate instead of methyl 4-(bromomethyl)benzoate. MS: 331.2 (M+H)⁺.

### Example I-5

### 2-amino-4-(butylamino)-6-(3-methoxy-4-(pyrrolidin-1-ylmethyl)benzyl)pyrido[4,3-d]pyrimidin-5(6H)-one

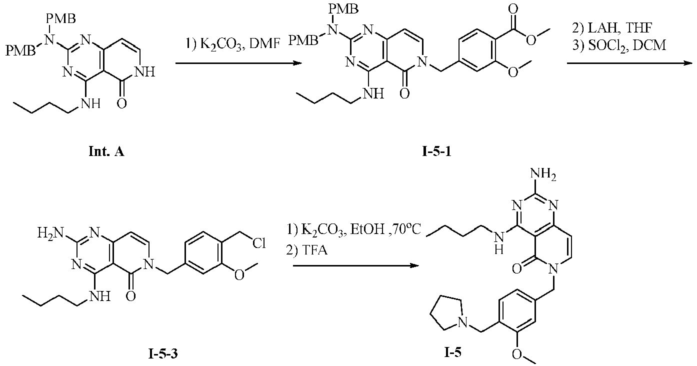

The title compound **I-5** was prepared in analogy to the preparation of compound **I-2** by using methyl 4-(bromomethyl)-2-methoxybenzoate instead of methyl 4-(bromomethyl)-3-methoxybenzoate. MS: 437.3 (M+H)⁺. ¹H NMR (400 MHz, DMSO-*d₆*) δ 13.57 (s, 1H), 10.11 (t, J = 5.8 Hz, 1H), 9.83 (s, 1H), 8.40 (s, 2H), 8.19 (d, J = 7.4 Hz, 1H), 7.40 (d, J = 7.7 Hz, 1H), 7.11 (d, J = 1.5 Hz, 1H), 6.84 (dd, J = 7.8, 1.5 Hz, 1H), 6.41 (d, J = 7.4 Hz, 1H), 5.19 (s, 2H), 4.26 (d, J = 4.6 Hz, 2H), 3.82 (s, 3H), 3.52 (q, J = 6.7 Hz, 2H), 3.36 (d, J = 11.7 Hz, 2H), 3.07 (s, 2H), 1.99 (s, 2H), 1.84 (s, 2H), 1.62 - 1.51 (m, 2H), 1.33 (q, J = 7.4 Hz, 2H), 0.90 (t, J = 7.3 Hz, 3H).

### Example I-6

### 2-amino-4-(butylamino)-6-(4-(pyrrolidin-1-ylmethyl)benzyl)-6,7-dihydro-5H-pyrrolo[3,4-d]pyrimidin-5-one

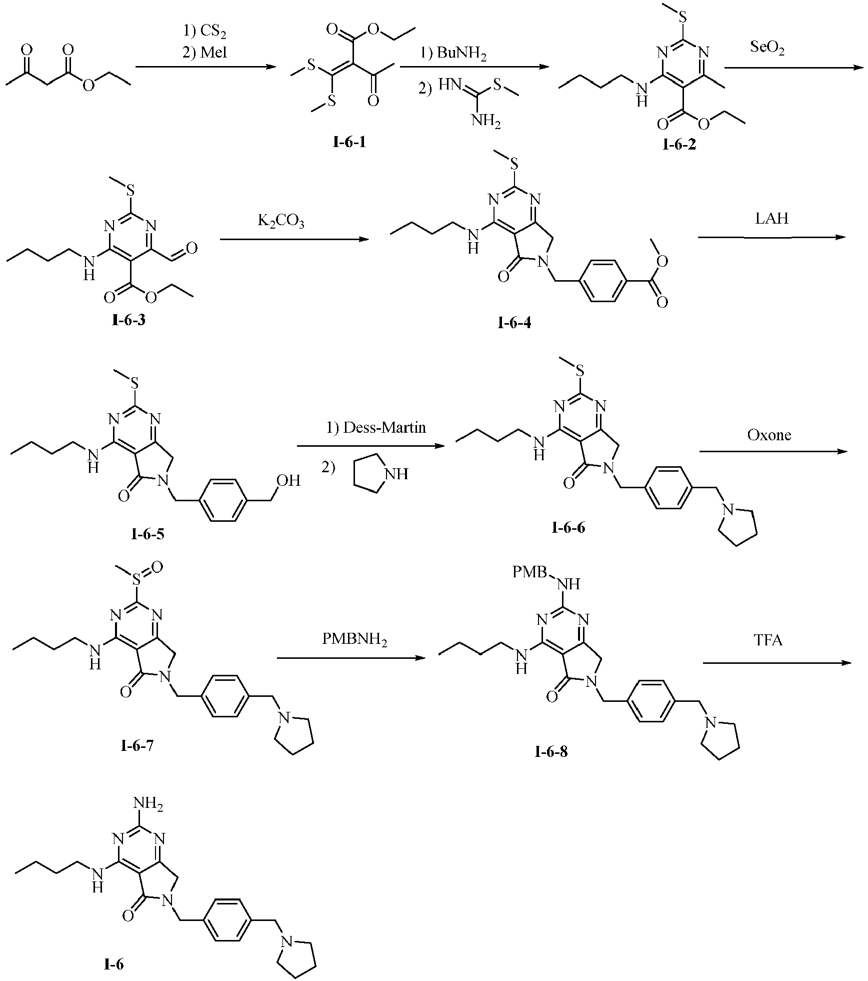

### Step 1: Preparation of Intermediate I-6-1

To a stirred mixture solution of CS₂(3.8 g, 49.9 mmol) and ethyl 3-oxobutanoate (6.5 g, 49.9 mmol) in DMF (100 mL) was added t-BuOK (11.21 g, 100 mmol) at 0 °C, then the resulting mixture was stirred at 20 °C for 1 hour. Then CH₃I (14.18 g, 100 mmol) was added to the above mixture solution at 0 °C and the resulting mixture was stirred at 20 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-6-1** (6.25 g, 53.4%). MS: 235.2 (M+H)⁺.

### Step 2: Preparation of Intermediate I-6-2

To a stirred mixture solution of **Intermediate I-6-1** (6.25 g, 26.7 mmol) and DIEA (6.89 g, 53.3 mmol) in DMF (100 mL) was added butan-1-amine (11.21 g, 100 mmol) at 0 °C, then the resulting mixture was stirred at 20 °C for 16 hours. Then K₂CO₃ (11.06 g, 80 mmol) and methyl carbamimidothioate (7.42 g, 26.7 mmol) was added to the above mixture solution at 0 °C and the resulting mixture was stirred at 80 °C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-2** (8.5 g, 73%). MS: 284.2 (M+H)⁺.

### Step 3: Preparation of Intermediate I-6-3

A mixture solution of **Intermediate I-6-2** (6.25 g, 26.7 mmol) and SeO₂ (2.232 g, 20.11 mmol) in Dioxane (100 mL) was stirred at 100 °C for 16 hours. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-3** (2 g, 99%). MS: 298.2 (M+H)⁺.

### Step 4: Preparation of Intermediate I-6-4

A mixture solution of **Intermediate I-6-3** (3.8 g, 12.8 mmol), methyl 4-(aminomethyl)benzoate (2.11 g, 12.8 mmol) and AcOH (80 mg, 1.3 mmol) in DCM (100 mL) was stirred at 20 °C for 1 hour. Then NaBH₃CN (11.21 g, 100 mmol) was added to the above mixture solution at 0 °C and the resulting mixture was stirred at 20 °C for 1 hour; the resulting mixture was concentrated in vacuo to give a crude product. Then the solution of the residue and K₂CO₃ (1.532 g, 11.08 mmol) in DMF (100 mL) was stirred at 80 °C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-4** (0.465 g, 9%). MS: 401.2 (M+H)⁺.

### Step 5: Preparation of Intermediate I-6-5

The title compound **Intermediate I-6-5** was prepared in analogy to the preparation of **Intermediate I-1-1** by using **Intermediate I-6-4** instead of **Intermediate I-11-2.** MS: 373.2 (M+H)⁺.

### Step 6: Preparation of Intermediate I-6-6

A mixture solution of **Intermediate I-6-5** (200 mg, 0.537 mmol) and Dess-Martin (342 mg, 0.8 mmol) in DCM (20 mL) was stirred at 20 °C for 1 hour. The resulting mixture was concentrated in vacuo to give a crude product, then the solution of the residue, AcOH (1mL) and pyrrolidine (71 mg, 1 mmol) in DCM (10 mL) was stirred at 20 °C for 0.5 hour, NaBH₃CN (63 mg, 1 mmol) was added to the above mixture solution at 0 °C and the resulting mixture was stirred at 20 °C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-6**(0.3 g, 99%). MS: 426.2 (M+H)⁺.

### Step 7: Preparation of Intermediate I-6-7

A mixture solution of **Intermediate I-6-6** (300 mg, 0.705 mmol) and Oxone (1.75 g, 2.82 mmol) in THF (10 mL) and water (10 mL) was stirred at 20 °C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-7** (0.3 g, 96%). MS: 442.2 (M+H)⁺.

### Step 8: Preparation of Intermediate I-6-8

A mixture solution of **Intermediate I-6-7** (300 mg, 0.679 mmol), K₂CO₃ (282 mg, 2.04 mmol) and (4-methoxyphenyl)methanamine (186 mg, 1.36 mmol) in DMF (5 mL) was stirred at 100°C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-6-8** (300 mg, 86%). MS: 515.2 (M+H)⁺

### Step 8: Preparation of Compound I-6

A mixture solution of **Intermediate I-6-8** (300 mg, 0.583 mmol) and anisole (2 mL) in TFA (5 mL) was stirred at 100°C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by prep-HPLC to afford **Compound I-6** (10.2 mg). MS: 395.4 (M+H)⁺. ¹H NMR (400 MHz, DMSO-*d₆*) δ 10.19 (s, 1H), 7.98 (s, 2H), 7.78 (s, 1H), 7.49 (d, J = 8.0 Hz, 2H), 7.34 (d, J = 8.0 Hz, 2H), 4.62 (s, 2H), 4.33 (d, J = 8.0 Hz, 2H), 4.28 (s, 2H), 3.46 (q, J = 5.0 Hz, 2H), 3.34 (s, 2H), 3.05 (m, 2H), 2.01 (s, 2H), 1.92 - 1.72 (m, 2H), 1.55 (m, 2H), 1.30 (p, J = 7.4 Hz, 2H), 0.90 (t, J = 8.0 Hz, 3H).

### Example I-7

### 2-amino-4-(butylamino)-6-(2-methoxy-4-(pyrrolidin-1-ylmethyl)benzyl)-6,7-dihydro-5H-pyrrolo[3,4-d]pyrimidin-5-one

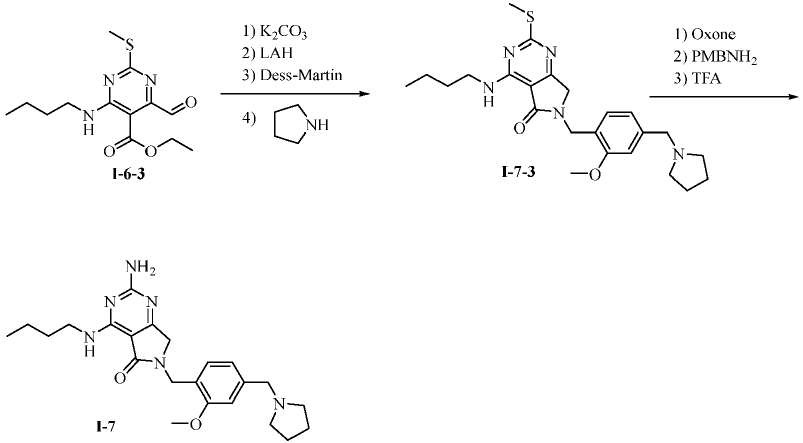

The title compound **I-7** was prepared in analogy to the preparation of compound **I-6** by using methyl 4-(aminomethyl)-3-methoxybenzoate instead of methyl 4-(aminomethyl)benzoate. MS: 425.4 (M+H)⁺. ¹H NMR (400 MHz, DMSO-*d₆*) δ 9.92 (s, 1H), 7.21 (s, 1H), 7.16 (s, 1H), 7.05 (s, 1H), 4.56 (s, 2H), 4.33 (d, J = 5.5 Hz, 2H), 4.25 (s, 2H), 3.87 (s, 3H), 3.48-3.41 (m, 2H), 3.38-3.34 (m, 2H), 3.14-3.06 (m, 2H), 2.07-2.00 (m, 2H), 1.90-1.85 (m, 2H), 1.57-1.53 (m, 2H), 1.35-1.29 (m, 2H), 0.91 (t, J = 6.0 Hz, 3H).

### Example I-8

### methyl 4-((2-amino-4-((2-methoxyethyl)amino)-5-oxopyrido[4,3-d]pyrimidin-6(5H)-yl)methyl)benzoate

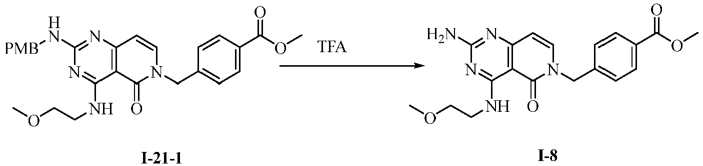

The title compound **I-8** was prepared in analogy to the preparation of **Intermediate I-11-2** by using **Intermediate I-21-1** instead of **Intermediate I-11-1.** MS: 384.1 (M+H)⁺. ¹H NMR (400 MHz, DMSO-*d₆*) δ 10.13 (s, 1H), 8.18 (d, J = 7.3 Hz, 1H), 7.96 (d, J = 7.8 Hz, 2H), 7.42 (d, J = 7.9 Hz, 2H), 6.47 (d, J = 7.3 Hz, 1H), 5.27 (s, 2H), 3.85 (s, 3H), 3.72-3.68 (m, 2H), 3.55-3.53 (m, 2H), 3.29 (s, 3H).

### Example I-9

### 2-amino-8-bromo-4-(butylamino)-6-(4-(pyrrolidin-1-ylmethyl)benzyl)pyrido[4,3-d]pyrimidin-5(6H)-one

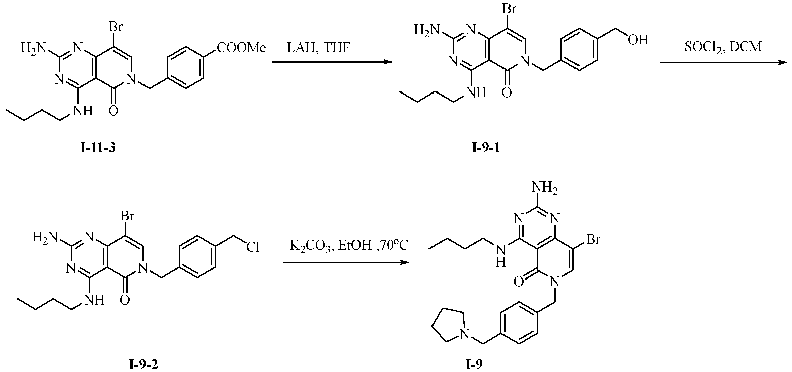

The title compound **I-9** was prepared in analogy to the preparation of compound **I-11** by using **Intermediate I-11-3** instead of **Intermediate I-11-4.** MS: 485.2 & 487.2 (M+H)⁺. ¹H NMR (400 MHz, DMSO-*d₆*) δ 9.49-9.47 (m, 1H), 8.21 (s, 1H), 7.28-7.20 (m, 4H), 6.95-6.78 (br, 2H), 5.02 (s, 2H), 3.52 (s, 2H), 3.41 (q, *J* = 6.6 Hz, 2H), 2.41-2.37 (m, 4H), 1.69-1.62 (m, 4H), 1.56-1.47 (m, 2H), 1.37-1.28 (m, 2H), 0.89 (t, *J* = 7.3 Hz, 3H).

### Example I-10

### 2-amino-4-(butylamino)-8-chloro-6-(4-(pyrrolidin-1-ylmethyl)benzyl)pyrido[4,3-d]pyrimidin-5(6H)-one

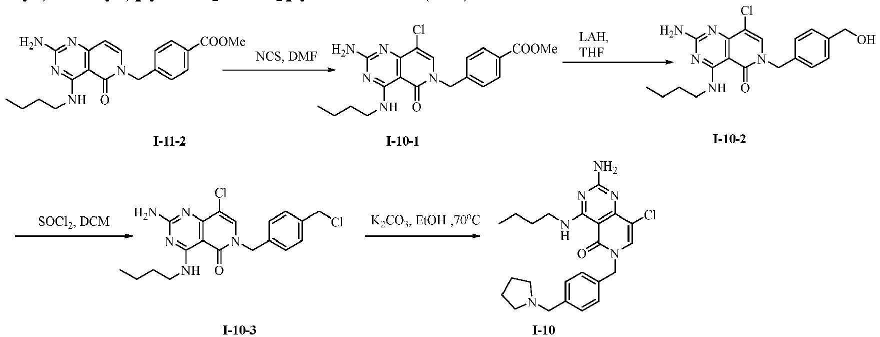

### Step 1: Preparation of Intermediate I-10-1

The title compound **Intermediate I-10-1** was prepared in analogy to the preparation of **Intermediate I-11-3** by using **NCS** instead of **NBS.** MS: 416.2 (M+H)⁺.

### Step 2: Preparation of Compound I-10

The title compound **I-10** was prepared in analogy to the preparation of compound **I-11** by using **Intermediate I-10-1** instead of **Intermediate I-11-4.** MS: 441.2 (M+H)⁺. ¹H NMR (400 MHz, DMSO-*d₆*) δ 9.49-9.47 (m, 1H), 8.17-8.12 (m, 1H), 7.30-7.21 (m, 4H), 7.02-6.78 (br, 2H), 5.02 (s, 2H), 3.56 (s, 2H), 3.41 (q, *J* = 6.6 Hz, 2H), 2.46-2.37 (m, 4H), 1.69-1.62 (m, 4H), 1.56-1.47 (m, 2H), 1.37-1.28 (m, 2H), 0.89 (t, *J* = 7.3 Hz, 3H).

### Example I-11

### 2-amino-4-(butylamino)-8-methyl-6-(4-(pyrrolidin-1-ylmethyl)benzyl)pyrido[4,3-d]pyrimidin-5(6H)-one

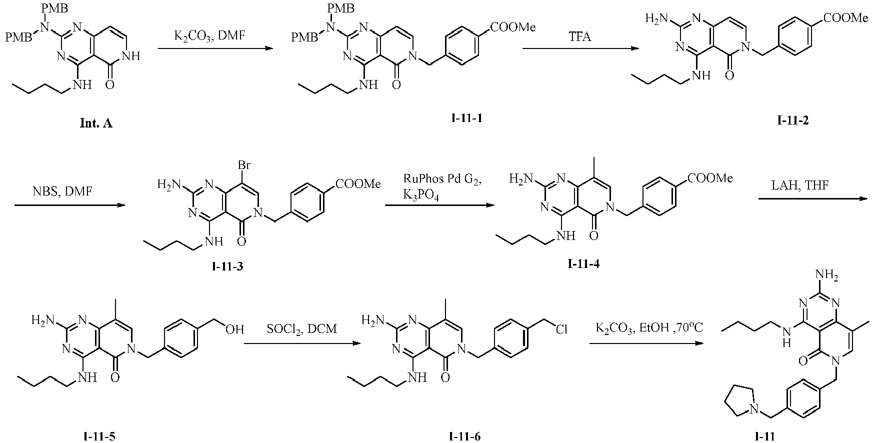

### Step 1: Preparation of Intermediate I-11-1

A mixture solution of **Intermediate Int. A** (4 g, 8.45 mmol), K₂CO₃ (2.335g, 16.89 mmol) and methyl 4-(bromomethyl)benzoate (3.87 g, 16.89 mmol) in DMF (40 mL) was stirred at 60°C for 12 hours. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by silica gel flash chromatography to afford **Intermediate I-11-1** (4.6 g, 88%). MS: 662.2 (M+H)⁺

### Step 2: Preparation of Intermediate I-11-2

A mixture solution of **Intermediate I-11-1** (3 g, 4.83 mmol) in TFA (20 mL) was stirred at 70°C for 2 hours. After the reaction was completed, the reaction mixture was concentrated in vacuo and extracted with EA. The combined organic layers were washed with NaHCO₃ solution, brine, dried over anhydrous Na₂SO₄, filtered and concentrated in vacuo to give a crude product, which was purified by silica gel flash chromatography to afford **Intermediate I-11-2** (1.6 g, 87%). MS: 382.2 (M+H)⁺

### Step 3: Preparation of Intermediate I-11-3

To a stirred mixture solution of **Intermediate I-11-2** (500 mg, 1.311 mmol) in DMF (100 mL) was added NBS (280 mg, 1.573 mmol) at 0 °C, then the resulting mixture was stirred at 20 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-11-3** (450 mg, 74.6%) as a yellow solid. MS: 460.1& 462.1 (M+H)⁺.

### Step 4: Preparation of Intermediate I-11-4

To a stirred mixture solution of **Intermediate I-11-3** (300 mg, 0.652 mmol) and trimethylboroxine (195 mg, 3.26 mmol) in Dioxane (2 mL) and water (0.4 mL) was added K₃PO₄ (414 mg, 1.955 mmol), RuPhos Pd G₂(50.6 mg, 0.065 mmol) at 0 °C, then the resulting mixture was stirred at 80 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-11-4** (140 mg, 54.3%) as a yellow solid. MS: 396.2 (M+H)⁺.

### Step 5: Preparation of Intermediate I-11-5

To a stirred mixture solution of **Intermediate I-11-4** (140 mg, 0.354 mmol) in THF (2 mL) was added a solution of 1.0 M LAH in THF (0.35 mL, 0.354 mmol) at 0 °C, then the resulting mixture was stirred at 20 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-11-5** (100 mg, 77%) as a white solid. MS: 368.2 (M+H)⁺.

### Step 6: Preparation of Intermediate I-11-6

To a stirred mixture solution of **Intermediate I-11-5** (100 mg, 0.272 mmol) in DCM (1 mL) was added SOCl₂ (48.6 mg, 0.408 mmol) at 0 °C, then the resulting mixture was stirred at 20 °C for 1 hour. After the reaction was completed, the reaction mixture was concentrated in vacuo to give a crude product **Intermediate I-11-6** (100 mg, 95%) as a white solid. MS: 386.2 (M+H)⁺.

### Step 7: Preparation of Compound I-11

To a stirred mixture solution of **Intermediate I-11-6** (100 mg, 0.259 mmol) and pyrrolidine (36.9 mg, 0.518 mmol) in EtOH (1 mL) was added K₂CO₃ (71.6 mg, 0.518 mmol) at 20 °C, then the resulting mixture was stirred at 75 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by Prep-HPL to afford **Compound I-11** (70 mg, 764.2%) as a white solid. MS: 421.2 (M+H)⁺. ¹H NMR (400 MHz, DMSO-*d₆*) δ 8.19 (s, 1H), 7.55 (s, 1H), 7.29 (d, *J* = 7.8 Hz, 2H), 7.22 (d, *J* = 7.9 Hz, 2H), 6.59 (s, 2H), 4.99 (s, 2H), 3.67 (s, 2H), 3.40 (q, *J* = 6.6 Hz, 2H), 2.57-2.52 (m, 4H), 1.98 (s, 3H), 1.73-1.64 (m, 4H), 1.56-1.47 (m, 2H), 1.36-1.29 (m, 2H), 0.89 (t, *J* = 7.3 Hz, 3H).

### Example I-12

### 2-amino-4-(pentan-2-ylamino)-6-(4-(pyrrolidin-1-ylmethyl)benzyl)pyrido[4,3-d]pyrimidin-5(6H)-one

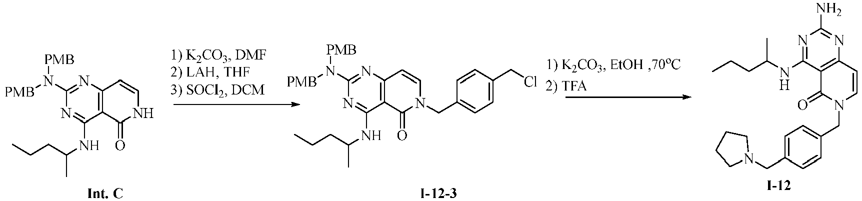

The title compound **I-12** was prepared in analogy to the preparation of compound **I-1** by using **Intermediate Int. C** instead of **Intermediate Int. A.** MS: 421.2 (M+H)⁺. ¹H NMR (400 MHz, DMSO-*d₆*) δ 10.18 (s, 1H), 10.04-10.02 (d, J = 8.0 Hz, 1H), 8.42 (br, 1H), 8.20-8.18 (d, J = 8.0 Hz, 1H), 7.50-7.48 (d, J = 8.0 Hz, 2H), 7.38-7.34 (m, 2H), 6.44-6.42 (d, J = 8.0 Hz, 1H), 5.25 - 5.14 (m, 2H), 4.32 - 4.25(m, 5H), 3.05 (br, 2H), 2.00 (br, 2H), 1.85-1.82 (m, 2H), 1.56-1.51 (m, 2H), 1.34 - 1.27 (m, 2H), 1.20 (d, J = 6.5 Hz, 3H), 0.87 (t, J = 7.3 Hz, 3H).

### Example I-13

### 4-((2-amino-4-(butylamino)-5-oxopyrido[4,3-d]pyrimidin-6(5H)-yl)methyl)-N-(2-(methylamino)ethyl)benzamide

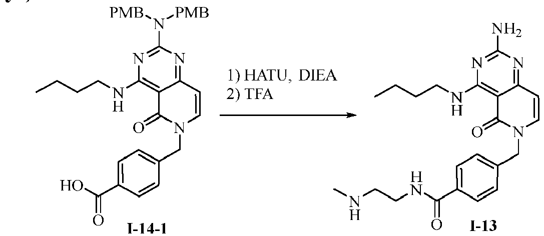

The title compound **I-13** was prepared in analogy to the preparation of compound **I-14** by using tert-butyl (2-aminoethyl)(methyl)carbamate instead of tert-butyl piperazine-1-carboxylate. MS: 424.5 (M+H)⁺. ¹H NMR (400 MHz, DMSO-*d*₆) δ 10.06 (t, *J* = 5.7 Hz, 1H), 8.68 (t, *J* = 5.7 Hz, 1H), 8.56 (s, 2H), 8.18 (d, *J* = 7.4 Hz, 3H), 7.88 - 7.81 (m, 2H), 7.37 (d, *J* = 8.1 Hz, 2H), 6.41 (d, *J* = 7.5 Hz, 1H), 5.22 (s, 2H), 3.07 (s, 2H), 2.48 (p, *J* = 1.9 Hz, 3H), 1.55 (p, *J* = 7.1 Hz, 2H), 1.32 (q, *J* = 7.5 Hz, 2H), 0.89 (t, *J* = 7.3 Hz, 3H).

### Example I-14

### 2-amino-4-(butylamino)-6-(4-(piperazine-1-carbonyl)benzyl)pyrido [4,3-d]pyrimidin-5(6H)-one

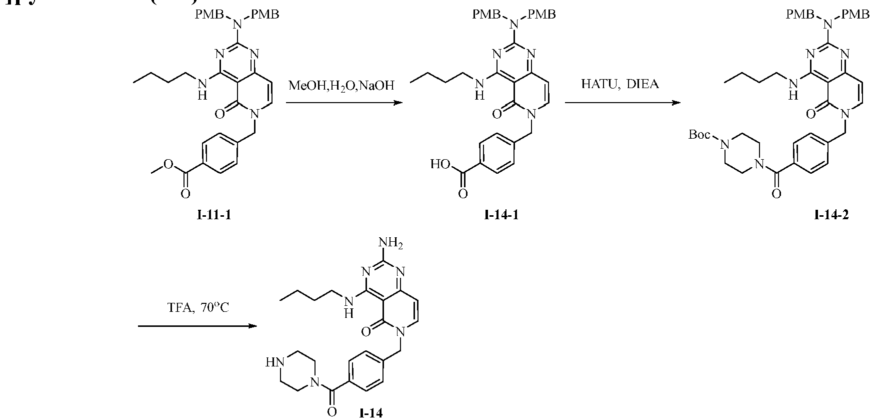

### Step 1: Preparation of Intermediate I-14-1

To a stirred mixture solution of **Intermediate I-11-1** (1 g, 1.61 mmol) in MeOH (2 mL) and water (10 mL) was added NaOH (193 mg, 4.83 mmol) at 0 °C, then the resulting mixture was stirred at 70 °C for 14 hours. After the reaction was completed, the reaction mixture was poured into ice-water, adjusted pH to 4 with 1N HCl and extracted with EA. The combined organic layers were washed with brine, dried over anhydrous Na₂SO₄, filtered and concentrated in vacuo to give a crude product, which was purified by silica gel flash chromatography to afford **Intermediate I-14-1** (928 mg, 95%) as a yellow oil. MS: 608.7 (M+H)⁺.

### Step 2: Preparation of Intermediate I-14-2

A mixture solution of **Intermediate I-14-1** (100 mg, 0.164 mmol), HATU (87.6 mg, 0.23 mmol), DIEA (42.3 mg, 0.328 mmol) and tert-butyl piperazine-1-carboxylate (45.7 mg, 0.246 mmol) in DMF (2 mL) was stirred at 50 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-14-2** (130 mg, 100%) as a yellow oil. MS: 776.9 (M+H)⁺.

### Step 3: Preparation of Compound I-14

The title compound **I-14** was prepared in analogy to the preparation of compound **I-1** by using **Intermediate I-14-2** instead of **Intermediate I-1-3.** MS: 436.5 (M+H)⁺. ¹H NMR (400 MHz, DMSO-*d₆*) δ 10.07 (t, *J* = 6.0 Hz, 1H), 9.07 (s, 1H), 8.18 (d, *J* = 7.4 Hz, 3H), 7.44 (d, *J* = 8.2 Hz, 2H), 7.36 (d, *J* = 8.2 Hz, 2H), 6.41 (d, *J* = 7.5 Hz, 1H), 5.21 (s, 2H), 3.85 - 3.01 (m, 10H), 1.60 - 1.51 (m, 2H), 1.37-1.28 (m, 2H), 0.89 (t, *J* = 7.4 Hz, 3H).

### Example I-15

### 2-amino-4-(butylamino)-6-(4-(morpholine-4-carbonyl)benzyl)pyrido[4,3-d]pyrimidin-5(6H)-one

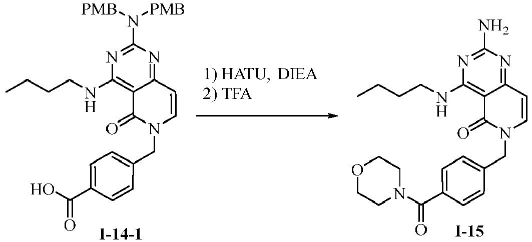

The title compound **I**-**15** was prepared in analogy to the preparation of **Compound I-14** by using morpholine instead of tert-butyl piperazine-1-carboxylate. MS: 437.5 (M+H)⁺. ¹H NMR (400 MHz, DMSO-*d*₆) δ 10.13 (t, *J* = 5.8 Hz, 1H), 8.20 (d, *J* = 7.4 Hz, 3H), 7.40-7.33 (m, 4H), 6.43 (d, *J* = 7.4 Hz, 1H), 5.20 (s, 2H), 3.54-3.29 (m, 10H), 1.58-1.54 (m, 2H), 1.35-1.30 (m, 2H), 0.89 (t, *J* = 7.3 Hz, 3H).

### Example I-16

### 4-((2-amino-4-(butylamino)-5-oxopyrido[4,3-d]pyrimidin-6(5H)-yl)methyl)-N,N-dimethylbenzamide

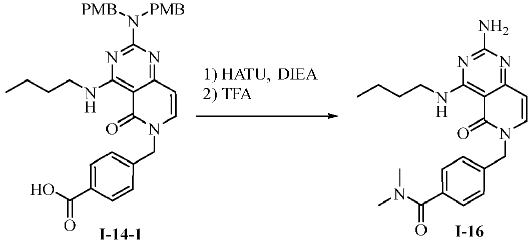

The title compound **I-16** was prepared in analogy to the preparation of **Compound I-14** by using dimethylamine hydrochloride instead of tert-butyl piperazine-1-carboxylate. MS: 395.4 (M+H)⁺. ¹H NMR (400 MHz, DMSO-*d*₆) δ 10.13 (t, *J* = 5.8 Hz, 1H), 8.52 - 8.02 (m, 3H), 7.38-7.31 (m, 4H), 6.43-6.41 (d, *J* = 7.4 Hz, 1H), 5.20 (s, 2H), 3.69-3.23(m, 2H), 2.94 (s, 3H), 2.86 (s, 3H), 1.60-1.52 (m, 2H), 1.37-1.28 (m, 2H), 0.89 (t, *J* = 7.3 Hz, 3H).

### Example I-17

### 4-((2-amino-4-(butylamino)-5-oxopyrido[4,3-d]pyrimidin-6(H)-yl)methyl)-N-(2-(pyrrolidin-2-yl)ethyl)benzamide

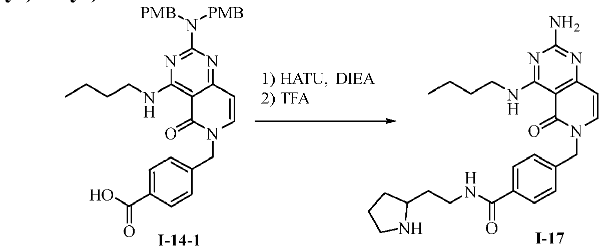

The title compound **I-17** was prepared in analogy to the preparation of **Compound I-14** by using tert-butyl 2-(2-aminoethyl)pyrrolidine-1-carboxylate instead of tert-butyl piperazine-1-carboxylate. MS: 464.6 (M+H)⁺. ¹H NMR (400 MHz, DMSO-*d*₆) δ 10.09 (t, *J* = 5.9 Hz, 1H), 8.95 (s, 1H), 8.62 - 8.14 (m, 5H), 7.80 (d, *J* = 8.1 Hz, 2H), 7.36 (d, *J* = 8.1 Hz, 2H), 6.42 (d, *J* = 7.5 Hz, 1H), 5.22 (s, 2H), 3.51 (m, 2H), 3.46 - 3.38 (m, 1H), 3.32 (m, 2H), 3.21 - 3.07 (m, 2H), 2.15-2.10 (m, 1H), 1.96-1.77 (m, 4H), 1.59 - 1.49 (m, 3H), 1.35-1.29 (m, 2H), 0.89 (t, *J* = 7.4 Hz, 3H).

### Example I-18

### 4-((2-amino-4-(butylamino)-5-oxopyrido[4,3-d]pyrimidin-6(5H)-yl)methyl)-N-(2-(1-methylpyrrolidin-2-yl)ethyl)benzamide

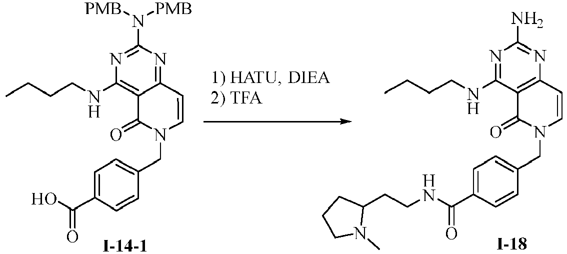

The title compound **I-18** was prepared in analogy to the preparation of **Compound I-14** by using 2-(1-methylpyrrolidin-2-yl)ethan-1-amine instead of tert-butyl piperazine-1-carboxylate. MS: 478.6 (M+H)⁺. ¹H NMR (400 MHz, DMSO-*d*₆) δ 10.09 (t, *J* = 5.8 Hz, 1H), 9.75 (s, 1H), 8.65 - 8.12 (m, 3H), 7.81 (d, *J* = 8.0 Hz, 2H), 7.36 (d, *J* = 8.2 Hz, 2H), 6.42 (d, *J* = 7.4 Hz, 1H), 5.21 (s, 2H), 3.51 (m, 4H), 3.36-3.21 (m, 3H), 3.07 - 2.96 (m, 1H), 2.79 (m, 2H), 2.31-2.10 (m, 2H), 2.02 - 1.78 (m, 2H), 1.78 - 1.50 (m, 4H), 1.31 (m, 2H), 0.89 (t, *J* = 7.3 Hz, 3H).

### Example I-19

### 6-(4-(3,6-diazabicyclo[3.1.1]heptane-3-carbonyl)benzyl)-2-amino-4-(butylamino)pyrido[4,3-d]pyrimidin-5(6H)-one

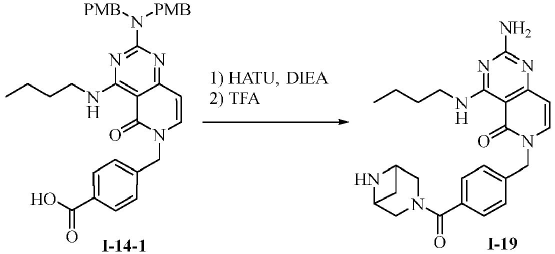

The title compound **I-19** was prepared in analogy to the preparation of **Compound I-14** by using tert-butyl 3,6-diazabicyclo[3.1.1]heptane-6-carboxylate instead of tert-butyl piperazine-1-carboxylate. MS: 448.5 (M+H)⁺. ¹H NMR (400 MHz, DMSO-*d₆*) δ 10.11 (t, *J* = 5.8 Hz, 1H), 9.63 (s, 1H), 8.50 - 8.14 (m, 3H), 7.45 (d, *J* = 7.9 Hz, 2H), 7.38 (d, *J* = 8.1 Hz, 2H), 6.43 (d, *J* = 7.4 Hz, 1H), 5.22 (s, 2H), 4.40 (s, 1H), 4.21 (s, 1H), 4.05-4.01 (m, 1H), 3.90 - 3.80 (m, 2H), 3.72-3.69 (m, 1H), 3.51 (q, *J* = 6.7 Hz, 2H), 2.85 - 2.74 (m, 1H), 1.83-1.79 (m, 1H), 1.58-1.53 (m, 2H), 1.38 - 1.20 (m, 3H), 0.90 (t, *J* = 7.3 Hz, 3H).

### Example I-20

### 4-((2-amino-4-(butylamino)-5-oxopyrido[4,3-d]pyrimidin-6(5H)-yl)methyl)benzoic acid

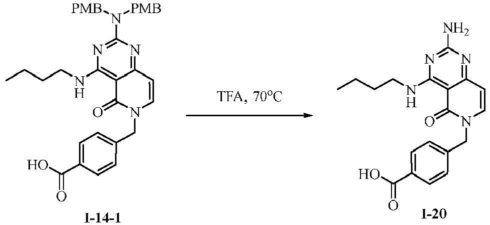

The title compound **I-20** was prepared in analogy to the preparation of **Compound I-1** by using **Intermediate I-14-1** instead of **Intermediate I-1-3.** MS: 368.4 (M+H)⁺. ¹H NMR (400 MHz, DMSO-*d*₆) δ 10.00 (s, 1H), 8.12 (d, *J* = 7.4 Hz, 1H), 7.90 (d, *J* = 8.2 Hz, 2H), 7.37 (d, *J* = 8.1 Hz, 2H), 6.40 (d, *J* = 7.4 Hz, 1H), 5.22 (s, 2H), 3.49 (q, *J* = 6.6 Hz, 2H), 1.59-1.51 (m, 2H), 1.37 - 1.27 (m, 2H), 0.89 (t, *J* = 7.3 Hz, 3H).

### Example I-21

### 2-amino-4-((2-methoxyethyl)amino)-6-(4-(pyrrolidin-1-ylmethyl)benzyl)pyrido[4,3-d]pyrimidin-5(6H)-one

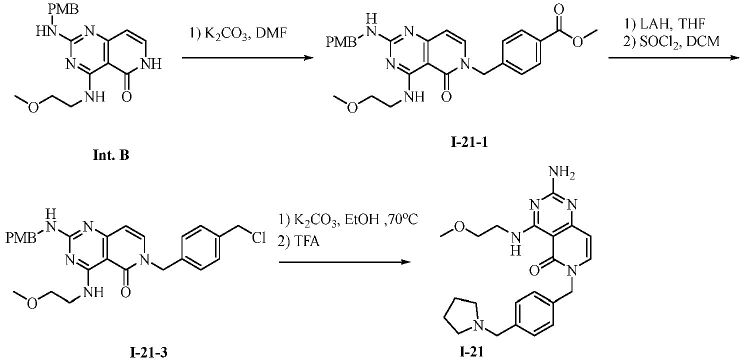

The title compound **I-21** was prepared in analogy to the preparation of **Compound I-2** by using **Intermediate Int. B** instead of **Intermediate Int. A** and methyl 4-(bromomethyl)benzoate instead of methyl 4-(bromomethyl)-3-methoxybenzoate. MS: 409.2 (M+H)⁺. ¹H NMR (400 MHz, DMSO-*d₆*) δ 10.17-10.14 (m, 1H), 10.10-10.05 (m, 1H), 8.58 - 8.25 (m, 2H), 8.19 (d, J = 7.4 Hz, 1H), 7.48 (d, J = 7.8 Hz, 2H), 7.35 (d, J = 7.9 Hz, 2H), 6.42 (d, J = 7.4 Hz, 1H), 5.20 (s, 2H), 4.62 - 4.46 (m, 1H), 4.31 (d, J = 5.3 Hz, 2H), 3.68 (q, J = 5.4 Hz, 2H), 3.52 (t, J = 5.3 Hz, 2H), 3.37-3.30 (m, 2H), 3.27 (s, 3H), 3.16 - 2.95 (m, 2H), 2.05-1.95 (d, J = 18.5 Hz, 2H), 1.92 - 1.75 (m, 2H).

### Example I-22

### 2-amino-4-(butylamino)-6-((1,2,3,4-tetrahydroisoquinolin-7-yl)methyl)pyrido[4,3-d]pyrimidin-5(6H)-one

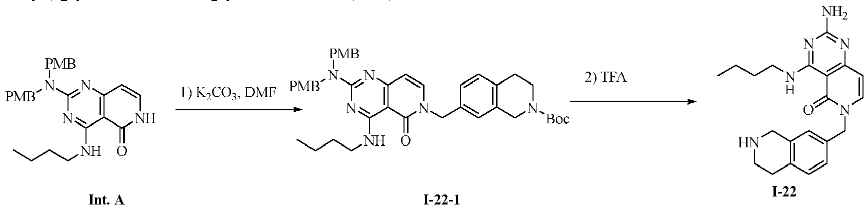

The title compound **I-22** was prepared in analogy to the preparation of **Compound I-1** by using tert-butyl 7-(bromomethyl)-3,4-dihydroisoquinoline-2(1H)-carboxylate instead of methyl 4-(bromomethyl)benzoate. MS: 379.2 (M+H)⁺. ¹H NMR (400 MHz, DMSO-*d₆*) δ 10.03 (s, 1H), 9.15 (s, 2H), 8.12 (d, J = 7.4 Hz, 3H), 7.20 - 7.10 (m, 3H), 6.36 (d, J = 7.4 Hz, 1H), 5.11 (s, 2H), 4.23 (s, 2H), 3.50 (q, J = 6.6 Hz, 2H), 3.36 - 3.34 (m, 2H), 2.93 (t, J = 6.2 Hz, 2H), 1.61 - 1.48 (m, 2H), 1.32 (dt, J = 14.5, 7.4 Hz, 2H), 0.89 (t, J = 7.3 Hz, 3H).

### Example I-23

### 2-amino-4-(butylamino)-6-(4-(pyrrolidin-1-ylmethyl)benzyl)-7,8-dihydropyrido[4,3-d]pyrimidin-5(6H)-one

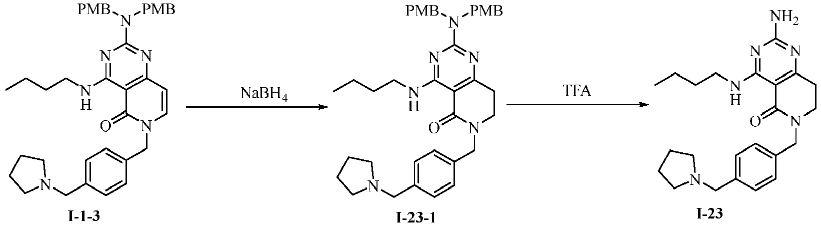

### Step 1: Preparation of Intermediate I-23-1

To a stirred mixture solution of **Intermediate I-1-3** (320 mg, 0.5 mmol) in MeOH (5 mL) was added NaBH₄ (0.5 mmol) at 0 °C, then the resulting mixture was stirred at 0 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-23-1** (160 mg, 49%). MS: 649.1 (M+H)⁺.

### Step 2: Preparation of Compound I-23

The title compound **I-23** was prepared in analogy to the preparation of **Intermediate I-11-2** by using **Intermediate I-23-1** instead of **Intermediate I-11-1.** MS: 409.3 (M+H)⁺.

### Example I-24

### 2-amino-4-(butylamino)-6-(3-(diethylamino)propyl)pyrido[4,3-d]pyrimidin-5(6H)-one

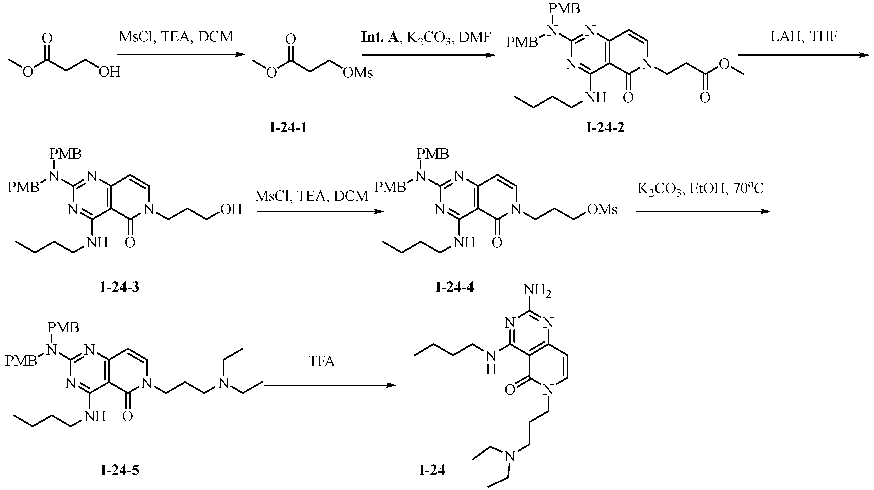

### Step 1: Preparation of Intermediate I-24-1

To a stirred mixture solution of methyl 3-hydroxypropanoate (500 mg, 4.8 mmol) and TEA (729 mg, 7.2 mmol) in DCM (10 mL) was added MsCl (660 mg, 5.76 mmol) at 0 °C, then the resulting mixture was stirred at 0 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product **Intermediate I-24-1** (750 mg, 86%) as a yellow solid.

### Step 2: Preparation of Compound I-24

The title compound **I-24** was prepared in analogy to the preparation of compound **I-1** by using **Intermediate I-24-1** instead of methyl 4-(bromomethyl)benzoate, MsCl instead of SOCl₂ and diethylamine instead of pyrrolidine. MS: 347.2 (M+H)⁺. ¹H NMR (400 MHz, DMSO-*d₆*) δ 9.44 (t, J = 5.6 Hz, 1H), 8.19 (s, 1H), 7.55 (d, J = 7.5 Hz, 1H), 6.59 (s, 2H), 6.03 (d, J = 7.5 Hz, 1H), 3.83 (t, J = 7.1 Hz, 2H), 3.42-3.38 (m, 2H), 2.64 (q, J = 7.1 Hz, 4H), 2.57 (t, J = 7.4 Hz, 2H), 1.84-1.77 (m, 2H), 1.57-1.49 (m, 2H), 1.40-1.29 (m, 2H), 0.98 (t, J = 7.1 Hz, 6H), 0.90 (t, J = 7.3 Hz, 3H).

### Example I-25

### 2-amino-4-(butylamino)-6-(5-(diethylamino)pentyl)pyrido[4,3-d]pyrimidin-5(6H)-one

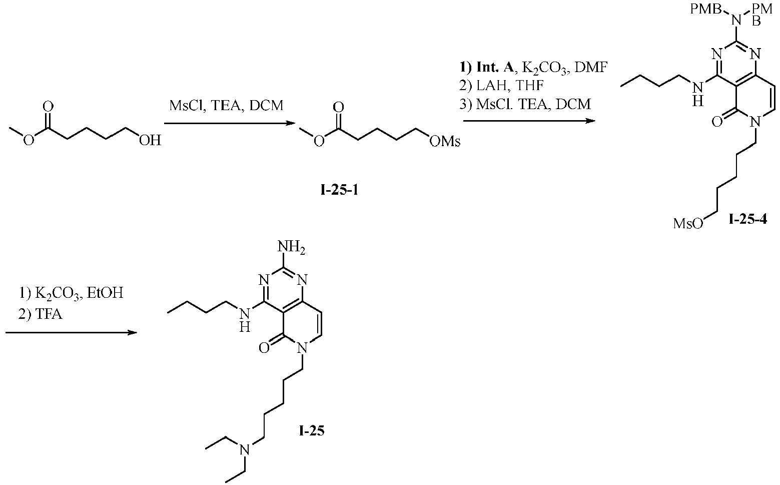

The title compound **I-25** was prepared in analogy to the preparation of compound **I-24** by using methyl 5-hydroxypentanoate instead of methyl 3-hydroxypropanoate. MS: 375.2 (M+H)⁺. ¹H NMR (400 MHz, DMSO-*d₆*) δ 9.46 (t, J = 5.5 Hz, 1H), 8.24 (s, 1H), 7.55 (d, J = 7.5 Hz, 1H), 6.02 (d, J = 7.5 Hz, 1H), 3.80 (t, J = 7.2 Hz, 2H), 3.42-3.38 (m, 2H), 2.65 (q, J = 7.1 Hz, 4H), 2.57-2.52 (m, 2H), 1.63-1.58 (m, 2H), 1.55-1.42 (m, 4H), 1.39-1.30 (m, 2H), 1.28-1.20 (m, 2H), 0.99 (t, J = 7.1 Hz, 6H), 0.90 (t, J = 7.3 Hz, 3H).

### Example I-26

### 2-amino-6-(4-(aminomethyl)benzyl)-4-(butylamino)pyrido[4,3-d]pyrimidin-5(6H)-one

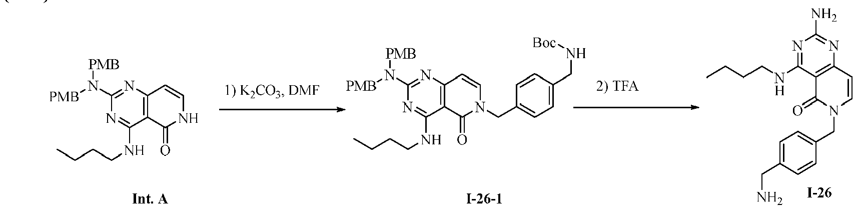

The title compound **I-26** was prepared in analogy to the preparation of compound **I-2** by using tert-butyl (4-(bromomethyl)benzyl)carbamate instead of methyl 4-(bromomethyl)-3-methoxybenzoate. MS: 353.3 (M+H)⁺. ¹H NMR (400 MHz, DMSO-*d₆*) δ 10.08 (s, 1H), 8.5 - 7.9 (m, 6H), 7.41 (d, J = 8.2 Hz, 2H), 7.33 (d, J = 8.2 Hz, 2H), 6.41 (d, J = 7.4 Hz, 1H), 5.17 (s, 2H), 4.05 - 3.95 (m, 2H), 3.55 - 3.46 (m, 3H), 1.62 - 1.50 (m, 2H), 1.38- 1.26 (m, 2H), 0.90 (t, J = 7.4 Hz, 3H).

### Example I-27

### 2-amino-4-(butylamino)-6-(3-fluoro-4-(pyrrolidin-1-ylmethyl)benzyl)pyrido [4,3-d]pyrimidin-5(6H)-one

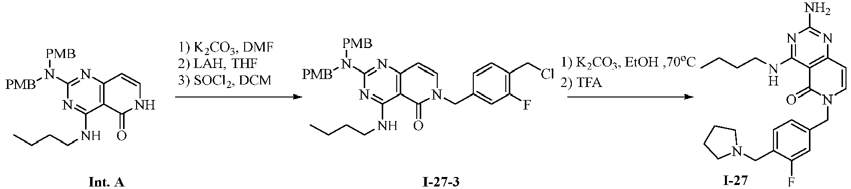

The title compound **I-27** was prepared in analogy to the preparation of compound **I-2** by using methyl 4-(bromomethyl)-2-fluorobenzoate instead of methyl 4-(bromomethyl)-3-methoxybenzoate. MS: 425.2 (M+H)⁺. ¹H NMR (400 MHz, DMSO-*d₆*) δ 10.20-10.12 (m, 1H), 10.07 (t, J = 5.8 Hz, 1H), 8.50-8.30 (m, 1H), 8.18 (d, J = 7.4 Hz, 2H), 7.58 (t, J = 7.8 Hz, 1H), 7.26 (d, J = 10.5 Hz, 1H), 7.20 (dd, J = 7.9, 1.7 Hz, 1H), 6.43 (d, J = 7.4 Hz, 1H), 5.21 (s, 2H), 4.38 (s, 2H),3.54-3.49 (q, J = 6.7 Hz, 3H), 3.45-3.35 (m, 2H), 3.15-3.00 (m, 2H), 2.07-1.93 (m, 2H), 1.88-1.76 (m, 2H), 1.63 - 1.51 (m, 2H), 1.37-1.28 (m, 2H), 0.89 (t, J = 7.3 Hz, 3H).

### Example I-28

### 2-amino-4-(butylamino)-6-(4-(hydroxymethyl)benzyl)pyrido [4,3-d] pyrimidin-5(6H)-one

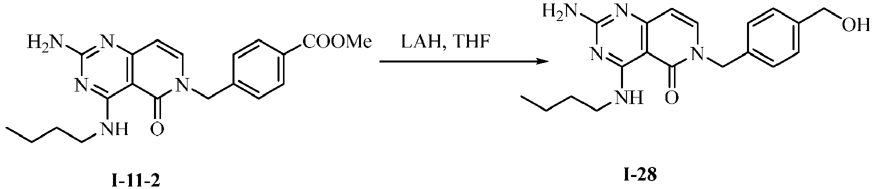

The title compound **I-28** was prepared in analogy to the preparation of **Intermediate I-11-5** by using **Intermediate I-11-2** instead of **Intermediate I-11-4.** MS: 354.2 (M+H)⁺. ¹H NMR (400 MHz, DMSO-*d₆*) δ 12.72 (s, 1H), 10.13 (s, 1H), 8.33 (s, 1H), 8.14 (d, J = 7.4 Hz, 1H), 7.80 (s, 1H), 7.32 - 7.20 (m, 4H), 6.40 (d, J = 7.4 Hz, 1H), 5.13 (s, 3H), 4.44 (s, 2H), 3.55 - 3.47 (m, 2H), 1.63 - 1.50 (m, 2H), 1.39 - 1.24 (m, 2H), 0.89 (t, J = 7.3 Hz, 3H).

### Example I-29

### 2-amino-4-(butylamino)-6-(4-((hexahydropyrrolo[3,4-c]pyrrol-2(1H)-yl)methyl)benzyl)pyrido[4,3-d]pyrimidin-5(6H)-one

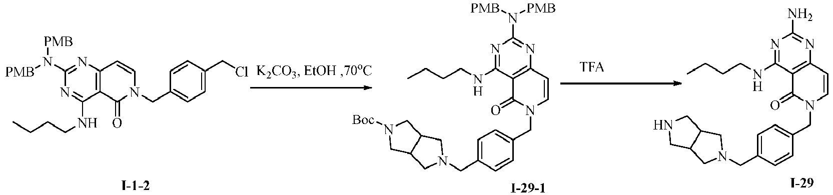

The title compound **I-29** was prepared in analogy to the preparation of compound **I-1** by using tert-butyl hexahydropyrrolo[3,4-c]pyrrole-2(1H)-carboxylate instead of pyrrolidine. MS: 448.2 (M+H)⁺. ¹H NMR (400 MHz, DMSO-*d₆*) δ 9.34 (s, 5H), 8.47 (s, 2H), 8.19 (d, J = 7.5 Hz, 1H), 7.42 (s, 2H), 7.35 (d, J = 7.9 Hz, 2H), 6.39 (d, J = 7.4 Hz, 1H), 5.19 (s, 2H), 4.27 (s, 2H), 3.36 (s, 4H), 3.10 (s, 4H), 2.87 (s, 1H), 2.71 (s, 1H), 1.63 - 1.49 (m, 2H), 1.32 (h, J = 7.3 Hz, 2H), 0.89 (t, J = 7.3 Hz, 3H).

### Example I-30

### 2-amino-4-(butylamino)-6-(4-((4-cyclohexylpiperazin-1-yl)methyl)benzyl)pyrido[4,3-d]pyrimidin-5(6H)-one

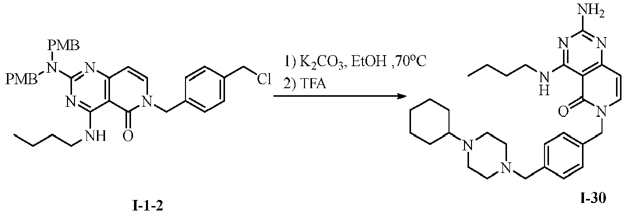

The title compound **I-30** was prepared in analogy to the preparation of compound **I-1** by using 1-cyclohexylpiperazine instead of pyrrolidine. MS: 504.2 (M+H)⁺. ¹H NMR (400 MHz, DMSO-*d₆*) δ 9.33 (s, 1H), 8.49 (s, 1H), 8.18 (d, J = 7.4 Hz, 1H), 7.30 (q, J = 8.1 Hz, 4H), 6.42 (d, J = 7.5 Hz, 1H), 5.16 (s, 2H), 3.67 (s, 3H), 3.51 (q, J = 6.7 Hz, 3H), 3.39 (s, 3H), 3.00 (s, 5H), 1.97 (s, 2H), 1.79 (d, J = 12.1 Hz, 2H), 1.56 (h, J = 7.1, 6.7 Hz, 3H), 1.40 - 1.14 (m, 6H), 1.07 (t, J = 12.2 Hz, 1H), 0.89 (t, J = 7.3 Hz, 3H).

### Example I-31

### (S)-N-(4-((2-amino-4-(butylamino)-5-oxopyrido[4,3-d]pyrimidin-6(5H)-yl)methyl)benzyl)pyrrolidine-2-carboxamide

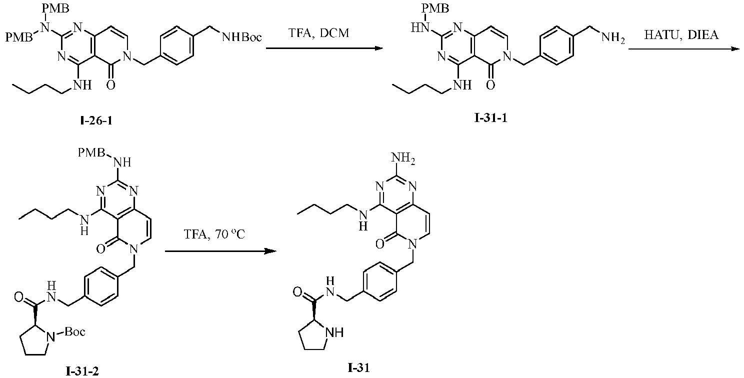

### Step 1: Preparation of Intermediate I-31-1

A mixture solution of **Intermediate I-26-1** (450 mg, 0.649 mmol) and TFA (3 mL) in DCM (10 mL) was stirred at 20 °C for 1 hour. After the reaction was completed, the reaction mixture was concentrated in vacuo to give a crude product, which was purified by silica gel flash chromatography to afford **Intermediate I-31-1** (290 mg, 94%). MS: 473.2 (M+H)⁺.

### Step 2: Preparation of Intermediate I-31-2

A mixture solution of (t-butyloxycarbonyl)-L-proline (34 mg, 0.159 mmol), DIEA (68 mg, 0.529 mmol) and HATU (60 mg, 0.159 mmol) in DMF (3 mL) was stirred at 20 °C for 1 hour. Then **Intermediate I-31-1** (50 mg, 0.106 mmol) was added to the above mixture solution and the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-31-2** (50 mg, 70.4%). MS: 670.2 (M+H)⁺.

### Step 3: Preparation of Compound I-31

The title compound **I-31** was prepared in analogy to the preparation of **Intermediate I-11-2** by using **Intermediate I-31-2** instead of **Intermediate I-11-1.** MS: 450.2 (M+H)⁺. ¹H NMR (400 MHz, DMSO-*d₆*) δ 10.09 (s, 1H), 9.23 (s, 1H), 8.97 (t, J = 5.8 Hz, 1H), 8.55 (s, 1H), 8.14 (d, J = 7.4 Hz, 1H), 7.30 - 7.17 (m, 4H), 6.40 (d, J = 7.4 Hz, 1H), 5.13 (s, 2H), 4.40 - 4.20 (m, 2H), 4.15 (br s, 1H), 3.55 - 3.45 (m, 3H), 3.20 (s, 2H), 1.97 - 1.75 (m, 3H), 1.61 - 1.48 (m, 2H), 1.40 - 1.22 (m, 2H), 0.89 (t, J = 7.3 Hz, 3H).

### Example I-32

### 2-amino-6-(4-(azetidin-1-ylmethyl)benzyl)-4-(butylamino)pyrido [4,3-d]pyrimidin-5(6H)-one

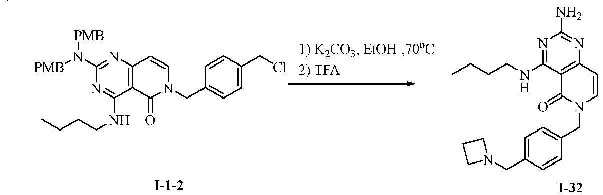

The title compound **I-32** was prepared in analogy to the preparation of compound **I-1** by using azetidine instead of pyrrolidine. MS: 393.2 (M+H)⁺. ¹H NMR (400 MHz, DMSO-*d₆*) δ 9.36 (t, J = 5.5 Hz, 1H), 8.25 (s, 2H), 7.67 (d, J = 7.5 Hz, 1H), 7.36 - 7.15 (m, 4H), 6.63 (s, 2H), 6.06 (d, J = 7.5 Hz, 1H), 5.00 (s, 2H), 3.90 (t, J = 8.2 Hz, 1H), 3.65 (d, J = 3.1 Hz, 2H), 3.38 (q, J = 6.8 Hz, 2H), 3.34 - 3.22 (m, 2H), 2.40 - 2.26 (m, 1H), 2.05-1.98 (m, 2H), 1.55-1.48 (m, 2H), 1.37-1.28 (m, 2H), 0.88 (t, J = 7.3 Hz, 2H).

### Example I-33

### 2-amino-4-(butylamino)-6-(4-(piperidin-1-ylmethyl)benzyl)pyrido[4,3-d]pyrimidin-5(6H)-one

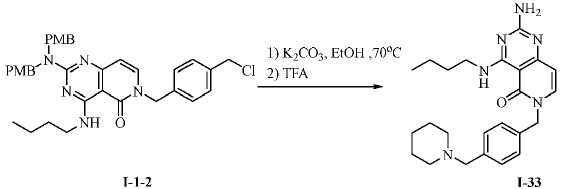

The title compound **I-33** was prepared in analogy to the preparation of compound **I-1** by using piperidine instead of pyrrolidine. MS: 421.2 (M+H)⁺. ¹H NMR (400 MHz, DMSO-*d₆*) δ 9.38 (t, J = 5.4 Hz, 1H), 7.69 (s, 1H), 7.32 (d, J = 7.9 Hz, 2H), 7.25 (d, J = 7.7 Hz, 2H), 6.80-6.50 (s, 2H), 6.25-6.00 (s, 2H), 5.03 (s, 2H), 3.73 (s, 2H), 3.39 (q, J = 6.6 Hz, 2H), 3.09-2.90 (m, 2H), 2.65-2.52 (m, 2H), 1.70-1.58 (m, 2H), 1.57 - 1.47 (m, 4H), 1.43-1.37 (m, 2H), 1.35-1.28 (m, 2H), 0.88 (t, J = 7.3 Hz, 3H).

### Example I-34

### 2-amino-4-(butylamino)-6-(4-(((2-hydroxyethyl)amino)methyl) benzyl)pyrido[4,3-d]pyrimidin-5(6H)-one

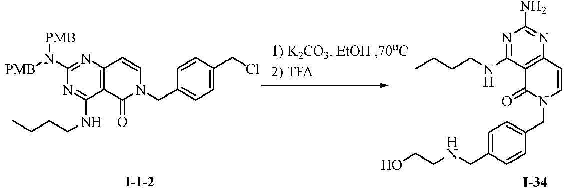

The title compound **I-34** was prepared in analogy to the preparation of compound **I-1** by using 2-aminoethan-1-ol instead of pyrrolidine. MS: 397.2 (M+H)⁺. ¹H NMR (400 MHz, DMSO-*d₆*) δ 10.07 (s, 1H), 8.86 (s, 2H), 8.16 (d, J = 7.4 Hz, 1H), 7.46 (d, J = 8.0 Hz, 2H), 7.33 (d, J = 8.0 Hz, 2H), 6.41 (d, J = 7.4 Hz, 1H), 5.17 (s, 2H), 3.62 (s, 2H), 3.55 - 3.42 (m, 3H), 2.92 (s, 2H), 1.61 - 1.48 (m, 2H), 1.39 - 1.25 (m, 2H), 0.89 (t, J = 7.3 Hz, 3H).

### Example I-35

### 2-amino-4-(butylamino)-6-(4-((dimethylamino)methyl)benzyl)pyrido[4,3-d]pyrimidin-5(6H)-one

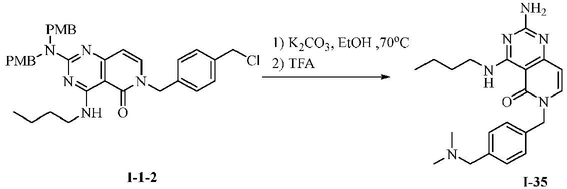

The title compound **I-35** was prepared in analogy to the preparation of compound **I-1** by using dimethylamine hydrochloride instead of pyrrolidine. MS: 381.2 (M+H)⁺. ¹H NMR (400 MHz, DMSO-*d₆*) δ 9.37 (t, J = 5.5 Hz, 1H), 8.16 (s, 1H), 7.68 (d, J = 7.5 Hz, 1H),7.33 - 7.20 (m, 4H), 6.65 (s, 2H), 6.07 (d, J = 7.5 Hz, 1H), 5.03 (s, 2H), 3.60 (s, 2H), 3.41-3.36 (m, 2H), 2.27 (s, 6H), 1.53-1.48 (m, 2H), 1.38 - 1.27 (m, 2H), 0.89 (t, J = 7.3 Hz, 3H).

### Example I-36

### 2-amino-4-(butylamino)-6-(4-(pyrrolidin-1-ylmethyl)phenyl)pyrido[4,3-d]pyrimidin-5(6H)-one

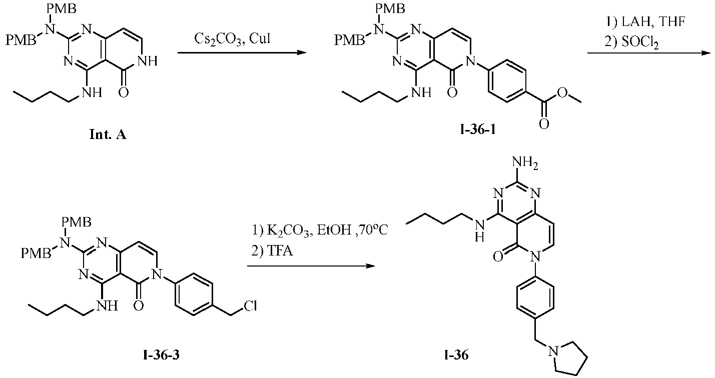

### Step 1: Preparation of Intermediate I-36-1

A mixture solution of **Intermediate Int. A** (200 mg, 0.422 mmol), dimethylglycine (65.3 mg, 0.633 mmol), Cs₂CO₃ (413 mg, 1.267 mmol), CuI (16.09 mg, 0.084 mmol) and methyl 4-iodobenzoate (166 mg, 0.633 mmol) in DMF (15 mL) was stirred at 100 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-36-1** (6.25 g, 53.4%) as a yellow solid. MS: 608.1 (M+H)⁺.

### Step 2: Preparation of Compound I-36

The title compound **I-36** was prepared in analogy to the preparation of compound **I-1** by using **Intermediate I-36-1** instead of **Intermediate I-11-1.** MS: 393.2 (M+H)⁺. ¹H NMR (400 MHz, DMSO-*d₆*) δ 10.48-10.23 (s, 1H), 10.06-10.04 (m, 1H), 8.68-8.22 (m, 2H), 8.01 (d, J = 7.5 Hz, 1H), 7.68 (d, J = 8.1 Hz, 2H), 7.55 (d, J = 8.0 Hz, 2H), 6.47 (d, J = 7.5 Hz, 1H), 4.43 (s, 2H), 3.53 (q, J = 6.6 Hz, 3H), 3.44-3.31 (m, 2H), 3.16-3.03 (m, 2H), 1.99-2.10 (m, 2H), 1.95-1.80 (m, 2H), 1.59-1.52 (m, 2H), 1.35-1.29 (m, 2H), 0.89 (t, J = 7.3 Hz, 3H).

### Example I-37

### 2-amino-4-(butylamino)-6-(4-(morpholinomethyl)benzyl)pyrido[4,3-d]pyrimidin-5(6H)-one

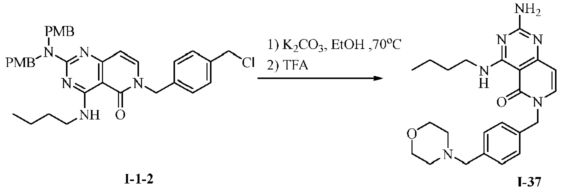

The title compound **I-37** was prepared in analogy to the preparation of compound **I-1** by using morpholine instead of pyrrolidine. MS: 423.2 (M+H)⁺. ¹H NMR (400 MHz, DMSO-*d₆*) δ 10.51-10.19 (m, 1H), 10.12-10.10 (m, 1H), 8.56-8.09 (m, 2 H), 8.18 (d, J = 7.4 Hz, 1H), 7.46 (d, J = 8.2 Hz, 2H), 7.37 (d, J = 8.1 Hz, 2H), 6.42 (d, J = 7.4 Hz, 1H), 5.20 (s, 2H), 4.30 (s, 2H), 4.03-3.56 (m, 4H), 3.53-3.49 (m, 2H), 3.28-2.95 (m, 4H), 1.59-1.52 (m, 2H), 1.38 - 1.27 (m, 2H), 0.89 (t, J = 7.4 Hz, 3H).

### Example I-38

### 2-amino-4-(butylamino)-6-(4-((cyclopropylamino)methyl)benzyl)pyrido[4,3-d]pyrimidin-5(6H)-one

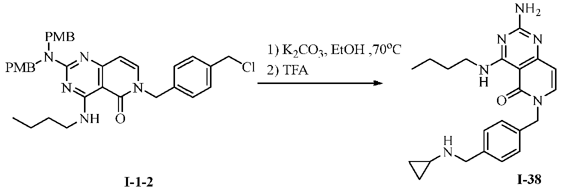

The title compound **I-38** was prepared in analogy to the preparation of compound **I-1** by using cyclopropanamine instead of pyrrolidine. MS: 393.2 (M+H)⁺. ¹H NMR (400 MHz, DMSO-*d₆*) δ 10.11 (t, J = 5.8 Hz, 1H), 9.40 (s, 2H), 8.59-8.51 (m, 2H), 8.21 (d, J = 7.4 Hz, 1H), 8.15 (s, 1H), 7.46 (d, J = 8.1 Hz, 2H), 7.33 (d, J = 7.8 Hz, 2H), 6.38 (d, J = 7.4 Hz, 1H), 5.18 (s, 2H), 4.19 (s, 2H), 3.50 (q, J = 6.7 Hz, 2H), 2.63 (s, 1H), 1.59-1.51 (m, 2H), 1.36-1.27 (m, 2H), 0.88 (t, J = 7.3 Hz, 3H), 0.84-0.79 (m, 2H), 0.72-0.67 (m, 2H).

### Example I-39

### 6-(4-((5-azaspiro[2.4]heptan-5-yl)methyl)benzyl)-2-amino-4-(butylamino)pyrido[4,3-d]pyrimidin-5(6H)-one

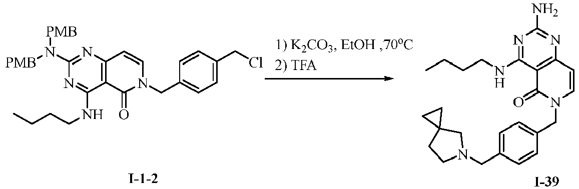

The title compound **I-39** was prepared in analogy to the preparation of compound **I-1** by using 5-azaspiro[2.4]heptane instead of pyrrolidine. MS: 433.3 (M+H)⁺. ¹H NMR (400 MHz, DMSO-*d₆*) δ 13.39 (s, 1H), 10.56 (s, 1H), 10.11 (t, J = 5.8 Hz, 1H), 8.39 (d, J = 39.3 Hz, 2H), 8.19 (d, J = 7.5 Hz, 1H), 7.54 - 7.44 (m, 2H), 7.39 - 7.30 (m, 2H), 6.42 (d, J = 7.4 Hz, 1H), 5.19 (s, 2H), 4.44 - 4.26 (m, 2H), 3.51 (q, J = 6.7 Hz, 3H), 3.29 (s, 1H), 3.16 (d, J = 6.8 Hz, 1H), 3.12 - 3.01 (m, 1H), 2.09 - 1.96 (m, 1H), 1.91 - 1.79 (m, 1H), 1.61 - 1.49 (m, 2H), 1.36 - 1.26 (m, 2H), 0.89 (t, J = 7.3 Hz, 3H), 0.65 (dd, J = 17.3, 6.4 Hz, 3H), 0.55 (d, J = 6.1 Hz, 1H).

### Example I-40

### 6-(4-((3,6-diazabicyclo[3.1.1]heptan-3-yl)methyl)benzyl)-2-amino-4-(butylamino)pyrido[4,3-d]pyrimidin-5(6H)-one

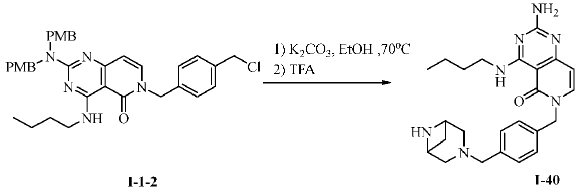

The title compound **I-40** was prepared in analogy to the preparation of compound **I-29** by using tert-butyl 3,6-diazabicyclo[3.1.1]heptane-6-carboxylate instead of tert-butyl hexahydropyrrolo[3,4-c]pyrrole-2(1H)-carboxylate. MS: 434.3 (M+H)⁺. ¹H NMR (400 MHz, DMSO-*d₆*) δ 13.41 (s, 1H), 10.14 (t, J = 5.8 Hz, 1H), 9.51 (s, 1H), 8.39 (d, J = 51.1 Hz, 2H), 8.19 (d, J = 7.4 Hz, 1H), 7.99 (s, 1H), 7.36 (d, J = 7.8 Hz, 2H), 7.30 (d, J = 7.9 Hz, 2H), 6.41 (d, J = 7.4 Hz, 1H), 5.17 (s, 2H), 4.25 (d, J = 5.9 Hz, 2H), 3.91 (s, 2H), 3.51 (q, J = 6.7 Hz, 2H), 3.21 (d, J = 44.1 Hz, 4H), 2.67 (s, 1H), 2.12 (dd, J = 10.1, 5.5 Hz, 1H), 1.62 - 1.49 (m, 2H), 1.40 - 1.23 (m, 2H), 0.89 (t, J = 7.3 Hz, 3H).

### Example I-41

### 6-(4-((3,6-diazabicyclo[3.1.1]heptan-6-yl)methyl)benzyl)-2-amino-4-(butylamino)pyrido[4,3-d]pyrimidin-5(6H)-one

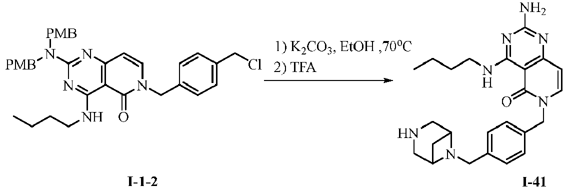

The title compound **I-41** was prepared in analogy to the preparation of compound **I-29** by using tert-butyl 3,6-diazabicyclo[3.1.1]heptane-3-carboxylate instead of tert-butyl hexahydropyrrolo[3,4-c]pyrrole-2(1H)-carboxylate. MS: 434.3 (M+H)⁺. ¹H NMR (400 MHz, DMSO-*d₆*) δ 10.26 - 9.62 (m, 3H), 8.41 (s, 2H), 8.19 (d, J = 7.4 Hz, 1H), 7.46 (s, 2H), 7.34 (d, J = 7.8 Hz, 2H), 6.41 (d, J = 7.4 Hz, 1H), 5.18 (s, 2H), 4.27 - 3.44 (m, 11H), 2.07 (s, 1H), 1.55 (p, J = 7.2 Hz, 2H), 1.32 (h, J = 7.4 Hz, 2H), 0.89 (t, J = 7.3 Hz, 3H).

### Example I-42

### 6-(4-((2,5-diazabicyclo[2.2.1]heptan-2-yl)methyl)benzyl)-2-amino-4-(butylamino)pyrido[4,3-d]pyrimidin-5(6H)-one

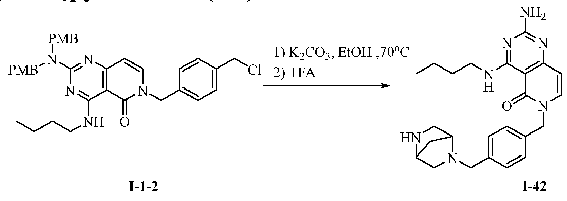

The title compound **I-42** was prepared in analogy to the preparation of compound **I-29** by using tert-butyl 2,5-diazabicyclo[2.2.1]heptane-2-carboxylate instead of tert-butyl hexahydropyrrolo[3,4-c]pyrrole-2(1H)-carboxylate. MS: 434.3 (M+H)⁺. ¹H NMR (400 MHz, DMSO-*d₆*) δ 13.33 (s, 1H), 10.12 (t, J = 5.8 Hz, 1H), 9.37 (m, 2H), 8.39 (m, 2H), 8.19 (d, J = 7.5 Hz, 1H), 7.45 (s, 2H), 7.33 (d, J = 7.7 Hz, 2H), 6.41 (d, J = 7.4 Hz, 1H), 5.18 (s, 2H), 4.29 (m, 5H), 3.51 (q, J = 6.7 Hz, 3H), 3.28 (m, 3H), 1.94 (s, 1H), 1.62 - 1.49 (m, 2H), 1.40 - 1.25 (m, 2H), 0.89 (t, J = 7.3 Hz, 3H).

### Example I-43

### 6-(4-((2,7-diazaspiro[4.4]nonan-2-yl)methyl)benzyl)-2-amino-4-(butylamino)pyrido[4,3-d]pyrimidin-5(6H)-one

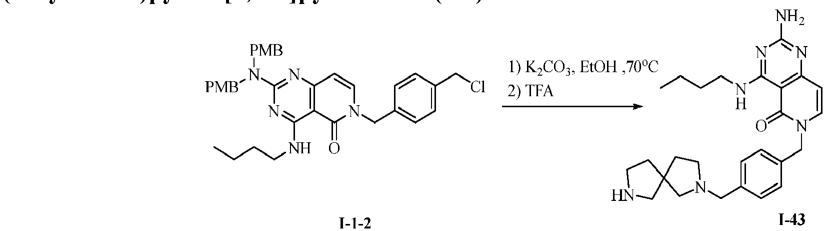

The title compound **I-43** was prepared in analogy to the preparation of compound **I-29** by using tert-butyl 2,7-diazaspiro[4.4]nonane-2-carboxylate instead of tert-butyl hexahydropyrrolo[3,4-c]pyrrole-2(1H)-carboxylate. MS: 462.3 (M+H)⁺. ¹H NMR (400 MHz, DMSO-*d₆*) δ 13.58 (s, 1H), 10.84 (s, 1H), 10.09 (t, J = 5.8 Hz, 1H), 9.19 (s, 2H), 8.38 (s, 2H), 8.18 (d, J = 7.4 Hz, 1H), 7.48 (d, J = 7.9 Hz, 2H), 7.35 (d, J = 7.9 Hz, 2H), 6.40 (d, J = 7.4 Hz, 1H), 5.19 (s, 2H), 4.35 (d, J = 28.4 Hz, 2H), 3.59 - 3.19 (m, 11H), 2.21 - 1.81 (m, 5H), 1.62 - 1.49 (m, 2H), 1.40 - 1.25 (m, 2H), 0.89 (t, J = 7.3 Hz, 3H).

### Example I-44

### 6-(4-((2,6-diazaspiro[3.4]octan-6-yl)methyl)benzyl)-2-amino-4-(butylamino)pyrido[4,3-d]pyrimidin-5(6H)-one

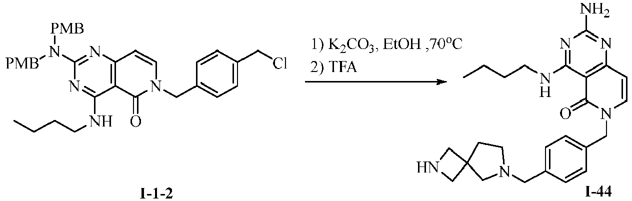

The title compound **I-44** was prepared in analogy to the preparation of compound **I-29** by using tert-butyl 2,6-diazaspiro[3.4]octane-2-carboxylate instead of tert-butyl hexahydropyrrolo[3,4-c]pyrrole-2(1H)-carboxylate. MS: 448.3 (M+H)⁺. ¹H NMR (400 MHz, DMSO-*d₆*) δ 13.51 (s, 1H), 10.67 (s, 1H), 10.09 (t, J = 5.7 Hz, 1H), 9.03 (s, 1H), 8.80 (s, 1H), 8.34 (s, 2H), 8.18 (d, J = 7.4 Hz, 1H), 7.46 (d, J = 8.2 Hz, 2H), 7.36 (d, J = 8.1 Hz, 2H), 6.41 (d, J = 7.4 Hz, 1H), 5.19 (s, 2H), 4.31 (d, J = 15.4 Hz, 2H), 4.01 (s, 3H), 3.82 (s, 1H), 3.62 (s, 1H), 3.51 (q, J = 6.7 Hz, 3H), 3.37 (s, 2H), 3.15 (s, 1H), 2.41 (s, 1H), 2.19 (s, 1H), 1.61 - 1.50 (m, 2H), 1.32 (h, J = 7.4 Hz, 2H), 0.89 (t, J = 7.3 Hz, 3H).

### Example I-45

### (4-((2-amino-4-(butylamino)-5-oxopyrido[4,3-d]pyrimidin-6(5H)-yl)methyl)benzyl)proline

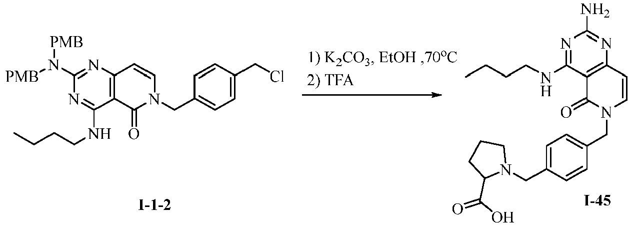

The title compound **I-45** was prepared in analogy to the preparation of compound **I-29** by using tert-butyl prolinate instead of tert-butyl hexahydropyrrolo[3,4-c]pyrrole-2(1H)-carboxylate. MS: 451.2 (M+H)⁺. ¹H NMR (400 MHz, DMSO-d6) δ 10.05 (t, J = 5.7 Hz, 1H), 8.34 (s, 2H), 8.16 (d, J = 7.5 Hz, 1H), 7.46 (d, J = 8.0 Hz, 2H), 7.33 (d, J = 7.9 Hz, 2H), 6.38 (d, J = 7.4 Hz, 1H), 5.17 (s, 2H), 4.36 (d, J = 12.9 Hz, 1H), 4.16 (d, J = 12.9 Hz, 1H), 4.05 (t, J = 8.3 Hz, 1H), 3.50 (q, J = 6.7 Hz, 2H), 3.31 (d, J = 11.3 Hz, 1H), 3.09 (q, J = 8.8 Hz, 1H), 2.33 (q, J = 9.1, 7.5 Hz, 1H), 2.03 - 1.89 (m, 2H), 1.80 (d, J = 12.1 Hz, 1H), 1.61 - 1.49 (m, 2H), 1.39 - 1.26 (m, 2H), 0.89 (t, J = 7.3 Hz, 3H).

### Example I-46

### 6-(4-((1H-imidazol-1-yl)methyl)benzyl)-2-amino-4-(butylamino)pyrido [4,3-d]pyrimidin-5(6H)-one

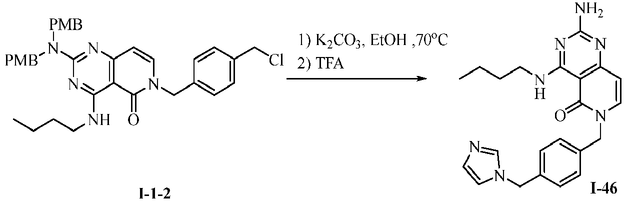

The title compound **I-46** was prepared in analogy to the preparation of compound **I-1** by using 1H-imidazole instead of pyrrolidine. MS: 404.2 (M+H)⁺. ¹H NMR (400 MHz, DMSO-*d₆*) δ 14.01 (s, 1H), 10.08 (t, J = 5.8 Hz, 1H), 9.17 (s, 1H), 8.32 (d, J = 65.0 Hz, 2H), 8.17 (d, J = 7.4 Hz, 1H), 7.77 - 7.69 (m, 1H), 7.63 (d, J = 2.0 Hz, 1H), 7.38 (d, J = 8.3 Hz, 2H), 7.32 (d, J = 8.3 Hz, 2H), 6.39 (d, J = 7.4 Hz, 1H), 5.38 (s, 2H), 5.16 (s, 2H), 3.50 (q, J = 6.7 Hz, 2H), 1.60 - 1.49 (m, 2H), 1.37 - 1.25 (m, 2H), 0.89 (t, J = 7.3 Hz, 3H).

### Example I-47

### 2-amino-4-(butylamino)-6-(4-(ethoxymethyl)benzyl)pyrido[4,3-d]pyrimidin-5(6H)-one

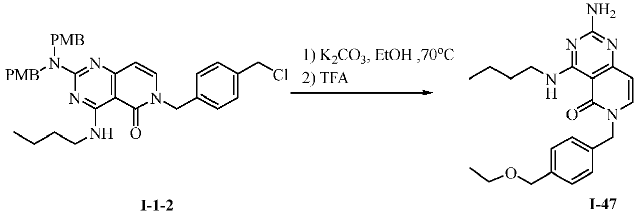

The title compound **I-47** was prepared in analogy to the preparation of compound **I-1** by using sodium ethanolate instead of pyrrolidine. MS: 382.2 (M+H)⁺. ¹H NMR (400 MHz, DMSO-*d₆*) δ 10.00 (s, 1H), 8.07 (d, J = 7.3 Hz, 1H), 7.44 - 7.23 (m, 4H), 6.35 (dd, J = 7.5, 2.0 Hz, 1H), 5.13 (d, J = 5.7 Hz, 2H), 4.39 (s, 2H), 3.46 (dq, J = 22.7, 6.8 Hz, 4H), 1.55 (p, J = 7.2 Hz, 2H), 1.32 (h, J = 7.4 Hz, 2H), 1.11 (t, J = 7.0 Hz, 2H), 0.89 (t, J = 7.3 Hz, 3H).

### Example I-48

### 2-amino-4-((2-cyclopropylethyl)amino)-6-(4-(pyrrolidin-1-ylmethyl)benzyl)pyrido[4,3-d]pyrimidin-5(6H)-one

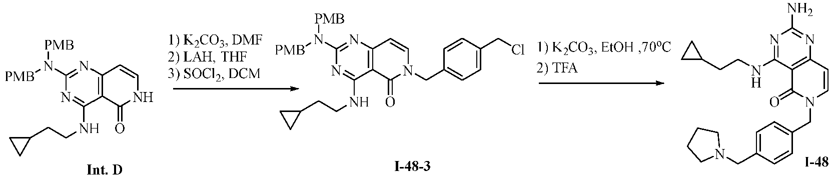

The title compound **I-48** was prepared in analogy to the preparation of compound **I-1** by using **Intermediate Int. D** instead of **Intermediate Int. A.** MS: 419.3 (M+H)⁺. ¹H NMR (400 MHz, DMSO-*d₆*) δ 11.39 (d, J = 4.4 Hz, 1H), 10.39 (s, 1H), 8.47 (s, 2H), 8.09 (d, J = 7.5 Hz, 1H), 7.51 - 7.44 (m, 2H), 7.39 - 7.32 (m, 2H), 6.26 (d, J = 7.5 Hz, 1H), 5.20 - 5.11 (m, 2H), 4.43 (d, J = 10.0 Hz, 1H), 4.31 (d, J = 5.6 Hz, 3H), 3.32 (d, J = 8.9 Hz, 2H), 3.06 (d, J = 7.7 Hz, 2H), 2.22 (d, J = 14.2 Hz, 1H), 1.99 (d, J = 7.5 Hz, 3H), 1.83 (p, J = 5.3 Hz, 3H), 1.71 (dt, J = 13.5, 6.5 Hz, 1H), 1.62 - 1.52 (m, 1H), 0.92 (t, J = 7.3 Hz, 3H).

### Example I-49

### 2-amino-4-(butylamino)-6-(4-((3-fluoropyrrolidin-1-yl)methyl)benzyl)pyrido[4,3-d]pyrimidin-5(6H)-one

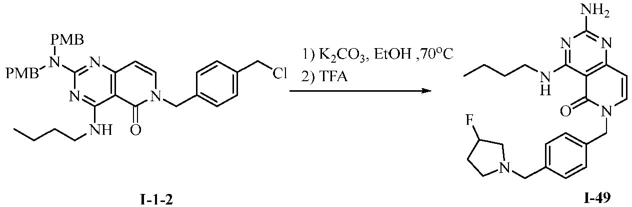

The title compound **I-49** was prepared in analogy to the preparation of compound **I-1** by using 3-fluoropyrrolidine instead of pyrrolidine. MS: 425.2 (M+H)⁺. ¹H NMR (400 MHz, DMSO-*d₆*) δ 13.47 (s, 1H), 10.88 (s, 1H), 10.11 (t, J = 5.7 Hz, 1H), 8.42 (s, 2H), 8.19 (d, J = 7.5 Hz, 1H), 7.50 (d, J = 7.7 Hz, 2H), 7.35 (d, J = 8.1 Hz, 2H), 6.41 (d, J = 7.4 Hz, 1H), 5.43 (d, J = 53.3 Hz, 1H), 5.20 (s, 2H), 4.37 (s, 2H), 3.64 - 3.21 (m, 6H), 2.25 (d, J = 50.7 Hz, 2H), 1.62 - 1.50 (m, 2H), 1.38 - 1.26 (m, 2H), 0.89 (t, J = 7.4 Hz, 3H).

### Example I-50

### 2-amino-6-(4-((bis(2-hydroxyethyl)amino)methyl)benzyl)-4-(butylamino)pyrido[4,3-d]pyrimidin-5(6H)-one

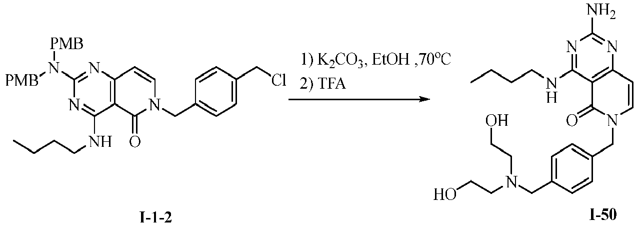

The title compound **I-50** was prepared in analogy to the preparation of compound **I-1** by using 2,2'-azanediylbis(ethan-1-ol) instead of pyrrolidine. MS: 441.2 (M+H)⁺. ¹H NMR (400 MHz, DMSO-*d₆*) δ 10.07 (s, 1H), 9.37 (s, 1H), 8.16 (d, J = 7.4 Hz, 1H), 8.00 (s, 1H), 7.52 (d, J = 8.0 Hz, 2H), 7.35 (d, J = 8.0 Hz, 2H), 6.42 (d, J = 7.4 Hz, 1H), 5.32 (s, 2H), 5.19 (s, 2H), 4.38 (s, 2H), 3.57 - 3.42 (m, 3H), 3.19 (s, 2H), 3.11 (s, 2H), 1.63 - 1.48 (m, 2H), 1.39 - 1.25 (m, 2H), 0.89 (t, J = 7.4 Hz, 3H).

### Example I-51

### 2-amino-4-(butylamino)-6-(2-fluoro-4-(pyrrolidin-1-ylmethyl)benzyl)pyrido[4,3-d]pyrimidin-5(6H)-one

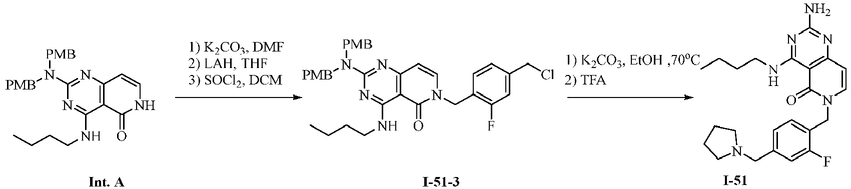

The title compound **I-51** was prepared in analogy to the preparation of compound **I-2** by using methyl 4-(bromomethyl)-3-fluorobenzoate instead of methyl 4-(bromomethyl)-3-methoxybenzoate. MS: 425.2 (M+H)⁺. ¹H NMR (400 MHz, DMSO-*d₆*) δ 9.98 (s, 2H), 8.40 (s, 1H), 8.12 (d, J = 7.4 Hz, 1H), 7.46 - 7.38 (m, 1H), 7.30 (s, 2H), 6.45 (d, J = 7.4 Hz, 1H), 5.22 (s, 2H), 4.38 - 4.28 (m, 2H), 3.58 - 3.45 (m, 3H), 3.34 (s, 2H), 3.06 (s, 2H), 2.01 (s, 2H), 1.84 (s, 2H), 1.60 - 1.48 (m, 2H), 1.38 - 1.25 (m, 2H), 0.88 (t, J = 7.4 Hz, 3H).

### Example I-52

### 6-(4-((2-oxa-6-azaspiro[3.3]heptan-6-yl)methyl)benzyl)-2-amino-4-(butylamino)pyrido[4,3-d]pyrimidin-5(6H)-one

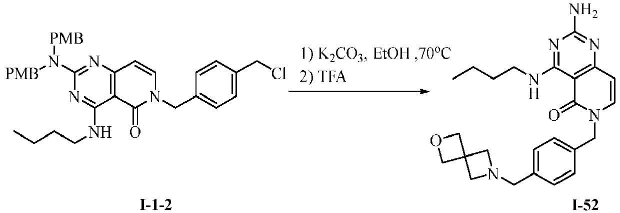

The title compound **I-52** was prepared in analogy to the preparation of compound **I-1** by using 2-oxa-6-azaspiro[3.3]heptane instead of pyrrolidine. MS: 435.2 (M+H)⁺. ¹H NMR (400 MHz, DMSO-*d₆*) δ 9.37 (t, J = 5.5 Hz, 1H), 8.21 (s, 1H), 7.66 (d, J = 7.5 Hz, 1H), 7.18 (s, 4H), 6.62 (s, 2H), 6.06 (d, J = 7.5 Hz, 1H), 4.99 (s, 2H), 4.56 (s, 4H), 3.42 - 3.34 (m, 2H), 3.25 (s, 4H), 1.57 - 1.46 (m, 2H), 1.39 - 1.26 (m, 2H), 0.89 (t, J = 7.4 Hz, 3H).

### Example I-53

### 2-amino-4-(butylamino)-6-(4-(4-isopropylpiperazine-1-carbonyl)benzyl)pyrido[4,3-d]pyrimidin-5(6H)-one

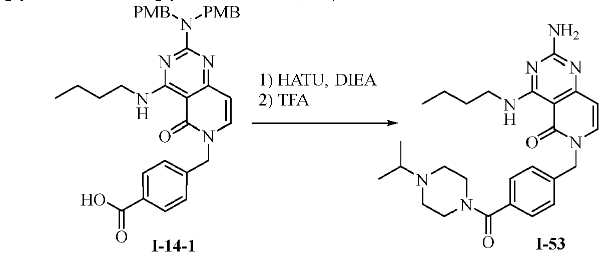

The title compound **I-53** was prepared in analogy to the preparation of compound **I-14** by using 1-isopropylpiperazine instead of tert-butyl piperazine-1-carboxylate. MS: 478.1 (M+H)⁺. ¹H NMR (400 MHz, DMSO-*d₆*) δ 10.15 - 9.96 (m, 2H), 8.19 (d, J = 7.4 Hz, 3H), 7.62 - 7.44 (m, 2H), 7.42 - 7.29 (m, 2H), 6.42 (d, J = 7.4 Hz, 1H), 5.21 (s, 2H), 3.79 - 3.00 (m, 10H), 1.58-1.54 (m, 2H), 1.37 - 1.29 (m, 2H), 1.24 (s, 3H), 1.22 (s, 3H) 0.89 (t, J = 7.3 Hz, 3H).

### Example I-54

### 2-amino-4-((2-ethoxyethyl)amino)-6-(4-(pyrrolidin-1-ylmethyl)benzyl)pyrido[4,3-d]pyrimidin-5(6H)-one

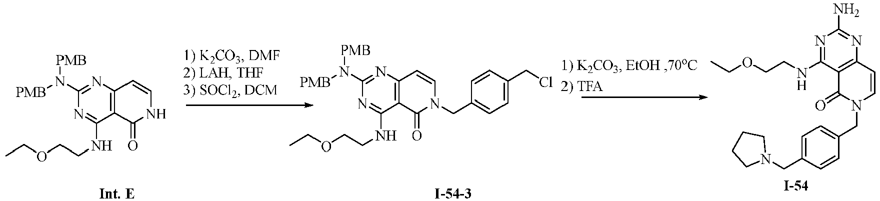

The title compound **I-54** was prepared in analogy to the preparation of compound **I-1** by using **Intermediate Int. E** instead of **Intermediate Int. A.** MS: 423.1 (M+H)⁺.

### Example I-55

### 2-amino-4-(butylamino)-6-(4-((4-(2-hydroxyethyl)piperazin-1-yl)methyl)benzyl)pyrido[4,3-d]pyrimidin-5(6H)-one

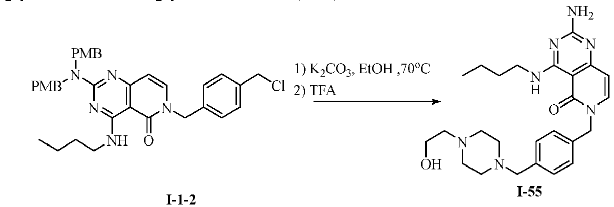

The title compound **I-55** was prepared in analogy to the preparation of compound **I-1** by using 2-(piperazin-1-yl)ethan-1-ol instead of pyrrolidine. MS: 466.6 (M+H)⁺. ¹H NMR (400 MHz, DMSO-*d₆*) δ 10.13 (t, J = 5.8 Hz, 1H), 8.45 (s, 2H), 8.19 (d, J = 7.4 Hz, 1H), 7.40 - 7.25 (m, 4H), 6.40 (d, J = 7.4 Hz, 1H), 5.17 (s, 2H), 3.90-3.68 (m, 4H), 3.57 - 3.49 (m, 2H), 3.13 (m, 11H), 1.58-1.54 (m, 2H), 1.38 - 1.27 (m, 2H), 0.89 (t, J = 7.3 Hz, 3H).

### Example I-56

### 2-amino-4-(butylamino)-6-(4-((4-cyclopentylpiperazin-1-yl)methyl)benzyl)pyrido[4,3-d]pyrimidin-5(6H)-one

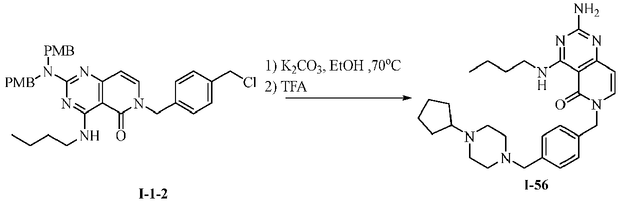

The title compound **I-56** was prepared in analogy to the preparation of compound **I-1** by using 1-cyclopentylpiperazine instead of pyrrolidine. MS: 490.6 (M+H)⁺. ¹H NMR (400 MHz, DMSO-*d₆*) δ 10.13 (t, J = 5.8 Hz, 1H), 8.38 (s, 2H), 8.18 (d, J = 7.4 Hz, 1H), 7.29 (t, J = 7.1 Hz, 4H), 6.40 (d, J = 7.4 Hz, 1H), 5.16 (s, 2H), 3.64 (s, 2H), 3.54-3.43 (m, 6H), 2.96 (s, 4H), 2.42 (s, 1H), 1.95-1.93 (m, 2H), 1.64-1.50 (m, 8H), 1.38 - 1.28 (m, 2H), 0.90 (t, J = 7.3 Hz, 3H).

### Example I-57

### 2-amino-4-(butylamino)-6-(4-(piperazin-1-ylmethyl)benzyl)pyrido[4,3-d]pyrimidin-5(6H)-one

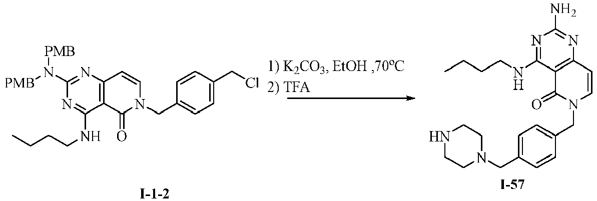

The title compound **I-57** was prepared in analogy to the preparation of compound **I-1** by using tert-butyl piperazine-1-carboxylate instead of pyrrolidine. MS: 422.2 (M+H)⁺. ¹H NMR (400 MHz, DMSO-*d₆*) δ 10.13 (t, J = 5.8 Hz, 1H), 8.98 (s, 2H), 8.55-8.23 (m, 2H), 8.19 (d, J = 7.5 Hz, 1H), 7.38 (d, J = 8.0 Hz, 1H), 7.31 (d, J = 8.0 Hz, 1H), 6.41 (d, J = 7.4 Hz, 1H), 5.17 (s, 2H), 3.89 (s, 2H), 3.51 (q, J = 6.7 Hz, 2H), 3.19 (s, 4H), 2.88 (s, 4H), 1.63 - 1.50 (m, 2H), 1.37-1.28 (m, 2H), 0.89 (t, J = 7.3 Hz, 3H).

### Example I-58

### 1-(4-((2-amino-4-(butylamino)-5-oxopyrido[4,3-d]pyrimidin-6(5H)-yl)methyl)phenyl)-N,N,N-trimethylmethanaminium iodide

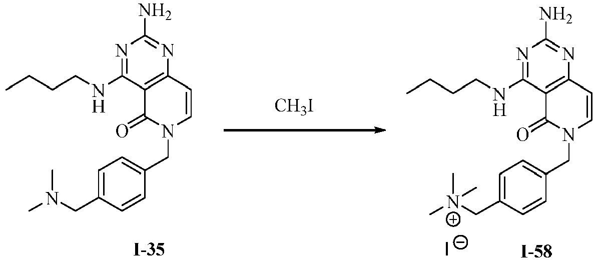

A mixture solution of **I-35** (10 mg, 0.026 mmol) and CH₃I (3.73 mg, 0.026 mmol) in ACN (10 mL) was stirred at 20°C for 1 hour. After the reaction was completed, the reaction mixture was poured into 10% KHCO₃ ice-water solution and extracted with EA. The combined organic layers were washed with brine, dried over anhydrous Na₂SO₄, filtered and concentrated in vacuo to give a crude product, which was purified by pre-HPLC to afford **I-58**. MS: 523.1 (M+H)⁺. ¹H NMR (400 MHz, DMSO-*d₆*) δ 9.34 (t, J = 5.5 Hz, 1H), 8.32 (s, 2H), 7.69 (d, J = 7.5 Hz, 1H), 7.49 (d, J = 7.9 Hz, 2H), 7.38 (d, J = 7.8 Hz, 2H), 6.63 (s, 2H), 6.09 (d, J = 7.5 Hz, 1H), 5.10 (s, 2H), 4.47 (s, 2H), 3.47 - 3.35 (m, 2H), 2.98 (s, 9H), 1.58 - 1.46 (m, 2H), 1.39 - 1.27 (m, 2H), 0.89 (t, J = 7.3 Hz, 3H).

### Example I-59

### 2-amino-4-(butylamino)-6-(1-(4-(pyrrolidin-1-ylmethyl)phenyl)ethyl)pyrido[4,3-d]pyrimidin-5(6H)-one

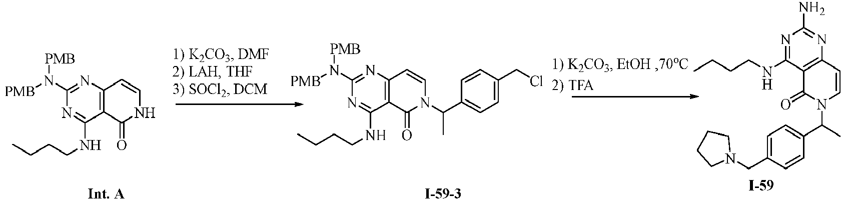

The title compound **I-59** was prepared in analogy to the preparation of compound **I-1** by using methyl 4-(1-chloroethyl)benzoate instead of methyl 4-(bromomethyl)benzoate. MS: 421.2 (M+H)⁺. ¹H NMR (400 MHz, DMSO-*d₆*) δ 10.19 (t, J = 5.8 Hz, 1H), 8.55-8.17 (m, 2H), 8.06 (d, J = 7.7 Hz, 1H), 7.50 (d, J = 8.0 Hz, 2H), 7.39 (d, J = 8.0 Hz, 2H), 6.40 (d, J = 7.6 Hz, 1H), 6.13 (q, J = 7.1 Hz, 1H), 4.32 (d, J = 5.3 Hz, 2H), 3.58-3.47 (m, 2H), 3.39-3.28 (m, 2H), 3.12-3.00 (m, 2H), 2.06-1.94 (m, 2H), 1.89-1.78 (m, 2H), 1.72 (d, J = 7.2 Hz, 3H), 1.63 - 1.50 (m, 2H), 1.42 - 1.27 (m, 2H), 0.90 (t, J = 7.3 Hz, 3H).

### Example I-60

### 2-amino-4-(butylamino)-6-(4-(2-(pyrrolidin-1-yl)ethoxy)benzyl)pyrido[4,3-d]pyrimidin-5(6H)-one

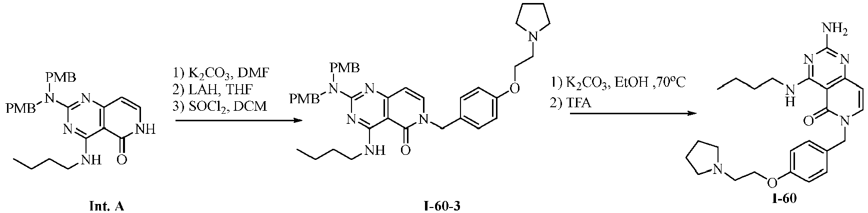

The title compound **I-60** was prepared in analogy to the preparation of compound **I-1** by using methyl 2-(4-(chloromethyl)phenoxy)acetate instead of methyl 4-(bromomethyl)benzoate. MS: 437.1 (M+H)⁺. ¹H NMR (400 MHz, DMSO-*d₆*) δ 10.15 (t, J = 5.9 Hz, 1H), 9.92 (s, 1H), 8.50-8.03 (m, 2H), 8.17 (d, J = 7.4 Hz, 1H), 7.36 - 7.24 (m, 2H), 7.01 - 6.92 (m, 2H), 6.39 (d, J = 7.4 Hz, 1H), 5.10 (s, 2H), 4.25 (t, J = 4.9 Hz, 2H), 3.59-3.47 (m, J = 6H), 3.16-3.03 (m, 2H), 2.07-1.94 (m, 2H), 1.92-1.78 (m, 2H), 1.63 - 1.50 (m, 2H), 1.38-1.29 (m, 2H), 0.90 (t, J = 7.3 Hz, 3H).

### Example I-61

### 2-amino-4-(butylamino)-6-(4-((4-methylpiperazin-1-yl)methyl)benzyl)pyrido[4,3-d]pyrimidin-5(6H)-one

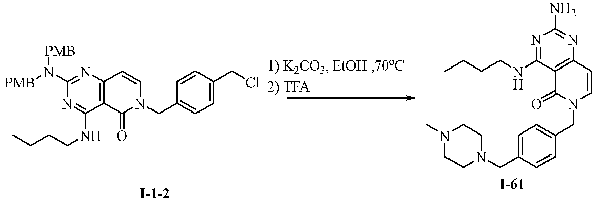

The title compound **I-61** was prepared in analogy to the preparation of compound **I-1** by using 1-methylpiperazine instead of pyrrolidine. MS: 436.6 (M+H)⁺.

### Example I-62

### 2-amino-4-(butylamino)-6-(4-((4-cyclobutylpiperazin-1-yl)methyl)benzyl)pyrido[4,3-d]pyrimidin-5(6H)-one

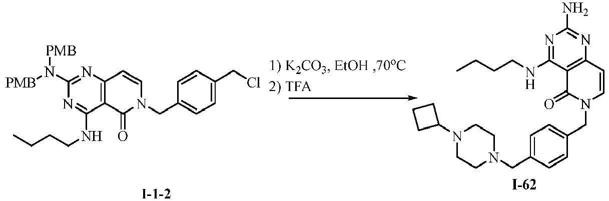

The title compound **I-62** was prepared in analogy to the preparation of compound **I-1** by using 1-cyclobutylpiperazine instead of pyrrolidine. MS: 476.6 (M+H)⁺. ¹H NMR (400 MHz, DMSO-*d₆*) δ 10.13 (t, J = 5.8 Hz, 1H), 8.57 - 8.13 (m, 3H), 7.32 (q, J = 8.0 Hz, 4H), 6.41 (d, J = 7.4 Hz, 1H), 5.16 (s, 2H), 4.51 - 2.58 (m, 13H), 2.20 - 2.03 (m, 4H), 1.73-1.52 (m, 4H), 1.35-1.30 (m, 2H), 0.89 (t, J = 7.3 Hz, 3H).

### Example I-63

### 2-amino-4-(butylamino)-6-(4-(4-methylpiperazine-1-carbonyl)benzyl)pyrido[4,3-d]pyrimidin-5(6H)-one

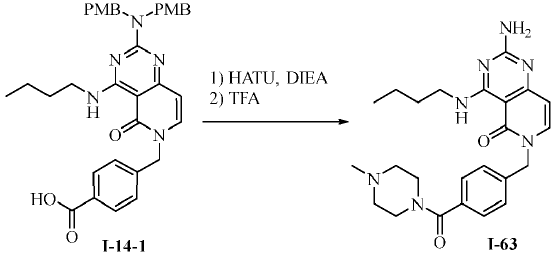

The title compound **I-63** was prepared in analogy to the preparation of compound **I-14** by using 1-methylpiperazine instead of tert-butyl piperazine-1-carboxylate. MS: 449.5 (M+H)⁺. ¹H NMR (400 MHz, DMSO-*d₆*) δ 10.08 (t, J = 5.7 Hz, 1H), 8.18 (d, J = 7.5 Hz, 3H), 7.76 - 7.35 (m, 4H), 6.42 (d, J = 7.4 Hz, 1H), 5.21 (s, 2H), 3.90 - 2.90 (m, 10H), 2.79 (s, 3H), 1.62 - 1.52 (m, 2H), 1.39 - 1.28 (m, 2H), 0.90 (t, J = 7.4, 3H).

### Example I-64

### 2-amino-4-(butylamino)-6-((2-methoxy-6-(pyrrolidin-1-ylmethyl)pyridin-3-yl)methyl)pyrido[4,3-d]pyrimidin-5(6H)-one

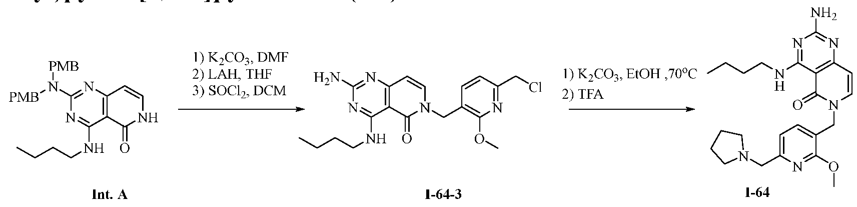

The title compound **I-64** was prepared in analogy to the preparation of compound **I-2** by using methyl 5-(chloromethyl)-6-methoxypicolinate instead of methyl 4-(bromomethyl)-3-methoxybenzoate. MS: 438.5 (M+H)⁺. 1H NMR (400 MHz, DMSO-*d₆*) δ 9.35 (t, J = 5.6 Hz, 1H), 8.14 (s, 1H), 7.68 (d, J = 7.6 Hz, 1H), 7.35 (d, J = 7.4 Hz, 1H), 7.04 (d, J = 7.5 Hz, 1H), 6.81 (s, 2H), 6.12 (d, J = 7.4 Hz, 1H), 4.96 (s, 2H), 4.33 (s, 2H), 3.97 (s, 3H), 3.39 (td, J = 6.9, 5.4 Hz, 2H), 3.26 (s, 4H), 1.97 - 1.87 (m, 4H), 1.56 - 1.45 (m, 2H), 1.37 - 1.25 (m, 2H), 0.88 (t, J = 7.3 Hz, 3H).

### Example I-65

### 6-(4-(3,6-diazabicyclo[3.1.1]heptane-6-carbonyl)benzyl)-2-amino-4-(butylamino)pyrido[4,3-d]pyrimidin-5(6H)-one

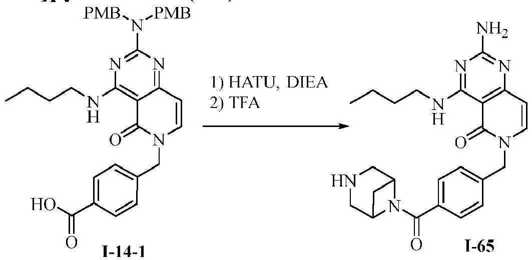

The title compound **I-65** was prepared in analogy to the preparation of compound **I-14** by using tert-butyl 3,6-diazabicyclo[3.1.1]heptane-3-carboxylate instead of tert-butyl piperazine-1-carboxylate. MS: 448.5 (M+H)⁺. ¹H NMR (400 MHz, DMSO-*d₆*) δ 10.08 (s, 1H), 9.62 (s, 1H), 8.50 - 7.98 (m, 3H), 7.50 - 7.34 (m, 4H), 6.42 (d, J = 7.4 Hz, 1H), 5.22 (d, J = 5.0 Hz, 2H), 4.69 - 3.08 (m, 8H), 2.82-2.78 (m, 1H), 1.87-1.80 (m, 1H), 1.60-1.53 (m, 2H), 1.38-1.30 (m, 2H), 0.90 (t, J = 7.4 Hz, 3H).

### Example I-66

### 6-(4-(2,5-diazabicyclo[2.2.1]heptane-2-carbonyl)benzyl)-2-amino-4-(butylamino)pyrido[4,3-d]pyrimidin-5(6H)-one

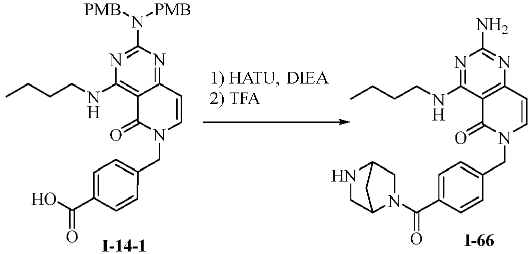

The title compound **I-66** was prepared in analogy to the preparation of compound **I-14** by using tert-butyl 2,5-diazabicyclo[2.2.1]heptane-2-carboxylate instead of tert-butyl piperazine-1-carboxylate. MS: 448.5 (M+H)⁺. ¹H NMR (400 MHz, DMSO-*d₆*) δ 10.05 (s, 1H), 9.28 (m, 1H), 8.89-8.16 (m, 3H), 7.54-7.35(m, 4H), 6.41 (d, J = 7.4 Hz, 1H), 5.22 (d, J = 5.1 Hz, 2H), 4.86 - 4.28 (m, 2H), 3.77 - 3.18 (m, 6H), 2.11-2.02 (m, 1H), 1.90-1.73 (m, 1H), 1.60-1.52 (m, 2H), 1.37-1.30 (m, 2H), 0.90 (t, J = 7.3 Hz, 3H).

### Example I-67

### 2-amino-4-(butylamino)-6-(4-(4-(2-hydroxyethyl)piperazine-1-carbonyl)benzyl)pyrido[4,3-d]pyrimidin-5(6H)-one

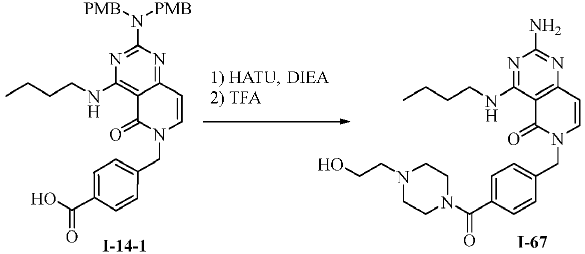

The title compound **I-67** was prepared in analogy to the preparation of compound **I-14** by using 2-(piperazin-1-yl)ethan-1-ol instead of tert-butyl piperazine-1-carboxylate. MS: 480.1 (M+H)⁺.

### Example I-68

### 2-amino-4-(butylamino)-6-(3-(pyrrolidin-1-ylmethyl)benzyl)pyrido[4,3-d]pyrimidin-5(6H)-one

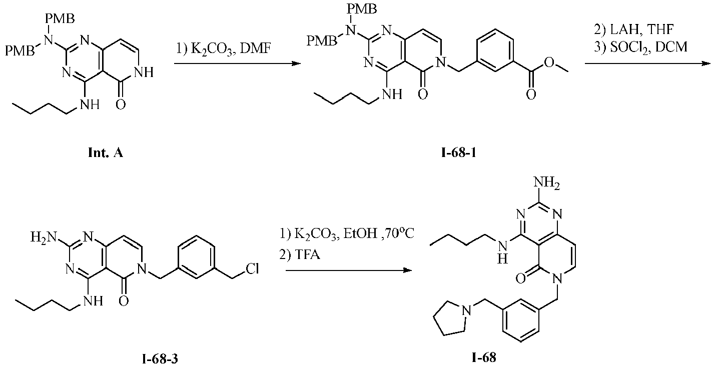

The title compound **I-68** was prepared in analogy to the preparation of compound **I-2** by using methyl 3-(bromomethyl)benzoate instead of methyl 4-(bromomethyl)-3-methoxybenzoate. MS: 407.1 (M+H)⁺. ¹H NMR (400 MHz, DMSO-*d₆*) δ 10.08 (s, 1H), 9.96 (s, 1H), 8.35 (s, 1H), 8.13 (d, J = 7.4 Hz, 1H), 7.49 -7.38 (m, 3H), 6.43 (d, J = 7.4 Hz, 1H), 5.20 (s, 2H), 4.32 (d, J = 5.2 Hz, 2H), 3.55 - 3.47 (m, 3H), 3.38 - 3.27 (m, 2H), 3.12 - 2.97 (m, 2H), 2.07 - 1.93 (m, 2H), 1.90 - 1.75 (m, 2H), 1.61 - 1.50 (m, 2H), 1.39 - 1.27 (m, 2H), 0.90 (t, J = 7.4 Hz, 3H).

### Example I-69

### 2-amino-4-(butylamino)-6-(4-(pyrrolidin-1-ylmethyl)phenethyl)pyrido[4,3-d]pyrimidin-5(6H)-one

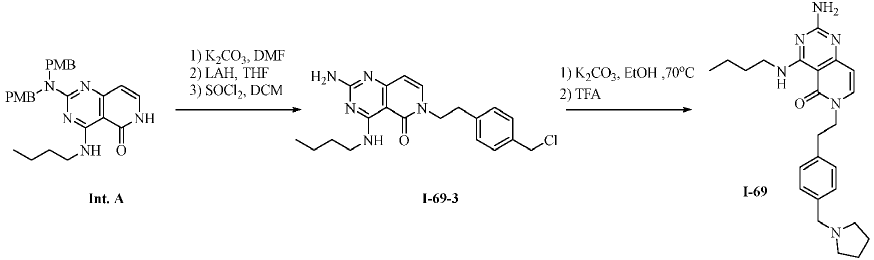

The title compound **I-69** was prepared in analogy to the preparation of compound **I-2** by using methyl 4-(2-bromoethyl)benzoate instead of methyl 4-(bromomethyl)-3-methoxybenzoate. MS: 421.1 (M+H)⁺. ¹H NMR (400 MHz, DMSO-*d₆*) δ 13.22 (s, 1H), 10.20 (s, 1H), 9.99 (s, 1H), 8.50 - 8.00 (m, 2H), 7.84 (d, J = 7.4 Hz, 1H), 7.43 (d, J = 7.8 Hz, 2H), 7.30 (d, J = 7.8 Hz, 2H), 6.26 (d, J = 7.4 Hz, 1H), 4.30 (d, J = 4.8 Hz, 2H), 4.17 (t, J = 7.4 Hz, 2H), 3.58 - 3.50 (m, 2H), 3.31 (s, 2 H), 3.15 - 3.02 (m, 2H), 2.99 (t, J = 7.4 Hz, 2H), 2.07 - 1.92 (m, 2H), 1.92 - 1.75 (m, 2H), 1.66 - 1.53 (m, 2H), 1.42 - 1.28 (m, 2H), 0.92 (t, J = 7.4 Hz, 3H).

### Example I-70

### 2-amino-4-(butylamino)-6-(3-(piperazine-1-carbonyl)benzyl)pyrido[4,3-d]pyrimidin-5(6H)-one

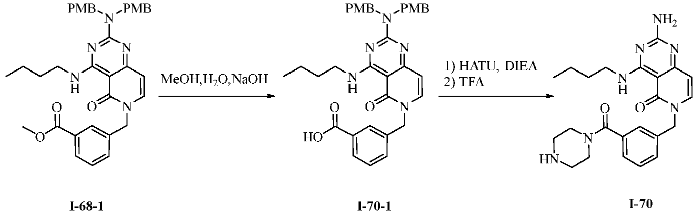

The title compound **I-70** was prepared in analogy to the preparation of compound **I-14** by using **Intermediate I-68-1** instead of **Intermediate I-11-1.** MS: 436.1 (M+H)⁺. ¹H NMR (400 MHz, DMSO-*d₆*) δ 13.13 (s, 1H), 10.07 (s, 1H), 8.98 (s, 1H), 8.49 - 7.88 (m, 3H), 7.49 - 7.36 (m, 6H), 6.41 (d, J = 7.4 Hz, 1H), 5.20 (s, 2H), 3.93 - 3.26 (m, 7H), 3.14 (s, 2H), 1.60 - 1.50 (m, 2H), 1.40 - 1.26 (m, 2H), 0.90 (t, J = 7.4 Hz, 3H).

### Example I-71

### (S)-2,6-diamino-N-(4-((2-amino-4-(butylamino)-5-oxopyrido[4,3-d]pyrimidin-6(5H)-yl)methyl)benzyl)hexanamide

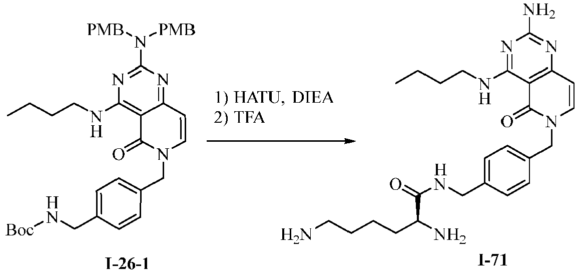

The title compound **I-71** was prepared in analogy to the preparation of compound **I-14** by using **Intermediate I-26-1** instead of tert-butyl piperazine-1-carboxylate and N²,N⁶-bis(tert-butoxycarbonyl)-L-lysine instead of **Intermediate I-14-1.** MS: 481.1 (M+H)⁺.

### Example I-72

### 2-amino-4-(butylamino)-6-(2-methoxy-4-(piperazine-1-carbonyl)benzyl)pyrido[4,3-d]pyrimidin-5(6H)-one

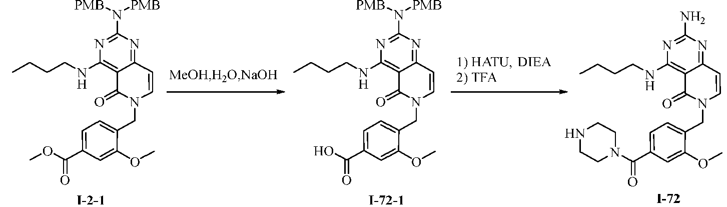

The title compound **I-72** was prepared in analogy to the preparation of compound **I-14** by using **Intermediate I-2-1** instead of **Intermediate I-11-1.** MS: 466.1 (M+H)⁺. ¹H NMR (400 MHz, DMSO-*d₆*) δ 13.05 (s, 1H), 10.12 (s, 1H), 8.94 (s, 2H), 8.45 (s, 1H), 8.09 (d, J = 7.5 Hz, 1H), 7.14 - 7.06 (m, 2H), 6.98 (dd, J = 7.6, 1.4 Hz, 1H), 6.43 (d, J = 7.5 Hz, 1H), 5.11 (s, 2H), 3.96 - 3.66 (m, 7H), 3.57 - 3.47 (m, 3H), 3.14 (s, 4H), 1.62 - 1.49 (m, 2H), 1.39 - 1.28 (m, 2H), 0.89 (t, J = 7.4 Hz, 3H).

### Example I-73

### 2-amino-4-(butylamino)-6-(2-methoxy-4-(4-methylpiperazine-1-carbonyl)benzyl)pyrido[4,3-d]pyrimidin-5(6H)-one

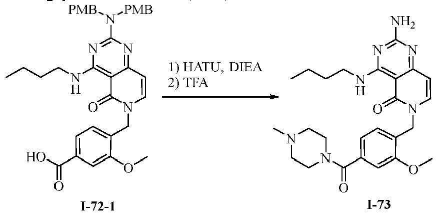

The title compound **I-73** was prepared in analogy to the preparation of compound **I-72** by using 1-methylpiperazine instead of tert-butyl piperazine-1-carboxylate. MS: 480.1 (M+H)⁺. ¹H NMR (400 MHz, DMSO-*d₆*) δ 10.30 (s, 1H), 10.18 - 10.08 (m, 1H), 8.61 - 8.20 (m, 2H), 8.10 (d, J = 7.5 Hz, 1H), 7.12 (d, J = 7.8 Hz, 1H), 7.07 (d, J = 1.4 Hz, 1H), 6.97 (dd, J = 7.6, 1.6 Hz, 1H), 6.42 (d, J = 7.6 Hz, 1H), 5.11 (s, 2H), 3.85 (s, 3H), 3.56 - 3.47 (m, 2H), 3.07 (s, 2H), 2.88 (s, 2H), 2.81 (s, 3H), 2.72 (d, J = 0.6 Hz, 1H), 2.67 (s, 2H), 1.61 - 1.50 (m, 2H), 1.38 - 1.28 (m, 2H), 1.28 - 1.20 (m, 2H), 0.89 (t, J = 7.3 Hz, 3H).

### Example I-74

### 2-amino-4-((2-methoxyethyl)amino)-6-(4-(piperazine-1-carbonyl)benzyl)pyrido[4,3-d]pyrimidin-5(6H)-one

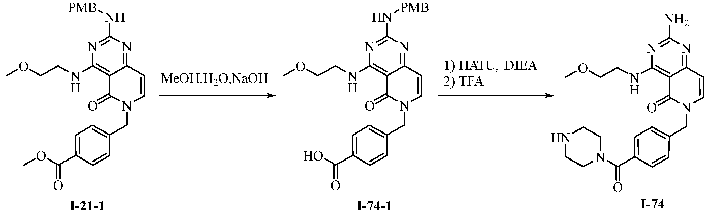

The title compound **I-74** was prepared in analogy to the preparation of compound **I-14** by using **Intermediate I-21-1** instead of **Intermediate I-11-1.** MS: 438.1 (M+H)⁺. ¹H NMR (400 MHz, DMSO-*d₆*) δ 10.00 (s, 1H), 9.07 (s, 2H), 8.10 (d, J = 7.4 Hz, 1H), 8.01 (s, 1H), 7.40 (d, J = 8.2 Hz, 2H), 7.35 (d, J = 8.2 Hz, 2H), 6.35 (d, J = 7.4 Hz, 1H), 5.19 (s, 2H), 3.66 (q, J = 5.4 Hz, 3H), 3.52 (t, J = 5.4 Hz, 3H), 3.86-3.22 (m, 4H), 3.28 (s, 3H), 3.20-3.07(m, 4H).

### Example I-75

### 2-amino-4-((2-methoxyethyl)amino)-6-(4-(4-methylpiperazine-1-carbonyl)benzyl)pyrido[4,3-d]pyrimidin-5(6H)-one

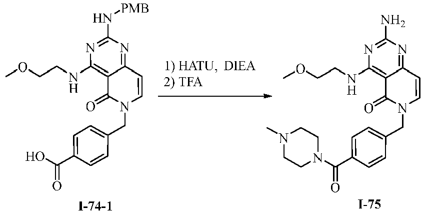

The title compound **I-75** was prepared in analogy to the preparation of compound **I-74** by using 1-methylpiperazine instead of tert-butyl piperazine-1-carboxylate. MS: 452.2 (M+H)⁺. ¹H NMR (400 MHz, DMSO-*d₆*) δ 10.14 (t, J = 5.5 Hz, 1H), 8.53-8.22 (m, 2H), 8.19 (d, J = 7.4 Hz, 1H), 7.44 (d, J = 8.2 Hz, 2H), 7.37 (d, J = 8.2 Hz, 2H), 6.42 (d, J = 7.4 Hz, 1H), 5.22 (s, 2H), 3.68 (q, J = 5.4 Hz, 3H), 3.53 (t, J = 5.4 Hz, 3H), 3.85 - 2.95 (m, 8H), 3.28 (s, 3H), 2.79 (s, 3H).

### Example I-76

### 2-amino-4-((2-(ethylamino)ethyl)amino)-6-(4-(pyrrolidin-1-ylmethyl)benzyl)pyrido[4,3-d]pyrimidin-5(6H)-one

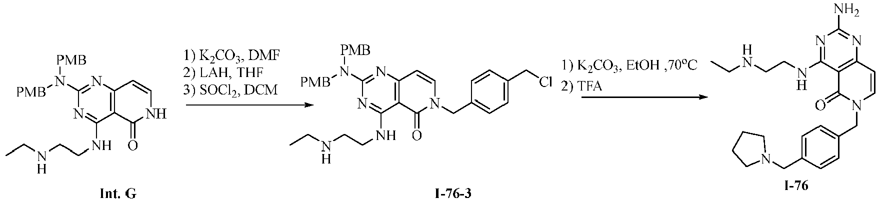

The title compound **I-76** was prepared in analogy to the preparation of compound **I-1** by using **Intermediate Int. G** instead of **Intermediate Int. A.** MS: 422.2 (M+H)⁺. ¹H NMR (400 MHz, DMSO-*d₆*) δ 13.94 (s, 1H), 10.74 (s, 1H), 10.10 (t, J = 6.1 Hz, 1H), 9.02 (s, 1H), 8.81 (s, 2H), 8.59 (d, J = 48.3 Hz, 2H), 8.26 (d, J = 7.5 Hz, 1H), 7.50 (d, J = 8.0 Hz, 2H), 7.36 (d, J = 7.9 Hz, 2H), 6.41 (d, J = 7.4 Hz, 1H), 5.20 (s, 2H), 4.32 (d, J = 4.8 Hz, 2H), 3.80 (q, J = 5.8 Hz, 2H), 3.41 - 3.27 (m, 2H), 3.20 (q, J = 5.8 Hz, 2H), 3.15 - 2.94 (m, 4H), 1.99 (q, J = 7.0, 6.3 Hz, 2H), 1.83 (td, J = 8.4, 7.1, 4.7 Hz, 2H), 1.15 (t, J = 7.2 Hz, 3H).

### Example I-77

### 2-amino-4-(butylamino)-6-(3-methoxy-4-(piperazine-1-carbonyl)benzyl)pyrido[4,3-d]pyrimidin-5(6H)-one

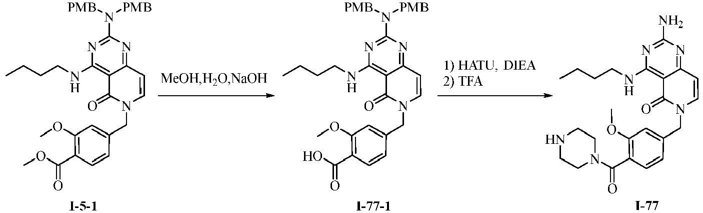

The title compound **I-77** was prepared in analogy to the preparation of compound **I-14** by using **Intermediate I-5-1** instead of **Intermediate I-11-1.** MS: 466.2 (M+H)⁺. ¹H NMR (400 MHz, DMSO-*d₆*) δ 13.42 (s, 1H), 10.11 (t, J = 5.8 Hz, 1H), 9.15 (s, 2H), 8.34 (s, 2H), 8.19 (d, J = 7.4 Hz, 1H), 7.22 (d, J = 7.7 Hz, 1H), 7.11 (d, J = 1.5 Hz, 1H), 6.86 (dd, J = 7.8, 1.4 Hz, 1H), 6.41 (d, J = 7.4 Hz, 1H), 5.19 (s, 2H), 3.79 (s, 5H), 3.52 (q, J = 6.7 Hz, 2H), 3.38 - 3.23 (m, 2H), 3.15 (s, 2H), 3.02 (s, 2H), 1.62 - 1.52 (m, 2H), 1.40 - 1.28 (m, 2H), 0.90 (t, J = 7.3 Hz, 3H).

### Example I-78

### 2-amino-4-(butylamino)-6-((4-(pyrrolidin-1-ylmethyl)naphthalen-1-yl)methyl)pyrido[4,3-d]pyrimidin-5(6H)-one

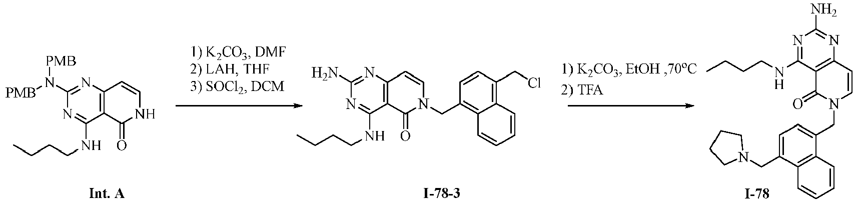

The title compound **I-78** was prepared in analogy to the preparation of compound **I-2** by using methyl 4-(bromomethyl)-1-naphthoate instead of methyl 4-(bromomethyl)-3-methoxybenzoate. MS: 457.1 (M+H)⁺. ¹H NMR (400 MHz, DMSO-*d₆*) δ 13.64 (s, 1H), 10.19 - 10.03 (m, 2H), 8.57 - 8.32 (m, 3H), 8.22 (dt, J = 8.0, 2.8 Hz, 1H), 8.08 (d, J = 7.4 Hz, 1H), 7.72 (ddd, J = 12.6, 8.7, 6.1 Hz, 3H), 7.11 (d, J = 7.4 Hz, 1H), 6.47 (d, J = 7.4 Hz, 1H), 5.73 (s, 2H), 4.87 (d, J = 5.0 Hz, 2H), 3.53 (q, J = 6.7 Hz, 2H), 3.40 (d, J = 9.2 Hz, 2H), 3.21 (d, J = 9.7 Hz, 2H), 2.04 (s, 2H), 1.85 (d, J = 9.8 Hz, 2H), 1.63 - 1.48 (m, 2H), 1.38 - 1.28 (m, 2H), 0.89 (t, J = 7.4 Hz, 3H).

### Example I-79

### 2-amino-4-(butylamino)-6-(3-methoxy-4-(4-methylpiperazine-1-carbonyl)benzyl)pyrido[4,3-d]pyrimidin-5(6H)-one

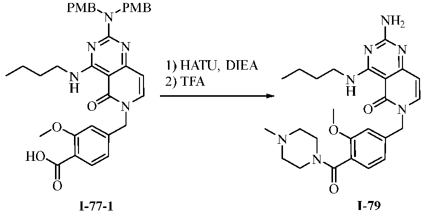

The title compound **I-79** was prepared in analogy to the preparation of compound **I-77** by using 1-methylpiperazine instead of tert-butyl piperazine-1-carboxylate. MS: 480.1 (M+H)⁺. ¹H NMR (400 MHz, DMSO-*d₆*) δ 13.50 (s, 1H), 10.48 (s, 1H), 10.14 (t, J = 5.8 Hz, 1H), 8.45 (s, 2H), 8.21 (d, J = 7.4 Hz, 1H), 7.21 (d, J = 7.7 Hz, 1H), 7.11 (d, J = 1.5 Hz, 1H), 6.92 - 6.84 (m, 1H), 6.42 (d, J = 7.5 Hz, 1H), 5.20 (s, 2H), 4.52 (s, 1H), 3.80 (s, 3H), 3.53 (q, J = 6.7 Hz, 2H), 3.38 (m, 7H), 2.82 (s, 3H), 1.63 - 1.52 (m, 2H), 1.40 - 1.28 (m, 2H), 0.90 (t, J = 7.4 Hz, 3H).

### Example I-80

### 2-amino-4-((4-fluorobutyl)amino)-6-(4-(pyrrolidin-1-ylmethyl)benzyl)pyrido[4,3-d]pyrimidin-5(6H)-one

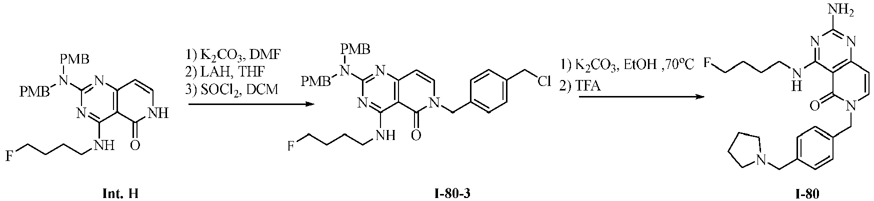

The title compound **I-80** was prepared in analogy to the preparation of compound **I-1** by using **Intermediate Int. H** instead of **Intermediate Int. A.** MS: 425.2 (M+H)⁺. ¹H NMR (400 MHz, DMSO-*d₆*) δ 13.55 (s, 1H), 10.38 (s, 1H), 10.11 (s, 1H), 8.37 (m, 2H), 8.19 (d, J = 7.5 Hz, 1H), 7.49 (d, J = 7.9 Hz, 2H), 7.35 (d, J = 7.9 Hz, 2H), 6.41 (dd, J = 7.4, 3.9 Hz, 1H), 5.22 (d, J = 16.5 Hz, 2H), 4.53 (q, J = 5.5 Hz, 2H), 4.45 - 4.37 (m, 2H), 4.31 (s, 2H), 3.60 - 3.49 (m, 2H), 3.33 (m, 2H), 3.05 (m, 2H), 2.00 (m, 2H), 1.83 (d, J = 8.4 Hz, 2H), 1.75 - 1.57 (m, 4H).

### Example I-81

### 2-amino-4-butoxy-6-(4-(pyrrolidin-1-ylmethyl)benzyl)pyrido[4,3-d]pyrimidin-5(6H)-one

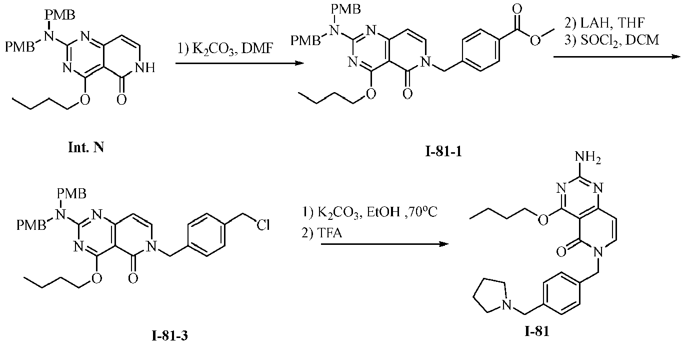

The title compound **I-81** was prepared in analogy to the preparation of compound **I-1** by using **Intermediate Int. S** instead of **Intermediate Int. A.** MS: 408.2 (M+H)⁺. ¹H NMR (400 MHz, DMSO-*d₆*) δ 10.10 (s, 1H), 7.94 (d, J = 7.5 Hz, 1H), 7.89 - 7.44 (m, 3H), 7.34 (d, J = 8.0 Hz, 2H), 6.21 (s, 1H), 5.08 (s, 2H), 4.43 - 4.18 (m, 4H), 3.33 (m, 2H), 3.06 (m, 2H), 2.01 (m, 2H), 1.83 (m, 2H), 1.70 (m, 2H), 1.50 - 1.39 (m, 2H), 0.92 (t, J = 7.4 Hz, 3H).

### Example I-82

### 4-((2-amino-4-(butylamino)-5-oxopyrido[4,3-d]pyrimidin-6(5H)-yl)methyl)-N-(2-(pyrrolidin-1-yl)ethyl)benzamide

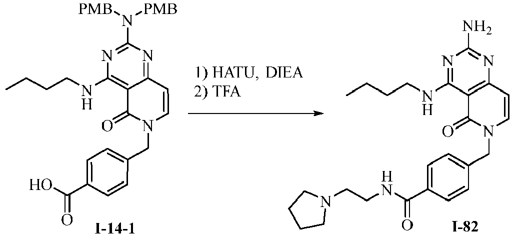

The title compound **I-82** was prepared in analogy to the preparation of compound **I-14** by using 2-(pyrrolidin-1-yl)ethan-1-amine instead of tert-butyl piperazine-1-carboxylate. MS: 464.1 (M+H)⁺. ¹H NMR (400 MHz, DMSO-*d₆*) δ 10.23 - 9.86 (m, 2H), 8.78 (d, J = 5.6 Hz, 1H), 8.19 (d, J = 7.4 Hz, 3H), 7.90 - 7.79 (m, 2H), 7.38 (d, J = 8.0 Hz, 2H), 6.42 (d, J = 7.4 Hz, 1H), 5.23 (s, 2H), 3.78 - 2.99 (m, 9H), 2.00 (s, 2H), 1.85 (s, 2H), 1.61 - 1.52 (m, 2H), 1.34 (p, J = 7.5 Hz, 2H), 0.95 - 0.87 (m, 3H).

### Example I-83

### 2-amino-4-(butylamino)-6-(4-(4-methylpiperazin-1-yl)-4-oxobutyl)pyrido[4,3-d]pyrimidin-5(6H)-one

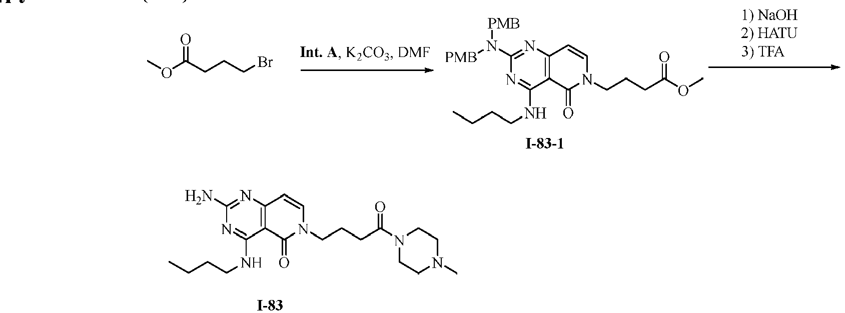

The title compound **I-83** was prepared in analogy to the preparation of compound **I-14** by using methyl 4-bromobutanoate instead of methyl 4-(bromomethyl)benzoate and 1-methylpiperazine instead of tert-butyl piperazine-1-carboxylate. MS: 402.2 (M+H)⁺. ¹H NMR (400 MHz, DMSO-*d₆*) δ 10.20 (t, J = 5.8 Hz, 1H), 8.44 - 8.06 (m, 2H), 7.99 (d, J = 7.4 Hz, 1H), 6.35 (d, J = 7.4 Hz, 1H), 4.24 - 3.07 (m, 13H), 2.78 (s, 3H), 2.40 (t, J = 7.2 Hz, 2H), 1.89 (m, 2H), 1.59 (m, 2H), 1.42 - 1.31 (m, 2H), 0.92 (t, J = 7.3 Hz, 3H).

### Example I-84

### 2-amino-4-(butylamino)-6-(4-oxo-4-(piperazin-1-yl)butyl)pyrido[4,3-d]pyrimidin-5(6H)-one

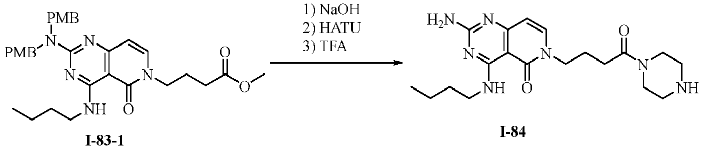

The title compound **I-84** was prepared in analogy to the preparation of compound **I-83** by using tert-butyl piperazine-1-carboxylate instead of 1-methylpiperazine. MS: 388.2 (M+H)⁺. ¹H NMR (400 MHz, DMSO-*d₆*) δ 10.25 (t, J = 5.7 Hz, 1H), 8.38 (s, 2H), 8.02 (d, J = 7.4 Hz, 1H), 6.36 (d, J = 7.4 Hz, 1H), 3.96 (t, J = 7.1 Hz, 2H), 3.69 - 3.47 (m, 6H), 3.11 (s, 2H), 3.05 (s, 2H),2.68 (s, 1H), 2.40 (t, J = 7.2 Hz, 2H), 1.95 - 1.81 (m, 2H), 1.58 (m, 2H), 1.42 - 1.29 (m, 2H), 0.92 (t, J = 7.3 Hz, 3H).

### Example I-86

### 2-amino-4-(butylamino)-6-((3-(piperazine-1-carbonyl)bicyclo[1.1.1]pentan-1-yl)methyl)pyrido[4,3-d]pyrimidin-5(6H)-one

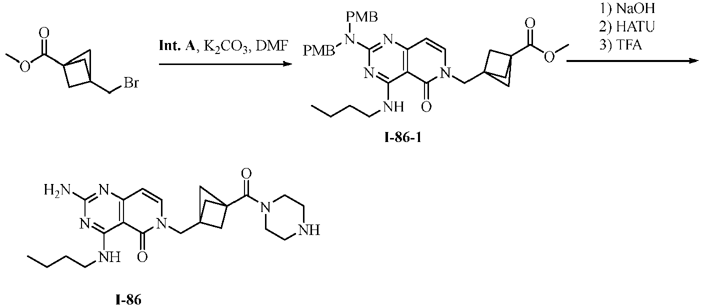

The title compound **I-86** was prepared in analogy to the preparation of compound **I-14** by using methyl 3-(bromomethyl)bicyclo[1.1.1]pentane-1-carboxylate instead of methyl 4-(bromomethyl)benzoate. MS: 426.2 (M+H)⁺.

### Example I-88

### (R)-2-amino-4-((1-hydroxy-2-methylhexan-2-yl)amino)-6-(4-(pyrrolidin-1-ylmethyl)benzyl)pyrido[4,3-d]pyrimidin-5(6H)-one

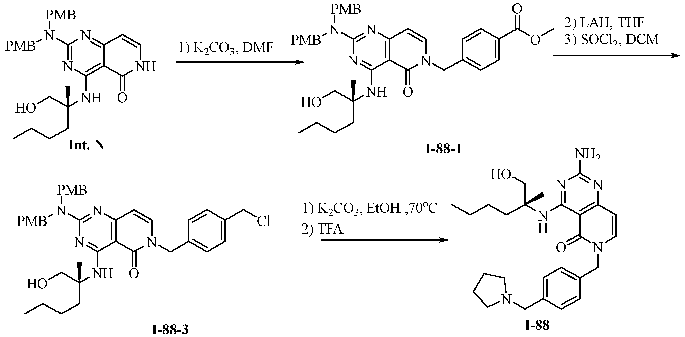

The title compound **I-88** was prepared in analogy to the preparation of compound **I-1** by using **Intermediate Int. N** instead of **Intermediate Int. A.** MS: 465.2 (M+H)⁺.

### Example I-90

### 2-amino-4-(butylamino)-6-(4-(2-(dimethylamino)ethyl)benzyl)pyrido[4,3-d]pyrimidin-5(6H)-one

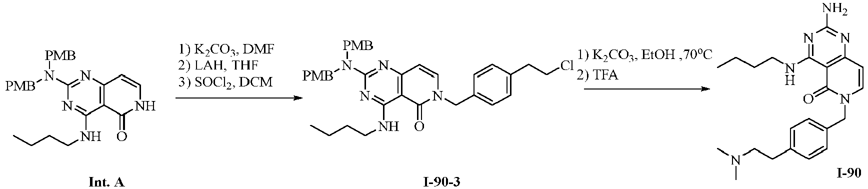

The title compound **I-90** was prepared in analogy to the preparation of compound **I-2** by using methyl 2-(4-(bromomethyl)phenyl)acetate instead of methyl 4-(bromomethyl)benzoate and dimethylamine hydrochloride instead of pyrrolidine. MS: 395.2 (M+H)⁺. ¹H NMR (400 MHz, DMSO-*d₆*) δ 10.11 (s, 1H), 9.66 (s, 1H), 8.23 - 7.90 (m, 2H), 7.39 - 7.15 (m, 4H), 5.14 (s, 2H), 3.55 - 3.48 (m, 2H), 3.28 - 3.21 (m, 2H), 2.97 - 2.88 (m, 2H), 2.81 (s, 6H), 1.64 - 1.50 (m, 2H), 1.40 - 1.25 (m, 2H), 0.90 (t, J = 7.3 Hz, 3H).

### Example I-91

### (R)-2-amino-4-((1-hydroxy-2-methylhexan-2-yl)amino)-6-(4-(piperazine-1-carbonyl)benzyl)pyrido[4,3-d]pyrimidin-5(6H)-one

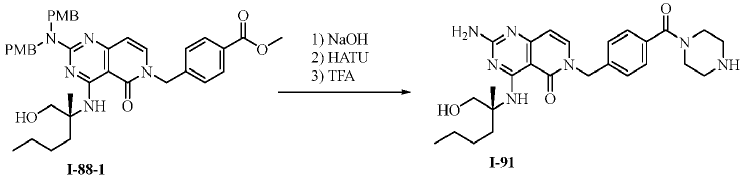

The title compound **I-91** was prepared in analogy to the preparation of compound **I-83** by using **Intermediate I-88-1** instead of **Intermediate I-83-1** and tert-butyl piperazine-1-carboxylate instead of 1-methylpiperazine. MS: 494.2 (M+H)⁺. ¹H NMR (400 MHz, DMSO-*d₆*) δ 10.23 (s, 2H), 8.45 - 8.14 (m, 3H), 7.55 - 7.44 (m, 2H), 7.35 (d, J = 8.2 Hz, 2H), 6.41 (d, J = 7.4 Hz, 1H), 5.20 (s, 2H), 4.32 (d, J = 4.8 Hz, 2H), 3.68-3.06 (m, 3H), 2.00 (s, 2H), 1.93 - 1.70 (m, 4H), 1.36 (s, 3H), 1.31 - 1.16 (m, 4H), 0.85 (t, J = 6.9 Hz, 3H).

### Example I-93

### 2-amino-4-(butylamino)-6-((4-(pyrrolidin-1-ylmethyl)cyclohexyl)methyl)pyrido[4,3-d]pyrimidin-5(6H)-one

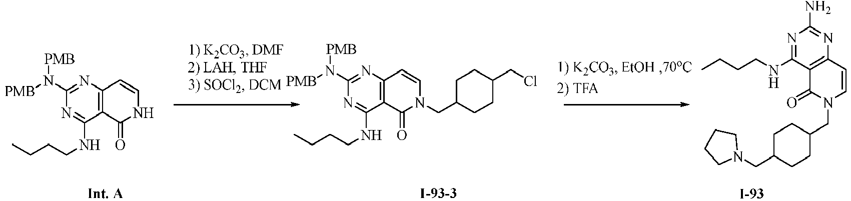

The title compound **I-93** was prepared in analogy to the preparation of compound **I-2** by using methyl 4-(bromomethyl)cyclohexane-1-carboxylate instead of methyl 4-(bromomethyl)-3-methoxybenzoate. MS: 413.1 (M+H)⁺.

### Example I-94

### 2-amino-4-(butylamino)-6-(4-(piperazin-1-yl)benzyl)pyrido[4,3-d]pyrimidin-5(6H)-one

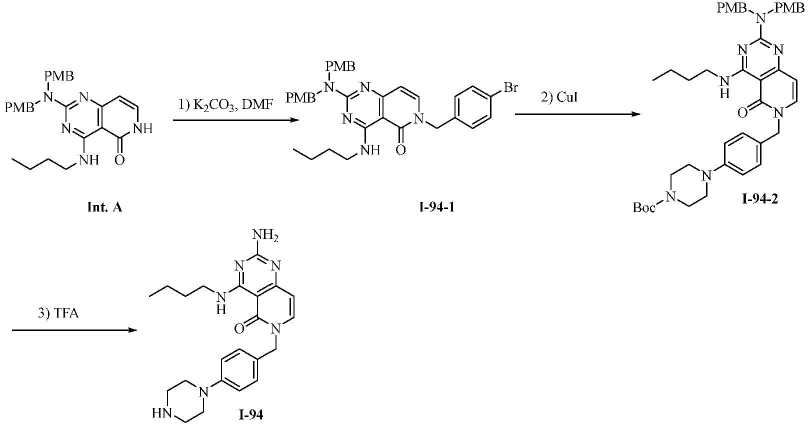

The title compound **Intermediate I-94-1** was prepared in analogy to the preparation of **Intermediate I-2-1** by using 1-bromo-4-(bromomethyl)benzene instead of methyl 4-(bromomethyl)-3-methoxybenzoate.

The title compound **Intermediate I-94-2** was prepared in analogy to the preparation of **Intermediate I-36-1** by using tert-butyl piperazine-1-carboxylate instead of **Intermediate Int. A** and **Intermediate I-94-1** instead of methyl 4-iodobenzoate.

The title compound **I-94** was prepared in analogy to the preparation of compound **I-1** by using **Intermediate I-94-2** instead of **Intermediate I-1-3.** MS: 408.1 (M+H)⁺. ¹H NMR (400 MHz, DMSO-*d₆*) δ 9.99 (s, 1H), 8.71 (s, 2H), 8.04 (s, 1H), 7.23 (d, J = 8.4 Hz, 2H), 6.95 (d, J = 8.4 Hz, 2H), 6.31 (s, 1H), 5.03 (s, 2H), 3.56 - 3.45 (m, 2H), 3.29 - 3.17 (m, 9H), 2.97 - 2.85 (m, 1H), 1.62 - 1.50 (m, 2H), 1.41 - 1.28 (m, 2H), 0.90 (t, J = 7.3 Hz, 3H).

### Example I-95

### 2-amino-4-((1-hydroxyhexan-3-yl)amino)-6-(4-(pyrrolidin-1-ylmethyl)benzyl)pyrido[4,3-d]pyrimidin-5(6H)-one

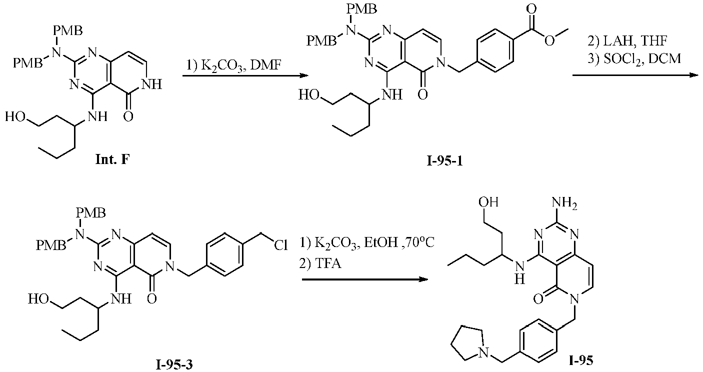

The title compound **I-95** was prepared in analogy to the preparation of compound **I-1** by using **Intermediate Int. F** instead of **Intermediate Int. A.** MS: 451.3 (M+H)⁺. ¹H NMR (400 MHz, DMSO-*d₆*) δ 13.34 (s, 1H), 10.08 (d, J = 9.2 Hz, 1H), 8.51 - 8.06 (m, 3H), 7.50 (d, J = 8.2 Hz, 2H), 7.36 (d, J = 8.1 Hz, 2H), 6.43 (d, J = 7.4 Hz, 1H), 5.20 (s, 2H), 4.40 (d, J = 6.3 Hz, 1H), 4.32 (d, J = 5.2 Hz, 2H), 3.45 (t, J = 6.5 Hz, 2H), 3.34 (dd, J = 10.2, 6.4 Hz, 2H), 3.15 - 2.98 (m, 2H), 1.99 (dt, J = 12.7, 6.6 Hz, 2H), 1.84 (dd, J = 16.4, 8.1 Hz, 2H), 1.78 - 1.63 (m, 2H), 1.63 - 1.48 (m, 2H), 1.37 - 1.20 (m, 2H), 0.87 (t, J = 7.3 Hz, 3H).

### Example I-96

### 3-((2-amino-5-oxo-6-(4-(pyrrolidin-1-ylmethyl)benzyl)-5,6-dihydropyrido[4,3-d]pyrimidin-4-yl)amino)hexyl 2,2,2-trifluoroacetate

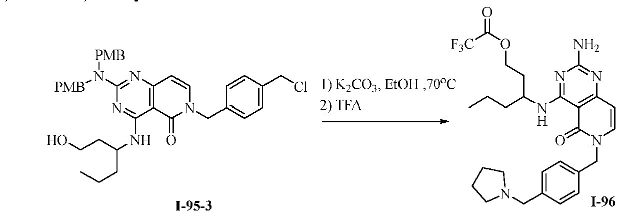

The title compound **I-96** was prepared in analogy to the preparation of compound **I-1** by using **Intermediate Int. F** instead of **Intermediate Int. A.** MS: 547.3 (M+H)⁺. ¹H NMR (400 MHz, DMSO-*d₆*) δ 13.33 (s, 1H), 10.07 (d, J = 9.0 Hz, 1H), 8.54 - 8.10 (m, 3H), 7.55 - 7.46 (m, 2H), 7.42 - 7.33 (m, 2H), 6.43 (d, J = 7.4 Hz, 1H), 5.20 (s, 2H), 4.40 (d, J = 7.1 Hz, 1H), 4.32 (d, J = 5.0 Hz, 2H), 3.45 (t, J = 6.4 Hz, 2H), 3.33 (d, J = 9.4 Hz, 2H), 3.07 (m, 2H), 2.01 (m, 2H), 1.83 (d, J = 5.9 Hz, 2H), 1.78 - 1.63 (m, 2H), 1.56 (dt, J = 13.4, 8.8 Hz, 2H), 1.38 - 1.21 (m, 2H), 0.87 (t, J = 7.3 Hz, 3H).

### Example I-97

### 2-amino-4-(butylamino)-6-(4-(piperazin-1-ylsulfonyl)benzyl)pyrido[4,3-d]pyrimidin-5(6H)-one

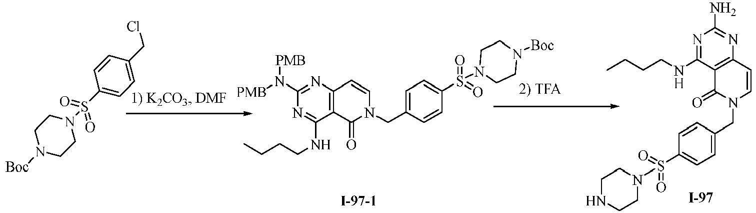

The title compound **I-97** was prepared in analogy to the preparation of compound **I-1** by using tert-butyl 4-((4-(chloromethyl)phenyl)sulfonyl)piperazine-1-carboxylate instead of methyl 4-(bromomethyl)benzoate. MS: 472.1 (M+H)⁺. ¹H NMR (400 MHz, DMSO-*d₆*) δ 9.35 (t, J = 5.7 Hz, 1H), 8.18 - 8.09 (m, 2H), 7.76 (m, 3H), 7.53 (d, J = 8.3 Hz, 2H), 6.78 (s, 2H), 6.15 (d, J = 7.5 Hz, 1H), 5.18 (s, 2H), 3.45 - 3.38 (m, 2H), 3.13 (d, J = 5.6 Hz, 4H), 3.07 (d, J = 5.6 Hz, 4H), 2.88 (m, 2H), 1.57 - 1.46 (m, 2H), 1.40 - 1.25 (m, 2H), 0.94 - 0.84 (m, 3H).

### Example I-98

### 2-amino-4-(butylamino)-6-(4-(((2-hydroxyethyl)(propyl)amino)methyl)benzyl)pyrido[4,3-d]pyrimidin-5(6H)-one

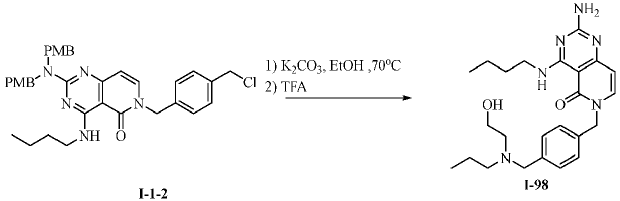

The title compound **I-98** was prepared in analogy to the preparation of compound **I-1** by using 2-(propylamino)ethan-1-ol instead of pyrrolidine. MS: 439.1 (M+H)⁺. ¹H NMR (400 MHz, DMSO-*d₆*) δ 10.07 (s, 1H), 9.51 (s, 1H), 8.40 - 8.03 (m, 2H), 7.51 (d, J = 8.0 Hz, 2H), 7.36 (d, J = 8.0 Hz, 2H), 6.42 (d, J = 7.4 Hz, 1H), 5.38 (s, 1H), 5.20 (s, 2H), 4.32 (s, 2H), 3.76 - 3.66 (m, 2H), 3.56 - 3.47 (m, 2H), 3.15 - 2.92 (m, 4H), 1.80 - 1.62 (m, 2H), 1.62 - 1.50 (m, 2H), 1.39 - 1.25 (m, 2H), 0.90 (t, J = 7.3 Hz, 3H), 0.85 (t, J = 7.3 Hz, 3H).

### Example I-99

### 2-amino-4-(butylamino)-6-(4-(4,5-dihydro-1H-imidazol-2-yl)benzyl)pyrido[4,3-d]pyrimidin-5(6H)-one

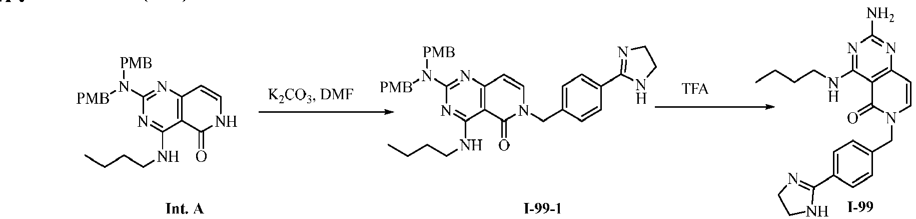

The title compound **I-99** was prepared in analogy to the preparation of compound **I-1** by using 2-(4-(chloromethyl)phenyl)-4,5-dihydro-1H-imidazole instead of methyl 4-(bromomethyl)benzoate. MS: 392.3 (M+H)⁺. ¹H NMR (400 MHz, DMSO-*d₆*) δ 10.60 (s, 2H), 10.06 (t, J = 5.6 Hz, 1H), 8.62-8.16 (m, 2H), 8.22 (d, J = 7.4 Hz, 1H), 7.89 (d, J = 8.0 Hz, 2H), 7.51 (d, J = 8.0 Hz, 2H), 6.45 (d, J = 7.4 Hz, 1H), 5.29 (s, 2H), 3.99 (s, 4H), 3.51 (q, J = 6.7 Hz, 2H), 1.55 (p, J = 7.2 Hz, 2H), 1.32 (h, J = 7.3 Hz, 2H), 0.89 (t, J = 7.4 Hz, 3H).

### Example I-100

### 2-amino-4-(butylamino)-6-(4-(4-(2,3-dihydroxypropyl)piperazine-1-carbonyl)benzyl)pyrido[4,3-d]pyrimidin-5(6H)-one

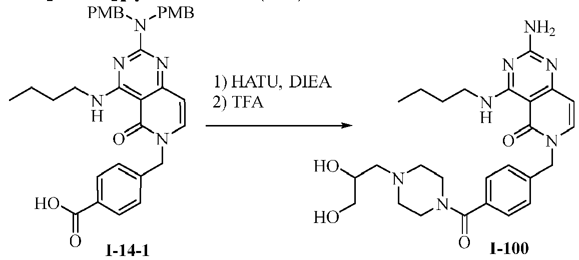

The title compound **I-100** was prepared in analogy to the preparation of compound **I-100** by using 1-((2,2-dimethyl-1,3-dioxolan-4-yl)methyl)piperazine instead of tert-butyl piperazine-1-carboxylate. MS: 510.1 (M+H)⁺. ¹H NMR (400 MHz, DMSO-*d₆*) δ 10.09 (s, 1H), 9.70 (s, 1H), 8.18 (d, 1H), 7.45 (d, J = 8.1 Hz, 2H), 7.38 (d, J = 8.1 Hz, 2H), 6.44 (d, J = 7.5 Hz, 1H), 5.59 (s, 1H), 5.22 (s, 2H), 4.99 (s, 1H), 3.88 (s, 1H), 3.75 - 3.47 (m, 7H), 3.35 - 3.23 (m, 4H), 3.23 - 2.98 (m, 4H), 1.65 - 1.49 (m, 2H), 1.43 - 1.27 (m, 2H), 0.91 (t, J = 7.4, 4.1 Hz, 3H).

### Example I-101

### (R)-2-amino-4-((1-hydroxy-2-methylhexan-2-yl)amino)-6-(4-(4-methylpiperazine-1-carbonyl)benzyl)pyrido[4,3-d]pyrimidin-5(6H)-one

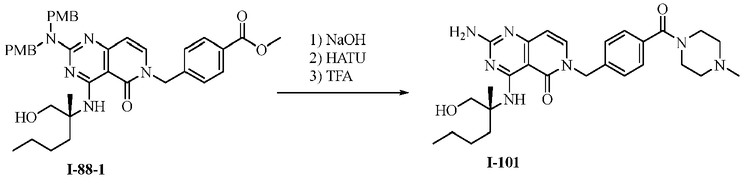

The title compound **I-101** was prepared in analogy to the preparation of compound **I-91** by using 1-methylpiperazine instead of tert-butyl piperazine-1-carboxylate. MS: 508.2 (M+H)⁺.

### Example I-102

### 2-amino-4-(butylamino)-6-((4-(pyrrolidin-1-ylmethyl)thiazol-2-yl)methyl)pyrido[4,3-d]pyrimidin-5(6H)-one

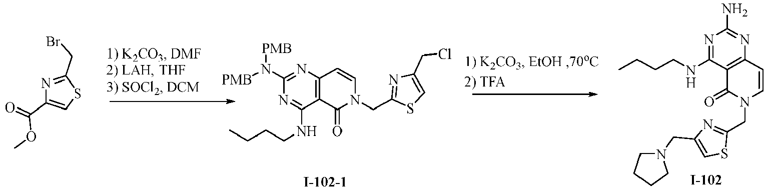

The title compound **I-102** was prepared in analogy to the preparation of compound **I-1** by using methyl 2-(bromomethyl)thiazole-4-carboxylate instead of methyl 4-(bromomethyl)benzoate. MS: 414.2 (M+H)⁺.

### Example I-103

### (R)-2-amino-4-(pentan-2-ylamino)-6-(4-(pyrrolidin-1-ylmethyl)benzyl)pyrido[4,3-d]pyrimidin-5(6H)-one

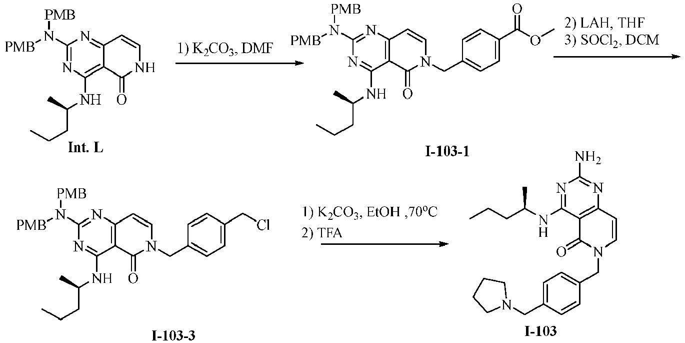

The title compound **I-103** was prepared in analogy to the preparation of compound **I-1** by using **Intermediate Int. L** instead of **Intermediate Int. A.** MS: 421.2 (M+H)⁺. ¹H NMR (400 MHz, DMSO-*d₆*) δ 10.18 (s, 1H), 10.04-10.02 (d, J = 8.0 Hz, 1H), 8.42 (br, 1H), 8.20-8.18 (d, J = 8.0 Hz, 1H), 7.50-7.48 (d, J = 8.0 Hz, 2H), 7.38-7.34 (m, 2H), 6.44-6.42 (d, J = 8.0 Hz, 1H), 5.25 - 5.14 (m, 2H), 4.32 - 4.25(m, 5H), 3.05 (br, 2H), 2.00 (br, 2H), 1.85-1.82 (m, 2H), 1.56-1.51 (m, 2H), 1.34 - 1.27 (m, 2H), 1.20 (d, J = 6.5 Hz, 3H), 0.87 (t, J = 7.3 Hz, 3H).

### Example I-104

### (S)-2-amino-4-(pentan-2-ylamino)-6-(4-(pyrrolidin-1-ylmethyl)benzyl)pyrido[4,3-d]pyrimidin-5(6H)-one

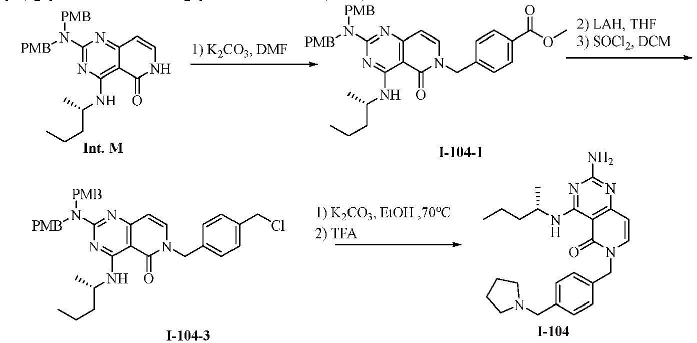

The title compound **I-104** was prepared in analogy to the preparation of compound **I-1** by using **Intermediate Int. M** instead of **Intermediate Int. A.** MS: 421.2 (M+H)⁺. ¹H NMR (400 MHz, DMSO-*d₆*) δ 10.18 (s, 1H), 10.04-10.02 (d, J = 8.0 Hz, 1H), 8.42 (br, 1H), 8.20-8.18 (d, J = 8.0 Hz, 1H), 7.50-7.48 (d, J = 8.0 Hz, 2H), 7.38-7.34 (m, 2H), 6.44-6.42 (d, J = 8.0 Hz, 1H), 5.25 - 5.14 (m, 2H), 4.32 - 4.25(m, 5H), 3.05 (br, 2H), 2.00 (br, 2H), 1.85-1.82 (m, 2H), 1.56-1.51 (m, 2H), 1.34 - 1.27 (m, 2H), 1.20 (d, J = 6.5 Hz, 3H), 0.87 (t, J = 7.3 Hz, 3H).

### Example I-105

### methyl 4-(4-((2-amino-4-(butylamino)-5-oxopyrido[4,3-d]pyrimidin-6(5H)-yl)methyl)phenyl)piperazine-1-carboxylate

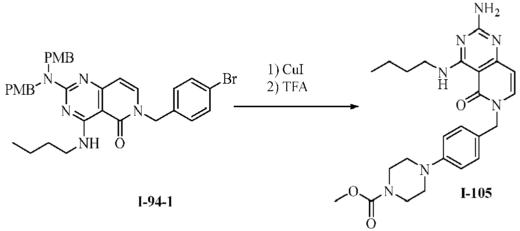

The title compound **I-105** was prepared in analogy to the preparation of compound **I-94** by using methyl piperazine-1-carboxylate instead of tert-butyl piperazine-1-carboxylate. MS: 466.1 (M+H)⁺. ¹H NMR (400 MHz, DMSO-*d₆*) δ 10.19 (s, 1H), 8.39 - 7.76 (m, 2H), 7.21 (d, J = 8.6 Hz, 2H), 6.92 (d, J = 8.6 Hz, 2H), 6.40 (d, J = 7.5 Hz, 1H), 5.05 (s, 2H), 3.60 (s, 3H), 3.56 - 3.43 (m, 7H), 3.13 - 3.02 (m, 4H), 1.63 - 1.52 (m, 2H), 1.41 - 1.26 (m, 2H), 0.91 (t, J = 7.3 Hz, 3H).

### Example I-107

### 2-amino-6-(2-methoxy-4-(pyrrolidin-1-ylmethyl)benzyl)-4-((2-methoxyethyl)amino)pyrido[4,3-d]pyrimidin-5(6H)-one

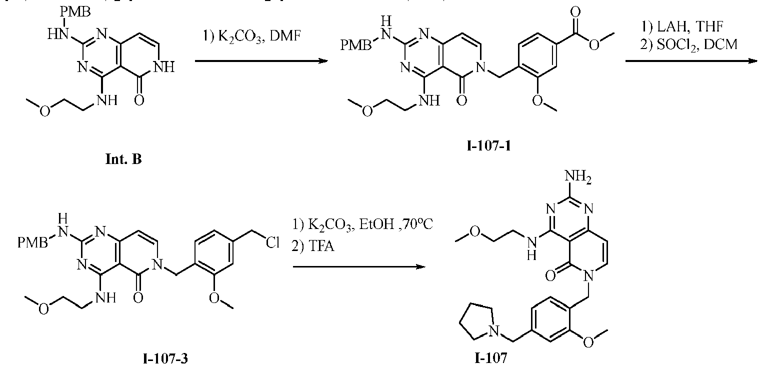

The title compound **I-107** was prepared in analogy to the preparation of compound **I-21** by using methyl 4-(bromomethyl)-3-methoxybenzoate instead of methyl 4-(bromomethyl)benzoate. MS: 439.2 (M+H)⁺.

### Example I-109

### methyl 4-(4-((2-amino-4-(butylamino)-5-oxopyrido[4,3-d]pyrimidin-6(5H)-yl)methyl)benzyl)piperazine-1-carboxylate

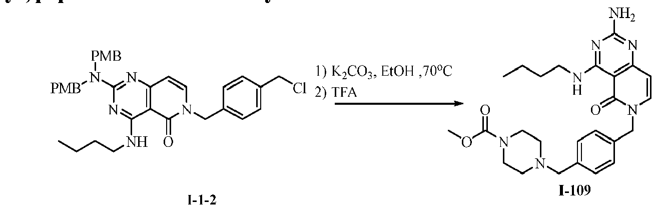

The title compound **I-109** was prepared in analogy to the preparation of compound **I-1** by using methyl piperazine-1-carboxylate instead of pyrrolidine. MS: 480.1 (M+H)⁺. ¹H NMR (400 MHz, DMSO-*d₆*) δ 10.12 (s, 1H), 8.45 (s, 1H), 8.19 (d, J = 7.4 Hz, 1H), 7.98 (s, 1H), 7.45 (d, J = 7.9 Hz, 2H), 7.37 (d, J = 7.8 Hz, 2H), 6.45 (d, J = 7.4 Hz, 1H), 5.20 (s, 2H), 4.29 (s, 2H), 4.04 (s, 2H), 3.61 (s, 2H), 3.57 - 3.49 (m, 6H), 3.21 - 2.93 (m, 4H), 1.64 - 1.49 (m, 2H), 1.40 - 1.26 (m, 2H), 0.90 (t, J = 7.3 Hz, 3H).

### Example I-110

### 2-amino-4-(butylamino)-8-fluoro-6-(4-(pyrrolidin-1-ylmethyl)benzyl)pyrido[4,3-d]pyrimidin-5(6H)-one

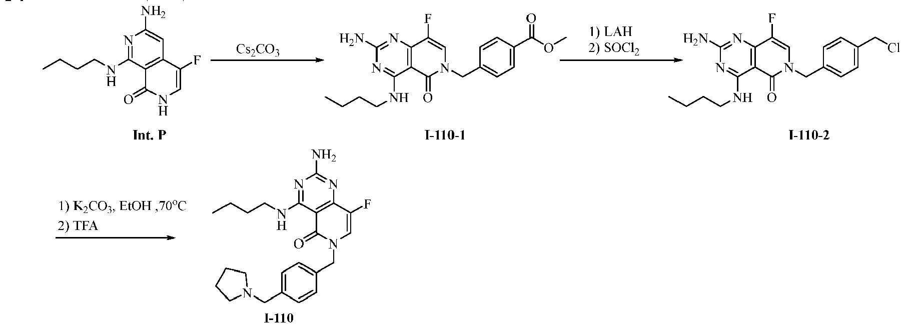

The title compound **I-110** was prepared in analogy to the preparation of compound **I-1** by using **Intermediate Int. P** instead of **Intermediate Int. A.** MS: 425.1 (M+H)⁺.

### Example I-112

### 2-amino-4-(heptan-4-ylamino)-6-(4-(piperazine-1-carbonyl)benzyl)pyrido[4,3-d]pyrimidin-5(6H)-one

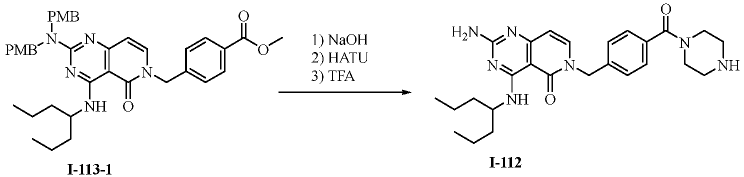

The title compound **I-112** was prepared in analogy to the preparation of compound **I-91** by using **Intermediate I-113-1** instead of **Intermediate I-88-1**. MS: 478.2 (M+H)⁺. ¹H NMR (400 MHz, DMSO-*d₆*) δ 9.99 (d, J = 9.0 Hz, 1H), 9.12 (s, 1H), 8.18 (d, J = 7.4 Hz, 2H), 7.46 (d, J = 8.2 Hz, 2H), 7.37 (d, J = 8.1 Hz, 2H), 6.43 (d, J = 7.4 Hz, 1H), 5.22 (s, 2H), 4.30 (d, J = 7.6 Hz, 1H), 3.34 (m, 9H), 1.52 (m, 4H), 1.37 - 1.19 (m, 4H), 0.87 (t, J = 7.3 Hz, 6H).

### Example I-113

### 2-amino-4-(heptan-4-ylamino)-6-(4-(pyrrolidin-1-ylmethyl)benzyl)pyrido[4,3-d]pyrimidin-5(6H)-one

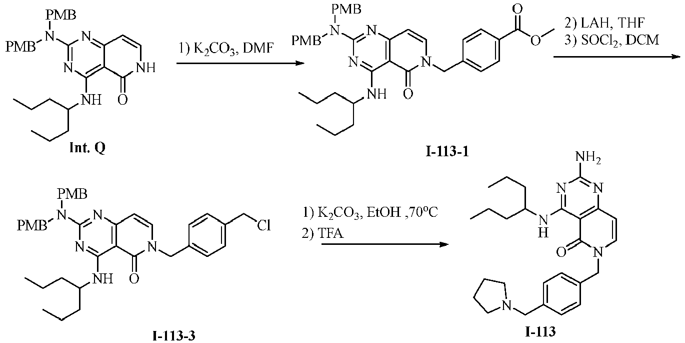

The title compound **I-113** was prepared in analogy to the preparation of compound **I-1** by using **Intermediate Int.Q** instead of **Intermediate Int.** A. MS: 449.2 (M+H)⁺. ¹H NMR (400 MHz, DMSO-*d₆*) δ 10.43 - 9.98 (m, 1H), 8.48 - 8.14 (m, 2H), 7.51 (d, J = 8.2 Hz, 2H), 7.36 (d, J = 8.1 Hz, 2H), 6.43 (d, J = 7.4 Hz, 1H), 5.20 (s, 2H), 4.32 (d, J = 5.3 Hz, 3H), 3.33 (s, 2H), 3.05 (s, 2H), 2.00 (s, 2H), 1.85 (d, J = 7.9 Hz, 2H), 1.62 - 1.44 (m, 4H), 1.37 - 1.20 (m, 4H), 0.87 (t, J = 7.3 Hz, 6H).

### Example I-114

### 2-amino-4-(heptan-4-ylamino)-6-(4-(4-methylpiperazine-1-carbonyl)benzyl)pyrido[4,3-d]pyrimidin-5(6H)-one

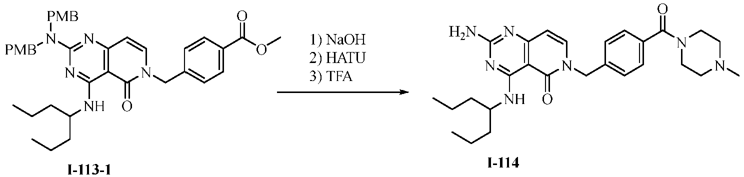

The title compound **I-114** was prepared in analogy to the preparation of compound **I-112** by using 1-methylpiperazine instead of tert-butyl piperazine-1-carboxylate. MS: 492.2 (M+H)⁺. ¹H NMR (400 MHz, DMSO-*d₆*) δ 9.99 (m, 1H), 8.18 (d, J = 7.5 Hz, 2H), 7.45 (d, J = 8.2 Hz, 2H), 7.38 (d, J = 8.3 Hz, 2H), 6.43 (d, J = 7.4 Hz, 1H), 5.22 (s, 2H), 3.44-2.98 (m, 10H) 2.79 (s, 3H), 1.52 (m,4H), 1.28 (m, 4H), 0.87 (t, J = 7.3 Hz, 6H).

### Example I-115

### 2-amino-4-((1-hydroxyhexan-3-yl)amino)-6-(4-(piperazine-1-carbonyl)benzyl)pyrido[4,3-d]pyrimidin-5(6H)-one

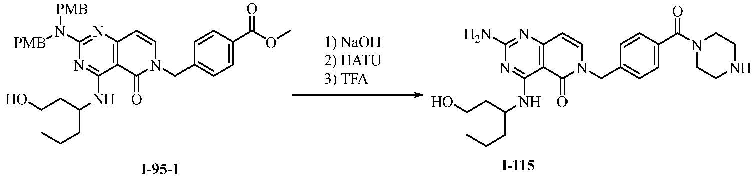

The title compound **I-115** was prepared in analogy to the preparation of compound **I-91** by using **Intermediate I-95-1** instead of **Intermediate I-88-1**. MS: 480.2 (M+H)⁺. ¹H NMR (400 MHz, DMSO-*d₆*) δ 13.39 (s, 1H), 9.91 (s, 1H), 9.20 (s, 2H), 8.09 (d, J = 7.5 Hz, 1H), 7.99-7.89 (m, 2H), 7.45 (d, J = 8.0 Hz, 2H), 7.36 (d, J = 8.0 Hz, 2H), 6.36 (d, J = 7.4 Hz, 1H), 5.25 - 5.12 (m, 2H), 4.37 (d, J = 6.7 Hz, 1H), 3.65-3.59 (m, 2H), 3.40-3.32 (m, 8H), 1.79 - 1.61 (m, 2H), 1.55 (td, J = 16.0, 7.9 Hz, 2H), 1.28 (tq, J = 13.3, 6.7, 6.2 Hz, 2H), 0.87 (t, J = 7.3 Hz, 3H).

### Example I-116

### 2-amino-4-((1-hydroxyhexan-3-yl)amino)-6-(4-(4-methylpiperazine-1-carbonyl)benzyl)pyrido[4,3-d]pyrimidin-5(6H)-one

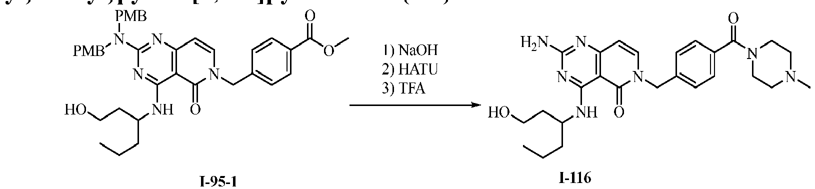

The title compound **I-116** was prepared in analogy to the preparation of compound **I-115** by using 1-methylpiperazine instead of tert-butyl piperazine-1-carboxylate. MS: 494.2 (M+H)⁺. ¹H NMR (400 MHz, DMSO-*d₆*) δ 13.36 (s, 1H), 9.93 (s, 1H), 9.18 (s, 2H), 8.19 (d, J = 7.5 Hz, 1H), 7.99-7.88 (m, 2H), 7.45 (d, J = 8.0 Hz, 2H), 7.35 (d, J = 8.0 Hz, 2H), 6.33 (d, J = 7.4 Hz, 1H), 5.26 - 5.15 (m, 2H), 4.37 (d, J = 6.7 Hz, 1H), 3.65-3.59 (m, 2H), 3.40-3.32 (m, 8H), 2.75 (s, 3H), 1.79 - 1.61 (m, 2H), 1.57-1.53 (m, 2H), 1.30-1.26 (m, 2H), 0.87 (t, J = 7.2 Hz, 3H).

### Example I-117

### (R)-2-amino-4-(pentan-2-ylamino)-6-(4-(piperazine-1-carbonyl)benzyl)pyrido[4,3-d]pyrimidin-5(6H)-one

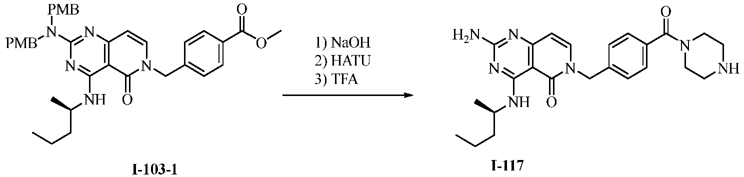

The title compound **I-117** was prepared in analogy to the preparation of compound **I-115** by using **Intermediate I-103-1** instead of **Intermediate I-95-1**. MS: 450.2 (M+H)⁺. ¹H NMR (400 MHz, DMSO-*d₆*) δ 13.61 (s, 1H), 9.98 (d, J = 8.3 Hz, 1H), 9.24 (s, 2H), 8.35 (s, 2H), 8.18 (d, J = 7.4 Hz, 1H), 7.45 (d, J = 8.0 Hz, 2H), 7.37 (d, J = 8.0 Hz, 2H), 6.40 (d, J = 7.4 Hz, 1H), 5.21 (q, J = 15.0 Hz, 2H), 4.27 (p, J = 6.9 Hz, 1H), 3.93 - 3.35 (m, 8H), 1.59 - 1.47 (m, 2H), 1.39 - 1.26 (m, 2H), 1.20 (dd, J = 6.6, 3.3 Hz, 3H), 0.88 (t, J = 7.3 Hz, 3H).

### Example I-118

### (S)-2-amino-4-((1-hydroxyhexan-3-yl)amino)-6-(4-(pyrrolidin-1-ylmethyl)benzyl)pyrido[4,3-d]pyrimidin-5(6H)-one

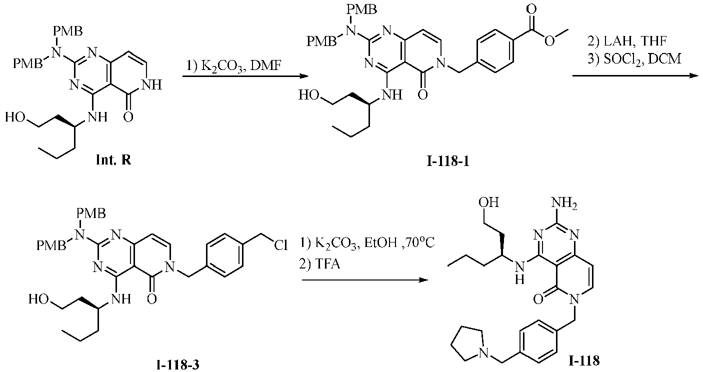

The title compound **I-118** was prepared in analogy to the preparation of compound **I-1** by using **Intermediate Int. R** instead of **Intermediate Int. A**. MS: 451.3 (M+H)⁺. ¹H NMR (400 MHz, DMSO-*d₆*) δ 13.19 (s, 1H), 9.35 (d, J = 8.9 Hz, 1H), 8.25 (s, 2H), 7.68 (d, J = 7.5 Hz, 1H), 7.34 (s, 1H), 7.33 (d, J = 7.8 Hz, 2H), 7.23 (d, J = 7.8 Hz, 2H), 6.08 (d, J = 7.5 Hz, 1H), 5.10 - 4.96 (m, 2H), 4.29 (q, J = 6.4, 5.9 Hz, 1H), 3.77 (d, J = 13.6 Hz, 3H), 3.41 (tq, J = 7.5, 4.0 Hz, 2H), 2.66-2.62 (m, 3H), 1.75-1.71 m, 5H), 1.62 - 1.40 (m, 3H), 1.37 - 1.22 (m, 2H), 0.85 (t, J = 7.2 Hz, 3H).

### Example I-119

### 2-amino-4-(butylamino)-6-((5-methoxy-1,2,3,4-tetrahydroisoquinolin-7-yl)methyl)pyrido[4,3-d]pyrimidin-5(6H)-one

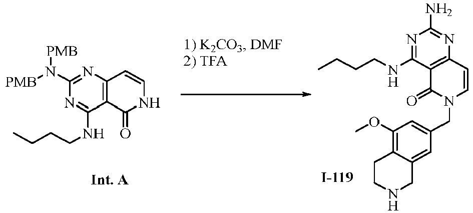

The title compound **I-119** was prepared in analogy to the preparation of compound **I-2** by using tert-butyl 7-(bromomethyl)-5-methoxy-3,4-dihydroisoquinoline-2(1H)-carboxylate instead of methyl 4-(bromomethyl)-3-methoxybenzoate. MS: 409.2 (M+H)⁺. ¹H NMR (400 MHz, DMSO-*d₆*) δ 10.13 (s, 1H), 9.04 (s, 2H), 8.43 (s, 1H), 8.16 (d, J = 7.5 Hz, 2H), 6.95 (s, 1H), 6.65 (s, 1H), 6.42 (d, J = 7.4 Hz, 1H), 5.13 (s, 2H), 4.18 (s, 2H), 3.79 (s, 3H), 3.56 - 3.49 (m, 2H), 3.34 (s, 2H), 2.79 - 2.71 (m, 2H), 1.61 - 1.51 (m, 2H), 1.39 - 1.29 (m, 2H), 0.91 (t, J = 7.3 Hz, 3H).

### Example I-120

### 2-amino-4-(butylamino)-6-(4-(4-(2-hydroxyethyl)piperazine-1-carbonyl)-2-methoxybenzyl)pyrido[4,3-d]pyrimidin-5(6H)-one

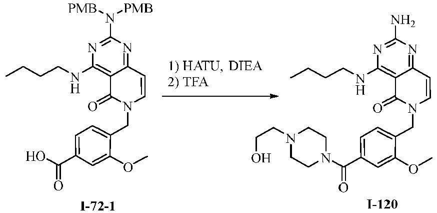

The title compound **I-120** was prepared in analogy to the preparation of compound **I-72** by using 2-(piperazin-1-yl)ethan-1-ol instead of tert-butyl piperazine-1-carboxylate. MS: 510.1 (M+H)⁺. ¹H NMR (400 MHz, DMSO-*d₆*) δ 13.33 (s, 1H), 10.09 (s, 1H), 9.96 (s, 1H), 8.50 - 8.12 (m, 2H), 8.08 (d, J = 7.5 Hz, 1H), 7.12 (d, J = 7.7 Hz, 1H), 7.08 (s, 1H), 6.98 (d, J = 7.8 Hz, 1H), 6.41 (d, J = 7.4 Hz, 1H), 5.42 (s, 1H), 5.11 (s, 2H), 3.86 (s, 3H), 3.77 - 3.70 (m, 2H), 3.56 - 3.45 (m, 3H), 3.45 - 2.95 (m, 7H), 1.65 - 1.50 (m, 2H), 1.44 - 1.25 (m, 2H), 0.90 (t, J = 7.3 Hz, 3H).

### Example I-121

### 2-amino-4-(butylamino)-8-fluoro-6-(4-(piperazine-1-carbonyl)benzyl)pyrido[4,3-d]pyrimidin-5(6H)-one

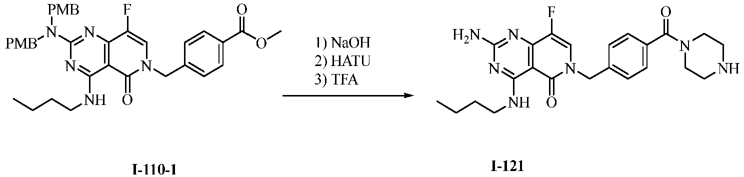

The title compound **I-121** was prepared in analogy to the preparation of compound **I-115** by using **Intermediate I-110-1** instead of **Intermediate I-95-1**. MS: 454.2 (M+H)⁺. ¹H NMR (400 MHz, DMSO-*d₆*) δ 9.86 (s, 1H), 8.91 (s, 2H), 8.43 (d, J = 5.6 Hz, 1H), 7.89 (s, 1H), 7.44 (d, J = 8.0 Hz, 2H), 7.39 (d, J = 8.1 Hz, 2H), 5.12 (s, 2H), 3.60 - 3.45 (m, 5H), 3.14 (s, 5H), 1.61 - 1.49 (m, 2H), 1.39 - 1.27 (m, 2H), 0.90 (t, J = 7.3 Hz, 3H).

### Example I-122

### 2-amino-4-(butylamino)-6-(4-(piperidin-4-yl)benzyl)pyrido[4,3-d]pyrimidin-5(6H)-one

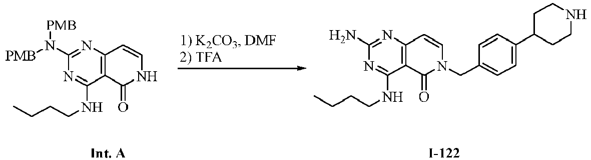

The title compound **I-122** was prepared in analogy to the preparation of compound **I-2** by using tert-butyl 4-(4-(bromomethyl)phenyl)piperidine-1-carboxylate instead of methyl 4-(bromomethyl)-3-methoxybenzoate. MS: 407.2 (M+H)⁺. ¹H NMR (400 MHz, DMSO-*d₆*) δ 13.21 (s, 1H), 10.12 (d, J = 6.2 Hz, 1H), 8.67 (s, 1H), 8.44 (s, 2H), 8.18 (d, J = 7.4 Hz, 1H), 7.27 (d, J = 8.1 Hz, 2H), 7.20 (d, J = 8.1 Hz, 2H), 6.41 (d, J = 7.4 Hz, 1H), 5.14 (s, 2H), 3.51 (q, J = 6.8 Hz, 2H), 3.35 (d, J = 12.5 Hz, 2H), 2.98 (q, J = 12.2 Hz, 2H), 2.81 (t, J = 12.0 Hz, 1H), 1.88 (d, J = 13.7 Hz, 2H), 1.82 - 1.67 (m, 2H), 1.63 - 1.51 (m, 2H), 1.41 - 1.26 (m, 2H), 0.90 (t, J = 7.3 Hz, 3H).

### Example I-123

### (R)-2-amino-6-(4-(4-(2-hydroxyethyl)piperazine-1-carbonyl)-2-methoxybenzyl)-4-(pentan-2-ylamino)pyrido[4,3-d]pyrimidin-5(6H)-one

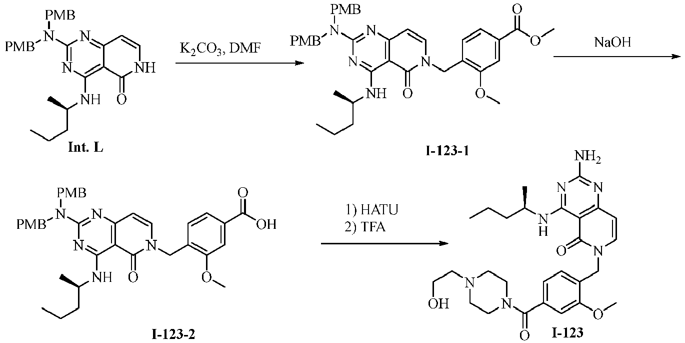

The title compound **I-123** was prepared in analogy to the preparation of compound **I-117** by using methyl 4-(bromomethyl)-3-methoxybenzoate instead of methyl 4-(bromomethyl)benzoate and 2-(piperazin-1-yl)ethan-1-ol instead of tert-butyl piperazine-1-carboxylate. MS: 524.2 (M+H)⁺. ¹H NMR (400 MHz, DMSO-*d₆*) δ 13.43 (s, 1H), 10.03 (d, J = 8.3 Hz, 1H), 8.40 (d, J = 57.9 Hz, 2H), 8.09 (d, J = 7.5 Hz, 1H), 7.15 - 7.06 (m, 2H), 6.99 (d, J = 7.7 Hz, 1H), 6.43 (d, J = 7.4 Hz, 1H), 5.43 (s, 1H), 5.18 - 5.03 (m, 2H), 4.48 (s, 1H), 4.33 - 4.21 (m, 1H), 3.86 (s, 3H), 3.73 (t, J = 5.0 Hz, 2H), 3.19 (t, J = 5.1 Hz, 2H), 3.12 (s, 2H), 1.60 - 1.44 (m, 2H), 1.38 - 1.21 (m, 2H), 1.19 (d, J = 6.5 Hz, 3H), 0.87 (t, J = 7.3 Hz, 3H).

### Example I-124

### 2-amino-4-(pentan-2-yloxy)-6-(4-(pyrrolidin-1-ylmethyl)benzyl)pyrido[4,3-d]pyrimidin-5(6H)-one

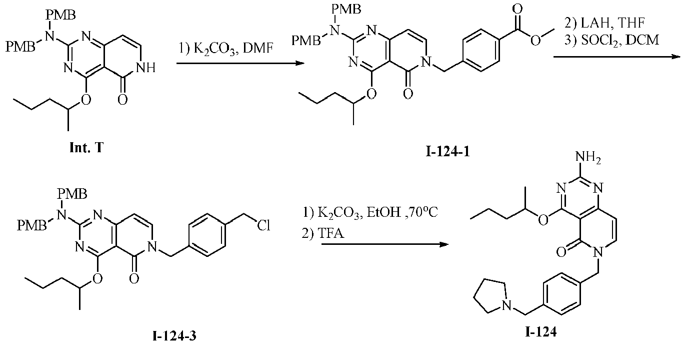

The title compound **I-124** was prepared in analogy to the preparation of compound **I-1** by using **Intermediate Int. T** instead of **Intermediate Int. A**. MS: 422.2 (M+H)⁺. ¹H NMR (400 MHz, DMSO-*d₆*) δ 9.86 (s, 1H), 8.18 - 7.85 (m, 2H), 7.48 (d, J = 8.1 Hz, 2H), 7.35 (d, J = 7.9 Hz, 2H), 6.24 (d, J = 7.5 Hz, 1H), 5.41 - 5.33 (m, 1H), 5.18 - 4.98 (m, 2H), 4.32 (d, J = 5.6 Hz, 2H), 3.34 (s, 2H), 3.06 (s, 2H), 2.07 - 1.92 (m, 2H), 1.90 - 1.76 (m, 2H), 1.74 - 1.49 (m, 2H), 1.45 - 1.33 (m, 2H), 1.32 - 1.21 (m, 3H), 0.87 (t, J = 7.3 Hz, 3H).

### Example I-126

### (S)-2-amino-4-((1-hydroxypentan-2-yl)amino)-6-(4-(piperazine-1-carbonyl)benzyl)pyrido[4,3-d]pyrimidin-5(6H)-one

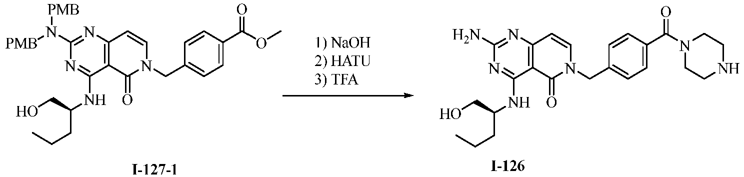

The title compound **I-126** was prepared in analogy to the preparation of compound **I-115** by using **Intermediate I-127-1** instead of **Intermediate I-95-1**. MS: 466.2 (M+H)⁺. ¹H NMR (400 MHz, DMSO-*d₆*) δ 9.38 (d, J = 8.6 Hz, 1H), 8.17 (s, 1H), 7.71 (d, J = 7.5 Hz, 1H), 7.42-7.29 (m, 4H), 6.59 (br, 2H), 6.09 (d, J = 7.5 Hz, 1H), 5.17-4.97 (m, 2H), 4.23-4.13 (m, 1H), 3.50-3.35 (m, 4H), 2.98-2.79 (m, 6H), 1.63-1.52 (m, 1H), 1.50-1.38 (m, 1H), 1.35-1.23 (m, 2H), 0.88 (td, J = 7.3, 3.1 Hz, 3H).

### Example I-127

### (S)-2-amino-4-((1-hydroxypentan-2-yl)amino)-6-(4-(pyrrolidin-1-ylmethyl)benzyl)pyrido[4,3-d]pyrimidin-5(6H)-one

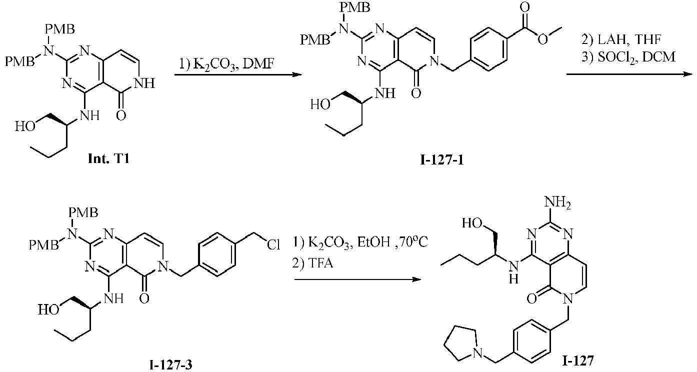

The title compound **I-127** was prepared in analogy to the preparation of compound **I-1** by using **Intermediate Int. T1** instead of **Intermediate Int. A**. MS: 437.3 (M+H)⁺. ¹H NMR (400 MHz, DMSO-*d₆*) δ 9.40 (d, J = 8.5 Hz, 1H), 8.23 (s, 1H), 7.67 (d, J = 7.5 Hz, 1H), 7.29-7.16 (m, 4H), 6.56 (br, 2H), 6.06 (d, J = 7.5 Hz, 1H), 5.10-4.90 (m, 2H), 4.23-4.14 (m, 1H), 3.54 (s, 2H), 3.49-3.38 (m, 2H), 2.45-2.35 (m, 4H), 1.70-1.63 (m, 4H), 1.63-1.53 (m, 1H), 1.50-1.38 (m, 1H), 1.35-1.25 (m, 2H), 0.87 (t, J = 7.3 Hz, 3H).

### Example I-128

### 2-amino-4-(butylamino)-6-((5-(pyrrolidin-1-ylmethyl)pyridin-2-yl)methyl)pyrido[4,3-d]pyrimidin-5(6H)-one

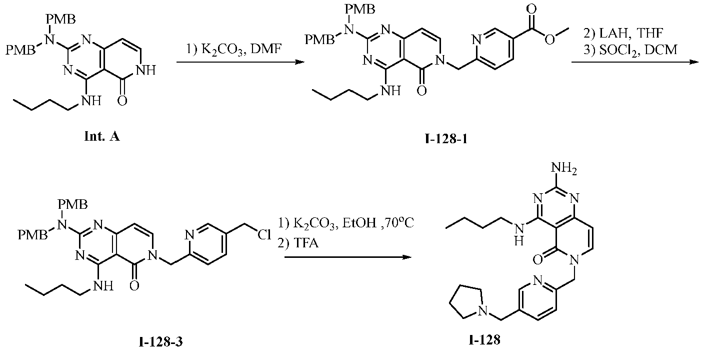

The title compound **I-128** was prepared in analogy to the preparation of compound **I-2** by using methyl 6-(bromomethyl)nicotinate instead of methyl 4-(bromomethyl)-3-methoxybenzoate. MS: 408.2 (M+H)⁺. ¹H NMR (400 MHz, DMSO-*d₆*) δ 13.23 (s, 1H), 10.24 (s, 1H), 10.06 (t, J = 5.8 Hz, 1H), 8.59 (d, J = 2.1 Hz, 1H), 8.49 (s, 1H), 8.41 (s, 1H), 8.17 (d, J = 7.4 Hz, 1H), 7.95 (dd, J = 8.1, 2.3 Hz, 1H), 7.46 (d, J = 8.0 Hz, 1H), 6.46 (d, J = 7.4 Hz, 1H), 5.32 (s, 2H), 4.38 (d, J = 5.2 Hz, 2H), 3.50 (q, J = 6.7 Hz, 2H), 3.09 (s, 2H), 2.02 (s, 2H), 1.86 - 1.81 (m, 2H), 1.60 - 1.48 (m, 2H), 1.38 - 1.24 (m, 2H), 0.88 (t, J = 7.3 Hz, 3H).

### Example I-129

### 2-amino-4-(butylamino)-6-((5-(pyrrolidin-1-ylmethyl)pyrazin-2-yl)methyl)pyrido[4,3-d]pyrimidin-5(6H)-one

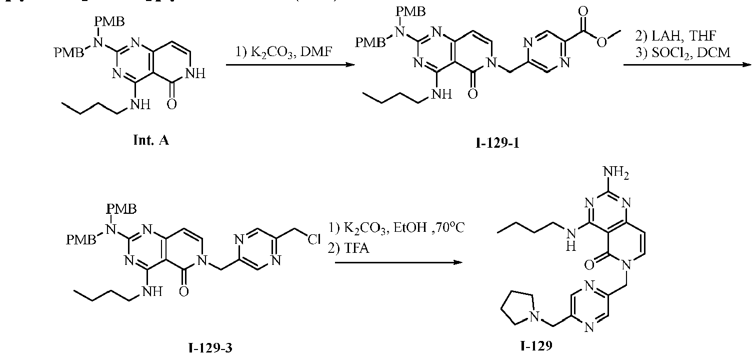

The title compound **I-129** was prepared in analogy to the preparation of compound **I-2** by using methyl 5-(bromomethyl)pyrazine-2-carboxylate instead of methyl 4-(bromomethyl)-3-methoxybenzoate. MS: 409.2 (M+H)⁺.

### Example I-130

### N-(2-((2-amino-5-oxo-6-(4-(piperazine-1-carbonyl)benzyl)-5,6-dihydropyrido[4,3-d]pyrimidin-4-yl)amino)pentyl)acetamide

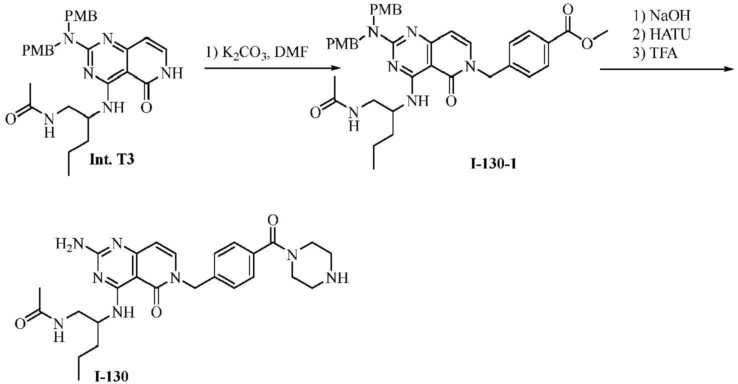

The title compound **I-130** was prepared in analogy to the preparation of compound **I-113** by using **Intermediate Int. T3** instead of **Intermediate Int. Q.** MS: 507.2 (M+H)⁺. ¹H NMR (400 MHz, DMSO-*d₆*) δ 9.28 (d, J = 8.4 Hz, 1H), 8.21 (s, 1H), 7.88 (t, J = 5.8 Hz, 1H), 7.71 (d, J = 7.5 Hz, 1H), 7.40-7.29 (m, 4H), 6.62 (br, 2H), 6.10 (d, J = 7.5 Hz, 1H), 5.16-5.00 (m, 2H), 4.28-4.16 (m, 1H), 3.69-3.50 (m, 2H), 3.36-3.28 (m, 3H), 3.10-3.02 (m, 1H), 2.90-2.72 (m, 4H), 1.76 (s, 3H), 1.57-1.36 (m, 2H), 1.35-1.22 (m, 2H), 0.86 (t, J = 7.2 Hz, 3H).

### Example I-131

### N-(2-((2-amino-5-oxo-6-(4-(pyrrolidin-1-ylmethyl)benzyl)-5,6-dihydropyrido[4,3-d]pyrimidin-4-yl)amino)pentyl)acetamide

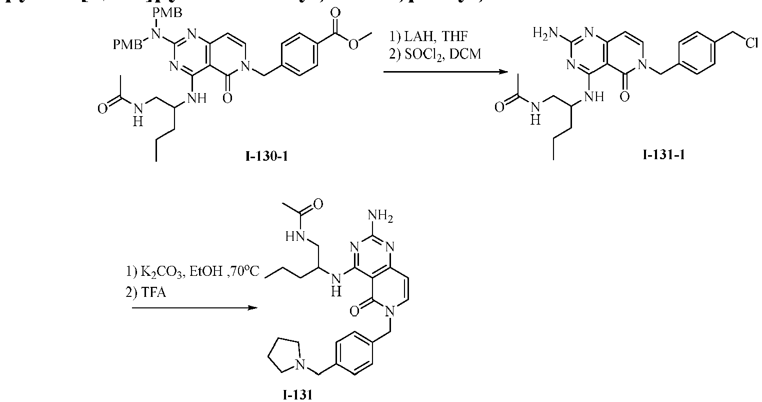

The title compound **I-131** was prepared in analogy to the preparation of compound **I-5** by using **Intermediate I-130-1** instead of **Intermediate I-5-1**. MS: 478.3 (M+H)⁺. ¹H NMR (400 MHz, DMSO-*d₆*) δ 9.31 (d, J = 8.4 Hz, 1H), 8.17 (d, J = 1.5 Hz, 1H), 7.88 (t, J = 5.8 Hz, 1H), 7.68 (d, J = 7.5 Hz, 1H), 7.34-7.18 (m, 4H), 6.07 (d, J = 7.5 Hz, 1H), 6.60 (br, 2H), 5.10-4.95 (m, 2H), 4.28-4.17 (m, 1H), 3.68 (s, 2H), 3.37-3.30 (m, 1H), 3.10-3.02 (m, 1H), 2.60-2.52 (m, 4H), 1.76 (s, 3H), 1.74-1.65 (m, 4H), 1.55-1.37 (m, 2H), 1.35-1.22 (m, 2H), 0.86 (t, J = 7.3 Hz, 3H).

### Example I-132

### 2-amino-4-(heptan-4-ylamino)-6-(2-methoxy-4-(piperazine-1-carbonyl)benzyl)pyrido[4,3-d]pyrimidin-5(6H)-one

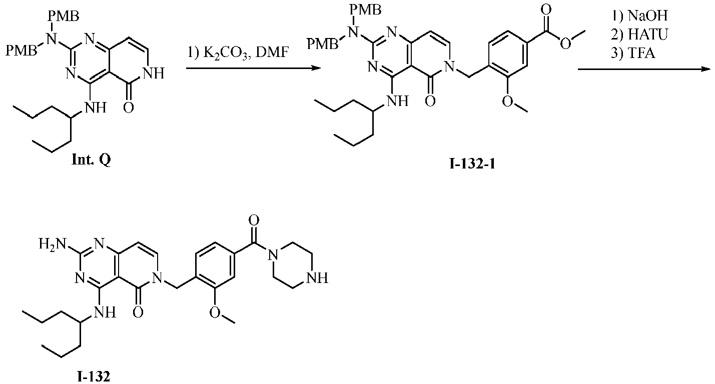

The title compound **I-132** was prepared in analogy to the preparation of compound **I-113** by using methyl 4-(bromomethyl)-3-methoxybenzoate instead of methyl 4-(bromomethyl)benzoate. MS: 508.2 (M+H)⁺. ¹H NMR (400 MHz, DMSO-*d₆*) δ 9.25 (d, J = 8.8 Hz, 1H), 8.14 (d, J = 1.5 Hz, 1H), 7.58 (d, J = 7.6 Hz, 1H), 7.03 (s, 1H), 6.92 (s, 2H), 6.57 (br, 2H), 6.08 (d, J = 7.5 Hz, 1H), 4.98 (s, 2H), 4.30-4.20 (m, 1H), 3.86 (s, 3H), 3.69-3.48 (m, 4H), 2.92-2.77 (m, 4H), 1.53-1.35 (m, 4H), 1.34-1.18 (m, 4H), 0.85 (t, J = 7.2 Hz, 6H).

### Example I-133

### 2-amino-4-(heptan-4-ylamino)-6-(2-methoxy-4-(4-methylpiperazine-1-carbonyl)benzyl)pyrido[4,3-d]pyrimidin-5(6H)-one

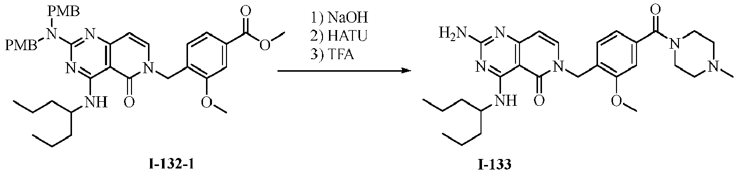

The title compound **I-133** was prepared in analogy to the preparation of compound **I-132** by using 1-methylpiperazine instead of tert-butyl piperazine-1-carboxylate. MS: 522.2 (M+H)⁺. ¹H NMR (400 MHz, DMSO-*d₆*) δ 9.32 (d, J = 8.8 Hz, 1H), 7.62 (d, J = 7.5 Hz, 1H), 7.02 (d, J = 1.3 Hz, 1H), 6.97-6.90 (m, 2H), 6.70 (br, 2H), 6.11 (d, J = 7.5 Hz, 1H), 4.99 (s, 2H), 4.30-4.20 (m, 1H), 3.86 (s, 3H), 3.50-3.35 (m, 4H), 2.68-2.50 (m, 4H), 2.36 (s, 3H), 1.54-1.37 (m, 4H), 1.35-1.19 (m, 4H), 0.85 (t, J = 7.3 Hz, 6H).

### Example I-134

### 6-(4-((4-acetylpiperazin-1-yl)methyl)benzyl)-2-amino-4-(butylamino)pyrido[4,3-d]pyrimidin-5(6H)-one

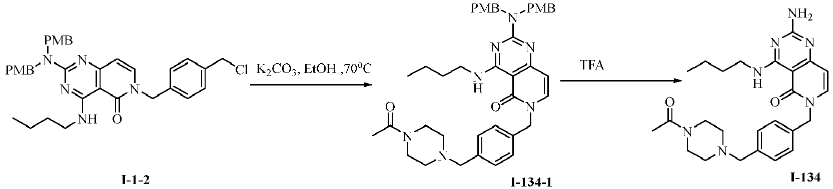

The title compound **I-134** was prepared in analogy to the preparation of compound **I-1** by using 1-(piperazin-1-yl)ethan-1-one instead of pyrrolidine. MS: 464.2 (M+H)⁺. ¹H NMR (400 MHz, DMSO-*d₆*) δ 10.40 (s, 1H), 10.12 (t, J = 5.8 Hz, 1H), 8.62-8.25 (m, 2H), 8.20 (d, J = 7.4 Hz, 1H), 7.46 (d, J = 7.8 Hz, 2H), 7.37 (d, J = 7.8 Hz, 2H), 6.43 (d, J = 7.4 Hz, 1H), 5.21 (s, 2H), 4.65-4.35 (m, 2H), 4.30 (s, 2H), 3.97 (s, 1H), 3.52 (q, J = 6.7 Hz, 2H), 3.45-2.75 (m, 6H), 2.01 (s, 3H), 1.56 (p, J = 7.1 Hz, 2H), 1.33 (h, J = 7.4 Hz, 2H), 0.90 (t, J = 7.3 Hz, 3H).

### Example I-135

### 7-amino-5-(butylamino)-3-(4-(piperazine-1-carbonyl)benzyl)pyrimido[4,5-d]pyrimidin-4(3H)-one

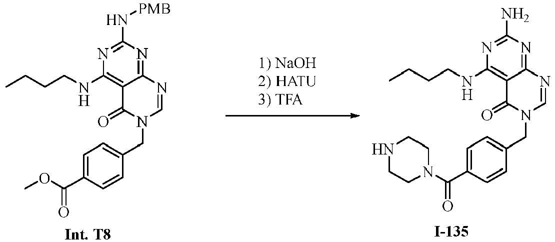

The title compound **I-135** was prepared in analogy to the preparation of compound **I-132** by using **Intermediate Int.T8** instead of **Intermediate I-132-1**. MS: 437.2 (M+H)⁺. ¹H NMR (400 MHz, DMSO-*d₆*) δ 9.41 (t, J = 5.9 Hz, 1H), 9.14 (s, 2H), 8.96 (s, 1H), 8.57 (s, 1H), 8.28 (s, 1H), 7.45 (s, 4H), 5.21 (s, 2H), 3.94 - 3.36 (m, 6H), 3.14 (s, 4H), 1.62 - 1.47 (m, 2H), 1.31 (q, J = 7.5 Hz, 2H), 0.89 (t, J = 7.3 Hz, 3H).

### Example I-136

### 2-amino-4-(heptan-4-ylamino)-6-(4-(4-(2-hydroxyethyl)piperazine-1-carbonyl)-2-methoxybenzyl)pyrido[4,3-d]pyrimidin-5(6H)-one

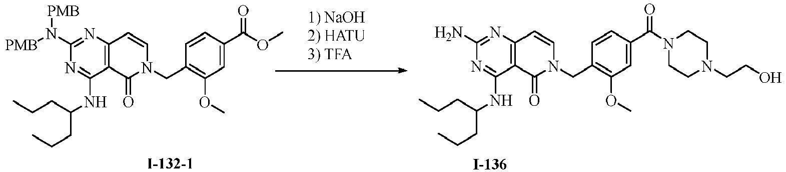

The title compound **I-136** was prepared in analogy to the preparation of compound **I-132** by using 2-(piperazin-1-yl)ethan-1-ol instead of tert-butyl piperazine-1-carboxylate. MS: 552.2 (M+H)⁺. ¹H NMR (400 MHz, DMSO-*d₆*) δ 10.02 (d, J = 7.3 Hz, 1H ) 8.08 (d, J = 7.3 Hz, 1H), 7.14 (d, J = 7.7 Hz, 1H), 7.09 (s, 1H), 7.00 (dd, J = 7.5, 1.5 Hz, 1H), 6.44 (d, J = 7.2 Hz, 1H), 5.11 (s, 2H), 4.34-4.26 (m, 1H), 3.86 (s, 3H), 3.75-3.70 (m, 2H), 3.60-3.55 (m, 4H), 3.22-3.06 (m, 6H), 1.60-1.45 (m, 4H), 1.35-1.20 (m, 4H), 0.87 (t, J = 7.2 Hz, 6H).

### Example I-137

### (R)-2-amino-4-(hexan-3-ylamino)-6-(4-(piperazine-1-carbonyl)benzyl)pyrido[4,3-d]pyrimidin-5(6H)-one

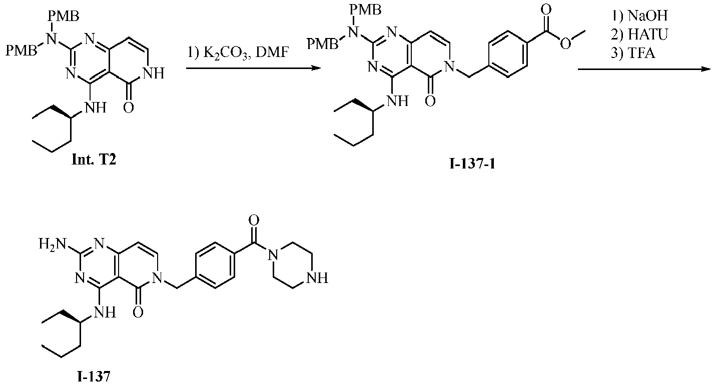

The title compound **I-137** was prepared in analogy to the preparation of compound **I-126** by using **Intermediate Int. T2** instead of **Intermediate Int. M**. MS: 464.2 (M+H)⁺. ¹H NMR (400 MHz, DMSO-*d₆*) δ 9.30 (d, J = 8.7 Hz, 1H), 8.21 (d, J = 1.0 Hz, 1H), 7.70 (d, J = 7.5 Hz, 1H), 7.38-7.29(m, 4H), 6.60 (br, 2H), 6.09 (d, J = 7.5 Hz, 1H), 5.08 (s, 2H), 4.20-4.10 (m, 1H), 3.62-3.48 (m, 2H), 3.28 (s, 2H), 2.82-2.65 (m, 4H), 1.61 - 1.37 (m, 4H), 1.35-1.20 (m, 2H), 0.86 (dt, J = 9.2, 7.3 Hz, 6H).

### Example I-138

### (R)-2-amino-4-(hexan-3-ylamino)-6-(4-(pyrrolidin-1-ylmethyl)benzyl)pyrido[4,3-d]pyrimidin-5(6H)-one

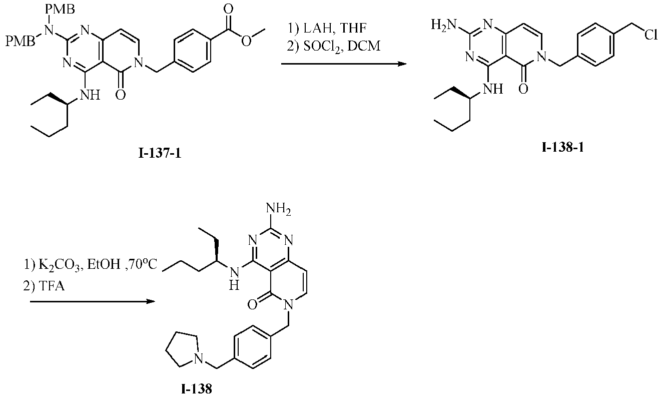

The title compound **I-138** was prepared in analogy to the preparation of compound **I-5** by using **Intermediate I-137-1** instead of **Intermediate I-5-1**. MS: 435.3 (M+H)⁺. ¹H NMR (400 MHz, DMSO-*d₆*) δ 9.33 (d, J = 8.8 Hz, 1H), 8.19 (s, 1H), 7.67 (d, J = 7.5 Hz, 1H), 7.33-7.19 (m, 4H), 6.59 (br, 2H), 6.07 (d, J = 7.5 Hz, 1H), 5.03 (s, 2H), 4.20-4.10 (m, 1H), 3.63 (s, 2H), 2.52-2.46 (m, 4H), 1.74-1.65 (m, 4H), 1.61-1.38 (m, 4H), 1.36-1.23 (m, 2H), 0.86 (dt, J = 9.0, 7.3 Hz, 6H).

### Example I-139

### (R)-2-amino-6-(4-(((2-hydroxyethyl)(methyl)amino)methyl)benzyl)-4-(pentan-2-ylamino)pyrido[4,3-d]pyrimidin-5(6H)-one

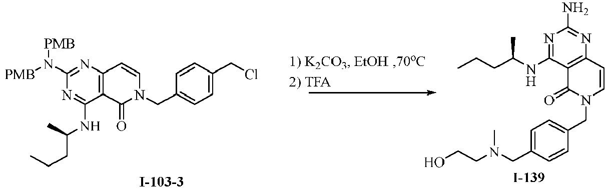

The title compound **I-139** was prepared in analogy to the preparation of compound **I-103** by using 2-(methylamino)ethan-1-ol instead of pyrrolidine. MS: 425.2 (M+H)⁺. ¹H NMR (400 MHz, DMSO-*d₆*) δ 9.33 (d, J = 8.2 Hz, 1H), 7.67 (d, J = 7.6 Hz, 1H), 7.29 - 7.17 (m, 4H), 6.60 (br, 2H), 6.07 (d, J = 7.5 Hz, 1H), 5.10 - 4.94 (m, 2H), 4.21 (p, J = 6.8 Hz, 1H), 3.49 - 3.45 (m, 2H), 3.44 (s, 2H), 2.38 (t, J = 6.4 Hz, 2H), 2.11 (s, 3H), 1.54 - 1.43 (m, 2H), 1.37 - 1.25 (m, 2H), 1.14 (d, J = 6.5 Hz, 3H), 0.88 (t, J = 7.3 Hz, 3H).

### Example I-140

### (R)-2-amino-6-(4-(((2-hydroxyethyl)(propyl)amino)methyl)benzyl)-4-(pentan-2-ylamino)pyrido[4,3-d]pyrimidin-5(6H)-one

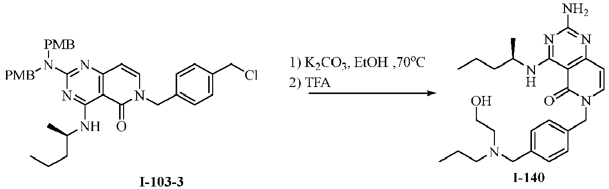

The title compound **I-140** was prepared in analogy to the preparation of compound **I-103** by using 2-(propylamino)ethan-1-ol instead of pyrrolidine. MS: 453.2 (M+H)⁺. ¹H NMR (400 MHz, DMSO-*d₆*) δ 9.34 (d, J = 8.2 Hz, 1H), 8.18 (s, 1H), 7.68 (d, J = 7.5 Hz, 1H), 7.31 - 7.15 (m, 4H), 6.63 (br, 2H), 6.07 (d, J = 7.5 Hz, 1H), 5.09 - 4.93 (m, 2H), 4.21 (p, J = 6.8 Hz, 1H), 3.55 (s, 2H), 3.44 (t, J = 6.6 Hz, 2H), 2.46 (t, J = 6.6 Hz, 2H), 2.39 - 2.33 (m, 2H), 1.53 - 1.38 (m, 4H), 1.37 - 1.26 (m, 2H), 1.14 (d, J = 6.5 Hz, 3H), 0.88 (t, J = 7.3 Hz, 3H), 0.79 (t, J = 7.3 Hz, 3H).

### Example I-141

### (R)-2-amino-6-(4-((bis(2-hydroxyethyl)amino)methyl)benzyl)-4-(pentan-2-ylamino)pyrido[4,3-d]pyrimidin-5(6H)-one

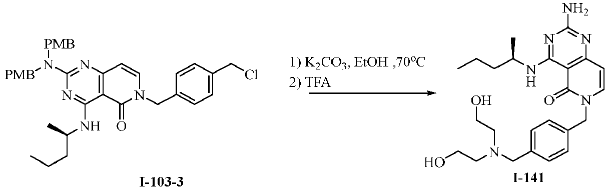

The title compound **I-141** was prepared in analogy to the preparation of compound **I-103** by using 2,2'-azanediylbis(ethan-1-ol) instead of pyrrolidine. MS: 455.2 (M+H)⁺. ¹H NMR (400 MHz, DMSO-*d₆*) δ 9.34 (d, J = 8.2 Hz, 1H), 8.15 (s, 1H), 7.68 (d, J = 7.6 Hz, 1H), 7.34 - 7.16 (m, 4H), 6.63 (br, 2H), 6.08 (d, J = 7.5 Hz, 1H), 5.09 - 4.93 (m, 2H), 4.21 (p, J = 6.8 Hz, 1H), 3.62 (s, 2H), 3.44 (t, J = 6.3 Hz, 4H), 2.53 (t, J = 6.3 Hz, 4H), 1.54 - 1.42 (m, 2H), 1.38 - 1.25 (m, 2H), 1.14 (d, J = 6.5 Hz, 3H), 0.88 (t, J = 7.3 Hz, 3H).

### Example I-142

### (S)-6-(4-((2-oxa-6-azaspiro[3.3]heptan-6-yl)methyl)benzyl)-2-amino-4-((1-hydroxyhexan-3-yl)amino)pyrido[4,3-d]pyrimidin-5(6H)-one

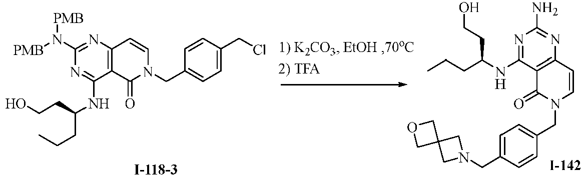

The title compound **I-142** was prepared in analogy to the preparation of compound **I-118** by using 2-oxa-6-azaspiro[3.3]heptane instead of pyrrolidine. MS: 479.3 (M+H)⁺. ¹H NMR (400 MHz, DMSO-*d₆*) δ 9.75 (s, 1H), 7.98 (s, 1H), 7.58 (s, 1H), 7.41 (d, J = 8.2 Hz, 2H), 7.33 (d, J = 7.9 Hz, 2H), 6.29 (d, J = 7.4 Hz, 1H), 5.19 - 5.08 (m, 2H), 4.70 - 4.10 (m, 13H), 3.44 (t, J = 6.6 Hz, 2H), 1.79 - 1.45 (m, 4H), 1.38 - 1.21 (m, 2H), 0.87 (t, J = 7.3 Hz, 3H).

### Example I-143

### (S)-2-amino-4-((1-hydroxyhexan-3-yl)amino)-6-(4-((4-methylpiperazin-1-yl)methyl)benzyl)pyrido[4,3-d]pyrimidin-5(6H)-one

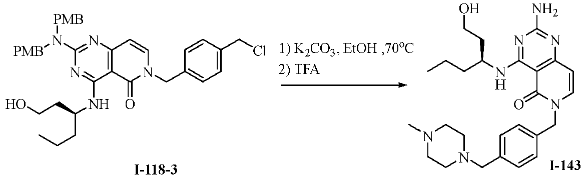

The title compound **I-143** was prepared in analogy to the preparation of compound **I-118** by using 1-methylpiperazine instead of pyrrolidine. MS: 480.6 (M+H)⁺.

### Example I-144

### (S)-2-amino-4-((1-hydroxyhexan-3-yl)amino)-6-(4-(piperazin-1-ylmethyl)benzyl)pyrido[4,3-d]pyrimidin-5(6H)-one

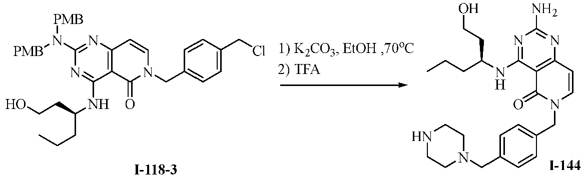

The title compound **I-144** was prepared in analogy to the preparation of compound **I-118** by using tert-butyl piperazine-1-carboxylate instead of pyrrolidine. MS: 466.6 (M+H)⁺. ¹H NMR (400 MHz, DMSO-*d₆*) δ 10.11 (d, J = 8.8 Hz, 1H), 9.03 (s, 2H), 8.55 - 8.16 (m, 3H), 7.45 - 7.27 (m, 4H), 6.43 (d, J = 7.4 Hz, 1H), 5.18 (s, 2H), 4.91 - 3.06 (m, 10H), 2.84 (s, 3H), 1.82 - 1.48 (m, 4H), 1.39 - 1.22 (m, 2H), 0.88 (t, J = 7.3 Hz, 3H).

### Example I-147

### 7-amino-5-(butylamino)-3-(4-(piperazin-1-ylmethyl)benzyl)pyrimido[4,5-d]pyrimidin-4(3H)-one

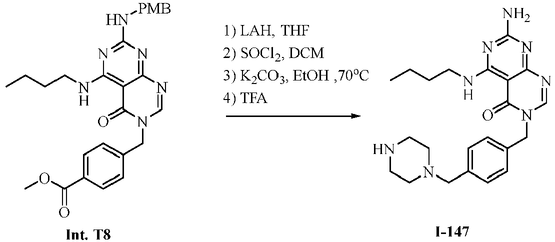

The title compound **I-147** was prepared in analogy to the preparation of compound **I-107** by using **Intermediate Int.T8** instead of **Intermediate I-107-1**. MS: 423.2 (M+H)⁺. ¹H NMR (400 MHz, DMSO-*d₆*) δ 9.42 (s, 1H), 8.95 (s, 1H), 8.81 (s, 2H), 8.56 (s, 1H), 7.91 (s, 1H), 7.38 (s, 4H), 5.16 (s, 2H), 3.51 (q, J = 6.7 Hz, 2H), 3.17 (s, 4H), 2.78 (s, 4H), 1.55 (q, J = 7.3 Hz, 2H), 1.30 (dt, J = 14.6, 7.4 Hz, 2H), 0.90 (t, J = 7.4 Hz, 3H).

### Example I-148

### (R)-2-amino-6-(4-((4-(2-hydroxyethyl)piperazin-1-yl)methyl)benzyl)-4-(pentan-2-ylamino)pyrido[4,3-d]pyrimidin-5(6H)-one

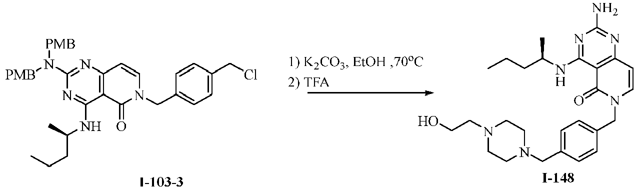

The title compound **I-148** was prepared in analogy to the preparation of compound **I-103** by using 2-(piperazin-1-yl)ethan-1-ol instead of pyrrolidine. MS: 480.2 (M+H)⁺. ¹H NMR (400 MHz, DMSO-*d₆*) δ 9.34 (d, J = 8.2 Hz, 1H), 8.20 (s, 1H), 7.68 (d, J = 7.5 Hz, 1H), 7.35 - 7.14 (m, 4H), 6.68 (s, 2H), 6.08 (d, J = 7.5 Hz, 1H), 5.09 - 4.94 (m, 2H), 4.31 - 4.12 (m, 1H), 3.50 (t, J = 6.1 Hz, 2H), 3.43 (s, 2H), 2.67 - 2.49 (m, 6H), 2.39 (s, 4H), 1.56 - 1.40 (m, 2H), 1.39 - 1.23 (m, 2H), 1.14 (d, J = 6.5 Hz, 3H), 0.87 (t, J = 7.2 Hz, 3H).

### Example I-149

### 2-amino-4-(butylthio)-6-(4-(piperazin-1-ylmethyl)benzyl)pyrido[4,3-d]pyrimidin-5(6H)-one

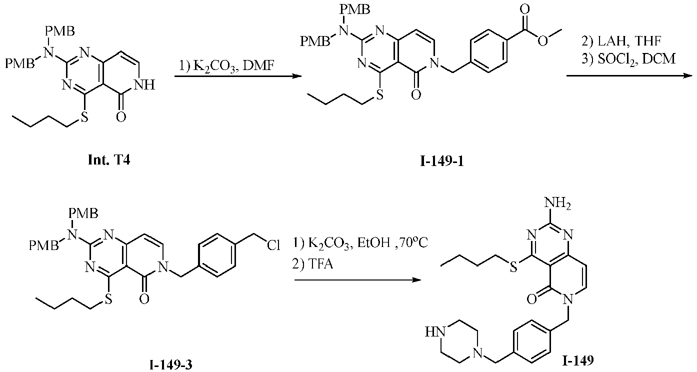

The title compound **I-149** was prepared in analogy to the preparation of compound **I-1** by using **Intermediate Int. T4** instead of **Intermediate Int. A**. MS: 439.3 (M+H)⁺. ¹H NMR (400 MHz, DMSO-*d₆*) δ 8.78 (d, J = 4.3 Hz, 1H), 8.12 (d, J = 4.3 Hz, 1H), 7.92 (d, J = 7.6 Hz, 1H), 7.30-7.23 (m, 4H), 6.32 (d, J = 7.4 Hz, 1H), 5.10 (s, 2H), 3.47 (s, 2H), 3.07 (t, J = 7.3 Hz, 2H), 3.03-2.90 (m, 4H), 2.50-2.41 (m, 4H), 1.68 - 1.54 (m, 2H), 1.44-1.37 (m, 2H), 0.90 (t, J = 7.3 Hz, 3H).

### Example I-150

### (R)-6-(4-((2-oxa-6-azaspiro[3.3]heptan-6-yl)methyl)benzyl)-2-amino-4-(pentan-2-ylamino)pyrido[4,3-d]pyrimidin-5(6H)-one

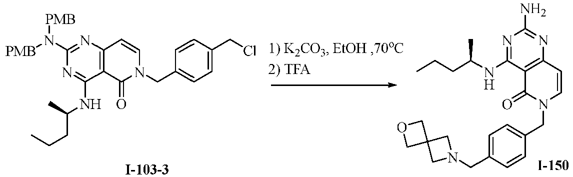

The title compound **I-150** was prepared in analogy to the preparation of compound **I-103** by using 2-oxa-6-azaspiro[3.3]heptane instead of pyrrolidine. MS: 449.3 (M+H)⁺. ¹H NMR (400 MHz, DMSO-*d₆*) δ 9.33 (d, J = 8.2 Hz, 1H), 8.17 (s, 2H), 7.68 (d, J = 7.5 Hz, 1H), 7.26 - 7.16 (m, 4H), 6.65 (s, 2H), 6.08 (d, J = 7.5 Hz, 1H), 5.10 - 4.93 (m, 2H), 4.58 (s, 4H), 4.27 - 4.15 (m, 1H), 3.55 (s, 2H), 2.51 (d, J = 1.8 Hz, 1H), 1.52 - 1.42 (m, 2H), 1.39 - 1.24 (m, 2H), 1.14 (d, J = 6.5 Hz, 3H), 0.88 (t, J = 7.3 Hz, 3H).

### Example I-151

### 6-(4-(3,6-diazabicyclo[3.1.1]heptane-3-carbonyl)benzyl)-2-amino-4-(((R)-pentan-2-yl)amino)pyrido[4,3-d]pyrimidin-5(6H)-one

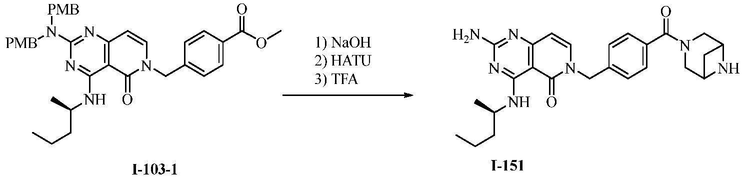

The title compound **I-151** was prepared in analogy to the preparation of compound **I-117** by using tert-butyl 3,6-diazabicyclo[3.1.1]heptane-6-carboxylate instead of tert-butyl piperazine-1-carboxylate. MS: 462.2 (M+H)⁺. ¹H NMR (400 MHz, DMSO-*d₆*) δ 10.03 (d, J = 8.3 Hz, 1H), 9.74 (s, 1H), 8.37 (s, 3H), 8.22 (d, J = 7.4 Hz, 1H), 7.47 (d, J = 8.2 Hz, 2H), 7.39 (d, J = 8.0 Hz, 2H), 6.44 (d, J = 7.4 Hz, 1H), 5.23 (q, J = 15.0 Hz, 2H), 4.48 - 2.77 (m, 7H), 1.82 (dd, J = 10.4, 4.6 Hz, 1H), 1.63 - 1.48 (m, 2H), 1.32 (dtd, J = 10.4, 6.7, 3.3 Hz, 2H), 1.21 (d, J = 6.5 Hz, 3H), 0.89 (t, J = 7.3 Hz, 3H).

### Example I-152

### (R)-2-amino-6-(2-chloro-4-(pyrrolidin-1-ylmethyl)benzyl)-4-(pentan-2-ylamino)pyrido[4,3-d]pyrimidin-5(6H)-one

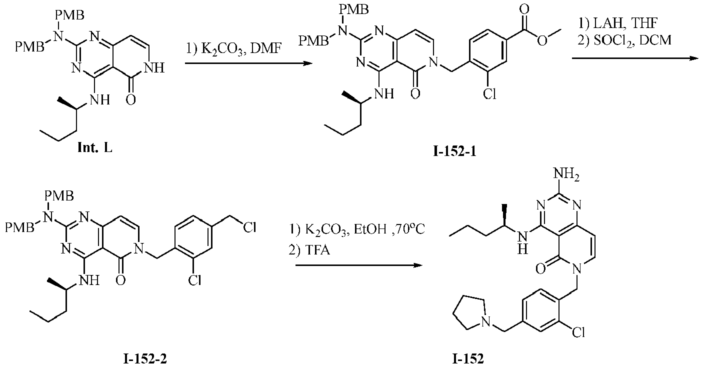

The title compound **I-152** was prepared in analogy to the preparation of compound **I-5** by using **Intermediate Int. L** instead of **Intermediate Int. A** and methyl 4-(bromomethyl)-3-chlorobenzoate instead of methyl 4-(bromomethyl)-2-methoxybenzoate. MS: 456.3 (M+H)⁺. ¹H NMR (400 MHz, DMSO-*d₆*) δ 9.24 (d, J = 8.2 Hz, 1H), 8.17 (s, 1H), 7.61 (d, J = 7.6 Hz, 1H), 7.44 (d, J = 1.6 Hz, 1H), 7.24 (dd, J = 7.9, 1.7 Hz, 1H), 6.86 (d, J = 7.9 Hz, 1H), 6.67 (br, 2H), 6.13 (d, J = 7.5 Hz, 1H), 5.09 (q, J = 16.0 Hz, 2H), 4.25-4.15 (m, 1H), 3.62 (s, 2H), 2.49-2.44 (m, 4H), 1.73-1.66 (m, 4H), 1.50-1.42 (m, 2H), 1.36-1.24 (m, 2H), 1.13 (d, J = 6.5 Hz, 3H), 0.87 (t, J = 7.3 Hz, 3H).

### Example I-153

### (R)-2-amino-6-((6-(2-(methylamino)ethoxy)pyridin-3-yl)methyl)-4-(pentan-2-ylamino)pyrido[4,3-d]pyrimidin-5(6H)-one

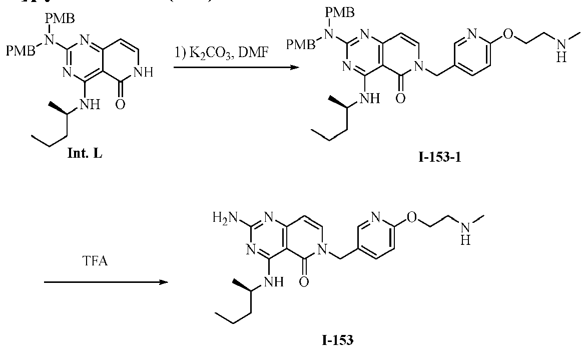

The title compound **I-153** was prepared in analogy to the preparation of compound **I-152** by using 2-((5-(bromomethyl)pyridin-2-yl)oxy)-N-methylethan-1-amine instead of methyl 4-(bromomethyl)-3-chlorobenzoate. MS: 412.3 (M+H)⁺. ¹H NMR (400 MHz, DMSO-*d₆*) δ 10.03 (d, J= 8.6 Hz, 1H), 8.69 (s, 1H), 8.26-8.13 (m 3H), 7.76 (d, J = 8.6 Hz, 1H), 6.85 (d, J = 8.6 Hz, 1H), 6.42 (d, J = 7.4 Hz, 1H), 5.13 (q, J = 14.6 Hz, 2H), 4.45 (d, J = 5.8 Hz, 2H), 4.32-4.22 (m, 1H), 3.35-3.26 (m, 2H), 2.61 (s, 3H), 1.61-1.50 (m, 2H), 1.37-1.26 (m, 2H), 1.21 (d, J = 6.5 Hz, 3H), 0.89 (t, J = 7.3 Hz, 3H).

### Example I-154

### (R)-2-amino-6-(2-methoxy-4-(pyrrolidin-1-ylmethyl)benzyl)-4-(pentan-2-ylamino)pyrido[4,3-d]pyrimidin-5(6H)-one

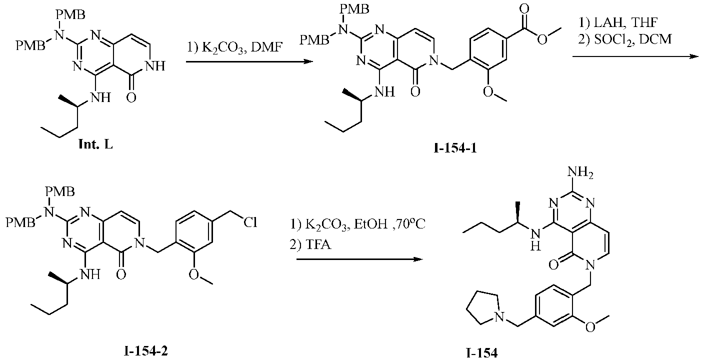

The title compound **I-154** was prepared in analogy to the preparation of compound **I-5** by using **Intermediate Int. L** instead of **Intermediate Int. A.** MS: 451.3 (M+H)⁺. ¹H NMR (400 MHz, DMSO-*d₆*) δ 9.32 (d, J = 8.2 Hz, 1H), 8.19 (s, 1H), 7.56 (d, J = 7.5 Hz, 1H), 6.98 (s, 1H), 6.85 (d, J = 1.5 Hz, 2H), 6.59 (br, 2H), 6.07 (d, J = 7.5 Hz, 1H), 5.02-4.87 (m, 2H), 4.20 (p, J = 6.8 Hz, 1H), 3.82 (s, 3H), 3.60 (s, 2H), 2.49-2.45 (m, 4H), 1.73-1.65 (m, 4H), 1.52-1.43 (m, 2H), 1.38-1.22 (m, 2H), 1.13 (d, J = 6.5 Hz, 3H), 0.87 (t, J = 7.2 Hz, 3H).

### Example I-156

### 2-amino-4-(butylamino)-6-((5-(pyrrolidin-1-ylmethyl)pyrimidin-2-yl)methyl)pyrido[4,3-d]pyrimidin-5(6H)-one

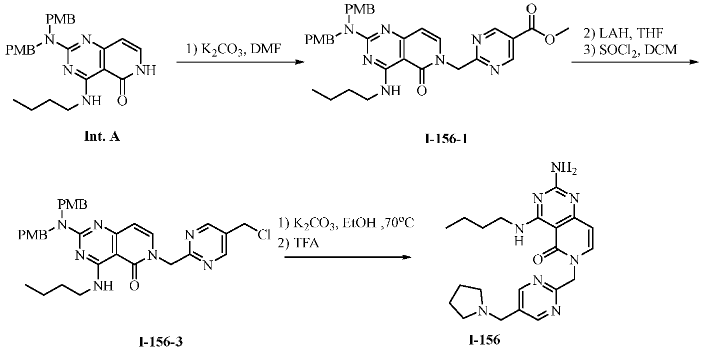

The title compound **I-156** was prepared in analogy to the preparation of compound **I-2** by using methyl 2-(bromomethyl)pyrimidine-5-carboxylate instead of methyl 4-(bromomethyl)-3-methoxybenzoate. MS: 409.2 (M+H)⁺.

### Example I-157

### (R)-2-amino-4-((1-hydroxypentan-2-yl)amino)-6-(2-methoxy-4-(pyrrolidin-1-ylmethyl)benzyl)pyrido[4,3-d]pyrimidin-5(6H)-one

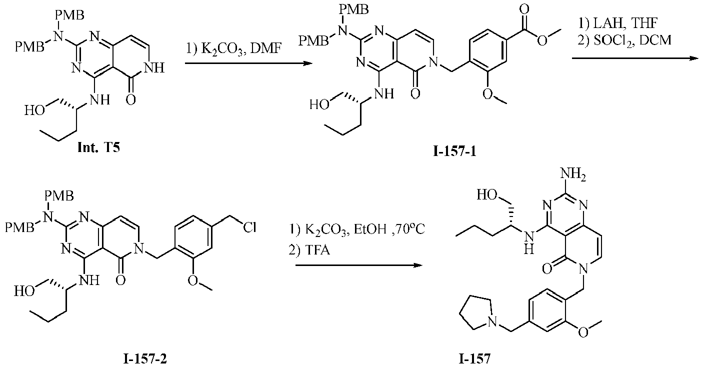

The title compound **I-157** was prepared in analogy to the preparation of compound **I-5** by using **Intermediate Int. T5** instead of **Intermediate Int. A**. MS: 467.3 (M+H)⁺.

### Example I-158

### (S)-2-amino-4-((1-hydroxypentan-2-yl)amino)-6-(2-methoxy-4-(pyrrolidin-1-ylmethyl)benzyl)pyrido[4,3-d]pyrimidin-5(6H)-one

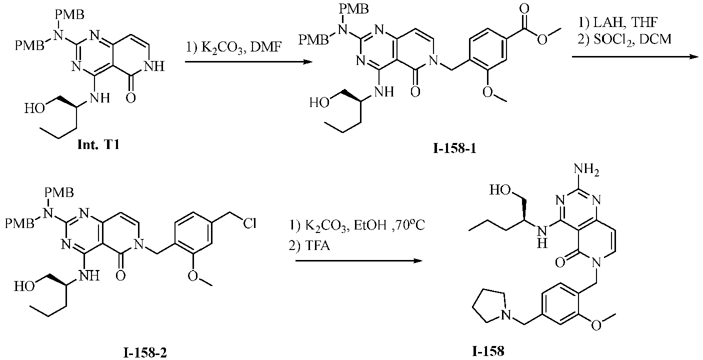

The title compound **I-158** was prepared in analogy to the preparation of compound **I-5** by using **Intermediate Int. T1** instead of **Intermediate Int. A**. MS: 467.3 (M+H)⁺.

### Example I-159

### (R)-2-amino-6-(2-methyl-4-(pyrrolidin-1-ylmethyl)benzyl)-4-(pentan-2-ylamino)pyrido[4,3-d]pyrimidin-5(6H)-one

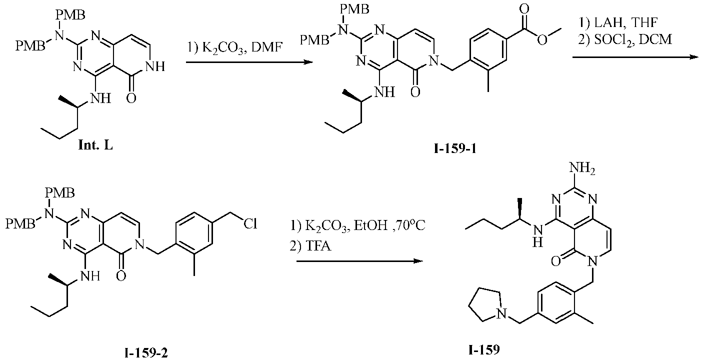

The title compound **I-159** was prepared in analogy to the preparation of compound **I-5** by using **Intermediate Int. L** instead of **Intermediate Int. A** and methyl 4-(bromomethyl)-3-methylbenzoate instead of methyl 4-(bromomethyl)-3-methoxybenzoate. MS: 435.3 (M+H)⁺. ¹H NMR (400 MHz, DMSO-*d₆*) δ 9.33 (d, J = 8.2 Hz, 1H), 8.19 (s, 1H), 7.51 (d, J = 7.6 Hz, 1H), 7.18 (s, 1H), 7.10 (d, J = 7.8, 1H), 6.74 (d, J = 7.8 Hz, 1H), 6.64 (br, 2H), 6.11 (d, J = 7.5 Hz, 1H), 5.02 (q, J = 15.7 Hz, 2H), 4.25-4.15 (m, 1H), 3.62 (s, 2H), 2.55-2.52 (m, 4H), 2.29 (s, 3H), 1.74-1.67 (m, 4H), 1.51-1.43 (m, 2H), 1.37-1.26 (m, 2H), 1.14 (d, J = 6.5 Hz, 3H), 0.87 (t, J = 7.3 Hz, 3H).

### Example I-160

### (R)-2-amino-6-(4-(((2-hydroxyethyl)(methyl)amino)methyl)-2-methoxybenzyl)-4-(pentan-2-ylamino)pyrido[4,3-d]pyrimidin-5(6H)-one

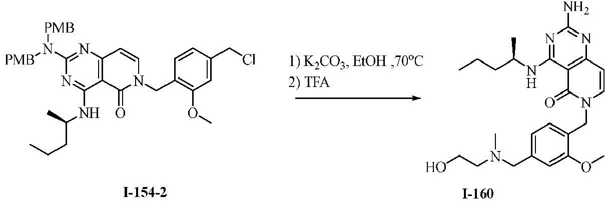

The title compound **I-160** was prepared in analogy to the preparation of compound **I-154** by using 2-(methylamino)ethan-1-ol instead of pyrrolidine. MS: 455.3 (M+H)⁺. ¹H NMR (400 MHz, DMSO-*d₆*) δ 9.32 (d, J = 8.2 Hz, 1H), 8.17 (s, 1H), 7.57 (d, J = 7.5 Hz, 1H), 7.01 (s, 1H), 6.87-6.82 (M, 2H), 6.62 (br, 2H), 6.07 (d, J = 7.5 Hz, 1H), 5.01-4.87 (m, 2H), 4.25-4.15 (m, 1H), 3.82 (s, 3H), 3.54-3.48 (m, 4H), 2.48-2.41 (m, 2H), 2.20 (s, 3H), 1.51-1.42 (m, 2H), 1.36-1.25 (m, 2H), 1.13 (d, J = 6.4 Hz, 3H), 0.87 (t, J = 7.2 Hz, 3H).

### Example I-161

### 6-(4-(3,6-diazabicyclo[3.1.1]heptane-6-carbonyl)benzyl)-2-amino-4-(((R)-pentan-2-yl)amino)pyrido[4,3-d]pyrimidin-5(6H)-one

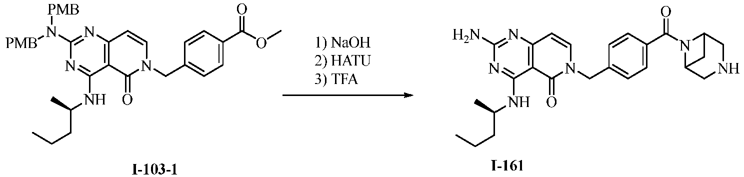

The title compound **I-161** was prepared in analogy to the preparation of compound **I-117** by using tert-butyl 3,6-diazabicyclo[3.1.1]heptane-3-carboxylate instead of tert-butyl piperazine-1-carboxylate. MS: 462.2 (M+H)⁺. ¹H NMR (400 MHz, DMSO-*d₆*) δ 10.02 (t, J = 8.4 Hz, 1H), 9.66-9.29 (m , 2H), 8.51 - 8.15 (m, 3H), 7.70 - 7.63 (m, 1H), 7.47 (d, J = 8.2 Hz, 1H), 7.39 (dd, J = 8.2, 3.7 Hz, 2H), 6.45 (d, J = 7.4 Hz, 1H), 5.24 (qd, J = 15.1, 5.3 Hz, 2H), 4.71 - 2.77 (m, 7H), 1.86 (d, J = 9.7 Hz, 1H), 1.55 (dq, J = 8.7, 6.4 Hz, 2H), 1.40 - 1.26 (m, 2H), 1.21 (dd, J = 6.5, 1.8 Hz, 3H), 0.89 (td, J = 7.3, 1.3 Hz, 3H).

### Example I-162

### (S)-2-amino-4-((1-hydroxypentan-2-yl)amino)-6-(2-methoxy-4-((4-methylpiperazin-1-yl)methyl)benzyl)pyrido[4,3-d]pyrimidin-5(6H)-one

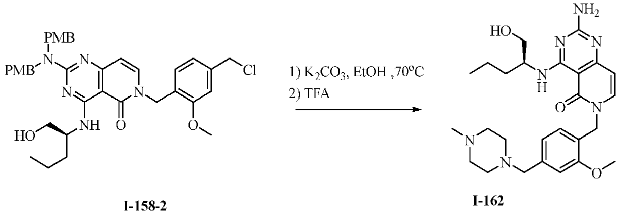

The title compound **I-162** was prepared in analogy to the preparation of compound **I-158** by using 1-methylpiperazine instead of pyrrolidine. MS: 496.3 (M+H)⁺. ¹H NMR (400 MHz, DMSO-*d₆*) δ 9.77 (s, 1H), 7.80 (s, 1H), 7.36 (s, 1H), 6.97 (d, J = 8.1 Hz, 2H), 6.86 (d, J = 7.6 Hz, 1H), 6.23 (d, J = 7.5 Hz, 1H), 5.08 - 4.84 (m, 3H), 4.26 - 4.16 (m, 1H), 3.83 (s, 3H), 3.61 - 2.59 (m, 16H), 1.60-1.43 (m, 2H), 1.35-1.28 (m, 2H), 0.88 (t, J = 7.3 Hz, 3H).

### Example I-163

### (S)-2-amino-6-(2-methoxy-4-(pyrrolidin-1-ylmethyl)benzyl)-4-((1-methoxypentan-2-yl)amino)pyrido[4,3-d]pyrimidin-5(6H)-one

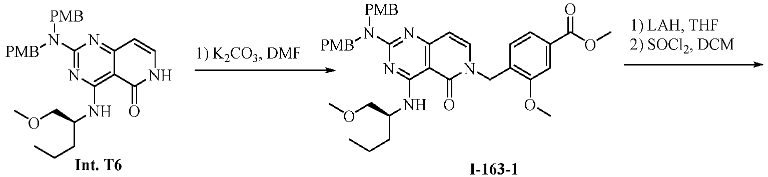

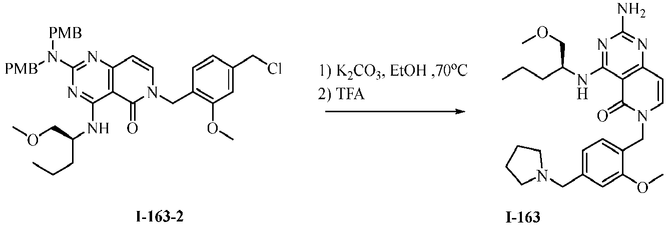

The title compound **I-163** was prepared in analogy to the preparation of compound **I-5** by using **Intermediate Int. T6** instead of **Intermediate Int. A**. MS: 481.3 (M+H)⁺.

### Example I-164

### (S)-2-amino-4-((1-hydroxyhexan-2-yl)amino)-6-(2-methoxy-4-(pyrrolidin-1-ylmethyl)benzyl)pyrido[4,3-d]pyrimidin-5(6H)-one

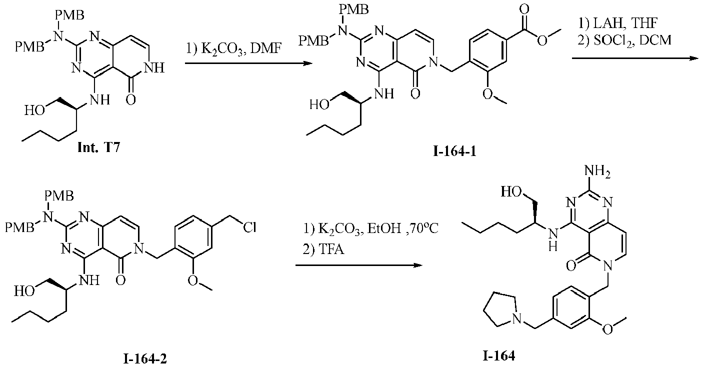

The title compound **I-164** was prepared in analogy to the preparation of compound **I-5** by using **Intermediate Int. T7** instead of **Intermediate Int. A**. MS: 481.3 (M+H)⁺. ¹H NMR (400 MHz, DMSO-*d₆*) δ 13.39 (s, 1H), 10.22 (s, 1H), 10.17 (d, J = 8.7 Hz, 1H), 8.30 (s, 2H), 8.09 (d, J = 7.5 Hz, 1H), 7.23 (d, J = 1.6 Hz, 1H), 7.12 (d, J = 7.7 Hz, 1H), 7.05 (dd, J = 7.6, 1.5 Hz, 1H), 6.42 (d, J = 7.5 Hz, 1H), 5.18 - 5.02 (m, 2H), 4.32 (d, J = 5.0 Hz, 2H), 4.23 (s, 1H), 3.91 - 3.81 (m, 3H), 3.57 - 3.44 (m, 2H), 3.36 (s, 2H), 3.08 (s, 2H), 2.11 - 1.95 (m, 2H), 1.93 - 1.77 (m, 2H), 1.72 - 1.58 (m, 1H), 1.58 - 1.46 (m, 1H), 1.38 - 1.18 (m, 4H), 0.89 - 0.80 (m, 3H).

### Example I-165

### (R)-2-amino-6-(4-(((2-hydroxyethyl)(propyl)amino)methyl)-2-methoxybenzyl)-4-(pentan-2-ylamino)pyrido[4,3-d]pyrimidin-5(6H)-one

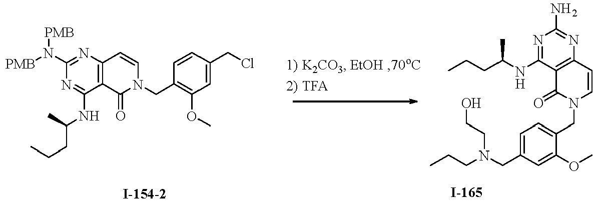

The title compound **I-165** was prepared in analogy to the preparation of compound **I-154** by using 2-(propylamino)ethan-1-ol instead of pyrrolidine. MS: 483.3 (M+H)⁺. ¹H NMR (400 MHz, DMSO-*d₆*) δ 9.33 (d, J = 8.2 Hz, 1H), 8.16 (s, 1H), 7.56 (d, J = 7.6 Hz, 1H), 7.01 (s, 1H), 6.84 (s, 2H), 6.60 (br, 2H), 6.07 (d, J = 7.5 Hz, 1H), 5.03- 4.86 (m, 2H), 4.25-4.15 (m, 1H), 3.81 (s, 3H), 3.57 (s, 2H), 3.46 (t, J = 6.5 Hz, 2H), 2.50-2.47 (m, 2H), 2.42-2.36 (m, 2H), 1.51-1.38 (m, 4H), 1.35-1.25 (m, 2H), 1.14 (d, J = 6.5 Hz, 3H), 0.87 (t, J = 7.3 Hz, 3H), 0.82 (t, J = 7.3 Hz, 3H).

### Example I-166

### (R)-2-amino-6-(4-((bis(2-hydroxyethyl)amino)methyl)-2-methoxybenzyl)-4-(pentan-2-ylamino)pyrido[4,3-d]pyrimidin-5(6H)-one

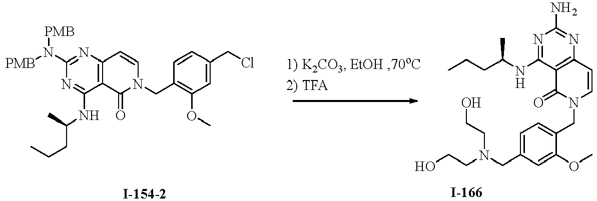

The title compound **I-166** was prepared in analogy to the preparation of compound **I-154** by using 2,2'-azanediylbis(ethan-1-ol) instead of pyrrolidine. MS: 485.3 (M+H)⁺. ¹H NMR (400 MHz, DMSO-*d₆*) δ 9.32 (d, J = 8.2 Hz, 1H), 8.16 (s, 1H), 7.56 (d, J = 7.5 Hz, 1H), 7.04 (s, 1H), 6.84 (s, 2H), 6.59 (br, 2H), 6.07 (d, J = 7.5 Hz, 1H), 5.04-4.84 (m, 2H), 4.25-4.15 (m, 1H), 3.82(s, 3H), 3.62 (s, 2H), 3.45 (t, J = 6.3 Hz, 4H), 2.55-2.50 (m, 4H), 1.52-1.43 (m, 2H), 1.38-1.24 (m, 2H), 1.14 (d, J = 6.5 Hz, 3H), 0.88 (t, J = 7.3 Hz, 3H).

### Example I-168

### (S)-2-amino-6-(2-chloro-4-(pyrrolidin-1-ylmethyl)benzyl)-4-((1-hydroxypentan-2-yl)amino)pyrido[4,3-d]pyrimidin-5(6H)-one

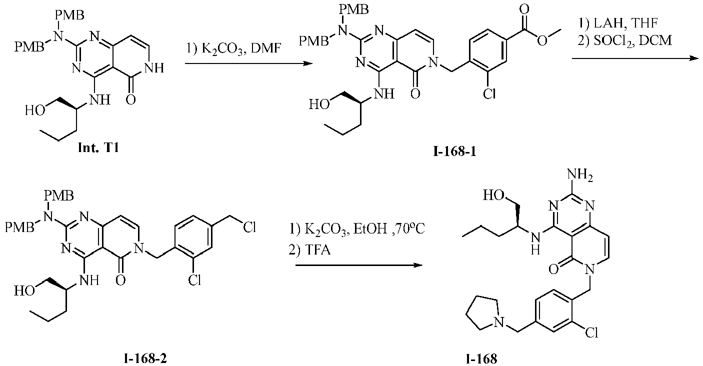

The title compound **I-168** was prepared in analogy to the preparation of compound **I-158** by using methyl 4-(bromomethyl)-3-chlorobenzoate instead of methyl 4-(bromomethyl)-3-methoxybenzoate. MS: 472.3 (M+H)⁺. ¹H NMR (400 MHz, DMSO-*d₆*) δ 9.33 (d, J = 8.5 Hz, 1H), 8.17 (s, 1H), 7.62 (d, J = 7.5 Hz, 1H), 7.44 (s, 1H), 6.87 (d, J = 7.9 Hz, 1H), 6.63 (s, 2H), 6.12 (d, J = 7.5 Hz, 1H), 5.09 (q, J = 16.0 Hz, 2H), 4.24 - 4.15 (m, 1H), 3.63 (s, 2H), 3.50 - 3.38 (m, 2H), 1.83 - 1.66 (m, 4H), 1.66 - 1.37 (m, 2H), 1.38 - 1.22 (m, 1H), 0.87 (t, J = 7.3 Hz, 3H).

### Example I-169

### (S)-2-amino-4-((1-hydroxypentan-2-yl)amino)-6-(2-methyl-4-(pyrrolidin-1-ylmethyl)benzyl)pyrido[4,3-d]pyrimidin-5(6H)-one

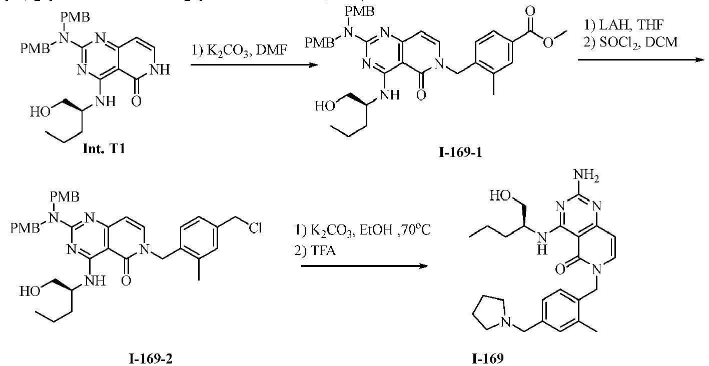

The title compound **1-169** was prepared in analogy to the preparation of compound **I-158** by using methyl 4-(bromomethyl)-3-methylbenzoate instead of methyl 4-(bromomethyl)-3-methoxybenzoate. MS: 451.3 (M+H)⁺. ¹H NMR (400 MHz, DMSO-*d₆*) *δ* 9.42 (d, J = 8.5 Hz, 1H), 8.20 (s, 1H), 7.51 (d, J = 7.5 Hz, 1H), 7.18 (s, 1H), 7.10 (d, J = 6.1 Hz, 1H), 6.75 (d, J = 7.8 Hz, 1H), 6.61 (s, 2H), 6.10 (d, J = 7.5 Hz, 1H), 5.10 - 4.93 (m, 2H), 4.25 - 4.15 (m, 1H), 3.52 - 3.37 (m, 2H), 2.54 (s, 3H), 2.51 (d, J = 1.9 Hz, 1H), 2.49 (d, J = 1.9 Hz, 1H), 2.30 (s, 3H), 1.75 - 1.67 (m, 4H), 1.65 - 1.52 (m, 1H), 1.51 - 1.37 (m, 1H), 1.39 - 1.23 (m, 2H), 0.87 (t, J = 7.3 Hz, 3H).

### Example 1-170

### 2-amino-4-((1-hydroxyhexan-3-yl)oxy)-6-((6-(2-(methylamino)ethoxy)pyridin-3-yl)methyl)pyrido[4,3-d]pyrimidin-5(6H)-one

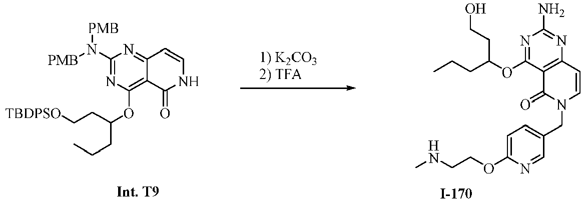

The title compound **1-170** was prepared in analogy to the preparation of compound **I-158** by using **Intermediate Int.T9** instead of **Intermediate Int. T1** and 2-((5-(bromomethyl)pyridin-2-yl)oxy)-N-methylethan-1-amine instead of methyl 4-(bromomethyl)-3-methylbenzoate. MS: 443.3 (M+H)⁺.

### Example 1-171

### 2-((4-((2-amino-4-(butylamino)-5-oxopyrido[4,3-d]pyrimidin-6(5H)-yl)methyl)benzyl)(methyl)amino)ethyl diethylcarbamate

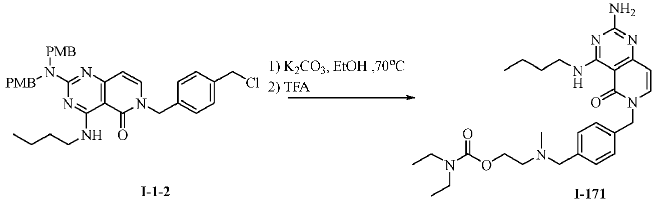

The title compound **1-171** was prepared in analogy to the preparation of compound **I-1** by using 2-(methylamino)ethyl diethylcarbamate instead of pyrrolidine. MS: 510.3 (M+H)⁺. ¹H NMR (400 MHz, DMSO-*d*₆) δ 10.12 (t, J = 5.8 Hz, 1H), 10.10-9.92 (brs, 1H), 8.55-8.25 (m, 2H), 8.20 (d, J = 7.4 Hz, 1H), 7.50 (d, J = 7.8 Hz, 2H), 7.38 (d, J = 7.8 Hz, 2H), 6.43 (d, J = 7.4 Hz, 1H), 5.21 (s, 2H), 4.44-4.21 (m, 4H), 3.53 (q, J = 6.7 Hz, 2H), 3.34 (s, 2H), 3.20 (q, J = 7.1 Hz, 5H), 2.74 (s, 3H), 1.56 (m, 2H), 1.34 (m, 2H), 1.08-0.96 (m, 6H), 0.91 (t, J = 7.3 Hz, 3H).

### Example 1-174

### 4-(butylamino)-6-(4-(piperazin-1-ylmethyl)benzyl)pyrido[4,3-d]pyrimidin-56H-one

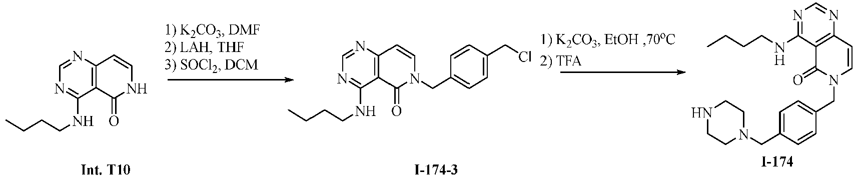

The title compound **1-174** was prepared in analogy to the preparation of compound **1-1** by using **Intermediate Int. T10** instead of **Intermediate Int. A** and tert-butyl piperazine-1-carboxylate instead of pyrrolidine. MS: 407.3 (M+H)⁺.

### Example 1-175

### diethyl (4-(4-(4-((2-amino-4-(butylamino)-5-oxopyrido[4,3-d]pyrimidin-6(5H)-yl)methyl)benzyl)piperazin-1-yl)butyl)phosphonate

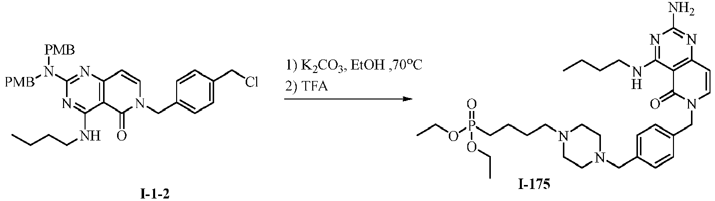

The title compound **1-175** was prepared in analogy to the preparation of compound **1-1** by using diethyl (4-(piperazin-1-yl)butyl)phosphonate instead of pyrrolidine. MS: 614.3 (M+H)⁺.

### Example 1-176

### (S)-(4-(4-(4-((2-amino-4-((1-hydroxypentan-2-yl)amino)-5-oxopyrido [4,3-d]pyrimidin-6(SH)-yl)methyl)benzyl)piperazin-1-yl)butyl)phosphonic acid

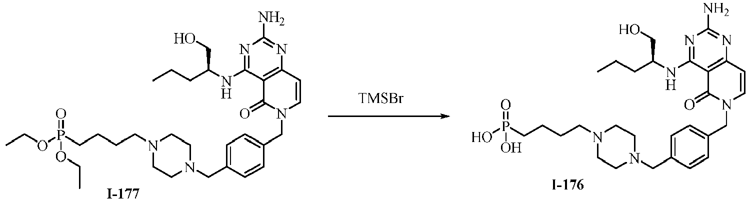

A mixture solution of **1-177** and TMSBr in DCM was stirred at 25 °C for 5 hours. After the reaction was completed, the reaction mixture was concentrated in vacuo to give a crude product, which was purified by pre-HPLC to afford **1-176.** MS: 588.3 (M+H)⁺. ¹H NMR (400 MHz, DMSO-*d*₆) δ 10.22 (d, J = 8.6 Hz, 1H), 8.42 (s, 1H), 8.20 (d, J = 7.5 Hz, 1H), 7.33 (q, J = 8.0 Hz, 4H), 6.44 (d, J = 7.4 Hz, 1H), 5.25 - 5.10 (m, 2H), 4.26 (s, 1H), 3.73 (s, 2H), 3.59 - 3.46 (m, 2H), 3.40 (s, 2H), 3.02 (s, 5H), 2.50 (p, J = 1.8 Hz, 5H), 1.67 (s, 2H), 1.63 - 1.54 (m, 1H), 1.54 - 1.43 (m, 2H), 1.32 (q, J = 7.5 Hz, 2H), 0.89 (t, J = 7.3 Hz, 3H).

### Example 1-177

### diethyl (S)-(4-(4-(4-((2-amino-4-((1-hydroxypentan-2-yl)amino)-5-oxopyrido[4,3-d]pyrimidin-6(5H)-yl)methyl)benzyl)piperazin-1-yl)butyl)phosphonate

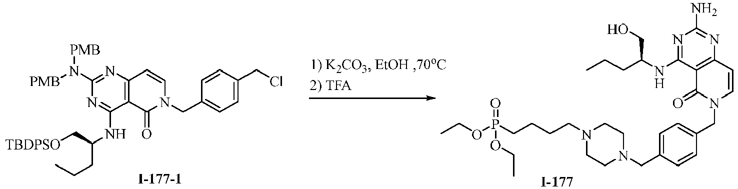

The title compound **1-177** was prepared in analogy to the preparation of compound **1-1** by using diethyl (4-(piperazin-1-yl)butyl)phosphonate instead of pyrrolidine. MS: 644.3 (M+H)⁺.

### Example 1-178

### (4-(4-(4-((2-amino-4-(butylamino)-5-oxopyrido[4,3-d]pyrimidin-6(5H)-yl)methyl)benzyl)piperazin-1-yl)butyl)phosphonic acid

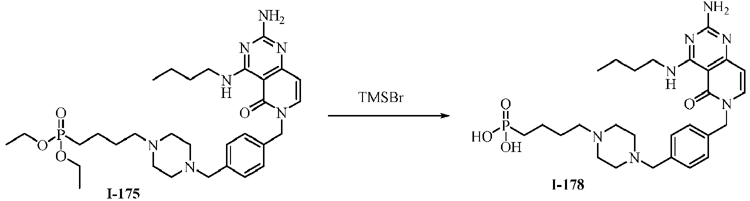

A mixture solution of **1-175** and TMSBr in DCM was stirred at 25 °C for 5 hours. After the reaction was completed, the reaction mixture was concentrated in vacuo to give a crude product, which was purified by pre-HPLC to afford **1-178.** MS: 558.4 (M+H)⁺. ¹H NMR (400 MHz, DMSO-*d*₆) δ 12.46 (s, 1H), 10.17 (t, J = 5.8 Hz, 1H), 8.53 (s, 1H), 8.26 (dd, J = 7.5, 2.2 Hz, 1H), 7.54 (s, 2H), 7.38 (d, J = 7.5 Hz, 2H), 6.51 (d, J = 7.4 Hz, 1H), 5.23 (s, 2H), 4.38 (s, 3H), 3.65 (s, 2H), 3.58 - 3.48 (m, 2H), 3.17 (s, 7H), 3.16 - 3.04 (m, 1H), 1.72 (s, 1H), 1.63 - 1.49 (m, 5H), 1.41 - 1.26 (m, 2H), 1.18 (t, J = 7.3 Hz, 1H), 0.91 (t, J = 7.3 Hz, 3H).

### Example 1-179

### (R)-2-amino-6-(2-methoxy-4-((4-methylpiperazin-1-yl)methyl)benzyl)-4-(pentan-2-ylamino)pyrido[4,3-d]pyrimidin-5(6H)-one

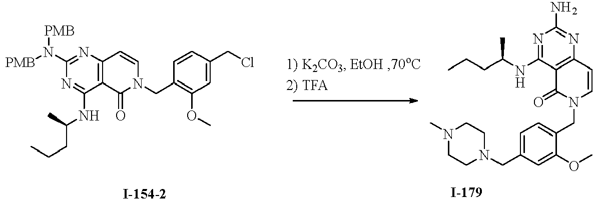

The title compound **1-179** was prepared in analogy to the preparation of compound **I-154** by using 1-methylpiperazine instead of pyrrolidine. MS: 480.3 (M+H)⁺.

### Example 1-180

### (R)-2-amino-6-(2-methoxy-4-(piperazine-1-carbonyl)benzyl)-4-(pentan-2-ylamino)pyrido[4,3-d]pyrimidin-5(6H)-one

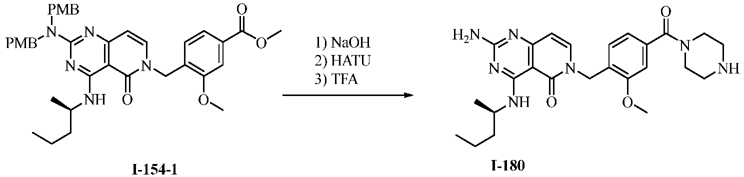

The title compound **1-180** was prepared in analogy to the preparation of compound I-**117** by using **Intermediate 1-154-1** instead of **Intermediate 1-103-1.** MS: 480.2 (M+H)⁺. ¹H NMR (400 MHz, DMSO-*d*₆) δ 9.52 (s, 1H), 8.87 (s, 2H), 7.75 (s, 1H), 7.08 (s, 1H), 6.98 (s, 2H), 6.20 (d, J = 7.6 Hz, 1H), 5.11 - 4.92 (m, 2H), 4.11-4.22 (m, 1H), 3.86 (s, 4H), 3.13 (s, 5H), 2.51 (s, 1H), 2.35 (s, 2H), 1.53 - 1.42 (m, 2H), 1.21-1.32 (m, 2H), 1.14 (d, J = 6.5 Hz, 3H), 0.86 (t, J = 7.3 Hz, 3H).

### Example 1-181

### 6-(4-(3,6-diazabicyclo[3.1.1]heptane-3-carbonyl)-2-methoxybenzyl)-2-amino-4-(((R)-pentan-2-yl)amino)pyrido[4,3-dlpyrimidin-5(6H)-one

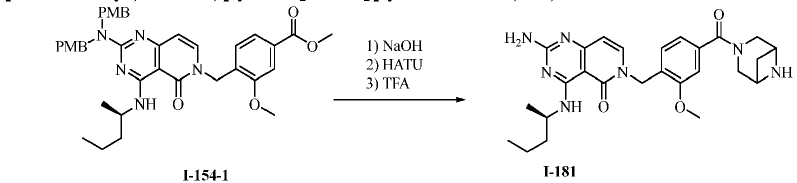

The title compound **1-181** was prepared in analogy to the preparation of compound **I-151** by using **Intermediate I-154-1** instead of **Intermediate I-103-1.** MS: 492.2 (M+H)⁺.

### Example 1-182

### (R)-2-amino-6-(2-methoxy-4-(4-methylpiperazine-1-carbonyl)benzyl)-4-(pentan-2-ylamino)pyrido[4,3-d]pyrimidin-5(6H)-one

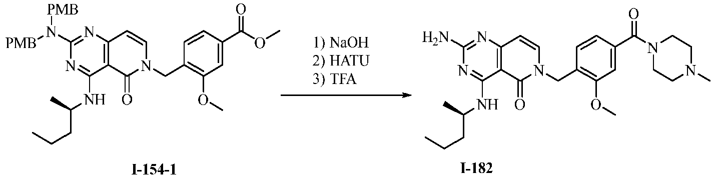

The title compound **1-182** was prepared in analogy to the preparation of compound **I-180** by using 1-methylpiperazine instead of tert-butyl piperazine-1-carboxylate. MS: 494.2 (M+H)⁺. ¹H NMR (400 MHz, DMSO-*d*₆) δ 9.57 (s, 1H), 7.78 (d, J = 7.5 Hz, 1H), 7.27 (s, 2H), 7.07 - 6.91 (m, 3H), 6.22 (d, J = 7.5 Hz, 1H), 5.11 - 4.95 (m, 2H), 4.28 - 4.16 (m, 1H), 3.85 (s, 3H), 3.50 (s, 3H), 2.99 (s, 5H), 2.65 (s, 3H), 1.53 - 1.43 (m, 2H), 1.34 - 1.26 (m, 2H), 1.15 (d, J = 6.5 Hz, 3H), 0.86 (t, J = 7.2 Hz, 3H).

### Example 1-183

### (R)-2-amino-6-(4-(4-isopropylpiperazine-1-carbonyl)-2-methoxybenzyl)-4-(pentan-2-ylamino)pyrido[4,3-d]pyrimidin-5(6H)-one

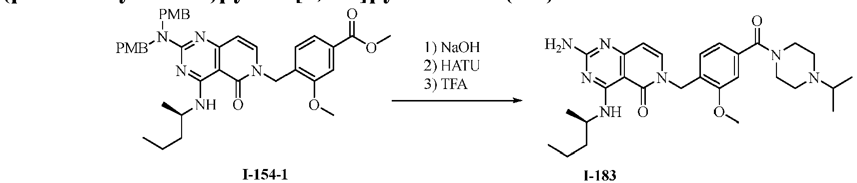

The title compound **1-183** was prepared in analogy to the preparation of compound **I-180** by using 1-isopropylpiperazine instead of tert-butyl piperazine-1-carboxylate. MS: 522.2 (M+H)⁺. ¹H NMR (400 MHz, DMSO-*d₆*) δ 9.60 (s, 1H), 7.81 (d, J = 7.5 Hz, 1H), 7.36 (s, 2H), 7.08 (s, 1H), 7.00 (d, J = 3.2 Hz, 2H), 6.23 (d, J = 7.4 Hz, 1H), 5.11 - 4.96 (m, 2H), 4.22 (p, J = 6.8 Hz, 1H), 3.86 (s, 3H), 3.03 (s, 3H), 1.55 - 1.41 (m, 2H), 1.37 - 1.25 (m, 2H), 1.17 (dd, J = 15.5, 6.4 Hz, 10H), 0.86 (t, J = 7.2 Hz, 3H).

### Example 1-184

### (R)-2-amino-6-(2-methoxy-4-(piperazin-1-ylmethyl)benzyl)-4-(pentan-2-ylamino)pyrido[4,3-d]pyrimidin-5(6H)-one

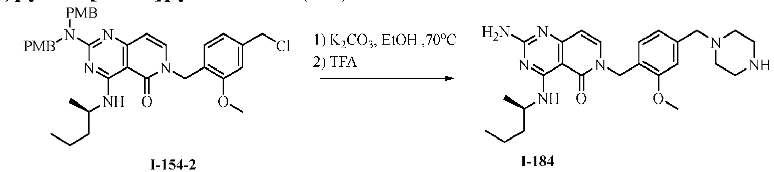

The title compound **1-184** was prepared in analogy to the preparation of compound **I-154** by using tert-butyl piperazine-1-carboxylate instead of pyrrolidine. MS: 466.2 (M+H)⁺. ¹H NMR (400 MHz, DMSO-*d₆*) δ 9.58 (s, 1H), 7.79 (d, J = 7.5 Hz, 1H), 7.23 (s, 2H), 7.08 - 6.92 (m, 3H), 6.25 (d, J = 7.5 Hz, 1H), 5.12 - 4.91 (m, 2H), 4.27 - 4.13(m, 1H), 3.85 (s, 3H), 2.96 (s, 5H), 2.63 (s, 3H), 1.53 - 1.44 (m, 2H), 1.34 - 1.26 (m, 2H), 1.14 (d, J = 6.5 Hz, 3H), 0.86 (t, J = 7.2 Hz, 3H).

### Example 1-185

### 6-(4-((3,6-diazabicyclo[3.1.1]heptan-3-yl)methyl)-2-methoxybenzyl)-2-amino-4-(((R)-pentan-2-yl)amino)pyrido[4,3-d]pyrimidin-5(6H)-one

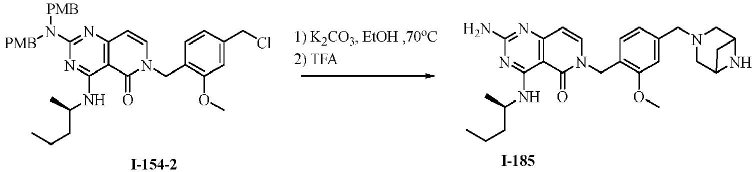

The title compound I**-185** was prepared in analogy to the preparation of compound **I-154** by using tert-butyl 3,6-diazabicyclo[3.1.1]heptane-6-carboxylate instead of pyrrolidine. MS: 478.2 (M+H)⁺. ¹H NMR (400 MHz, DMSO-*d*₆) δ 13.50 (s, 2H), 10.06 (d, J = 8.3 Hz, 1H), 9.53 (s, 1H), 8.38 (d, J = 42.3 Hz, 2H), 8.06 (d, J = 7.4 Hz, 2H), 7.07 (d, J = 8.4 Hz, 2H), 6.94 (d, J = 7.7 Hz, 1H), 6.41 (d, J = 7.4 Hz, 1H), 5.15 - 5.00 (m, 2H), 4.33 - 4.18 (m, 3H), 3.94 (s, 2H), 3.83 (s, 3H), 3.25 (d, J = 50.4 Hz, 4H), 2.69 (s, 1H), 2.15 (dd, J = 10.0, 5.2 Hz, 1H), 1.53 (q, J = 7.0 Hz, 2H), 1.31 (ddt, J = 13.4, 10.4, 4.8 Hz, 2H), 1.20 (d, J = 6.5 Hz, 3H), 0.88 (t, J = 7.3 Hz, 3H).

### Example 1-187

### 2-amino-4-(butylamino)-7-methyl-6-(4-(pyrrolidin-1-ylmethyl)benzyl)pyrido[4,3-d]pyrimidin-5(6H)-one

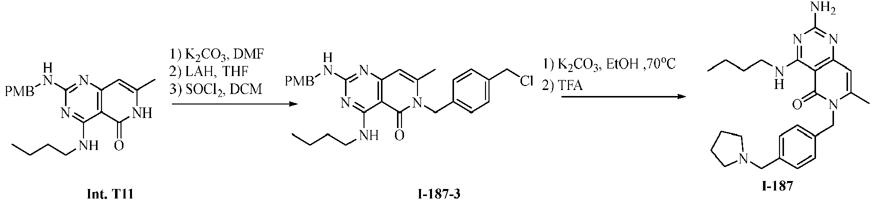

The title compound **1-187** was prepared in analogy to the preparation of compound **I-1** by using **Intermediate Int. T11** instead of **Intermediate Int. A.** MS: 421.3 (M+H)⁺. ¹H NMR (400 MHz, DMSO-*d*₆) δ 9.64, (s, 1H), 7.32 (m, 6H), 6.15 (s, 1H), 5.29 (s, 2H), 4.29 (s, 2H), 3.45 (q, J = 6.6 Hz, 3H), 3.17 (m, 4H), 2.28 (d, J = 2.2 Hz, 3H), 1.91 (s, 4H), 1.54 (p, J = 7.3 Hz, 2H), 1.34 (p, J = 7.4 Hz, 2H), 0.89 (t, J = 7.3 Hz, 3H).

### Example 1-188

### (R)-6-((6-((2-oxa-6-azaspiro[3.3]heptan-6-yl)methyl)-2-methoxypyridin-3-yl)methyl)-2-amino-4-(pentan-2-ylamino)pyrido[4,3-d]pyrimidin-5(6H)-one

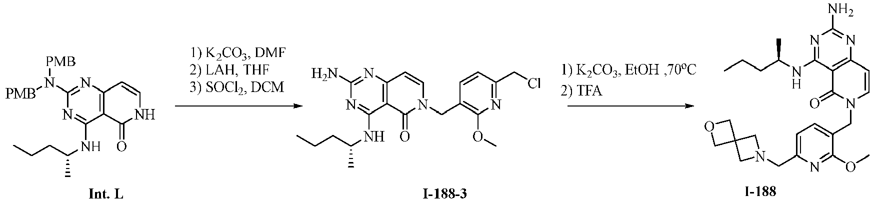

The title compound **1-188** was prepared in analogy to the preparation of compound **I-64** by using **Intermediate Int. L** instead of **Intermediate Int. A** and 2-oxa-6-azaspiro[3.3]heptane instead of pyrrolidine. MS: 480.3 (M+H)⁺. ¹H NMR (400 MHz, DMSO-*d*₆) δ 13.28 (s, 1H), 10.74 (s, 1H), 10.15 (s, 1H), 10.02 (d, J = 8.3 Hz, 1H), 8.32 (d, J = 84.0 Hz, 2H), 8.07 (d, J = 7.4 Hz, 1H), 7.16 (d, J = 8.7 Hz, 1H), 7.13 - 7.06 (m, 1H), 7.01 (t, J = 9.0 Hz, 1H), 6.42 (d, J = 7.4 Hz, 1H), 5.16 - 5.01 (m, 2H), 4.39 (s, 1H), 4.27 (p, J = 7.1 Hz, 3H), 4.05 (d, J = 7.5 Hz, 4H), 3.95 - 3.77 (m, 6H), 3.45 (d, J = 26.3 Hz, 2H), 1.53 (dt, J = 13.0, 6.7 Hz, 2H), 1.31 (q, J = 7.2, 6.7 Hz, 2H), 1.19 (d, J = 6.5 Hz, 3H), 0.87 (t, J = 7.3 Hz, 3H).

### Example I-191

### (S)-2-amino-4-((1-hydroxypentan-2-yl)amino)-6-(2-methoxy-4-(piperazin-1-ylmethyl)benzyl)pyrido[4,3-d]pyrimidin-5(6H)-one

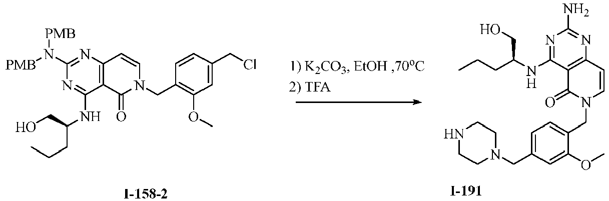

The title compound **1-191** was prepared in analogy to the preparation of compound **I-158** by using tert-butyl piperazine-1-carboxylate instead of pyrrolidine. MS: 482.3 (M+H)⁺. ¹H NMR (400 MHz, Methanol-*d₄*) δ 8.53 (s, 2H), 7.73 (d, J = 7.6 Hz, 1H), 7.24 (d, J = 7.7 Hz, 1H), 7.04 (s, 1H), 6.94 (d, J = 7.7 Hz, 1H), 6.25 (d, J = 7.6 Hz, 1H), 5.16 - 5.03 (m, 2H), 4.38 (t, J = 6.6 Hz, 1H), 3.91 (s, 3H), 3.74 - 3.55 (m, 4H), 3.34 (d, J = 2.1 Hz, 2H), 3.23 (t, J = 5.1 Hz, 4H), 2.70 (d, J = 5.5 Hz, 4H), 1.77 - 1.56 (m, 2H), 1.55 - 1.38 (m, 2H), 0.99 (t, J = 7.4 Hz, 3H).

### Example 1-192

### 2-amino-4-(butylamino)-7-phenyl-6-(4-(pyrrolidin-1-ylmethyl)benzyl)pyrido[4,3-d]pyrimidin-5(6H)-one

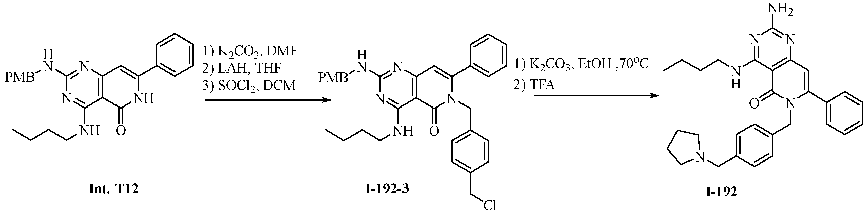

The title compound **1-192** was prepared in analogy to the preparation of compound **1-187** by using **Intermediate Int. T12** instead of **Intermediate Int. T11.** MS: 483.3 (M+H)⁺. ¹H NMR (400 MHz, DMSO-*d*₆) δ 11.19 (s, 1H), 9.62 (s, 1H), 7.45 (d, J = 8.2 Hz, 3H), 7.31 (d, J = 7.2 Hz, 2H), 7.23 (d, J = 7.7 Hz, 2H), 6.99 (d, J = 7.7 Hz, 2H), 6.55 (s, 2H), 4.05 (s, 2H), 3.87 (s, 2H), 3.48 (m, 2H), 2.97 (s, 4H), 1.87 (s, 4H), 1.64 - 1.54 (m, 2H), 1.46 - 1.36 (m, 2H), 1.27 (s, 2H), 0.96 (t, J = 7.4 Hz, 3H).

### Example 1-194

### (R)-2-amino-4-((1-hydroxypentan-2-yl)amino)-6-(2-methoxy-4-((4-methylpiperazin-1-yl)methyl)benzyl)pyrido[4,3-d]pyrimidin-5(6H)-one

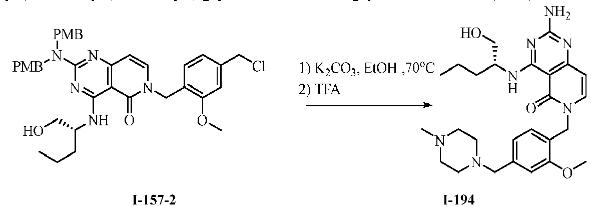

The title compound **1-194** was prepared in analogy to the preparation of compound **I-157** by using 1-methylpiperazine instead of pyrrolidine. MS: 496.3 (M+H)⁺.

### Example 1-195

### (S)-2-amino-6-(4-((dimethylamino)methyl)-2-methoxybenzyl)-4-((1-hydroxypentan-2-yl)amino)pyrido[4,3-d]pyrimidin-5(6H)-one

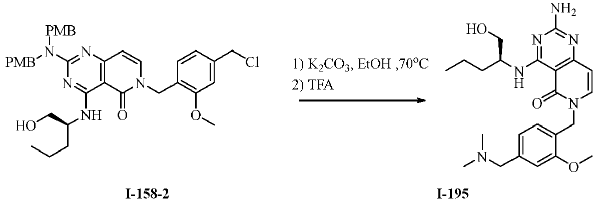

The title compound **1-195** was prepared in analogy to the preparation of compound **I-158** by using dimethylamine hydrochloride instead of pyrrolidine. MS: 441.5 (M+H)⁺.

### Example 1-198

### (R)-2-amino-6-(2-methoxy-4-((methylamino)methyl)benzyl)-4-(pentan-2-ylamino)pyrido[4,3-d]pyrimidin-5(6H)-one

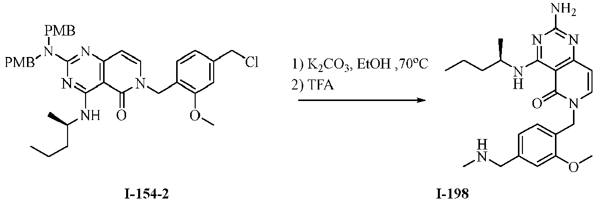

The title compound **1-198** was prepared in analogy to the preparation of compound **I-154** by using methanamine hydrochloride instead of pyrrolidine. MS: 411.5 (M+H)⁺. ¹H NMR (400 MHz, DMSO-*d*₆) δ 8.74 (s, 2H), 7.17 (s, 1H), 6.99 (t, J = 6.9 Hz, 2H), 6.23 (s, 1H), 5.02 (d, J = 7.3 Hz, 2H), 4.23 (s, 1H), 4.09 (s, 2H), 3.85 (s, 5H), 2.55 (s, 3H), 1.48 (t, J = 7.2 Hz, 2H), 1.30 (s, 1H), 1.23 (s, 2H), 1.15 (d, J = 6.5 Hz, 3H), 0.87 (t, J = 7.3 Hz, 3H).

### Example 1-207

### (S)-2-amino-6-(4-((4-(2-aminoethyl)piperazin-1-yl)methyl)-2-methoxybenzyl)-4-((1-hydroxypentan-2-yl)amino)pyrido[4,3-d]pyrimidin-5(6H)-one

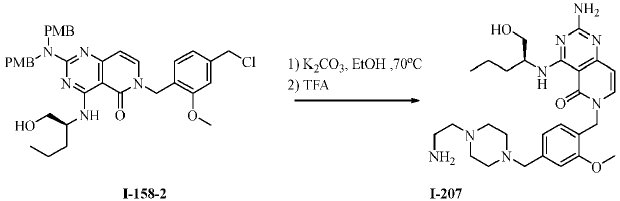

The title compound **1-207** was prepared in analogy to the preparation of compound **I-158** by using tert-butyl (2-(piperazin-1-yl)ethyl)carbamate instead of pyrrolidine. MS: 525.3 (M+H)⁺.

### Example 1-208

### (S)-2-amino-6-(4-(((2-aminoethyl)(methyl)amino)methyl)-2-methoxybenzyl)-4-((1-hydroxypentan-2-yl)amino)pyrido[4,3-d]pyrimidin-5(6H)-one

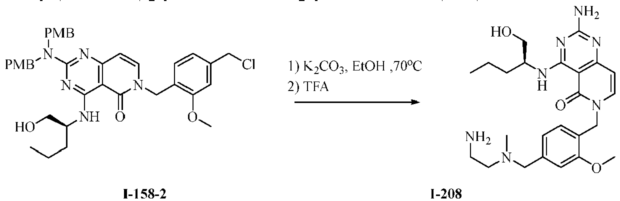

The title compound **1-208** was prepared in analogy to the preparation of compound **I-158** by using tert-butyl (2-(methylamino)ethyl)carbamate instead of pyrrolidine. MS: 470.28 (M+H)⁺. ¹H NMR (400 MHz, DMSO-*d*₆) δ 10.23 (s, 1H), 8.11 (d, J = 7.3 Hz, 1H), 7.14 (s, 2H), 7.02 (s, 1H), 6.48 (d, J = 7.3 Hz, 1H), 5.13 (d, J = 6.7 Hz, 2H), 4.30 (s, 1H), 3.90 (s, 4H), 3.55 (d, J = 6.6 Hz, 5H), 2.75 - 2.68 (m, 2H), 2.58 (s, 3H), 2.40 - 2.33 (m, 2H), 1.70 - 1.62 (m, 1H), 1.60 - 1.49 (m, 1H), 1.36 (q, J = 7.6 Hz, 2H), 0.93 (d, J = 7.4 Hz, 3H).

### Example 1-209

### (R)-2-amino-6-(2-methoxy-4-((4-(methylamino)piperidin-1-yl)methyl)benzyl)-4-(pentan-2-ylamino)pyrido[4,3-d]pyrimidin-5(6H)-one

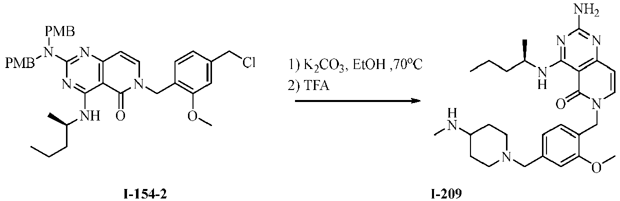

The title compound **1-209** was prepared in analogy to the preparation of compound **1-154** by using tert-butyl methyl(piperidin-4-yl)carbamate instead of pyrrolidine. MS: 494.5 (M+H)⁺. ¹H NMR (400 MHz, DMSO-*d*₆) δ 9.31 (d, J = 8.1 Hz, 1H), 8.32 (s, 2H), 7.55 (d, J = 7.6 Hz, 1H), 6.93 (s, 1H), 6.86 (d, J = 7.6 Hz, 1H), 6.80 (d, J = 7.7 Hz, 1H), 6.59 (s, 1H), 6.07 (d, J = 7.6 Hz, 1H), 5.00 - 4.88 (m, 2H), 4.20 (p, J = 6.8 Hz, 2H), 4.02 - 3.85 (m, 4H), 3.81 (s, 3H), 2.82 (d, J = 11.5 Hz, 2H), 2.45 (s, 3H), 1.93 (d, J = 12.6 Hz, 2H), 1.89 (s, 1H), 1.49 - 1.44 (m, 3H), 1.30 (dt, J = 13.6, 7.0 Hz, 2H), 1.13 (d, J = 6.5 Hz, 3H), 0.87 (t, J = 7.3 Hz, 3H).

### Example I-211

### 6-(4-(3,6-diazabicyclo[3.1.1]heptane-3-carbonyl)-2-methoxybenzyl)-2-amino-4-(((S)-1-hydroxypentan-2-yl)amino)pyrido[4,3-d]pyrimidin-5(6H)-one

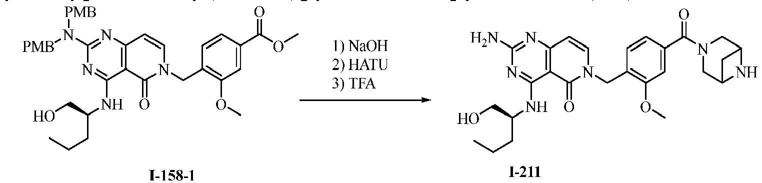

The title compound **1-211** was prepared in analogy to the preparation of compound **I-115** by using **Intermediate 1-158-1** instead of **Intermediate 1-95-1.** MS: 508.3 (M+H)⁺. ¹H NMR (400 MHz, DMSO-*d₆*) δ 9.42 (d, J = 8.5 Hz, 1H), 8.24 (s, 1H), 7.65 (d, J = 7.5 Hz, 1H), 7.12 (s, 1H), 7.00 (d, J = 2.3 Hz, 2H), 6.62 (s, 1H), 6.14 (d, J = 7.5 Hz, 1H), 5.04 (q, J = 15.7 Hz, 2H), 4.23 (s, 1H), 3.87 (s, 1H), 3.73 (t, J = 11.8 Hz, 4H), 3.59 - 3.43 (m, 9H), 1.60 (d, J = 9.2 Hz, 2H), 1.55 - 1.42 (m, 1H), 1.34 (dd, J = 14.2, 6.0 Hz, 2H), 0.92 (t, J = 7.3 Hz, 3H).

### Example 1-212

### (S)-2-amino-4-((1-hydroxypentan-2-yl)amino)-6-(2-methoxy-4-(4-methylpiperazine-1-carbonyl)benzyl)pyrido[4,3-d]pyrimidin-5(6H)-one

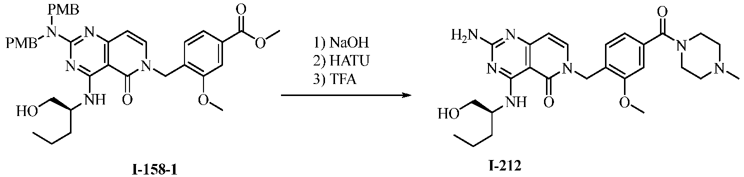

The title compound **1-212** was prepared in analogy to the preparation of compound **I-115** by using **Intermediate I-158-1** instead of **Intermediate I-95-1.** MS: 510.3 (M+H)⁺. ¹H NMR (400 MHz, DMSO-*d*₆) δ 10.00 (s, 1H), 8.00 (d, J = 7.6 Hz, 1H), 7.17 - 7.08 (m, 2H), 7.01 (dd, J = 7.6, 1.4 Hz, 1H), 6.38 (d, J = 7.4 Hz, 1H), 5.23 - 5.04 (m, 2H), 4.97 (s, 1H), 4.27 (s, 1H), 3.90 (s, 3H), 3.53 (s, 3H), 3.37 (s, 12H), 2.79 (s, 3H), 1.34 (q, J = 7.5 Hz, 2H), 0.91 (t, J = 7.3 Hz, 3H).

### Example 1-213

### (R)-2-amino-6-(4-((4-(2-aminoethyl)piperazin-1-yl)methyl)-2-methoxybenzyl)-4-(pentan-2-ylamino)pyrido[4,3-d]pyrimidin-5(6H)-one

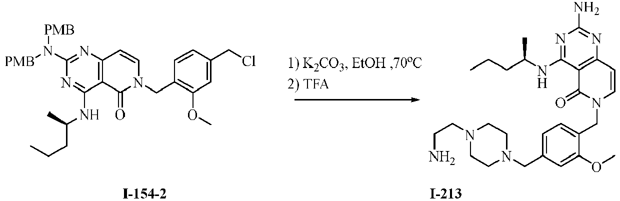

The title compound **I-213** was prepared in analogy to the preparation of compound **I-207** by using **Intermediate I-154-2** instead of **Intermediate I-158-2.** MS: 509.5 (M+H)⁺. ¹H NMR (400 MHz, DMSO-*d*₆) δ 10.47 (s, 1H), 9.98 (d, J = 8.4 Hz, 1H), 8.27 (s, 1H), 8.05 (d, J = 7.4 Hz, 1H), 7.77 (s, 2H), 7.23 (s, 1H), 7.12 - 7.01 (m, 2H), 6.40 (d, J = 7.4 Hz, 1H), 5.15 - 5.03 (m, 2H), 4.30 - 4.24 (m, 2H), 3.85 (s, 3H), 3.55 - 3.25 (m, 8H), 2.92 (s, 4H), 2.56 (s, 1H), 1.57 - 1.43 (m, 2H), 1.36 - 1.27 (m, 2H), 1.19 (d, J = 6.5 Hz, 3H), 0.88 (t, J = 7.3 Hz, 3H).

### Example 1-214

### (S)-2-amino-4-((1-hydroxypentan-2-yl)amino)-6-(2-methoxy-4-((m ethylamino)methyl)benzyl)pyrido [4,3-d] pyrimidin-5(6H)-one

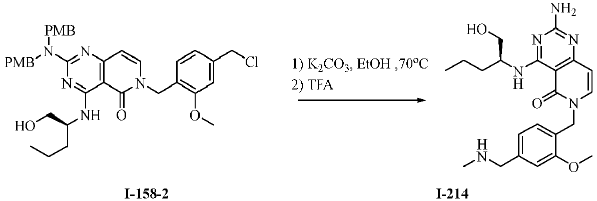

The title compound **I-214** was prepared in analogy to the preparation of compound **I-158** by using methanamine hydrochloride instead of pyrrolidine. MS: 427.3 (M+H)⁺. ¹H NMR (400 MHz, DMSO-*d₆*) δ 9.41 (d, J = 8.6 Hz, 1H), 7.64 (d, J = 7.6 Hz, 1H), 7.20 (s, 1H), 7.03 - 6.98 (m, 2H), 6.64 (s, 1H), 6.12 (d, J = 7.5 Hz, 1H), 5.01 (q, J = 15.5 Hz, 2H), 4.81 (s, 1H), 4.28 - 4.17 (m, 1H), 4.14 - 4.07 (m, 4H), 3.88 (s, 3H), 3.54 - 3.42 (m, 2H), 2.59 (s, 3H), 1.60 (dq, J = 13.9, 6.8 Hz, 1H), 1.46 (td, J = 13.8, 8.5 Hz, 1H), 1.33 (s, 2H), 0.90 (t, J = 7.3 Hz, 3H).

### Example I-216

### (R)-2-amino-6-(4-((4-(3-hydroxypropyl)piperazin-1-yl)methyl)-2-methoxybenzyl)-4-(pentan-2-ylamino)pyrido[4,3-d]pyrimidin-5(6H)-one

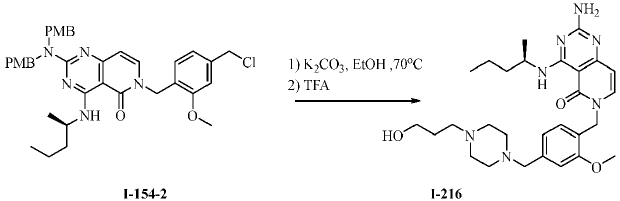

The title compound **1-216** was prepared in analogy to the preparation of compound **I-154.** MS: 524.6 (M+H)⁺. ¹H NMR (400 MHz, DMSO-*d₆*) δ 9.34 (d, J = 8.1 Hz, 1H), 7.58 (d, J = 7.6 Hz, 1H), 6.96 (d, J = 1.3 Hz, 1H), 6.92 - 6.81 (m, 2H), 6.63 (s, 2H), 6.08 (d, J = 7.5 Hz, 1H), 5.02 - 4.89 (m, 2H), 4.29 - 4.16 (m, 1H), 3.84 (d, J = 7.6 Hz, 3H), 3.46 - 3.40 (m, 4H), 2.72 (s, 1H), 2.51 (p, J = 1.9 Hz, 6H), 2.38 (t, J = 7.3 Hz, 4H), 1.63 - 1.54 (m, 2H), 1.53 - 1.45 (m, 2H), 1.38 - 1.28 (m, 2H), 1.15 (d, J = 6.5 Hz, 3H), 0.89 (t, J = 7.3 Hz, 3H).

### Example 1-217

### (S)-2-amino-6-(4-((4-(6-aminohexyl)piperazin-1-yl)methyl)-2-methoxybenzyl)-4-((1-hydroxypentan-2-yl)amino)pyrido[4,3-d]pyrimidin-5(6H)-one

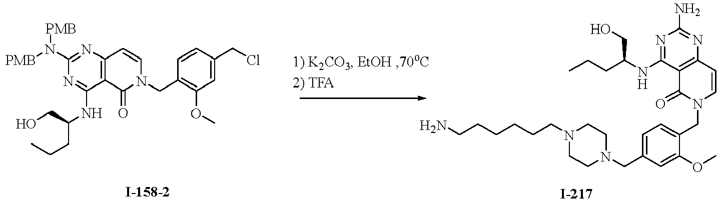

The title compound 1-217 was prepared in analogy to the preparation of compound **I-158.** MS: 581.3 (M+H)⁺.

### Example 1-220

### (S)-2-((2-amino-6-(2-methoxy-4-((4-methylpiperazin-1-yl)methyl)benzyl)-5-oxo-5,6-dihydropyrido[4,3-d]pyrimidin-4-yl)amino)pentyl 2,2-dimethylbutanoate

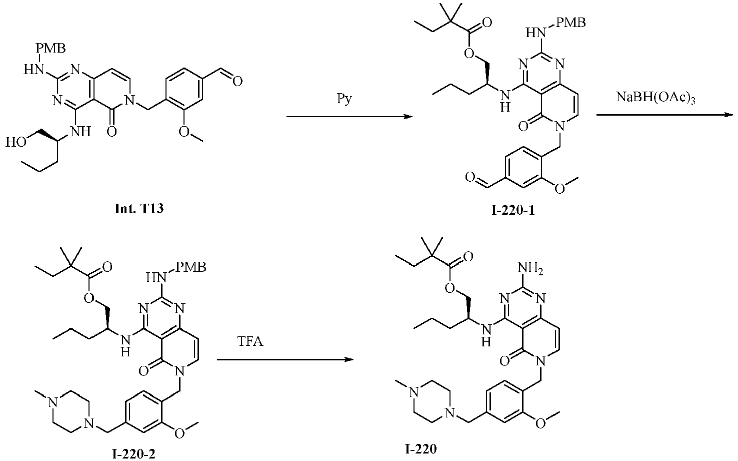

### Step 1: Preparation of Intermediate 1-220-1

To a stirred mixture solution of **Intermediate T13** (2.335 g, 16.89 mmol) in DCM (10 mL) was added 2,2-dimethylbutanoyl chloride (1.1 mL, 8.4 mmol) and pyridine (0.7 mL, 8.4 mmol) at 0 °C, then the resulting mixture was stirred at 20 °C for 12 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-220-1** (619 mg, 56%) as a yellow oil. MS: 630.2 (M+H)⁺.

### Step 2: Preparation of Intermediate 1-220-2

To a stirred mixture solution of **Intermediate I-220-1** (619 mg, 1 mmol) in ACN (5 mL) was added 1-methylpiperazine (164 uL, 1.5 mmol) and NaBH(OAc)₃ (312 mg, 1.5 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **Intermediate I-220-2** (238 mg, 34%) as a yellow oil. MS: 714.4 (M+H)⁺.

### Step 3: Preparation of compound 1-220

The title compound **I-220** was prepared in analogy to the preparation of compound **I-11-2** by using **Intermediate I-220-2** instead of **Intermediate I-11-1.** MS: 594.3 (M+H)⁺. ¹H NMR (400 MHz, CDCl₃) δ 15.41 (s, 1H), 10.05 (s, 1H), 7.72 (d, *J* = 7.3 Hz, 1H), 7.42 (d, *J* = 7.6 Hz, 1H), 7.10 (s, 1H), 6.95 (d, *J* = 7.5 Hz, 1H), 6.58 (d, *J* = 7.4 Hz, 1H), 5.61 (s, 1H), 5.22 - 4.95 (m, 2H), 4.63 (t, *J* = 4.3 Hz, 1H), 4.29 - 4.12 (m, 2H), 3.97 (s, 2H), 3.91 (s, 3H), 3.49 (s, 4H), 3.31 (s, 4H), 2.87 (s, 3H), 1.70 (q, *J* = 7.5 Hz, 2H), 1.61 (q, *J* = 7.5 Hz, 2H), 1.45 (dt, *J* = 14.9, 7.3 Hz, 2H), 1.20 (d, *J* = 4.4 Hz, 6H), 1.00 (t, *J* = 7.3 Hz, 3H), 0.84 (t, *J* = 7.5 Hz, 3H).

### Example 1-221

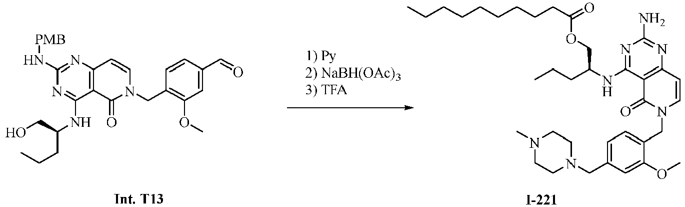

The title compound **I-221** was prepared in analogy to the preparation of compound **I-220.** MS: 650.4 (M+H)⁺. ¹H NMR (400 MHz, CDCl₃) δ 7.68 (d, *J* = 7.5 Hz, 1H), 7.36 (d, *J* = 7.7 Hz, 1H), 6.94 (d, *J =* 7.5 Hz, 2H), 6.56 (d, *J =* 7.5 Hz, 1H), 5.11 (s, 2H), 4.62 (s, 1H), 4.30 (dd, *J* = 11.3, 4.4 Hz, 1H), 4.16 (dd, *J* = 11.3, 5.3 Hz, 1H), 3.90 (s, 3H), 3.63 (s, 2H), 2.79 (s, 8H), 2.36 (t, *J* = 7.5 Hz, 2H), 1.67 (dt, *J* = 14.0, 8.0 Hz, 8H), 1.31 (d, *J* = 11.0 Hz, 14H), 1.00 (t, *J* = 7.3 Hz, 3H), 0.91 (t, *J* = 6.7 Hz, 3H).

### Example 1-222

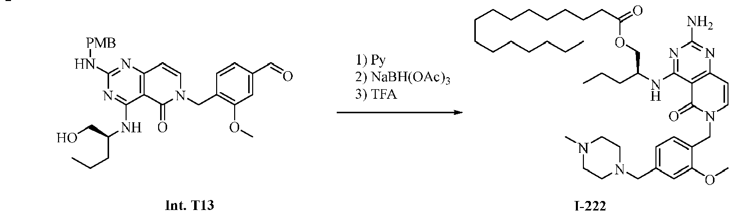

The title compound **1-222** was prepared in analogy to the preparation of compound **I-220.** MS: 734.5 (M+H)⁺. ¹H NMR (400 MHz, CDCl₃) δ 10.09 (s, 1H), 7.69 (d, J = 7.5 Hz, 1H), 7.37 (d, J = 7.6 Hz, 1H), 6.99 - 6.91 (m, 2H), 6.58 (d, J = 7.5 Hz, 1H), 5.57 (s, 1H), 5.10 (s, 2H), 4.61 (s, 1H), 4.30 (dd, J = 11.3, 4.4 Hz, 1H), 4.15 (dd, J = 11.4, 5.4 Hz, 1H), 3.90 (s, 3H), 3.72 (s, 2H), 3.51 (s, 2H), 3.00 (s, 6H), 2.82 (s, 3H), 2.36 (t, J = 7.5 Hz, 2H), 1.67 (dt, J = 10.9, 7.8 Hz, 4H), 1.46 (dd, J = 17.4, 8.3 Hz, 2H), 1.29 (d, J = 3.2 Hz, 26H), 1.00 (t, J = 7.3 Hz, 3H), 0.92 (t, J = 6.8 Hz, 3H).

### Example 1-223

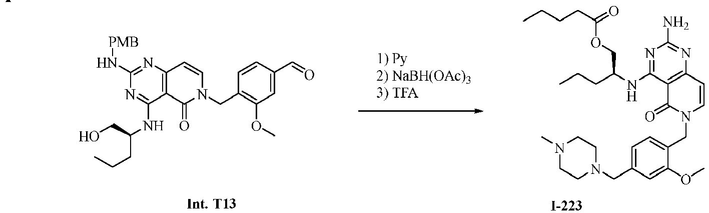

The title compound **1-223** was prepared in analogy to the preparation of compound **I-220.** MS: 580.3 (M+H)⁺. ¹H NMR (400 MHz, CDCl₃) δ 9.97 (s, 1H), 7.71 (d, *J* = 7.1 Hz, 1H), 7.41 (d, *J* = 7.5 Hz, 1H), 7.14 (s, 1H), 6.98 (d, *J* = 7.6 Hz, 1H), 6.58 (d, *J* = 7.3 Hz, 1H), 5.73 (s, 1H), 5.11 (s, 2H), 4.62 (d, *J* = 6.9 Hz, 1H), 4.29 (dd, *J* = 11.3, 4.3 Hz, 1H), 4.15 (dd, *J* = 11.4, 5.3 Hz, 1H), 4.08 (s, 2H), 3.91 (s, 3H), 3.57 (s, 4H), 3.43 (s, 4H), 2.89 (s, 3H), 2.36 (t, *J* = 7.5 Hz, 2H), 1.63 (p, *J* = 7.4 Hz, 4H), 1.53 - 1.31 (m, 4H), 0.99 (t, *J* = 7.3 Hz, 3H), 0.93 (t, *J* = 7.3 Hz, 3H).

### Example 1-224 & Example 1-225

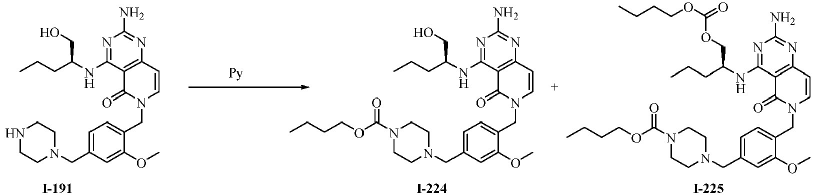

To a stirred mixture solution of **compound I-191** (333 mg, 0.7 mmol) in DCM (3 mL) was added butyl carbonochloridate (0.176 mL, 0.7 mmol) and pyridine (0.168 mL, 2.1 mmol) at -10 °C, then the resulting mixture was stirred at -10 °C for 0.5 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by pre-HPLC to afford **Compound I-224** (85.4 mg) and **Compound I-225** (65.3 mg) as a white solid.

**Compound 1-224,** MS: 582.3 (M+H)⁺.¹H NMR (400 MHz, DMSO-*d*₆) δ 9.57 (d, *J* = 8.5 Hz, 1H), 7.68 (d, *J* = 7.5 Hz, 1H), 7.02 (s, 1H), 6.94 (d, *J* = 7.7 Hz, 1H), 6.90 - 6.76 (m, 2H), 6.16 (d, *J* = 7.5 Hz, 1H), 5.11 - 4.91 (m, 2H), 4.35 - 4.15 (m, 1H), 4.00 (t, *J* = 6.5 Hz, 2H), 3.86 (s, 3H), 3.55 - 3.41 (m, 11H), 2.40 (s, 3H), 1.69 - 1.42 (m, 4H), 1.41 - 1.24 (m, 4H), 0.90 (t, *J* = 7.3 Hz, 6H).

**Compound I-225,** MS: 682.3 (M+H)⁺. ¹H NMR (400 MHz, DMSO-*d*₆) δ 9.41 (d, *J* = 8.5 Hz, 1H), 7.60 (d, *J* = 7.5 Hz, 1H), 6.98 (s, 1H), 6.91 (d, *J* = 7.7 Hz, 1H), 6.85 (d, *J* = 7.5 Hz, 1H), 6.65 (s, 1H), 6.11 (d, *J* = 7.5 Hz, 1H), 5.08 - 4.82 (m, 1H), 4.49 (dt, *J* = 8.6, 4.6 Hz, 1H), 4.25 (dd, *J* = 10.9, 4.4 Hz, 1H), 4.14 (dd, *J* = 10.9, 5.3 Hz, 1H), 4.05 (t, *J* = 6.6 Hz, 2H), 4.00 (t, *J* = 6.5 Hz, 2H), 3.85 (s, 3H), 3.47 (s, 8H), 2.34 (t, *J* = 5.1 Hz, 4H), 1.55 (ddt, *J* = 8.0, 5.6, 3.2 Hz, 6H), 1.44 - 1.19 (m, 6H), 0.88 (dt, *J* = 12.8, 7.4 Hz, 9H).

### Example 1-226

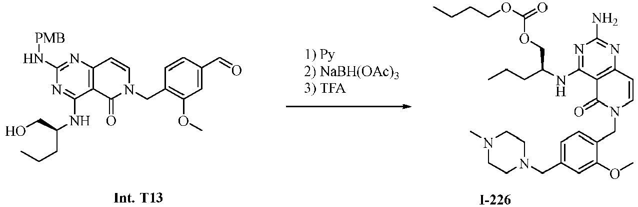

The title compound **I-226** was prepared in analogy to the preparation of compound **I-220.** MS: 596.3 (M+H)⁺. ¹H NMR (400 MHz, DMSO-*d*₆) δ 10.15 (d, J = 8.9 Hz, 1H), 8.08 (d, J = 7.5 Hz, 1H), 7.09 (d, J = 7.6 Hz, 1H), 7.04 (s, 1H), 6.91 (d, J = 7.7 Hz, 1H), 6.45 (d, J = 7.5 Hz, 1H), 5.16 - 5.00 (m, 2H), 4.58 (dq, J = 8.9, 5.4, 3.8 Hz, 1H), 4.31 (dd, J = 11.2, 4.1 Hz, 1H), 4.23 (dd, J = 11.2, 6.1 Hz, 1H), 4.07 (t, J = 6.6 Hz, 3H), 3.67 (s, 4H), 3.20 (d, J = 145.9 Hz, 8H), 2.79 (s, 3H), 1.68 - 1.47 (m, 4H), 1.44 - 1.20 (m, 4H), 0.89 (dt, J = 17.7, 7.3 Hz, 6H).

### Determination of the agonistic activity of the compound to human TLR7 or TLR8

HEK-Blue^{™} cells that stably express human TLR7 or TLR8 are used to evaluate the agonistic activity of the compounds on TLR7 or TLR8, which is determined by the induction of the expression of SEAP reporter gene under the control of minimum IFN-β promoter fusion to 5×NF-κB and AP-1 binding sites. The assay is conducted as below:
HEK-Blue^{™} hTLR7 (Invivogen, 80,000 cells per well) or HEK-Blue^{™} hTLR8 (Invivogen, 60,000 cells per well) were added to the 96-well cell culture plate, followed by adding of the test compound. The final concentration of the test compound in the medium ranged from 0.001 to 36µM, and was incubated for 16 to 22 hours. According to the manufacturer's instructions, the HEK-Blue assay reagent Quanti-blue (Invivogen) was used to measure SEAP levels in cell media. Optical density at 650nm (OD650) was measured by Neo2 multifunction plate reader (Bio-tek). The half effective concentration (EC₅₀) was calculated using GraphPad Prism.

**Table 1: EC₅₀ value of the compound of the invention for human TLR7/8 agonists**

| Compound No. | HEK-Blue TLR7 EC₅₀ (µM) | HEK-Blue TLR8 EC₅₀ (µM) | Compound No. | HEK-Blue TLR7 EC₅₀ (µM) | HEK-Blue TLR8 EC₅₀ (µM) |
|---|---|---|---|---|---|
| **I-1** | 0.081 | 0.058 | **I-51** | 0.098 | 0.088 |
| **I-2** | 0.064 | 0.107 | **I-52** | 0.083 | 0.056 |
| **I-12** | - | 0.097 | **I-53** | 0.061 | 0.092 |
| **I-14** | 0.085 | 0.077 | **I-63** | 0.082 | - |
| **I-19** | - | 0.055 | **I-64** | 0.067 | - |
| **I-21** | - | 0.051 | **I-65** | 0.203 | 0.064 |
| **I-27** | - | 0.095 | **I-72** | 0.064 | 0.081 |
| **I-34** | | 0.045 | **I-73** | 0.041 | - |
| **I-35** | 0.087 | 0.058 | **I-79** | 0.087 | 0.105 |
| **I-50** | - | 0.051 | **I-90** | 0.068 | 0.047 |
| **I-95** | 0.065 | 0.046 | **I-96** | 0.05 | 0.031 |
| **I-103** | 0.064 | 0.047 | **I-98** | 0.101 | 0.085 |
| **I-114** | 0.086 | - | **I-118** | 0.04 | 0.052 |
| **I-117** | 0.055 | 0.049 | **I-120** | 0.046 | 0.053 |
| **I-128** | - | 0.066 | **I-123** | 0.023 | 0.034 |
| **I-133** | 0.07 | - | **I-127** | 0.044 | 0.003 |
| **I-136** | 0.07 | | **I-129** | - | 0.077 |
| **I-138** | 0.058 | 0.097 | **I-132** | 0.084 | 0.660 |
| **I-140** | - | 0.03 | **I-137** | 0.065 | - |
| **I-142** | 0.07 | 0.04 | **I-139** | 0.03 | 0.02 |
| **I-148** | 0.04 | 0.04 | **I-141** | 0.05 | 0.02 |
| **I-151** | 0.08 | 0.03 | **I-150** | 0.04 | 0.03 |
| **I-153** | 0.09 | 0.04 | **I-152** | 0.08 | 0.11 |
| **I-158** | 0.01 | 0.004 | **I-154** | 0.03 | 0.04 |
| **I-161** | 0.11 | 0.04 | **I-160** | 0.02 | 0.02 |
| **I-163** | 0.03 | 0.07 | **I-162** | 0.02 | 0.01 |
| **I-165** | 0.04 | 0.06 | **I-164** | 0.04 | 0.03 |
| **I-168** | 0.04 | 0.01 | **I-166** | 0.03 | 0.03 |
| **I-179** | 0.091 | 0.058 | **I-169** | 0.07 | 0.02 |
| **I-181** | 0.03 | 0.007 | **I-180** | 0.038 | 0.012 |
| **I-183** | 0.091 | 0.083 | **I-182** | 0.054 | 0.058 |
| **I-184** | 0.096 | 0.107 | **I-187** | 0.145 | 0.064 |
| **I-191** | - | 0.018 | **I-188** | - | 0.029 |
| **I-198** | 0.1 | 0.005 | **I-195** | 0.031 | 0.001 |
| **I-208** | - | 0.07 | **I-207** | - | 0.044 |
| **I-213** | 0.1 | 0.009 | **I-212** | 0.06 | 0.004 |
| **I-216** | 0.063 | 0.04 | **I-214** | 0.086 | 0.003 |
| **I-221** | - | 0.03 | **I-217** | 0.1 | 0.035 |
| **I-226** | - | 0.073 | **I-222** | - | 0.009 |

| | | | | | |
|---|---|---|---|---|---|
| Result: It can be seen from Table 1 that the compounds of the invention have good TLR7/8 agonist activity. | | | | | |

### Pharmacokinetic (PK) evaluation in mice

The pharmacokinetic experiment was conducted by Medicilon Puya Pharmaceutical Technology (Shanghai) Co., LTD., and the animals were ICR mice (Shanghai Sipple-Bikai Laboratory Animal Co., LTD.). The test compound was administered to ICR mice by intravenous (iv) injection. The dose of iv was 2 mg/kg and the formulation is 100% Saline (0.9% saline). Animals were fasted overnight (10-14 hours) before dosing and were fed 4 hours after dosing. Blood was collected into heparin anticoagulant tubes at multiple time points (0.083, 0.25, 0.5, 1, 2, 4 h) after administration.

ICR mice were administered the test compound by oral gavage (po) administration. The dose of po is 5 mg/kg and the formulation is 100% Saline (0.9% saline). Animals were fasted overnight (10-14 hours) before dosing and were fed 4 hours after dosing. Blood was collected into heparin anticoagulant tubes at multiple time points (0.5, 1, 3, 5 hours) after administration.

Subcutaneous administration (sc) of the test compound to ICR mice. The dose of sc is 5 mg/kg and the formulation is 100% Saline (0.9% saline). Animals fasted overnight (10-14 hours) before dosing and were given food 4 hours after dosing. Blood was collected in heparin anticoagulant tubes at multiple time points (0.5, 1, 3, 5 hours) after administration. The plasma concentrations were analyzed by LC-MS/MS method, and the pharmacokinetic parameters were calculated by Phoenix WinNonlin.

The results show that the compound has unique pharmacokinetic properties. After intravenous (iv) administration of the prodrug molecule, the parent drug is rapidly and fully metabolized in the animal.

All references to the present invention are cited as references in this application as if each reference were cited separately. In addition, it is understood that after reading the above interpretation of the invention, professional persons in the art may make various changes or modifications to the invention, and these equivalent forms also fall within the limits of the claims attached to this application.

## Claims

1. A compound of formula I or formula II, or a solvate, a prodrug, a metabolite, or a pharmaceutically acceptable salt thereof, 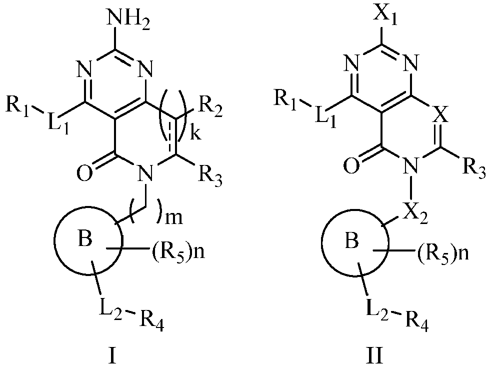 wherein:
L₁ is selected from the group consisting of: -O-, -NH-, -S-, -S(=O)- and -S(=O)₂-;
R₁ is selected from the group consisting of: H, C₁₋₁₂ alkyl, hydroxy substituted C₁₋₁₂ alkyl, C₁₋₁₂ alkoxy, C₂₋₁₂ alkenyl, C₂₋₁₂ alkynyl, C₃₋₁₂ cycloalkyl and 4-12 membered heterocycloalkyl; wherein, R₁ can be further substituted by one or more R^{a} substituents, and R^{a} is selected from the group consisting of: hydrogen, halogen, hydroxyl, cyano, C₁₋₆ alkyl, C₁₋₆ alkoxy, C₃₋₆ cycloalkyl, -NR^{a1}R^{a2}, -NHC(=O)-R^{a3}, 5-6-membered heteroaryl substituted with one or more R^{a4}, -OC(=O)R^{a5}, -C(=O)R^{a5}, -OC(=O)OR^{a5}, and -C(=O)OR^{a5};
R^{a1}, R^{a2}, R^{a3}, or R^{a4} is selected from the group consisting of: C₁₋₆ alkyl, C₁₋₆ haloalkyl, and C₃₋₆ cycloalkyl;
R^{a5} is selected from the group consisting of: C₁₋₂₄ alkyl, C₁₋₂₄ haloalkyl, and C₁₋₂₄ heteroalkyl having 1-10 heteroatoms, wherein the heteroatom is selected from one or more of NH, N, O and S;
X is N or CR₂;
X₁ is H or NH₂;
X₂ is selected from the group consisting of substituted or unsubstituted C₁- C₈ alkylene;
R₂ and R₃ are independently selected from the group consisting of: hydrogen, halogen, cyano, C₁₋₆ alkyl, C₁₋₆ alkoxy, C₃₋₆ cycloalkyl, 4-6 membered heterocycloalkyl, 5-8-membered heteroaryl and 5-8-membered aryl; wherein, R₂ and R₃ can be further substituted by one or more substituents selected from the group consisting of: halogen, hydroxyl, cyano, and amino;
K is 0 or 1;
m is 0, 1, 2, 3, 4, 5, 6,7 or 8; 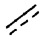
represents a single or double bond;
B is absent, or B is selected from the group consisting of: C₃₋₁₂ cycloalkyl, 4-12 membered heterocyclyl, C₆₋₁₂ aryl, 5-12 membered heteroaryl and 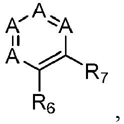 wherein, each A is selected from C, CH and N, and R₆ and R₇ together with the attached carbon atom jointly form a C₄₋₇ cycloalkylene or C₄₋₇ heterocycloalkylene, one or more methylene groups in the C₄₋₇ cycloalkylene or 4-7-membered heterocycloalkylene can be independently replaced by carbonyl or S(=O)₂; and the heteroatom in the 4-7-membered heterocycloalkylene is selected from N, O, and S; the number of heteroatom is 1 to 3;
L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O-), -S(=O)- and -S(=O)₂-; wherein, R^{b}, R^{c} are selected from the group consisting of: hydrogen, halogen, C₁₋₆ alkyl, C₃₋₆ cycloalkyl, 4-6 membered heterocycloalkyl and C₁₋₆ haloalkyl, R^{d} is selected from the group consisting of: hydrogen, C₁₋₆ alkyl, C₃₋₆ cycloalkyl, 4-6 membered heterocycloalkyl, C₁₋₆ haloalkyl and hydroxyl substituted C₁₋₆ alkyl; p is 0, 1, 2, 3, 4, 5, or 6; q is 0 or 1;
R₄ is selected from the group consisting of: hydrogen, halogen, cyano, amino, hydroxyl, C₁₋₁₂ alkyl, C₁₋₁₂ alkoxy, C₂₋₁₂ alkenyl, C₂₋₁₂ alkynyl, C₃₋₁₂ cycloalkyl, 4-12 membered heterocyclyl, C₆₋₁₂ aryl and 5-12 membered heteroaryl; and R₄ is optionally substituted by one or more R^{e}, wherein R^{e} is selected from the group consisting of: hydrogen, halogen, hydroxyl, carboxylic acid, amino, C₁₋₆alkyl, C₁₋₆alkyl substituted with one or more R^{e1}, C₁₋6 alkoxy, C₁₋₆ haloalkoxy, C₃₋₁₂ cycloalkyl, 4-12 membered heterocycloalkyl, C₆₋₁₂ aryl, 5-12 membered heteroaryl, -N(R^{e2}R^{e3}R^{e4}), -C(=O)O-R^{e2}, -C(=O)NH-R^{e2}, and -S(=O)₂-R^{e2};
R^{e1} is selected from the group consisting of: halogen, hydroxyl, and -P(O)(OR^{e2})₂; R^{e2} and R^{e3} are selected from the group consisting of: hydrogen, C₁₋₆ alkyl, C₃₋₆ cycloalkyl, C₁₋₆ haloalkyl and hydroxyl substituted C₁₋₆ alkyl;
R^{e4} is absent or selected from the group consisting of: C₁₋₆ alkyl, C₃₋₆ cycloalkyl and C₁₋₆ haloalkyl;
R₅ is selected from the group consisting of: halogen, hydroxyl, cyano, amino, C₁₋₆ alkyl, C₃₋₆ cycloalkyl, 4-6 membered heterocycloalkyl, C₁₋₆ haloalkyl, C₁₋₆ alkoxy, C₁₋₆ haloalkoxy, -C(=O)O-R^{f}, -C(=O)NH-R^{f}, and -S(=O)₂-R^{f}; wherein, R^{f} is selected from the group consisting of: hydrogen, C₁₋₆ alkyl, C₃₋₆ cycloalkyl and C₁₋₆ haloalkyl;
n is 1, 2, 3, 4, 5 or 6;
wherein, the heterocyclyl can be saturated or partially unsaturated (but without aromatic structure), and in the heterocyclyl, the heteroatom is selected from N, O and S, and the number of heteroatom is 1, 2, 3, or 4 (preferably 1 or 2); among the heteroaryl, the heteroatom is selected from N, O and S, and the number of heteroatom is 1, 2, or 3.

2. The compound of formula I according to claim 1, or a solvate, a prodrug, a metabolite, or a pharmaceutically acceptable salt thereof, wherein L₁ is selected from the group consisting of: -O-, -NH- and -S-.

3. The compound of formula I according to claim 1, or a solvate, a prodrug, a metabolite, or a pharmaceutically acceptable salt thereof, wherein R₁ is selected from the group consisting of: C₁₋₁₂ alkyl, hydroxy substituted C₁₋₁₂ alkyl, C₂₋₁₂ alkenyl, C₂₋₁₂ alkynyl, C₃₋₁₂ cycloalkyl and 4-12 membered heterocycloalkyl; wherein, R₁ can be further substituted by one or more R^{a}, and R^{a} is selected from the group consisting of: halogen, hydroxyl, cyano, C₁₋₆ alkyl, C₁₋₆ alkoxy, C₃₋₆ cycloalkyl, -NR^{a1}R^{a2}, -NHC(=O)-R^{a3}, 5-6-membered heteroaryl substituted with one or more R^{a4}, -OC(=O)R^{a5}, -C(=O)R^{a5}, - OC(=O)OR^{a5}, and -C(=O)OR^{a5}.

4. The compound of formula I according to claim 1, or a solvate, a prodrug, a metabolite, or a pharmaceutically acceptable salt thereof, wherein the compound of formula I has a structure selected from the group consisting of:

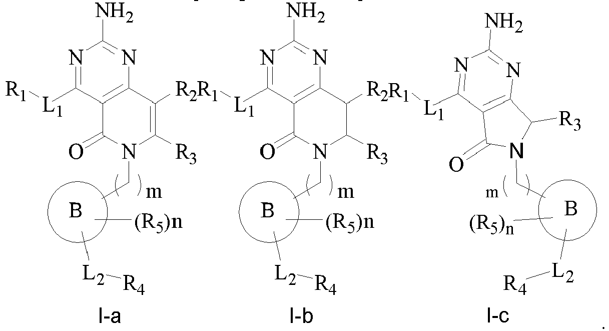

5. The compound of formula I according to claim 1, or a solvate, a prodrug, a metabolite, or a pharmaceutically acceptable salt thereof, wherein B is absent, or B is selected from the group consisting of: C₃₋₈ cycloalkyl, 4-7-membered heterocyclyl, C₆₋₁₀ aryl, and

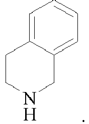

6. The compound of formula I according to claim 1, or a solvate, a prodrug, a metabolite, or a pharmaceutically acceptable salt thereof, wherein B is absent, or B is selected from the group consisting of: phenyl and pyridinyl.

7. The compound of formula I according to claim 1, or a solvate, a prodrug, a metabolite, or a pharmaceutically acceptable salt thereof, wherein L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O-), -S(=O)- and -S(=O)₂-; wherein, R^{b}, R^{c} are selected from the group consisting of: hydrogen, halogen, and C₁₋₆ alkyl; R^{d} is hydrogen, or C₁₋₆ alkyl; p is 0, 1, 2, or 3; q is 0 or 1.

8. The compound of formula I according to claim 1, or a solvate, a prodrug, a metabolite, or a pharmaceutically acceptable salt thereof, wherein L₂ is selected from the group consisting of: -(CH₂)ₚ, -(CH₂)ₚ-NR^{d}-, -O-, -S-, -(CH₂)ₚ-C(=O)-, -(CH₂)ₚ-C(=O)NH-, - (CH₂)ₚ-NHC(=O-), and -S(=O)₂-; wherein, R^{d} is hydrogen, or C₁₋₆ alkyl; p is 0, 1, 2, or 3.

9. The compound of formula I according to claim 1, or a solvate, a prodrug, a metabolite, or a pharmaceutically acceptable salt thereof, wherein R₄ is selected from the group consisting of: hydrogen, halogen, amino, hydroxyl, C₁₋₁₂ alkyl, C₁₋₁₂ alkoxy, C₂₋₁₂ alkenyl, C₂₋₁₂ alkynyl, C₃₋₁₀ cycloalkyl, 4-12 membered heterocyclyl, C₆₋₁₂ aryl and 5-12 membered heteroaryl; and R₄ is optionally substituted by one or more R^{e}, wherein R^{e} is selected from the group consisting of: hydrogen, halogen, hydroxyl, carboxylic acid, amino, C₁₋₆alkyl, C₁₋₆alkyl substituted with one or more R^{e1}, C₁₋₆ alkoxy, C₁₋₆ haloalkoxy, C₃₋₈ cycloalkyl, 4-7 membered heterocyclyl, phenyl, and 5-7 membered heteroaryl; wherein R^{e1} is selected from the group consisting of: halogen, hydroxyl and -P(O)(OR^{e2})₂.

10. The compound of formula I according to claim 1, or a solvate, a prodrug, a metabolite, or a pharmaceutically acceptable salt thereof, wherein R₄ is selected from the group consisting of: hydrogen, C₁₋₁₂ alkyl, C₁₋₁₂ alkoxy, cyclopropyl, cyclobutyl, cyclopentyl, cyclohexyl, 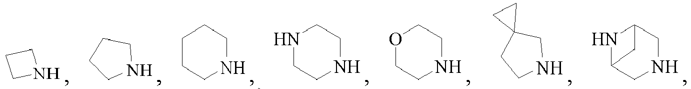 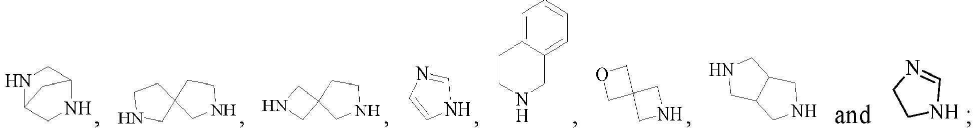 wherein, the loss of H atoms at any position on the above ring forms a connecting site; and R₄ is optionally substituted by one or more R^{e}.

11. The compound of formula I according to claim 1, or a solvate, a prodrug, a metabolite, or a pharmaceutically acceptable salt thereof, wherein R₅ is selected from the group consisting of: halogen, hydroxyl, cyano, amino, C₁₋₆ alkyl, C₁₋₆ alkoxy, and C₃₋₆ cycloalkyl.

12. The compound of formula I according to claim 1, or a solvate, a prodrug, a metabolite, or a pharmaceutically acceptable salt thereof, wherein the compound of formula I is selected from the group consisting of:

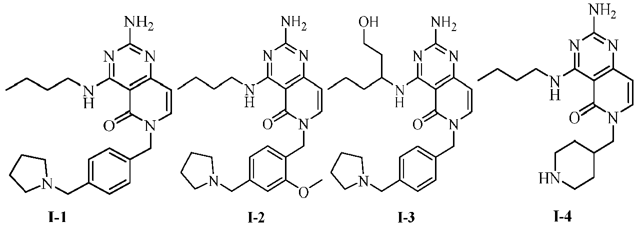

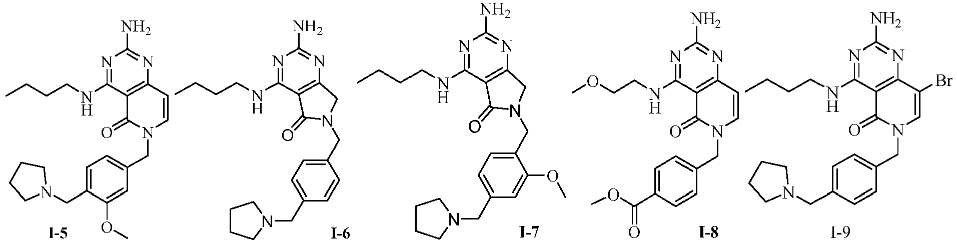

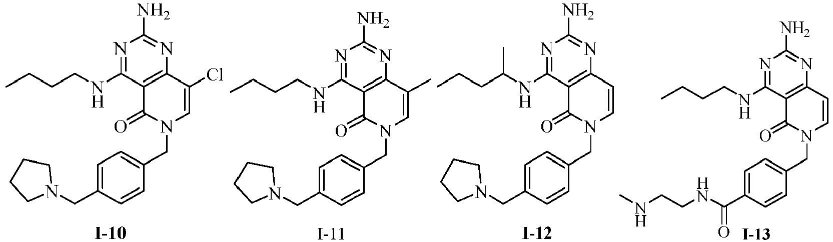

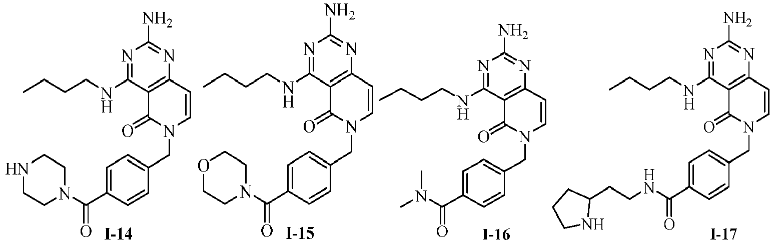

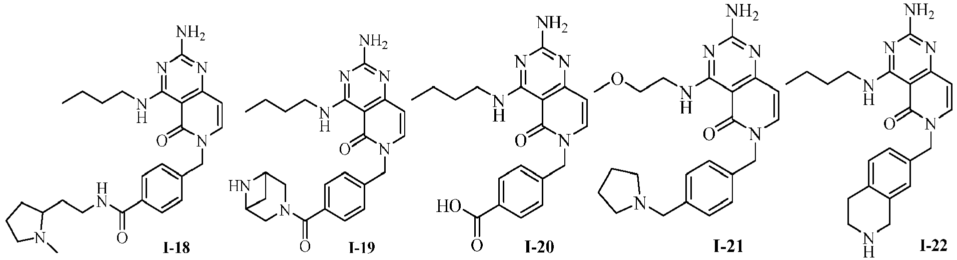

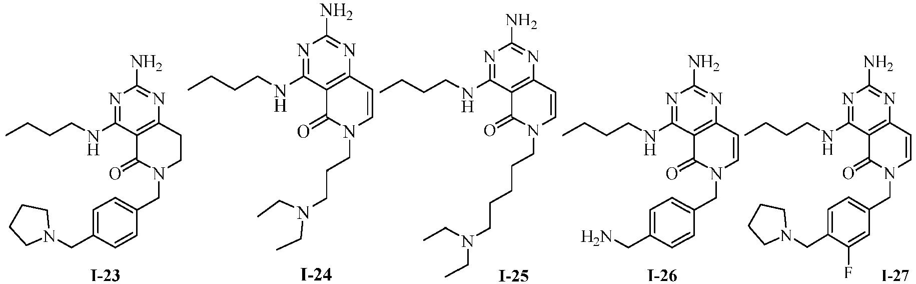

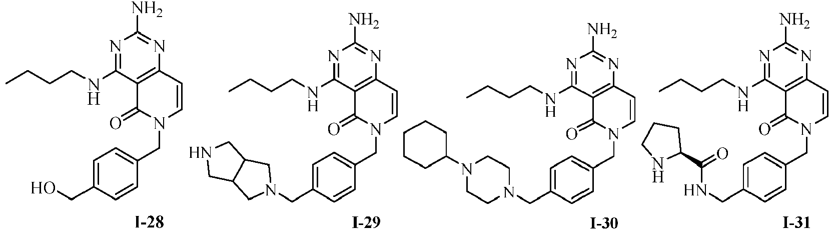

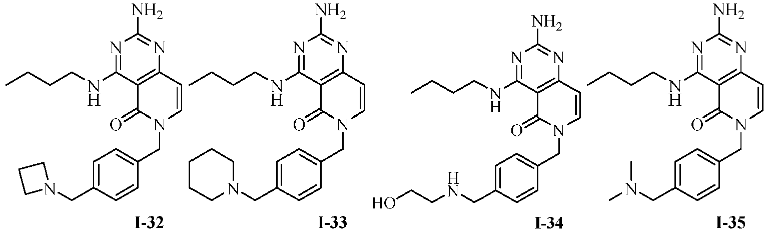

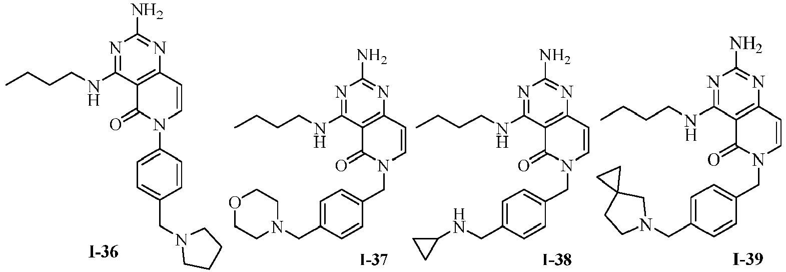

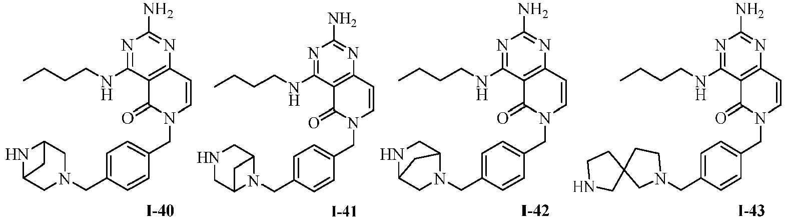

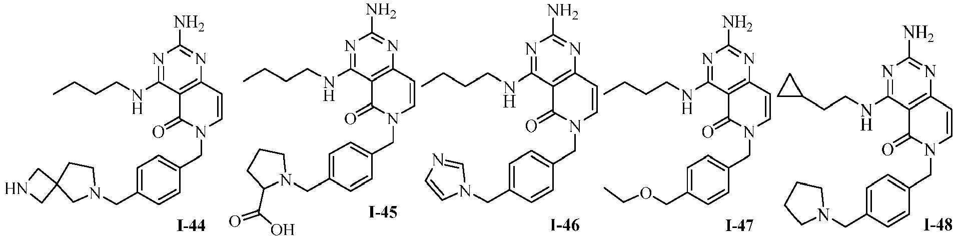

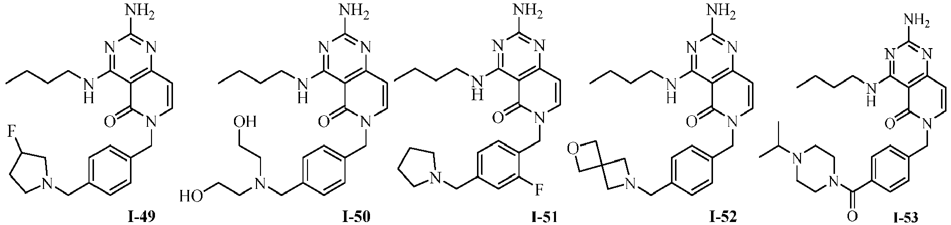

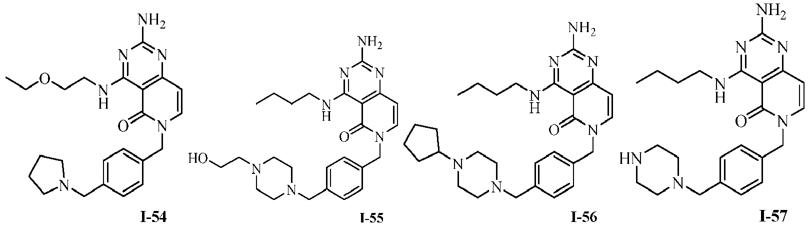

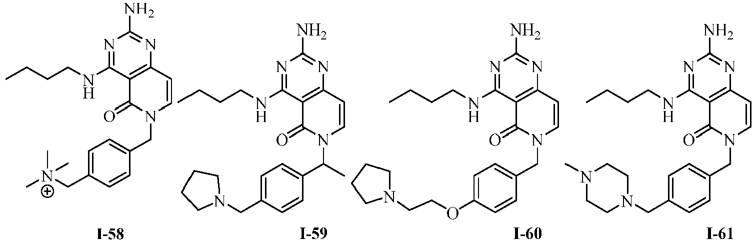

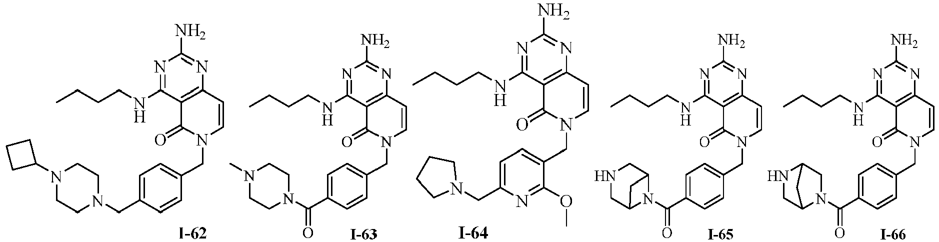

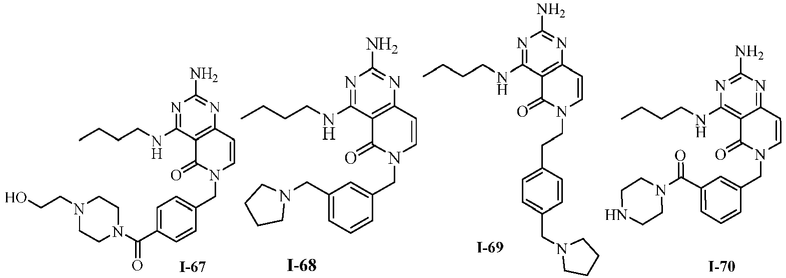

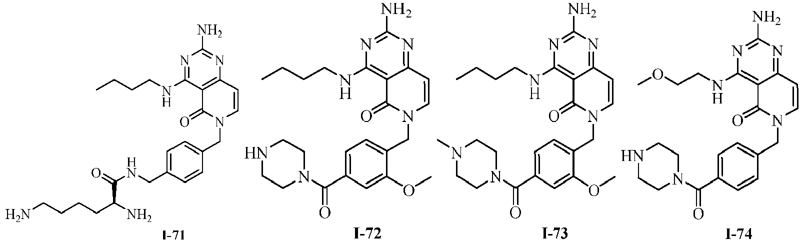

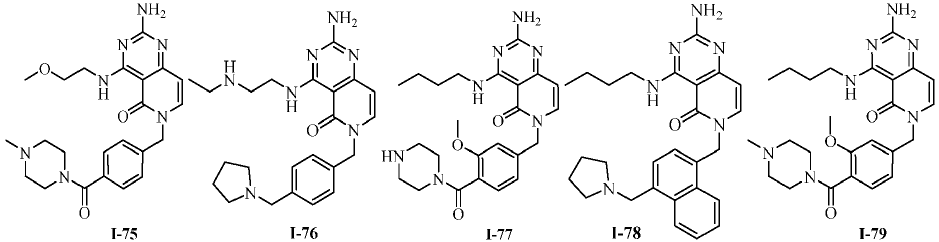

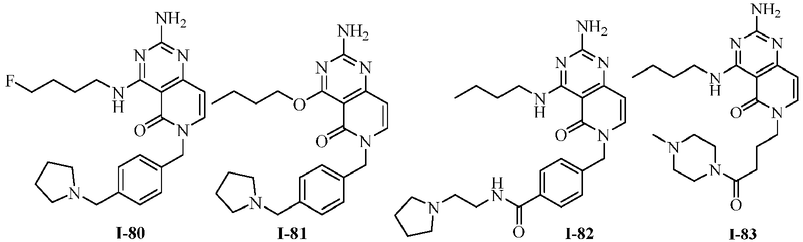

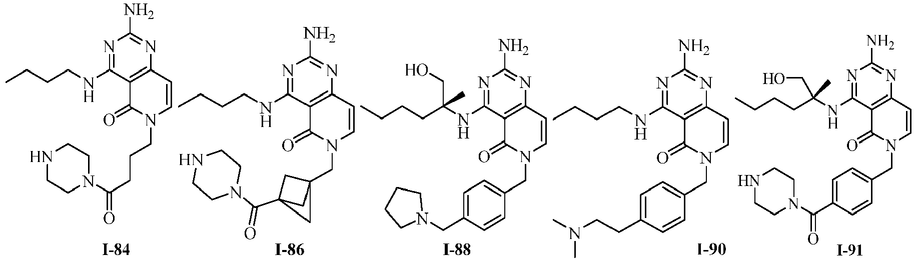

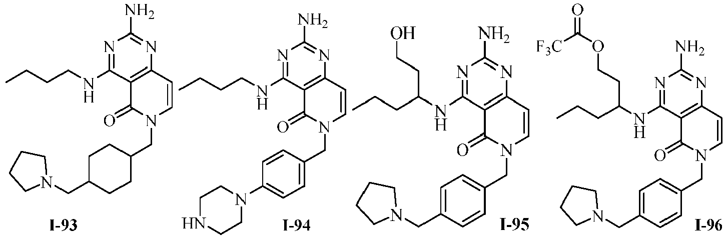

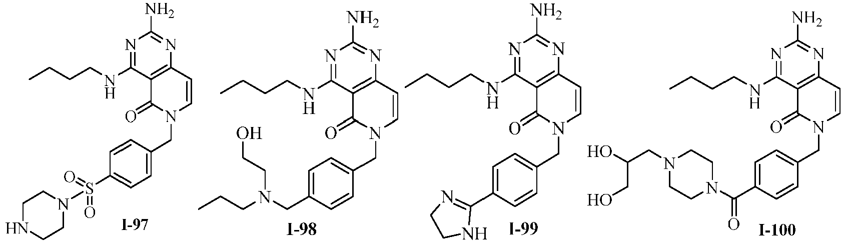

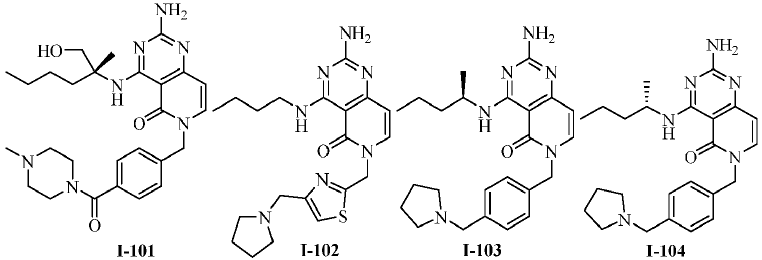

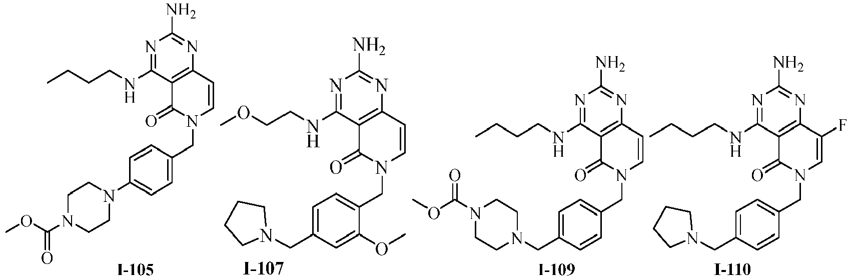

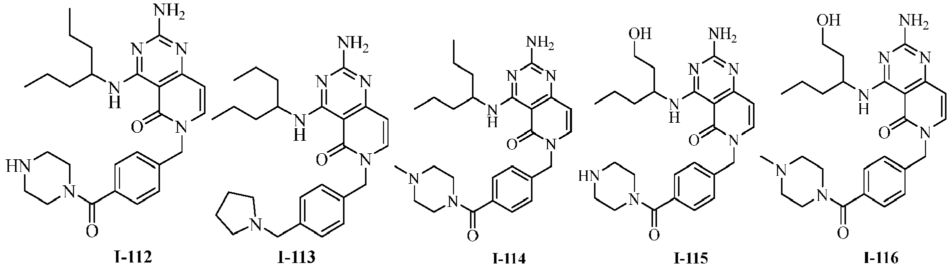

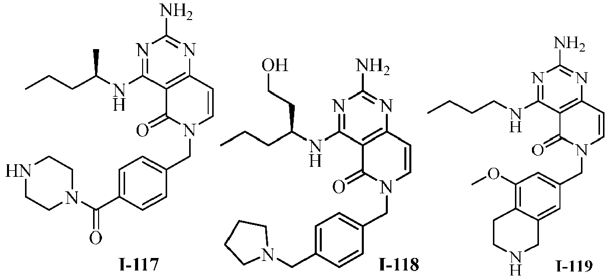

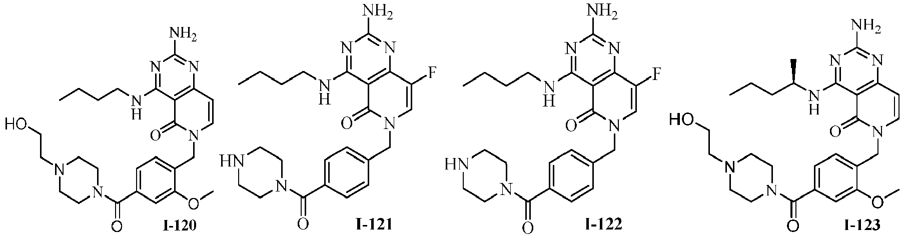

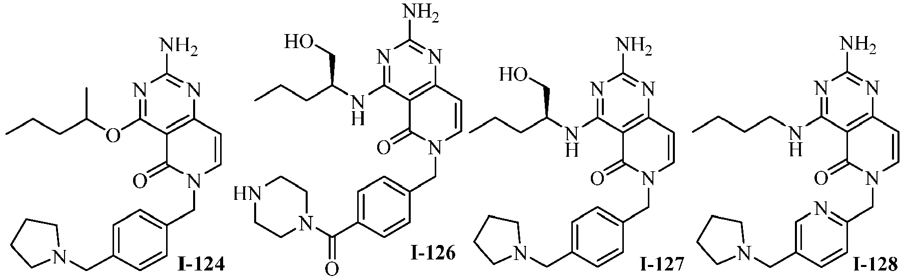

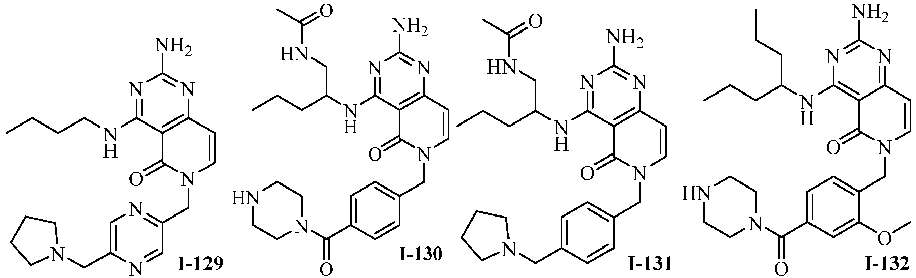

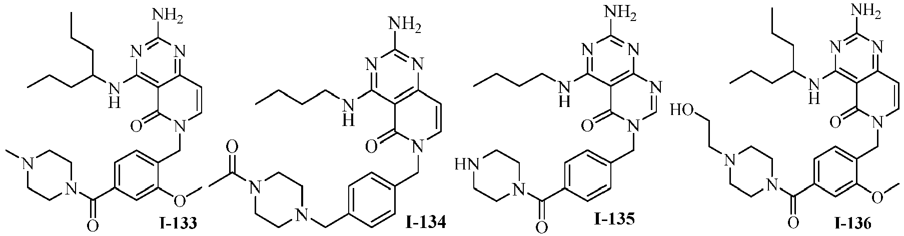

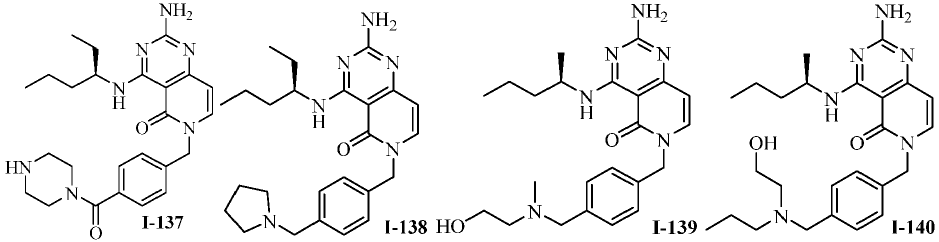

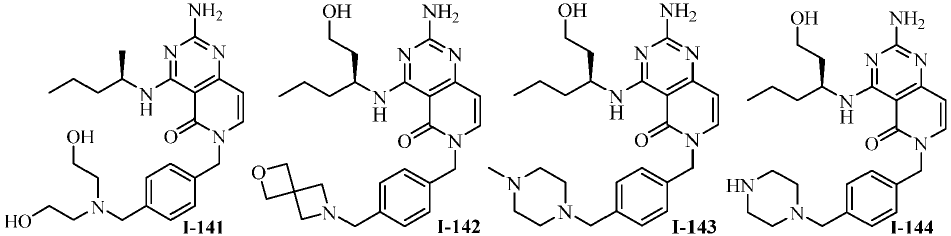

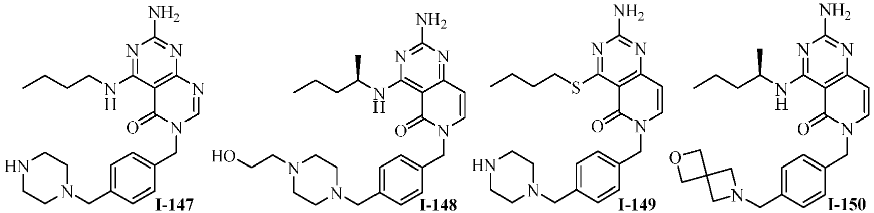

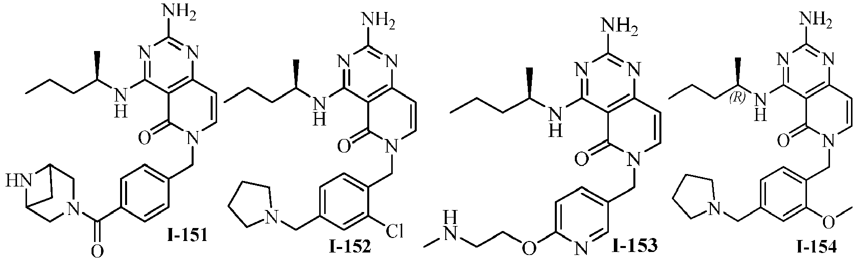

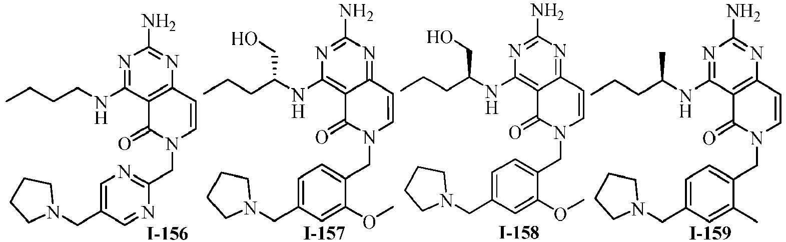

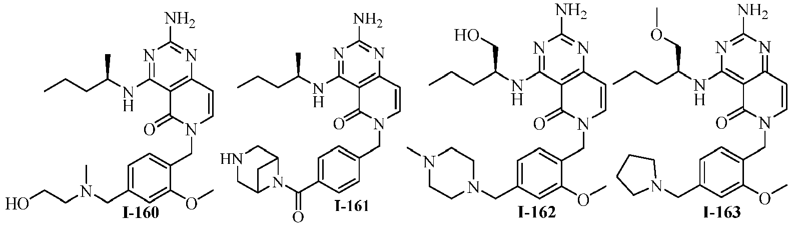

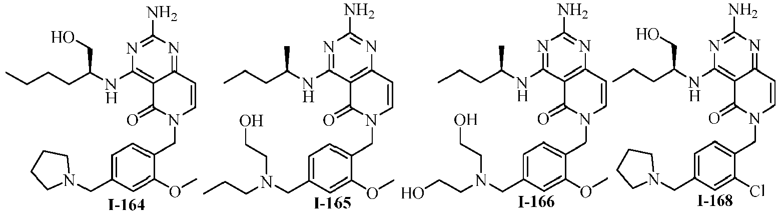

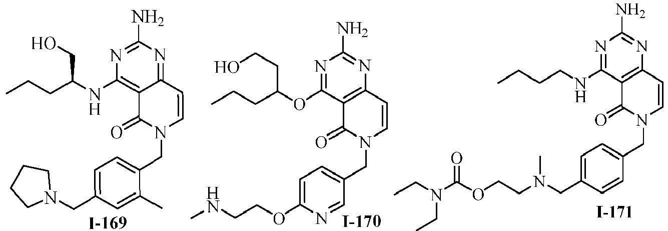

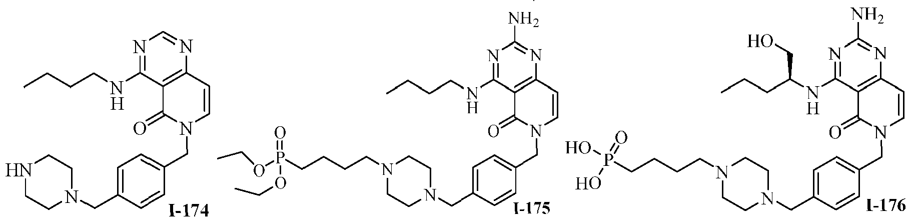

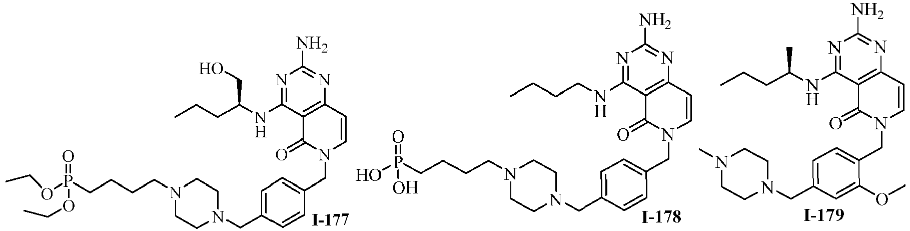

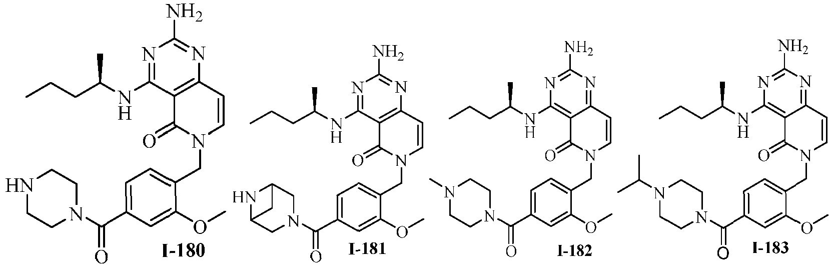

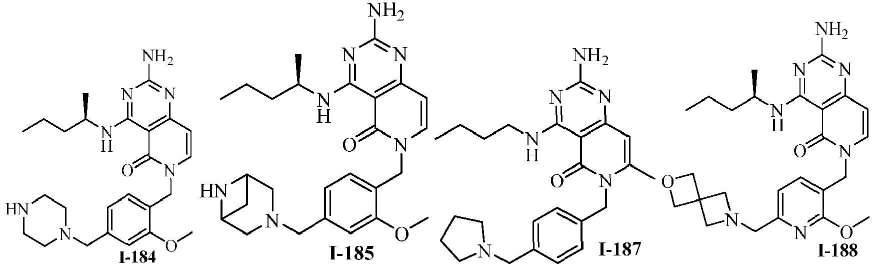

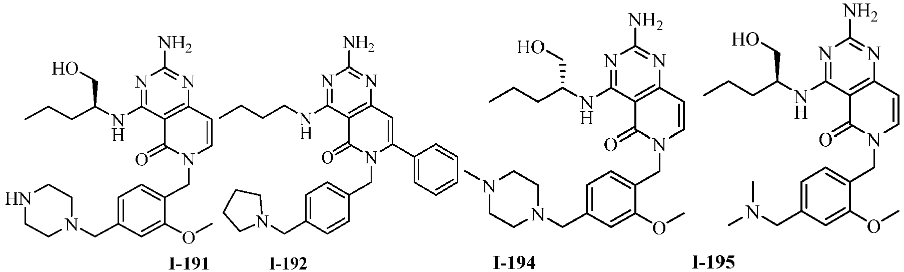

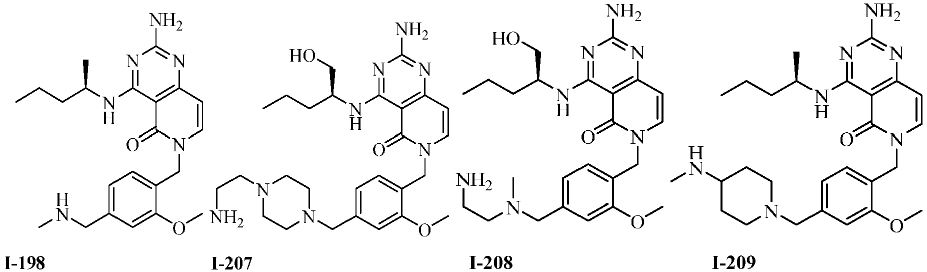

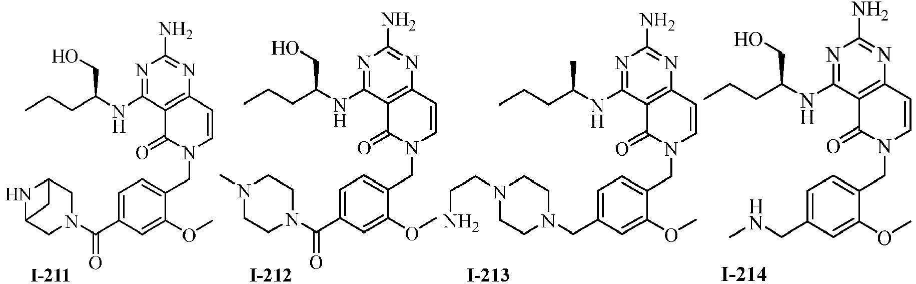

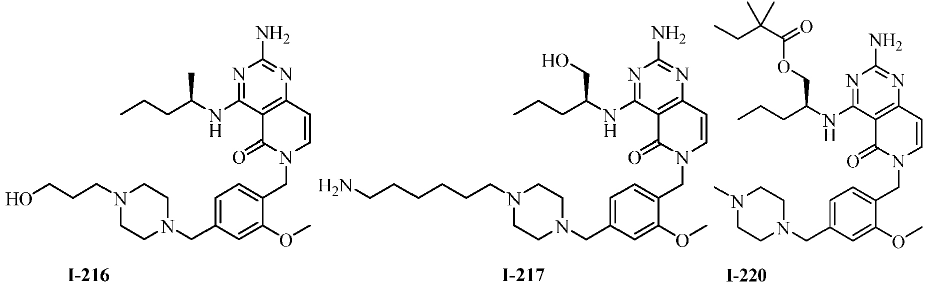

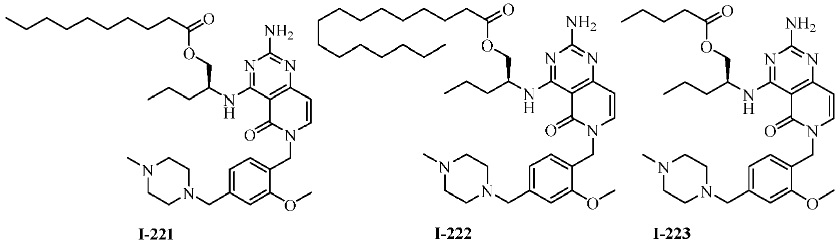

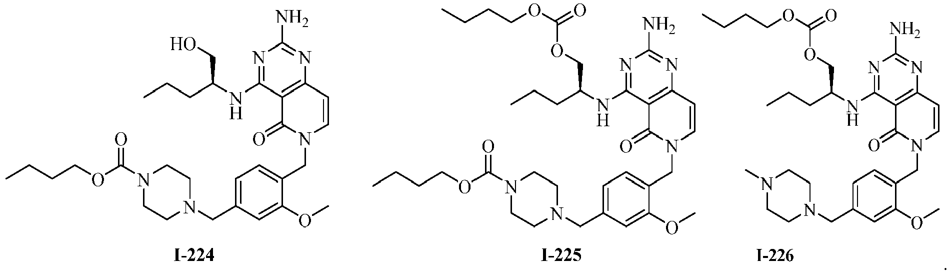

13. A pharmaceutical composition comprising one or more of the compound of formula I according to any one of claims 1-12, a pharmaceutically acceptable salt thereof, a racemate, a R-isomer, a S-isomers and a mixture thereof, as well as one or more pharmaceutically acceptable carriers, excipients, adjuvants, accessories, and/or diluents.

14. A use of the compound of any one of claims 1-12, a pharmaceutically acceptable salt, a racemate, a R-isomer, a S-isomer thereof, or a mixture thereof in the preparation of a pharmaceutical composition for the treatment or prevention of tumors or infections caused by viruses.

15. The use according to claim 14, wherein the virus is selected from the group consisting of: HBV, HCV, HIV, influenza virus, or a combination thereof; and/or
the tumor is preferably lung cancer, pancreatic cancer, kidney cancer, head and neck cancer, breast cancer, lymphoma, skin cancer, urothelial cancer, gastric cancer, hepatocellular carcinoma and colorectal cancer.
